# EUROPEAN PATENT APPLICATION

(11) **EP 4 328 225 A1**
(43) Date of publication of application: **28.02.2024**
(21) Application number: 22791130.2
(22) Date of filing: 22.04.2022
(51) Int. Cl.: C07D 471/04, C07D 401/14, C07D 403/14, A61K 31/4375, A61P 35/00

(54) **HETEROCYCLIC DERIVATIVE INHIBITOR AND PREPARATION METHOD THEREFOR AND APPLICATION THEREOF**

(30) Priority: 23.04.2021 CN 202110443582; 11.06.2021 CN 202110653169; 16.07.2021 CN 202110808316; 12.08.2021 CN 202110926676; 21.01.2022 CN 202210072358
(71) Applicant: Shanghai Hansoh Biomedical Co., Ltd., Shanghai 201203 (CN); Jiangsu Hansoh Pharmaceutical Group Co., Ltd., Lianyungang, Jiangsu 222047 (CN)
(72) Inventor: GAO, Peng, Shanghai 201203 (CN); ZENG, Mi, Shanghai 201203 (CN); WANG, Shaobao, Shanghai 201203 (CN); YU, Wensheng, Shanghai 201203 (CN)
(74) Representative: Cabinet Beau de Loménie
(86) International application number: PCT/CN2022/088466
(87) International publication number: WO 2022/223025

(57) **Abstract**

A heterocyclic derivative inhibitor and a preparation method therefor and an application thereof. Specifically, the present invention relates to a compound represented by general formula (I), a preparation method therefor, a pharmaceutical composition comprising said compound, and an application of same as an inhibitor for treatment of cancers, wherein substituents in general formula (I) are as defined in the description.

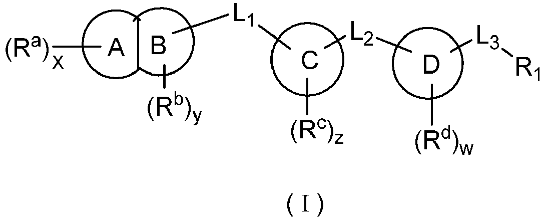

## Description

### FIELD OF THE INVENTION

The present invention belongs to the field of biomedicine, and specifically relates to a heterocyclic derivative inhibitor, a method for preparing the same, and a use thereof.

### BACKGROUND OF THE INVENTION

Poly(ADP-ribose) polymerase (PARP) is a protein superfamily that catalyzes the ribosylation of protein ADP in eukaryotic cells, including at least 17 protein subtypes. PARP can catalyze the cleavage of the substrate nicotinamide adenine dinucleotide NAD+ into nicotinamide and ADP-ribose, and enable the poly ADP-ribosylation of its target protein. PARP is located in the nucleus, and is a key enzyme in the repair of cellular DNA damage.

PARP1 is the earliest found and most studied PARP subtype, including three main domains, namely the N-terminal DNA-binding domain (DBD), the auto modification domain (AMD) and the C-terminal catalytic domain. PARP1 is an important functional protein in the process of DNA damage repair, and is a sensor of DNA single-strand damage. DNA damage can lead to PARP1 activation, poly-ADP-ribosylation modification of its target proteins such as histones, and recruitment of related repair proteins to promote DNA damage repair. PARP1 is very important for the stability of the genome of normal cells. However, in tumor therapy, PARP1 can antagonize the tumor killing effect produced by radiotherapy and chemotherapy by repairing the DNA of tumor cells damaged by radiotherapy and chemotherapy. Therefore, PARP1 inhibitors can be developed as sensitizers for tumor radiotherapy and chemotherapy.

Breast cancer susceptibility gene (BRCA) is an important tumor suppressor gene, which mainly includes two subtypes, BRCA1 and BRCA2. BRCA plays an important role in the repair of double-strand break DNA in DNA homologous recombination. Tumor cells often suffer from BRCA deficiency, which leads to the loss of double-strand break DNA damage repair function. If the function of PARP1 is simultaneously lost or inhibited, the repair of single-strand DNA damage will also be lost, which will eventually lead to the death of tumor cells and produce a "synthetic lethal" effect. Therefore, the use of PARP1 inhibitors blocks the repair function of the single-strand break DNA damage, which has a selective killing effect on BRCA-deficient tumors.

Currently, PARP inhibitors have achieved great success in the precision therapy of tumors, especially for tumors with BRCA mutations or defects. Currently marketed PARP inhibitors include Olaparib (AZD2281) from AstraZeneca, Rucaparib (CO-338) from Clovis, Niraparib (MK-4827) from Tesaro and Talazoparib (BMN-673) from Pfizer. The indications thereof are mainly ovarian cancer and breast cancer with BRCA mutations. There are also many PARP inhibitors in the clinical research stage. In the PARP family, PARP2 has the highest homology with PARP1. Therefore, most of the PARP inhibitors currently on the market or in the clinical stage are non-selective PARP inhibitors, which have potent inhibitory effects on both PARP1 and PARP2 subtypes. Studies have shown that PARP2 plays an important role in regulating red blood cell production. Inhibition of PARP2 is closely related to the clinical side effects of PARP inhibitor such as hematotoxicity, e.g., anemia.

### SUMMARY OF THE INVENTION

The objective of the present invention is to provide a compound of formula (I), a stereoisomer thereof or a pharmaceutically acceptable salt thereof, wherein the structure of the compound of formula (I) is as follows: 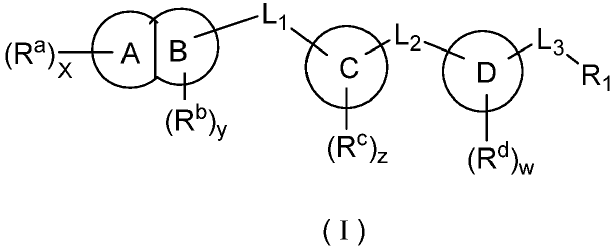 wherein, ring A, ring B, ring C and ring D are each independently selected from the group consisting of cycloalkyl, heterocyclyl, aryl and heteroaryl;
R₁ is selected from the group consisting of hydrogen, deuterium, halogen, nitro, hydroxy, thiol, cyano, amino, alkyl, deuterated alkyl, haloalkyl, hydroxyalkyl, alkoxy, alkenyl, alkynyl, cycloalkyl, heterocyclyl, aryl, heteroaryl, -(CH₂)ₙR₁₁, -(CH₂)ₙOR₁₁, -(CH₂)ₙC(O)R₁₁, -(CH₂)ₙC(O)OR₁₁, -(CH₂)ₙS(O)ₘR₁₁, -(CH₂)ₙNR₂₂R₃₃, -(CH₂)ₙNR₂₂C(O)OR₃₃, -(CH₂)ₙNR₂₂C(O)(CH₂)ₙ₁R₃₃, -(CH₂)ₙNR₂₂C(O)NR₂₂R₃₃, -(CH₂)ₙC(O)NR₂₂(CH₂)ₙ₁R₃₃, -OC(RₙR₂₂)ₙ(CH₂)ₙ₁R₃₃ and -(CH₂)ₙNR₂₂S(O)ₘR₃₃, the amino, alkyl, deuterated alkyl, haloalkyl, hydroxyalkyl, alkoxy, alkenyl, alkynyl, cycloalkyl, heterocyclyl, aryl and heteroaryl can be each optionally further substituted;
R₁₁, R₂₂ and R₃₃ are each independently selected from the group consisting of hydrogen, deuterium, halogen, amino, hydroxy, cyano, nitro, alkyl, alkenyl, alkynyl, deuterated alkyl, haloalkyl, alkoxy, haloalkoxy, hydroxyalkyl, cyano-substituted alkyl, cycloalkyl, heterocyclyl, aryl and heteroaryl, the amino, alkyl, alkenyl, alkynyl, deuterated alkyl, haloalkyl, alkoxy, haloalkoxy, hydroxyalkyl, cyano-substituted alkyl, cycloalkyl, heterocyclyl, aryl and heteroaryl can be each optionally further substituted;
L₁, L₂ and L₃ are each independently selected from the group consisting of a bond, substituted or unsubstituted alkenyl, substituted or unsubstituted alkynyl, -(CH₂)ₙ-, -(CH₂)ₙC(O)(CRₐₐR_{bb})ₙ₁-, -(CH₂)ₙC(O)NRₐₐ(CH₂)ₙ₁-, -(CH₂)ₙ(CRₐₐR_{bb})ₙ₂-, -(CRₐₐR_{bb})ₙO(CH₂)ₙ₁-, -(CH₂)ₙO(CRₐₐR_{bb})ₙ₁-, -(CRₐₐR_{bb})ₙ₃S(CH₂)ₙ₄-, -(CH₂)ₙS(CRₐₐR_{bb})ₙ₃-, -(CRₐₐR_{bb})ₙ₃(CH₂)ₙNR_{cc}-, -(CH2)ₙNRₐₐ(CR_{bb}R_{cc})ₙ-, -(CH₂)ₙNRₐₐC(O)-, -(CH₂)ₙP(O)ₚRₐₐ-, -(CH₂)ₙS(O)ₘ-, -(CH₂)ₙS(O)ₘNRₐₐ- and -(CH₂)ₙNRₐₐS(O)ₘ-;
Rₐₐ, R_{bb} and R_{cc} are each independently selected from the group consisting of hydrogen, deuterium, halogen, nitro, hydroxy, thiol, cyano, amino, alkyl, deuterated alkyl, haloalkyl, hydroxyalkyl, alkoxy, alkenyl, alkynyl, cycloalkyl, heterocyclyl, aryl and heteroaryl, the amino, alkyl, deuterated alkyl, haloalkyl, hydroxyalkyl, alkoxy, alkenyl, alkynyl, cycloalkyl, heterocyclyl, aryl and heteroaryl can be each optionally further substituted;
each R^{a} is independently selected from the group consisting of hydrogen, deuterium, halogen, nitro, hydroxy, thiol, oxo, cyano, amino, alkyl, deuterated alkyl, haloalkyl, hydroxyalkyl, alkoxy, alkenyl, alkynyl, cycloalkyl, heterocyclyl, aryl, heteroaryl, -(CH₂)ₙRₐ₁, -(CH₂)ₙORₐ₁, -(CH₂)ₙC(O)Rₐ₁, -(CH₂)ₙC(O)ORₐ₁, -(CH₂)ₙS(O)ₘRₐ₁, -(CH₂)ₙNRₐ₂Rₐ₃, -(CH₂)ₙNRₐ₂C(O)ORₐ₃, -(CH₂)ₙNRₐ₂C(O)(CH₂)ₙ₁Rₐ₃, -(CH₂)ₙNRₐ₂C(O)NRₐ₂Rₐ₃, -(CH₂)ₙC(O)NRₐ₂(CH₂)ₙ₁Rₐ₃, -OC(Rₐ₁Rₐ₂)ₙ(CH₂)ₙ₁Rₐ₃ and -(CH₂)ₙNRₐ₂S(O)ₘRₐ₃, the amino, alkyl, deuterated alkyl, haloalkyl, hydroxyalkyl, alkoxy, alkenyl, alkynyl, cycloalkyl, heterocyclyl, aryl and heteroaryl can be each optionally further substituted;
Rₐ₁, Rₐ₂ and Rₐ₃ are each independently selected from the group consisting of hydrogen, deuterium, halogen, nitro, hydroxy, thiol, cyano, amino, alkyl, deuterated alkyl, haloalkyl, hydroxyalkyl, alkoxy, alkenyl, alkynyl, cycloalkyl, heterocyclyl, aryl and heteroaryl, the amino, alkyl, deuterated alkyl, haloalkyl, hydroxyalkyl, alkoxy, alkenyl, alkynyl, cycloalkyl, heterocyclyl, aryl and heteroaryl can be each optionally further substituted;
each R^{b} is independently selected from the group consisting of hydrogen, deuterium, halogen, nitro, hydroxy, thiol, oxo, cyano, amino, alkyl, deuterated alkyl, haloalkyl, hydroxyalkyl, alkoxy, alkenyl, alkynyl, cycloalkyl, heterocyclyl, aryl, heteroaryl, -(CH₂)ₙR_{b1}, -(CH₂)ₙOR_{b1}, -(CH₂)ₙC(O)R_{b1}, -(CH₂)ₙC(O)OR_{b1}, -(CH₂)ₙS(O)ₘR_{b1}, -(CH₂)ₙNR_{b2}R_{b3}, -(CH₂)ₙNR_{b2}C(O)OR_{b3}, -(CH₂)ₙNR_{b2}C(O)(CH₂)ₙ₁R_{b3}, -(CH₂)ₙNR_{b2}C(O)NR_{b2}R_{b3}, -(CH₂)ₙC(O)NR_{b2}(CH₂)ₙ₁R_{b3}, -OC(R_{b1}R_{b2})ₙ(CH₂)ₙ₁R_{b3} and -(CH₂)ₙNR_{b2}S(O)ₘR_{b3}, the amino, alkyl, deuterated alkyl, haloalkyl, hydroxyalkyl, alkoxy, alkenyl, alkynyl, cycloalkyl, heterocyclyl, aryl and heteroaryl can be each optionally further substituted;
R_{b1}, R_{b2} and R_{b3} are each independently selected from the group consisting of hydrogen, deuterium, halogen, nitro, hydroxy, thiol, cyano, amino, alkyl, deuterated alkyl, haloalkyl, hydroxyalkyl, alkoxy, alkenyl, alkynyl, cycloalkyl, heterocyclyl, aryl and heteroaryl, the amino, alkyl, deuterated alkyl, haloalkyl, hydroxyalkyl, alkoxy, alkenyl, alkynyl, cycloalkyl, heterocyclyl, aryl and heteroaryl can be each optionally further substituted;
each R^{c} is independently selected from the group consisting of hydrogen, deuterium, halogen, nitro, hydroxy, thiol, oxo, cyano, amino, alkyl, deuterated alkyl, haloalkyl, hydroxyalkyl, alkoxy, alkenyl, alkynyl, cycloalkyl, heterocyclyl, aryl, heteroaryl, -(CH₂)ₙR_{c1}, -(CH₂)ₙOR_{c1}, -(CH₂)ₙC(O)R_{c1}, -(CH₂)ₙC(O)OR_{c1}, -(CH₂)ₙS(O)ₘR_{c1,} -(CH₂)ₙNR_{c2}R_{c3}, -(CH₂)ₙNR_{c2}C(O)OR_{c3}, -(CH₂)ₙNR_{c2}C(O)(CH₂)ₙ₁R_{c3}, -(CH₂)ₙNR_{c2}C(O)NR_{c2}R_{c3}, -(CH₂)ₙC(O)NR_{c2}(CH₂)ₙ₁R_{c3}, -OC(R_{c1}R_{c2})ₙ(CH₂)ₙ₁R_{c3} and -(CH₂)ₙNR_{c2}S(O)ₘR_{c3}, the amino, alkyl, deuterated alkyl, haloalkyl, hydroxyalkyl, alkoxy, alkenyl, alkynyl, cycloalkyl, heterocyclyl, aryl and heteroaryl can be each optionally further substituted;
R_{c1}, R_{c2} and R_{c3} are each independently selected from the group consisting of hydrogen, deuterium, halogen, nitro, hydroxy, thiol, cyano, amino, alkyl, deuterated alkyl, haloalkyl, hydroxyalkyl, alkoxy, alkenyl, alkynyl, cycloalkyl, heterocyclyl, aryl and heteroaryl, the amino, alkyl, deuterated alkyl, haloalkyl, hydroxyalkyl, alkoxy, alkenyl, alkynyl, cycloalkyl, heterocyclyl, aryl and heteroaryl can be each optionally further substituted;
each R^{d} is independently selected from the group consisting of hydrogen, deuterium, halogen, nitro, hydroxy, thiol, oxo, cyano, amino, alkyl, deuterated alkyl, haloalkyl, hydroxyalkyl, alkoxy, alkenyl, alkynyl, cycloalkyl, heterocyclyl, aryl, heteroaryl, -(CH₂)ₙR_{d1}, -(CH₂)ₙOR_{d1}, -(CH₂)ₙC(O)R_{d1}, -(CH₂)ₙC(O)OR_{d1}, -(CH₂)ₙS(O)ₘR_{d1}, -(CH₂)ₙNR_{d2}R_{d3}, -(CH₂)ₙNR_{d2}C(O)OR_{d3}, -(CH₂)ₙNR_{d2}C(O)(CH₂)ₙ₁R_{d3}, -(CH₂)ₙNR_{d2}C(O)NR_{d2}R_{d3}, -(CH₂)ₙC(O)NR_{d2}(CH₂)ₙ₁R_{d3}, -OC(R_{d1}R_{d2})ₙ(CH₂)ₙ₁R_{d3} and -(CH₂)ₙNR_{d2}S(O)ₘR_{d3}, the amino, alkyl, deuterated alkyl, haloalkyl, hydroxyalkyl, alkoxy, alkenyl, alkynyl, cycloalkyl, heterocyclyl, aryl and heteroaryl can be each optionally further substituted;
R_{d1}, R_{d2} and R_{d3} are each independently selected from the group consisting of hydrogen, deuterium, halogen, nitro, hydroxy, thiol, cyano, amino, alkyl, deuterated alkyl, haloalkyl, hydroxyalkyl, alkoxy, alkenyl, alkynyl, cycloalkyl, heterocyclyl, aryl and heteroaryl, the amino, alkyl, deuterated alkyl, haloalkyl, hydroxyalkyl, alkoxy, alkenyl, alkynyl, cycloalkyl, heterocyclyl, aryl and heteroaryl can be each optionally further substituted;
w, x, y and z are each independently 1, 2, 3 or 4;
each m is independently 0, 1 or 2;
each n is independently 0, 1, 2, 3 or 4;
each p is independently 0 or 1; and
n1, n2, n3 and n4 are each independently 0, 1, 2, 3 or 4.

In some embodiments of the present invention, the compound is further as shown in formula (III): 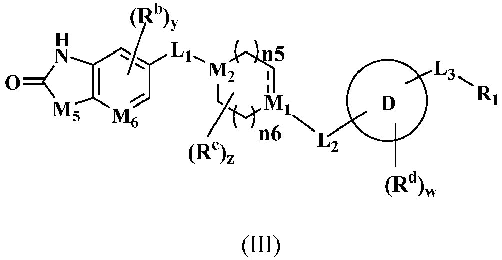 wherein,
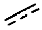 is a single bond or double bond;
L₁ is a bond, -(CH₂)ₙ(CRₐₐR_{bb})ₙ₂-, -(CH₂)ₙC(O)(CRₐₐR_{bb})ₙ₁- or -(CRₐₐR_{bb})ₙO(CH₂)ₙ₁-;
L₂ is a bond or -(CH₂)ₙ(CRₐₐR_{bb})ₙ₂-;
L₃ is selected from the group consisting of a bond, -(CH₂)ₙC(O)(CRₐₐR_{bb})ₙ₁-, -(CH₂)ₙC(O)NRₐₐ(CH₂)ₙ₁-, -(CH₂)ₙ(CRₐₐR_{bb})ₙ₂-, -(CRₐₐR_{bb})ₙO(CH₂)ₙ₁- and -(CH₂)ₙNRₐₐ(CR_{bb}R_{cc})ₙ-;
Rₐₐ, R_{bb} and R_{cc} are each independently selected from the group consisting of hydrogen, deuterium, halogen, nitro, hydroxy, thiol, cyano, amino, alkyl, deuterated alkyl, haloalkyl, hydroxyalkyl, alkoxy, alkenyl and alkynyl;
or, any two of Rₐₐ, R_{bb} and R_{cc} are bonded to form a cycloalkyl;
M₁ is N, C or CR₂;
M₂ is N or CR₃;
M₅ is -CR₆R₇-, -CR₆R₇-CR₈R₉-, -CR₆=CR₈-, -CR₆R₇-NR₈-, -NR₈-C(=O)-, -CR₆R₇-O- or -CR₆=N-;
M₆ is N or CR₁₀;
R₁ is selected from the group consisting of hydrogen, deuterium, halogen, nitro, hydroxy, thiol, cyano, amino, alkyl, deuterated alkyl, haloalkyl, hydroxyalkyl, alkoxy, alkenyl, alkynyl, cycloalkyl, heterocyclyl, aryl, heteroaryl, -(CH₂)ₙR₁₁, -(CH₂)ₙOR₁₁, -(CH₂)ₙC(O)R₁₁, -(CH₂)ₙC(O)OR₁₁, -(CH₂)ₙS(O)ₘR₁₁, -(CH₂)ₙNR₂₂R₃₃, -(CH₂)ₙNR₂₂C(O)OR₃₃, -(CH₂)ₙNR₂₂C(O)(CH₂)ₙ₁R₃₃, -(CH₂)ₙNR₂₂C(O)NR₂₂R₃₃, -(CH₂)ₙC(O)NR₂₂(CH₂)ₙ₁R₃₃, -OC(R₁₁R₂₂)ₙ(CH₂)ₙ₁R₃₃ and -(CH₂)ₙNR₂₂S(O)ₘR₃₃, the amino, alkyl, deuterated alkyl, haloalkyl, hydroxyalkyl, alkoxy, alkenyl, alkynyl, cycloalkyl, heterocyclyl, aryl and heteroaryl can be each optionally further substituted;
R₁₁, R₂₂ and R₃₃ are each independently selected from the group consisting of hydrogen, deuterium, halogen, amino, hydroxy, cyano, nitro, alkyl, alkenyl, alkynyl, deuterated alkyl, haloalkyl, alkoxy, haloalkoxy, hydroxyalkyl, cyano-substituted alkyl, cycloalkyl, heterocyclyl, aryl and heteroaryl, the amino, alkyl, alkenyl, alkynyl, deuterated alkyl, haloalkyl, alkoxy, haloalkoxy, hydroxyalkyl, cyano-substituted alkyl, cycloalkyl, heterocyclyl, aryl and heteroaryl can be each optionally further substituted;
R₂ and R₃ are identical or different, and are each independently selected from the group consisting of hydrogen, deuterium, halogen, nitro, hydroxy, thiol, cyano, amino optionally substituted by alkyl, alkyl, deuterated alkyl, haloalkyl, hydroxyalkyl, alkoxy, alkenyl, alkynyl, cycloalkyl, heterocyclyl, aryl and heteroaryl; preferably, R₂ and R₃ are each independently hydrogen, deuterium, halogen, alkyl or cycloalkyl;
R₆, R₇, R₈, R₉ and R₁₀ are identical or different, and are each independently selected from the group consisting of hydrogen, deuterium, halogen, nitro, hydroxy, thiol, cyano, amino optionally substituted by alkyl, alkyl, deuterated alkyl, haloalkyl, hydroxyalkyl, alkoxy, alkenyl, alkynyl, cycloalkyl, heterocyclyl, aryl and heteroaryl; preferably, R₆, R₇, R₈ and R₉ are each independently hydrogen, deuterium, halogen, cyano, alkyl, alkynyl or cycloalkyl;
or, R₆, R₇ together with the adjacent carbon atom are bonded to form a cycloalkyl, the cycloalkyl is optionally substituted by one or more substituents selected from the group consisting of hydrogen, deuterium, halogen, nitro, hydroxy, thiol, cyano and amino;
or, R₆, R₈ together with the adjacent carbon atom are bonded to form a cycloalkyl, the cycloalkyl is optionally substituted by one or more substituents selected from the group consisting of hydrogen, deuterium, halogen, nitro, hydroxy, thiol, cyano and amino;
each R^{b} is independently selected from the group consisting of hydrogen, deuterium, halogen, nitro, hydroxy, thiol, oxo, cyano, amino, alkyl, deuterated alkyl, haloalkyl, hydroxyalkyl, alkoxy, alkenyl, alkynyl, cycloalkyl, heterocyclyl, aryl, heteroaryl, -(CH₂)ₙR_{b1}, -(CH₂)ₙOR_{b1}, -(CH₂)ₙC(O)R_{b1} -(CH₂)ₙC(O)OR_{b1} -(CH₂)ₙS(O)ₘR_{b1}, -(CH₂)ₙNR_{b2}R_{b3}, -(CH₂)ₙNR_{b2}C(O)OR_{b3}, -(CH₂)ₙNR_{b2}C(O)(CH₂)ₙ₁R_{b3}, -(CH₂)ₙNR_{b2}C(O)NR_{b2}R_{b3}, -(CH₂)ₙC(O)NR_{b2}(CH₂)ₙ₁R_{b3}, -OC(R_{b1}R_{b2})ₙ(CH₂)ₙ₁R_{b3} and -(CH₂)ₙNR_{b2}S(O)ₘR_{b3}, the amino, alkyl, deuterated alkyl, haloalkyl, hydroxyalkyl, alkoxy, alkenyl, alkynyl, cycloalkyl, heterocyclyl, aryl and heteroaryl can be each optionally further substituted;
R_{b1}, R_{b2} and R_{b3} are each independently selected from the group consisting of hydrogen, deuterium, halogen, nitro, hydroxy, thiol, cyano, amino, alkyl, deuterated alkyl, haloalkyl, hydroxyalkyl, alkoxy, alkenyl, alkynyl, cycloalkyl, heterocyclyl, aryl and heteroaryl, the amino, alkyl, deuterated alkyl, haloalkyl, hydroxyalkyl, alkoxy, alkenyl, alkynyl, cycloalkyl, heterocyclyl, aryl and heteroaryl can be each optionally further substituted;
each R^{c} is independently selected from the group consisting of hydrogen, deuterium, halogen, nitro, hydroxy, thiol, oxo, cyano, amino, alkyl, deuterated alkyl, haloalkyl, hydroxyalkyl, alkoxy, alkenyl, alkynyl, cycloalkyl, heterocyclyl, aryl, heteroaryl, -(CH₂)ₙR_{c1}, -(CH₂)ₙOR_{c1}, -(CH₂)ₙC(O)R_{c1}, -(CH₂)ₙC(O)OR_{c1}, -(CH₂)ₙS(O)ₘR_{c1}, -(CH₂)ₙNR_{c2}R_{c3}, -(CH₂)ₙNR_{c2}C(O)OR_{c3}, -(CH₂)ₙNR_{c2}C(O)(CH₂)ₙ₁R_{c3}, -(CH₂)ₙNR_{c2}C(O)NR_{c2}R_{c3}, -(CH₂)ₙC(O)NR_{c2}(CH₂)ₙ₁R_{c3}, -OC(R_{c1}R_{c2})ₙ(CH₂)ₙ₁R_{c3} and -(CH₂)ₙNR_{c2}S(O)ₘR_{c3}, the amino, alkyl, deuterated alkyl, haloalkyl, hydroxyalkyl, alkoxy, alkenyl, alkynyl, cycloalkyl, heterocyclyl, aryl and heteroaryl can be each optionally further substituted;
R_{c1}, R_{c2} and R_{c3} are each independently selected from the group consisting of hydrogen, deuterium, halogen, nitro, hydroxy, thiol, cyano, amino, alkyl, deuterated alkyl, haloalkyl, hydroxyalkyl, alkoxy, alkenyl, alkynyl, cycloalkyl, heterocyclyl, aryl and heteroaryl, the amino, alkyl, deuterated alkyl, haloalkyl, hydroxyalkyl, alkoxy, alkenyl, alkynyl, cycloalkyl, heterocyclyl, aryl and heteroaryl can be each optionally further substituted;
each R^{d} is independently selected from the group consisting of hydrogen, deuterium, halogen, nitro, hydroxy, thiol, oxo, cyano, amino, alkyl, deuterated alkyl, haloalkyl, hydroxyalkyl, alkoxy, alkenyl, alkynyl, cycloalkyl, heterocyclyl, aryl, heteroaryl, -(CH₂)ₙR_{d1}, -(CH₂)ₙOR_{d1}, -(CH₂)ₙC(O)R_{d1}, -(CH₂)ₙC(O)OR_{d1}, -(CH₂)ₙS(O)ₘR_{d1}, -(CH₂)ₙNR_{d2}R_{d3}, -(CH₂)ₙNR_{d2}C(O)OR_{d3}, -(CH₂)ₙNR_{d2}C(O)(CH₂)ₙ₁R_{d3}, -(CH₂)ₙNR_{d2}C(O)NR_{d2}R_{d3}, -(CH₂)ₙC(O)NR_{d2}(CH₂)ₙ₁R_{d3}, -OC(R_{d1}R_{d2})ₙ(CH₂)ₙ₁R_{d3} and -(CH₂)ₙNR_{d2}S(O)ₘR_{d3}, the amino, alkyl, deuterated alkyl, haloalkyl, hydroxyalkyl, alkoxy, alkenyl, alkynyl, cycloalkyl, heterocyclyl, aryl and heteroaryl can be each optionally further substituted;
R_{d1}, R_{d2} and R_{d3} are each independently selected from the group consisting of hydrogen, deuterium, halogen, nitro, hydroxy, thiol, cyano, amino, alkyl, deuterated alkyl, haloalkyl, hydroxyalkyl, alkoxy, alkenyl, alkynyl, cycloalkyl, heterocyclyl, aryl and heteroaryl, the amino, alkyl, deuterated alkyl, haloalkyl, hydroxyalkyl, alkoxy, alkenyl, alkynyl, cycloalkyl, heterocyclyl, aryl and heteroaryl can be each optionally further substituted; w, y and z are each independently 1, 2, 3 or 4;
each m is independently 0, 1 or 2;
each n is independently 0, 1, 2, 3 or 4;
n1 and n2 are each independently 0, 1, 2, 3 or 4;
n5 and n6 are each independently 0, 1 or 2;
when M₁ is N and M₂ is N, then ring D is not a monocyclic ring, and when ring D is 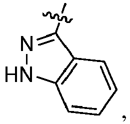 then R₁ is not hydrogen;
when M₂ is N, M₁ is CH and ring D is hydrogen; 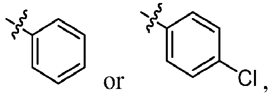 then R₁ is not
when M₁ is N, M₂ is CH and ring D is 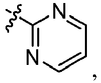 then R₁ is not hydrogen;
when 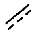 is a double bond, M₁ is C, M₂ is N and ring D is phenyl, then R₁ is not hydrogen.

In some embodiments of the present invention, the compound of formula (I) is a compound of formula (II):

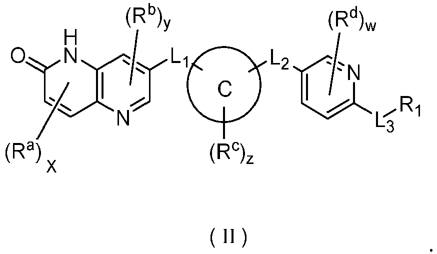

In some embodiments of the present invention, the compound of formula (I) is a compound of formula (IV): 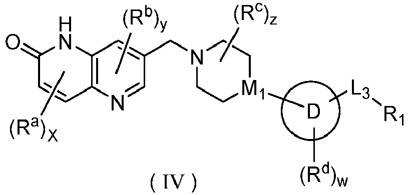 M₁ is N or CR^{a}.

In some embodiments of the present invention, ring C is a 3 to 10 membered heterocyclyl, and preferably, ring C is a 4 to 8 membered heterocyclyl; the heteroatoms in the 3 to 10 membered heterocyclyl and 4 to 8 membered heterocyclyl are each independently selected from the group consisting of nitrogen, oxygen and sulfur, and the number of heteroatoms is independently 1, 2 or 3; preferably, the rings of the 3 to 10 membered heterocyclyl and 4 to 8 membered heterocyclyl are each independently monocyclic ring, bridged ring, spiro ring or fused ring.

In some embodiments of the present invention, ring C is

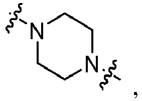

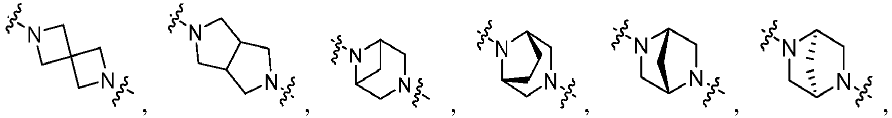

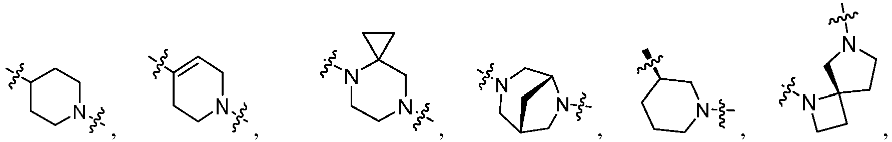

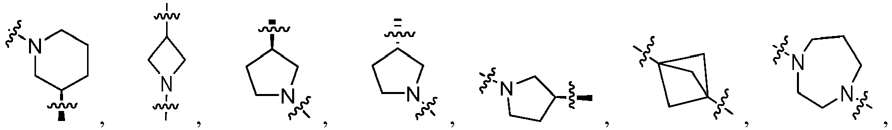

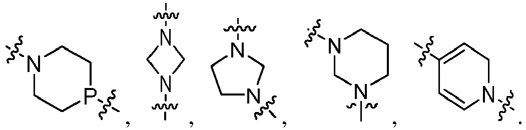

In some embodiments of the present invention, ring C is

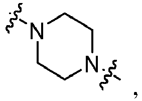

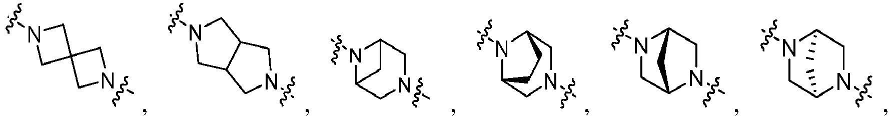

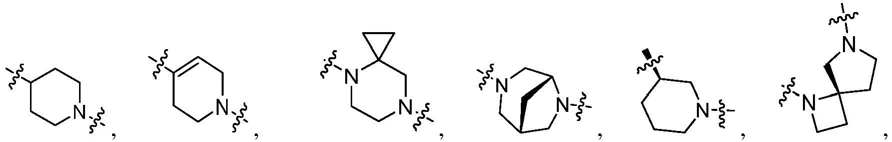

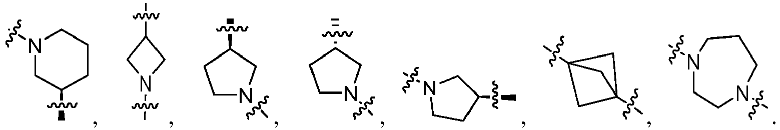

In some embodiments of the present invention, ring C is

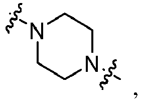

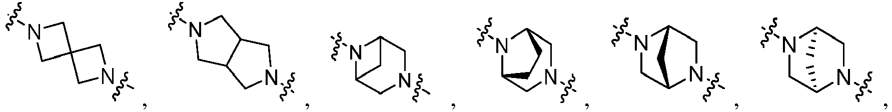

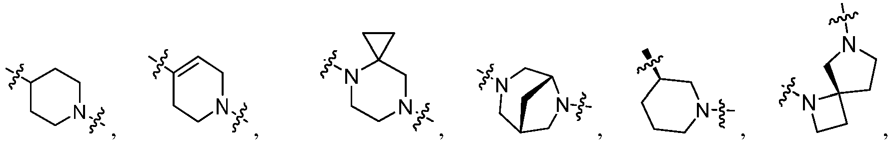

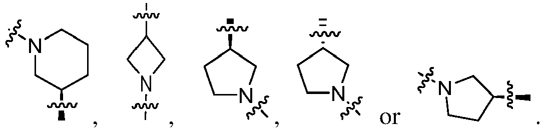

In some embodiments of the present invention, ring D is a 6 to 10 membered heterocyclyl, C₆₋₁₀ aryl or 5 to 10 membered heteroaryl; more preferably, ring D is a 5 membered heteroaromatic ring, 6 membered heteroaromatic ring, benzene ring, 5 membered heteroaromatic ring fused with 6 membered heteroaromatic ring, 6 membered heteroaromatic ring fused with 6 membered heteroaromatic ring, 6 membered heteroaromatic ring fused with 6 membered heterocyclic ring, 6 membered heteroaromatic ring fused with 5 membered heterocyclic ring, benzene ring fused with 5 membered heterocyclic ring, benzene ring fused with 6 membered heteroaromatic ring, benzene ring fused with 6 membered heterocyclic ring; wherein the heteroatoms in the 3 to 14 membered heterocyclyl, 6 to 10 membered heterocyclyl, 5 to 10 membered heteroaryl and 5 to 14 membered heteroaryl are each independently selected from the group consisting of nitrogen, oxygen and sulfur, and the number of heteroatoms is independently 1, 2 or 3.

In some embodiments of the present invention, ring D is the following groups:

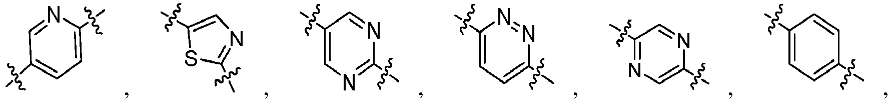

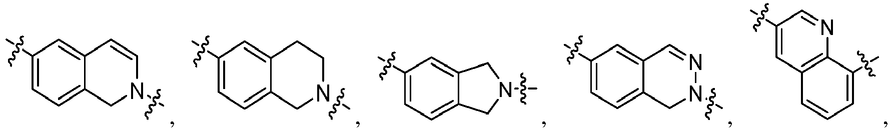

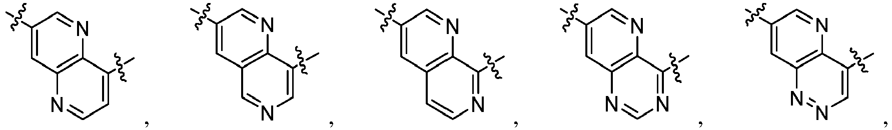

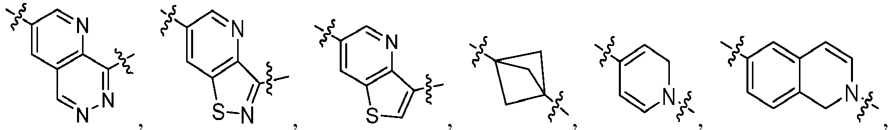

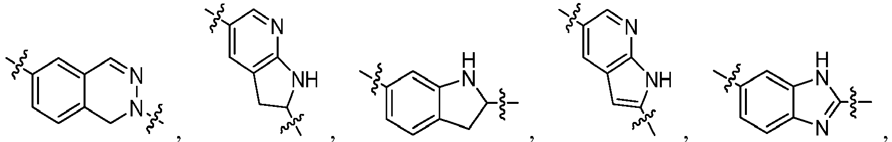

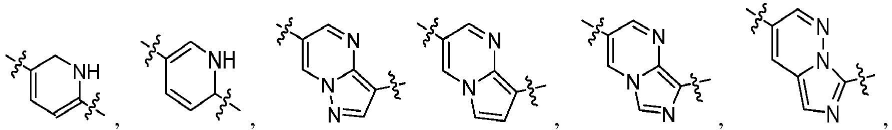

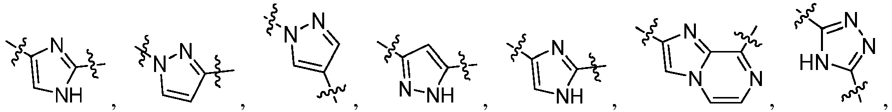

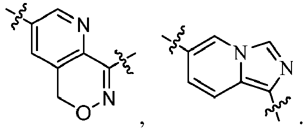

In some embodiments of the present invention, ring D is selected from the group consisting of:

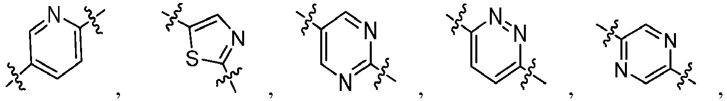

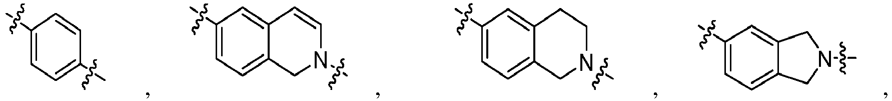

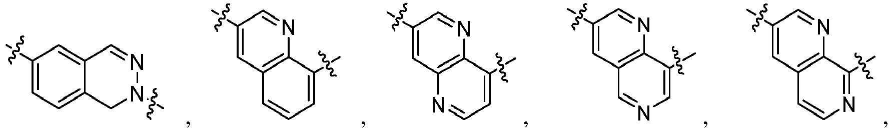

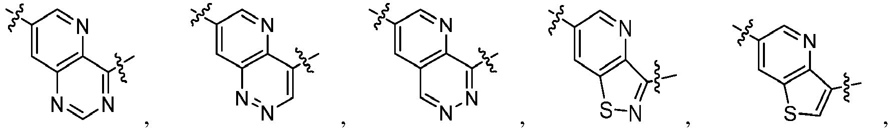

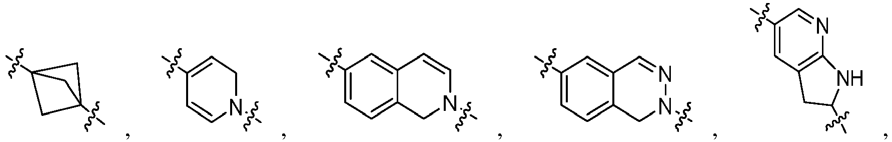

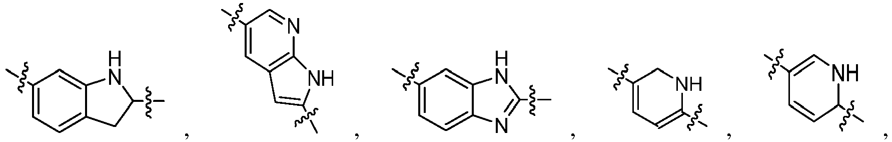

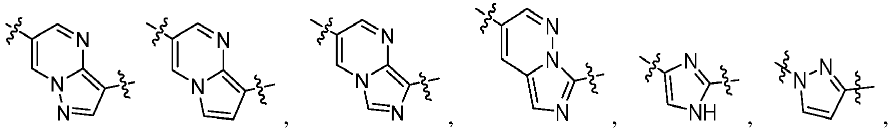

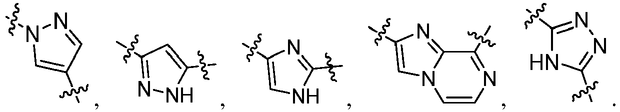

In some embodiments of the present invention, ring D is

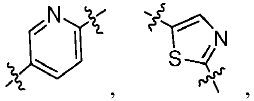

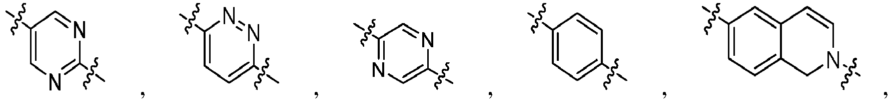

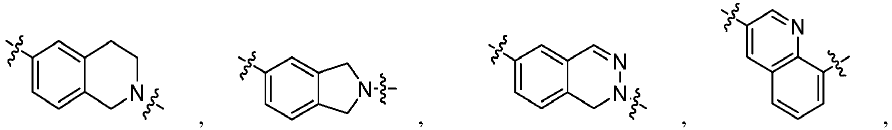

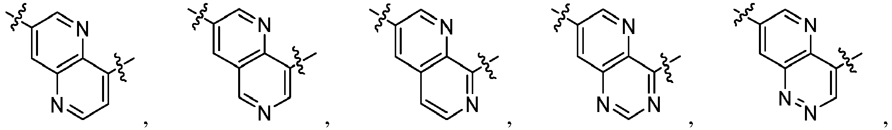

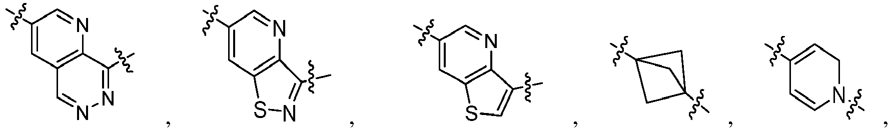

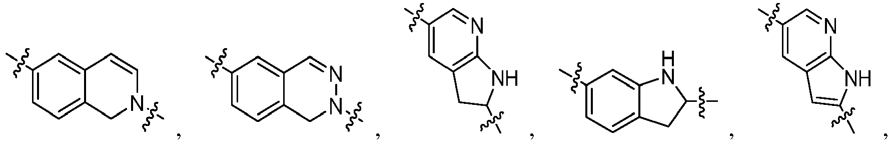

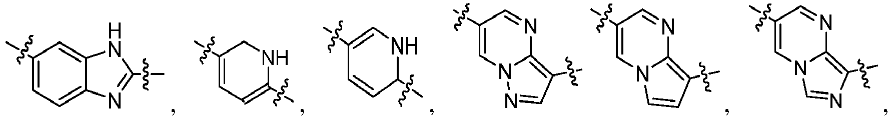

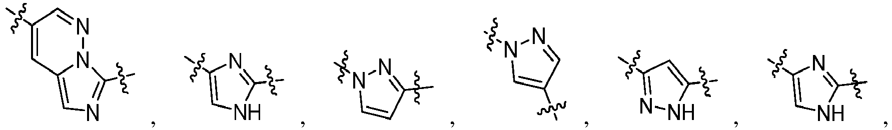

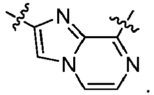

In some embodiments of the present invention, ring D is

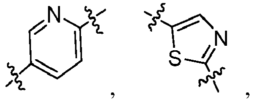

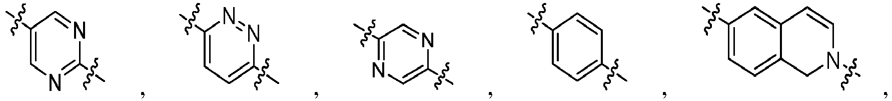

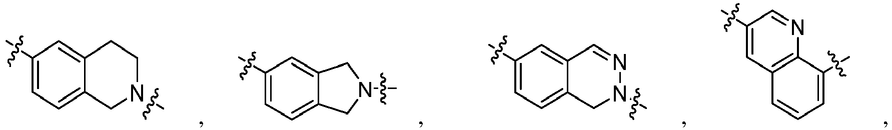

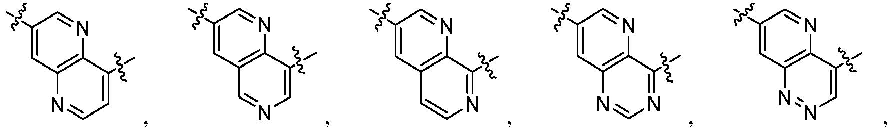

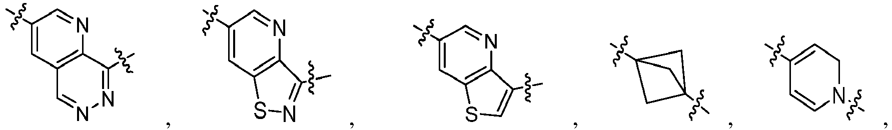

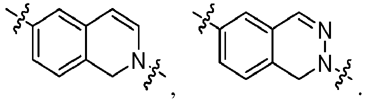

In some embodiments of the present invention, ring D is

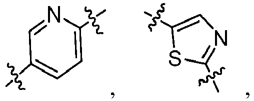

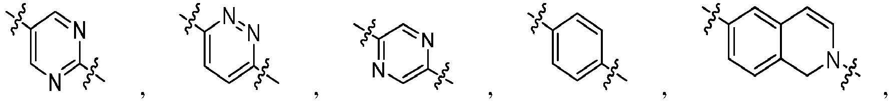

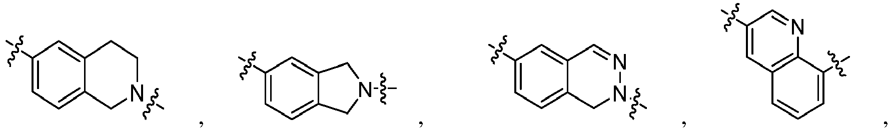

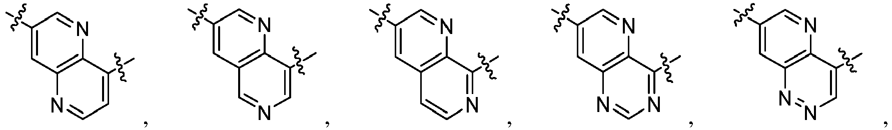

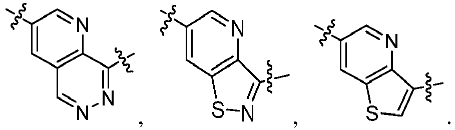

In some embodiments of the present invention, R₁ is selected from the group consisting of hydrogen, deuterium, halogen, nitro, hydroxy, thiol, cyano, amino, C₁₋₆ alkyl, C₁₋₆ deuterated alkyl, C₁₋₆ haloalkyl, C₁₋₆ hydroxyalkyl, C₁₋₆ alkoxy, C₂₋₆ alkenyl, C₂₋₆ alkynyl, C₃₋₁₂ cycloalkyl, 3 to 12 membered heterocyclyl, C₆₋₁₂ aryl, 5 to 12 membered heteroaryl, -(CH₂)ₙR₁₁, -(CH₂)ₙOR₁₁, -(CH₂)ₙC(O)R₁₁, -(CH₂)ₙC(O)OR₁₁, -(CH₂)ₙS(O)ₘR₁₁, -(CH₂)ₙNR₂₂R₃₃, -(CH₂)ₙNR₂₂C(O)OR₃₃, -(CH₂)ₙNR₂₂C(O)(CH₂)ₙ₁R₃₃, -(CH₂)ₙNR₂₂C(O)NR₂₂R₃₃, -(CH₂)ₙC(O)NR₂₂(CH₂)ₙ₁R₃₃, -OC(R₁₁R₂₂)ₙ(CH₂)ₙ₁R₃₃ and -(CH₂)ₙNR₂₂S(O)ₘR₃₃, the amino, C₁₋₆ alkyl, C₁₋₆ deuterated alkyl, C₁₋₆ haloalkyl, C₁₋₆ hydroxyalkyl, C₁₋₆ alkoxy, C₂₋₆ alkenyl, C₂₋₆ alkynyl, C₃₋₁₂ cycloalkyl, 3 to 12 membered heterocyclyl, C₆₋₁₂ aryl and 5 to 12 membered heteroaryl can be each optionally further substituted by one or more substituents selected from the group consisting of hydroxy, halogen, nitro, hydroxy, thiol, cyano, amino, oxo, C₁₋₆ alkyl, C₁₋₆ alkoxy, C₆₋₁₂ aryl, 5 to 12 membered heteroaryl and 3 to 12 membered heterocyclyl.

In some embodiments of the present invention, R₁ is selected from the group consisting of hydrogen, deuterium, halogen, nitro, hydroxy, thiol, cyano, amino, C₁₋₆ alkyl, C₁₋₆ deuterated alkyl, C₁₋₆ haloalkyl, C₁₋₆ hydroxyalkyl, C₁₋₆ alkoxy, C₂₋₆ alkenyl, C₂₋₆ alkynyl, C₃₋₁₂ cycloalkyl, 3 to 12 membered heterocyclyl, C₆₋₁₂ aryl, 5 to 12 membered heteroaryl, -(CH₂)ₙR₁₁, -(CH₂)ₙOR₁₁, -(CH₂)ₙC(O)R₁₁, -(CH₂)ₙC(O)OR₁₁, -(CH₂)ₙS(O)ₘR₁₁, -(CH₂)ₙNR₂₂R₃₃, -(CH₂)ₙNR₂₂C(O)OR₃₃, -(CH₂)ₙNR₂₂C(O)(CH₂)ₙ₁R₃₃, -(CH₂)ₙNR₂₂C(O)NR₂₂R₃₃, -(CH₂)ₙC(O)NR₂₂(CH₂)ₙ₁R₃₃, -OC(R₁₁R₂₂)ₙ(CH₂)ₙ₁R₃₃ and -(CH₂)ₙNR₂₂S(O)ₘR₃₃, the amino, C₁₋₆ alkyl, C₁₋₆ deuterated alkyl, C₁₋₆ haloalkyl, C₁₋₆ hydroxyalkyl, C₁₋₆ alkoxy, C₂₋₆ alkenyl, C₂₋₆ alkynyl, C₃₋₁₂ cycloalkyl, 3 to 12 membered heterocyclyl, C₆₋₁₂ aryl and 5 to 12 membered heteroaryl can be each optionally further substituted by one or more substituents selected from the group consisting of hydroxy, halogen, amino, C₁₋₆ alkyl and 3 to 12 membered heterocyclyl.

In some embodiments of the present invention, R₁₁, R₂₂ and R₃₃ are each independently selected from the group consisting of hydrogen, deuterium, halogen, amino, hydroxy, cyano, nitro, C₁₋₆ alkyl, C₂₋₆ alkenyl, C₂₋₆ alkynyl, C₁₋₆ deuterated alkyl, C₁₋₆ haloalkyl, C₁₋₆ alkoxy, C₁₋₆ haloalkoxy, C₁₋₆ hydroxyalkyl, cyano-substituted C₁₋₆ alkyl, C₃₋₁₂ cycloalkyl, 3 to 12 membered heterocyclyl, C₆₋₁₂ aryl, 5 to 12 membered heteroaryl, -O(CH₂)ₙ₁R₆₆, -OC(R₄₄R₅₅)ₘ₁(CH₂)ₙ₁R₆₆, -NR₄₄(CH₂)ₙ₁R₆₆, -(CH₂)ₙ₁-, -(CH₂)ₙ₁R₆₆, -(CH₂)ₙ₁OR₆₆, -(CH₂)ₙ₁SR₆₆, -(CH₂)ₙ₁C(O)R₆₆, -(CH₂)ₙ₁C(O)OR₆₆, -(CH₂)ₙ₁S(O)ₘ₁R₆₆, -(CH2)ₙ₁NR₄₄R₅₅, -(CH₂)ₙ₁C(O)NR₄₄R₅₅, -(CH₂)ₙ₁NR₄₄C(O)R₆₆ and -(CH₂)ₙ₁NRₐ₄S(O)ₘ₁R₆₆, each optionally substituted by one or more substituents selected from the group consisting of deuterium, halogen, amino, hydroxy, cyano, nitro, C₁₋₆ alkyl and C₃₋₁₂ cycloalkyl.

In some embodiments of the present invention, R₄₄, R₅₅ and R₆₆ are each independently selected from the group consisting of hydrogen, deuterium, halogen, amino, hydroxy, cyano, nitro, C₁₋₆ alkyl, C₂₋₆ alkenyl, C₂₋₆ alkynyl, C₁₋₆ deuterated alkyl, C₁₋₆ haloalkyl, C₁₋₆ alkoxy, C₁₋₆ haloalkoxy, C₁₋₆ hydroxyalkyl, cyano-substituted C₁₋₆ alkyl, C₃₋₁₂ cycloalkyl, 3 to 12 membered heterocyclyl, C₆₋₁₂ aryl and 5 to 12 membered heteroaryl, each optionally substituted by one or more substituents selected from the group consisting of deuterium, halogen, amino, hydroxy, cyano, nitro, C₁₋₆ alkyl and C₃₋₁₂ cycloalkyl.

In some embodiments of the present invention, R₁ is selected from the group consisting of hydrogen, deuterium, halogen, nitro, hydroxy, thiol, cyano, amino, C₁₋₆ alkyl, C₁₋₆ deuterated alkyl, C₁₋₆ haloalkyl, C₁₋₆ hydroxyalkyl, C₁₋₆ alkoxy, C₂₋₆ alkenyl, C₂₋₆ alkynyl, C₃₋₁₂ cycloalkyl, 3 to 12 membered heterocyclyl, C₆₋₁₂ aryl and 5 to 12 membered heteroaryl, each can be optionally further substituted by one or more substituents selected from the group consisting of hydroxy, halogen, amino, C₁₋₆ alkyl and 3 to 12 membered heterocyclyl.

In some embodiments of the present invention, R₁ is selected from the group consisting of hydrogen, deuterium, halogen, nitro, hydroxy, thiol, cyano, amino, C₁₋₆ alkyl, C₁₋₆ deuterated alkyl, C₁₋₆ haloalkyl, C₁₋₆ hydroxyalkyl, C₁₋₆ alkoxy, C₂₋₆ alkenyl, C₂₋₆ alkynyl, C₃₋₁₂ cycloalkyl, 3 to 12 membered heterocyclyl, C₆₋₁₂ aryl and 5 to 12 membered heteroaryl.

In some embodiments of the present invention, R₁ is selected from the group consisting of hydrogen, deuterium, cyano, C₁₋₃ deuterated alkyl, C₁₋₃ alkyl, C₁₋₃ alkoxy, C₃₋₆ cycloalkyl and 3 to 6 membered heterocyclyl, each can be optionally further substituted by one or more substituents selected from the group consisting of hydroxy, halogen, amino, C₁₋₃ alkyl and 3 to 6 membered heterocyclyl.

In some embodiments of the present invention, R₁ is selected from the group consisting of hydrogen, deuterium, C₁₋₃ deuterated alkyl, C₁₋₃ alkyl, C₃₋₆ cycloalkyl, 5 to 6 membered heteroaryl, 4 to 6 membered heterocyclyl and oxo-substituted 4 to 6 membered heterocyclyl.

In some embodiments of the present invention, R₁ is selected from the group consisting of hydrogen, deuterium, C₁₋₆ deuterated alkyl, C₁₋₆ alkyl, C₃₋₁₂ cycloalkyl and 3 to 12 membered heterocyclyl.

In some embodiments of the present invention, R₁ is selected from the group consisting of hydrogen, -CH₃, -CD₃, -CH₂CN, ethyl, methoxy, cyano, cyclopropyl,

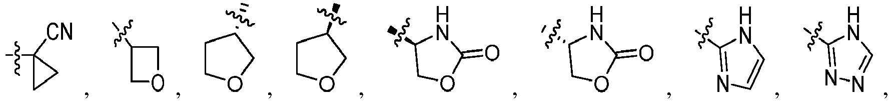

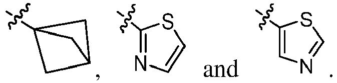

In some embodiments of the present invention, R₁ is selected from the group consisting of hydrogen, -CH₃, -CD₃, ethyl, methoxy, cyano, cyclopropyl,

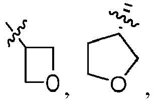

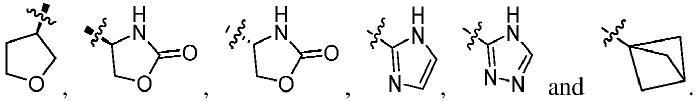

In some embodiments of the present invention, R₁ is selected from the group consisting of hydrogen, -CH₃, -CD₃, cyclopropyl,

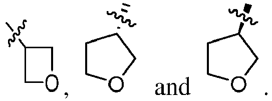

In some embodiments of the present invention, each R^{a} is independently selected from the group consisting of hydrogen, deuterium, halogen, nitro, hydroxy, thiol, oxo, cyano, amino, C₁₋₆ alkyl, C₁₋₆ deuterated alkyl, C₁₋₆ haloalkyl, C₁₋₆ hydroxyalkyl, C₁₋₆ alkoxy, C₂₋₆ alkenyl, C₂₋₆ alkynyl, C₃₋₁₂ cycloalkyl, 3 to 12 membered heterocyclyl, C₆₋₁₂ aryl, 5 to 12 membered heteroaryl, -(CH₂)ₙRₐ₁, -(CH₂)ₙORₐ₁, -(CH₂)ₙC(O)Rₐ₁, -(CH₂)ₙC(O)ORₐ₁, -(CH₂)ₙS(O)ₘRₐ₁, -(CH₂)ₙNRₐ₂Rₐ₃, -(CH₂)ₙNRₐ₂C(O)ORₐ₃, -(CH₂)ₙNRₐ₂C(O)(CH₂)ₙ₁Rₐ₃, -(CH₂)ₙNRₐ₂C(O)NRₐ₂Rₐ₃, -(CH₂)ₙC(O)NRₐ₂(CH₂)ₙ₁Rₐ₃, -OC(Rₐ₁Rₐ₂)ₙ(CH₂)ₙ₁Rₐ₃ and -(CH₂)ₙNRₐ₂S(O)ₘRₐ₃, the amino, C₁₋₆ alkyl, C₁₋₆ deuterated alkyl, C₁₋₆ haloalkyl, C₁₋₆ hydroxyalkyl, C₁₋₆ alkoxy, C₂₋₆ alkenyl, C₂₋₆ alkynyl, C₃₋₁₂ cycloalkyl, 3 to 12 membered heterocyclyl, C₆₋₁₂ aryl and 5 to 12 membered heteroaryl can be each optionally further substituted by one or more substituents selected from the group consisting of hydroxy, nitro, thiol, oxo, cyano, halogen, C₁₋₆ deuterated alkyl, C₁₋₆ haloalkyl, C₁₋₆ hydroxyalkyl, amino, C₁₋₆ alkyl, C₁₋₆ alkoxy, C₂₋₆ alkenyl, C₂₋₆ alkynyl, C₃₋₈ cycloalkyl, C₆₋₁₀ aryl, 5 to 10 membered heteroaryl and 3 to 12 membered heterocyclyl.

In some embodiments of the present invention, each R^{a} is independently selected from the group consisting of hydrogen, deuterium, halogen, nitro, hydroxy, thiol, oxo, cyano, amino, C₁₋₆ alkyl, C₁₋₆ deuterated alkyl, C₁₋₆ haloalkyl, C₁₋₆ hydroxyalkyl, C₁₋₆ alkoxy, C₂₋₆ alkenyl, C₂₋₆ alkynyl, C₃₋₁₂ cycloalkyl, 3 to 12 membered heterocyclyl, C₆₋₁₂ aryl, 5 to 12 membered heteroaryl, -(CH₂)ₙRₐ₁, -(CH₂)ₙORₐ₁, -(CH₂)ₙC(O)Rₐ₁, -(CH₂)ₙC(O)ORₐ₁, -(CH₂)ₙS(O)ₘRₐ₁, -(CH₂)ₙNRₐ₂Rₐ₃, -(CH₂)ₙNRₐ₂C(O)ORₐ₃, -(CH₂)ₙNRₐ₂C(O)(CH₂)ₙ₁Rₐ₃, -(CH₂)ₙNRₐ₂C(O)NRₐ₂Rₐ₃, -(CH₂)ₙC(O)NRₐ₂(CH₂)ₙ₁Rₐ₃, -OC(Rₐ₁Rₐ₂)ₙ(CH₂)ₙ₁Rₐ₃ and -(CH₂)ₙNRₐ₂S(O)ₘRₐ₃, the amino, C₁₋₆ alkyl, C₁₋₆ deuterated alkyl, C₁₋₆ haloalkyl, C₁₋₆ hydroxyalkyl, C₁₋₆ alkoxy, C₂₋₆ alkenyl, C₂₋₆ alkynyl, C₃₋₁₂ cycloalkyl, 3 to 12 membered heterocyclyl, C₆₋₁₂ aryl and 5 to 12 membered heteroaryl can be each optionally further substituted by one or more substituents selected from the group consisting of hydroxy, halogen, amino, C₁₋₆ alkyl and 3 to 12 membered heterocyclyl.

In some embodiments of the present invention, each R^{a} is independently selected from the group consisting of hydrogen, deuterium, halogen, nitro, hydroxy, thiol, oxo, cyano, amino, C₁₋₃ alkyl, C₁₋₃ deuterated alkyl, C₁₋₃ haloalkyl, C₁₋₃ hydroxyalkyl, C₁₋₃ alkoxy, C₂₋₃ alkenyl, C₂₋₃ alkynyl, C₃₋₆ cycloalkyl, 3 to 6 membered heterocyclyl, C₆₋₁₀ aryl, 5 to 10 membered heteroaryl, -(CH₂)ₙRₐ₁, -(CH₂)ₙORₐ₁, -(CH₂)ₙC(O)Rₐ₁, -(CH₂)ₙC(O)ORₐ₁, -(CH₂)ₙS(O)ₘRₐ₁, -(CH₂)ₙNRₐ₂Rₐ₃, -(CH₂)ₙNRₐ₂C(O)ORₐ₃, -(CH₂)ₙNRₐ₂C(O)(CH₂)ₙ₁Rₐ₃, -(CH₂)ₙNRₐ₂C(O)NRₐ₂Rₐ₃, -(CH₂)ₙC(O)NRₐ₂(CH₂)ₙ₁Rₐ₃, -OC(Rₐ₁Rₐ₂)ₙ(CH₂)ₙ₁Rₐ₃ and -(CH₂)ₙNRₐ₂S(O)ₘRₐ₃, the amino, C₁₋₃ alkyl, C₁₋₃ deuterated alkyl, C₁₋₃ haloalkyl, C₁₋₃ hydroxyalkyl, C₁₋₃ alkoxy, C₂₋₃ alkenyl, C₂₋₃ alkynyl, C₃₋₆ cycloalkyl, 3 to 6 membered heterocyclyl, C₆₋₁₀ aryl and 5 to 10 membered heteroaryl can be each optionally further substituted by one or more substituents selected from the group consisting of hydroxy, halogen, amino, C₁₋₃ alkyl and 3 to 6 membered heterocyclyl.

In some embodiments of the present invention, Rₐ₁, Rₐ₂ and Rₐ₃ are each independently selected from the group consisting of hydrogen, deuterium, halogen, nitro, hydroxy, thiol, cyano, amino, C₁₋₆ alkyl, C₁₋₆ deuterated alkyl, C₁₋₆ haloalkyl, C₁₋₆ hydroxyalkyl, C₁₋₆ alkoxy, C₂₋₆ alkenyl, C₂₋₆ alkynyl, C₃₋₁₂ cycloalkyl, 3 to 12 membered heterocyclyl, C₆₋₁₂ aryl and 5 to 12 membered heteroaryl, the amino, C₁₋₆ alkyl, C₁₋₆ deuterated alkyl, C₁₋₆ haloalkyl, C₁₋₆ hydroxyalkyl, C₁₋₆ alkoxy, C₂₋₆ alkenyl, C₂₋₆ alkynyl, C₃₋₁₂ cycloalkyl, 3 to 12 membered heterocyclyl, C₆₋₁₂ aryl and 5 to 12 membered heteroaryl can be each optionally further substituted by one or more substituents selected from the group consisting of hydroxy, halogen, amino, C₁₋₆ alkyl and 3 to 12 membered heterocyclyl.

In some embodiments of the present invention, each R^{a} is independently selected from the group consisting of hydrogen, deuterium, halogen, nitro, hydroxy, thiol, oxo, cyano, amino optionally substituted by C₁₋₃ alkyl, C₁₋₃ haloalkyl, C₁₋₃ alkyl, C₁₋₃ alkoxy, C₂₋₄ alkynyl and C₃₋₆ cycloalkyl.

In some embodiments of the present invention, each R^{a} is independently selected from the group consisting of hydrogen, deuterium, halogen, nitro, hydroxy, thiol, oxo, cyano, amino, C₁₋₃ haloalkyl, C₁₋₃ alkyl and C₃₋₆ cycloalkyl.

In some embodiments of the present invention, each R^{a} is independently selected from the group consisting of hydrogen, deuterium, halogen, nitro, hydroxy, thiol, oxo, cyano, amino, C₁₋₃ alkyl and C₃₋₆ cycloalkyl.

In some embodiments of the present invention, each R^{a} is independently selected from the group consisting of hydrogen, ethyl, oxo, cyclopropyl, methyl, methoxy, ethynyl, propynyl, trifluoromethyl and cyano.

In some embodiments of the present invention, each R^{a} is independently selected from the group consisting of hydrogen, ethyl, oxo, cyclopropyl, methyl, methoxy, ethynyl, trifluoromethyl and cyano.

In some embodiments of the present invention, each R^{a} is independently selected from the group consisting of hydrogen, ethyl, oxo, cyclopropyl, methyl, trifluoromethyl and cyano.

In some embodiments of the present invention, each R^{a} is independently selected from the group consisting of hydrogen, ethyl, oxo, cyclopropyl and methyl.

In some embodiments of the present invention, x is 1, 2 or 3.

In some embodiments of the present invention, each R^{b} is independently selected from the group consisting of hydrogen, deuterium, halogen, nitro, hydroxy, thiol, oxo, cyano, amino, C₁₋₆ alkyl, C₁₋₆ deuterated alkyl, C₁₋₆ haloalkyl, C₁₋₆ hydroxyalkyl, C₁₋₆ alkoxy, C₂₋₆ alkenyl, C₂₋₆ alkynyl, C₃₋₁₂ cycloalkyl, 3 to 12 membered heterocyclyl, C₆₋₁₂ aryl, 5 to 12 membered heteroaryl, -(CH₂)ₙR_{b1}, -(CH₂)ₙOR_{b1}, -(CH₂)ₙC(O)R_{b1}, -(CH₂)ₙC(O)OR_{b1}, -(CH₂)ₙS(O)ₘR_{b1}, -(CH₂)ₙNR_{b2}R_{b3}, -(CH₂)ₙNR_{b2}C(O)OR_{b3}, -(CH₂)ₙNR_{b2}C(O)(CH₂)ₙ₁R_{b3}, -(CH₂)ₙNR_{b2}C(O)NR_{b2}R_{b3}, -(CH₂)ₙC(O)NR_{b2}(CH₂)ₙ₁R_{b3}, -OC(R_{b1}R_{b2})ₙ(CH₂)ₙ₁R_{b3} and -(CH₂)ₙNR_{b2}S(O)ₘR_{b3}, the amino, C₁₋₆ alkyl, C₁₋₆ deuterated alkyl, C₁₋₆ haloalkyl, C₁₋₆ hydroxyalkyl, C₁₋₆ alkoxy, C₂₋₆ alkenyl, C₂₋₆ alkynyl, C₃₋₁₂ cycloalkyl, 3 to 12 membered heterocyclyl, C₆₋₁₂ aryl and 5 to 12 membered heteroaryl can be each optionally further substituted by one or more substituents selected from the group consisting of hydroxy, nitro, thiol, oxo, cyano, halogen, C₁₋₆ deuterated alkyl, C₁₋₆ haloalkyl, C₁₋₆ hydroxyalkyl, amino, C₁₋₆ alkyl, C₁₋₆ alkoxy, C₂₋₆ alkenyl, C₂₋₆ alkynyl, C₃₋₈ cycloalkyl, C₆₋₁₀ aryl, 5 to 10 membered heteroaryl and 3 to 12 membered heterocyclyl.

In some embodiments of the present invention, each R^{b} is independently selected from the group consisting of hydrogen, deuterium, halogen, nitro, hydroxy, thiol, oxo, cyano, amino, C₁₋₆ alkyl, C₁₋₆ deuterated alkyl, C₁₋₆ haloalkyl, C₁₋₆ hydroxyalkyl, C₁₋₆ alkoxy, C₂₋₆ alkenyl, C₂₋₆ alkynyl, C₃₋₁₂ cycloalkyl, 3 to 12 membered heterocyclyl, C₆₋₁₂ aryl, 5 to 12 membered heteroaryl, -(CH₂)ₙR_{b1}, -(CH₂)ₙOR_{b1}, -(CH₂)ₙC(O)R_{b1}, -(CH₂)ₙC(O)OR_{b1}, -(CH₂)ₙS(O)ₘR_{b1}, -(CH₂)ₙNR_{b2}R_{b3}, -(CH₂)ₙNR_{b2}C(O)OR_{b3}, -(CH₂)ₙNR_{b2}C(O)(CH₂)ₙ₁R_{b3}, -(CH₂)ₙNR_{b2}C(O)NR_{b2}R_{b3}, -(CH₂)ₙC(O)NR_{b2}(CH₂)ₙ₁R_{b3}, -OC(R_{b1}R_{b2})ₙ(CH₂)ₙ₁R_{b3} and -(CH₂)ₙNR_{b2}S(O)ₘR_{b3}, the amino, C₁₋₆ alkyl, C₁₋₆ deuterated alkyl, C₁₋₆ haloalkyl, C₁₋₆ hydroxyalkyl, C₁₋₆ alkoxy, C₂₋₆ alkenyl, C₂₋₆ alkynyl, C₃₋₁₂ cycloalkyl, 3 to 12 membered heterocyclyl, C₆₋₁₂ aryl and 5 to 12 membered heteroaryl can be each optionally further substituted by one or more substituents selected from the group consisting of hydroxy, halogen, amino, C₁₋₆ alkyl and 3 to 12 membered heterocyclyl.

In some embodiments of the present invention, each R^{b} is independently selected from the group consisting of hydrogen, deuterium, halogen, nitro, hydroxy, thiol, oxo, cyano, amino, C₁₋₃ alkyl, C₁₋₃ deuterated alkyl, C₁₋₃ haloalkyl, C₁₋₃ hydroxyalkyl, C₁₋₃ alkoxy, C₂₋₃ alkenyl, C₂₋₃ alkynyl, C₃₋₆ cycloalkyl, 3 to 6 membered heterocyclyl, C₆₋₁₀ aryl, 5 to 10 membered heteroaryl, -(CH₂)ₙR_{b1}, -(CH₂)ₙOR_{b1}, -(CH₂)ₙC(O)R_{b1}, -(CH₂)ₙC(O)OR_{b1}, -(CH₂)ₙS(O)ₘR_{b1}, -(CH₂)ₙNR_{b2}R_{b3}, -(CH₂)ₙNR_{b2}C(O)OR_{b3}, -(CH₂)ₙNR_{b2}C(O)(CH₂)ₙ₁R_{b3}, -(CH₂)ₙNR_{b2}C(O)NR_{b2}R_{b3}, -(CH₂)ₙC(O)NR_{b2}(CH₂)ₙ₁R_{b3}, -OC(R_{b1}R_{b2})ₙ(CH₂)ₙ₁R_{b3} and -(CH₂)ₙNR_{b2}S(O)ₘR_{b3}, the amino, C₁₋₃ alkyl, C₁₋₃ deuterated alkyl, C₁₋₃ haloalkyl, C₁₋₃ hydroxyalkyl, C₁₋₃ alkoxy, C₂₋₃ alkenyl, C₂₋₃ alkynyl, C₃₋₆ cycloalkyl, 3 to 6 membered heterocyclyl, C₆₋₁₀ aryl and 5 to 10 membered heteroaryl can be each optionally further substituted by one or more substituents selected from the group consisting of hydroxy, halogen, amino, C₁₋₃ alkyl and 3 to 6 membered heterocyclyl.

In some embodiments of the present invention, R_{b1}, R_{b2} and R_{b3} are each independently selected from the group consisting of hydrogen, deuterium, halogen, nitro, hydroxy, thiol, cyano, amino, C₁₋₆ alkyl, C₁₋₆ deuterated alkyl, C₁₋₆ haloalkyl, C₁₋₆ hydroxyalkyl, C₁₋₆ alkoxy, C₂₋₆ alkenyl, C₂₋₆ alkynyl, C₃₋₁₂ cycloalkyl, 3 to 12 membered heterocyclyl, C₆₋₁₂ aryl and 5 to 12 membered heteroaryl, the amino, C₁₋₆ alkyl, C₁₋₆ deuterated alkyl, C₁₋₆ haloalkyl, C₁₋₆ hydroxyalkyl, C₁₋₆ alkoxy, C₂₋₆ alkenyl, C₂₋₆ alkynyl, C₃₋₁₂ cycloalkyl, 3 to 12 membered heterocyclyl, C₆₋₁₂ aryl and 5 to 12 membered heteroaryl can be each optionally further substituted by one or more substituents selected from the group consisting of hydroxy, halogen, amino, C₁₋₆ alkyl and 3 to 12 membered heterocyclyl.

In some embodiments of the present invention, each R^{b} is independently selected from the group consisting of hydrogen, deuterium, halogen, nitro, hydroxy, thiol, oxo, cyano, amino, C₁₋₃ alkyl, C₁₋₃ haloalkyl and C₃₋₆ cycloalkyl.

In some embodiments of the present invention, each R^{b} is independently selected from the group consisting of hydrogen, F, -CF₃, cyano, methyl and cyclopropyl.

In some embodiments of the present invention, y is 1.

In some embodiments of the present invention, each R^{c} is independently selected from the group consisting of hydrogen, deuterium, halogen, nitro, hydroxy, thiol, oxo, cyano, amino, C₁₋₆ alkyl, C₁₋₆ deuterated alkyl, C₁₋₆ haloalkyl, C₁₋₆ hydroxyalkyl, C₁₋₆ alkoxy, C₂₋₆ alkenyl, C₂₋₆ alkynyl, C₃₋₁₂ cycloalkyl, 3 to 12 membered heterocyclyl, C₆₋₁₂ aryl, 5 to 12 membered heteroaryl, -(CH₂)ₙR_{c1}, -(CH₂)ₙOR_{c1}, -(CH₂)ₙC(O)R_{c1}, -(CH₂)ₙC(O)OR_{c1}, -(CH₂)ₙS(O)ₘR_{c1}, -(CH₂)ₙNR_{c2}R_{c3}, -(CH₂)ₙNR_{c2}C(O)OR_{c3}, -(CH₂)ₙNR_{c2}C(O)(CH₂)ₙ₁R_{c3}, -(CH₂)ₙNRC₂C(O)NR_{c2}R_{c3}, -(CH₂)ₙC(O)NR_{c2}(CH₂)ₙ₁R_{c3}, -OC(R_{c1}R_{c2})ₙ(CH₂)ₙ₁R_{c3} and -(CH₂)ₙNR_{c2}S(O)ₘR_{c3}, the amino, C₁₋₆ alkyl, C₁₋₆ deuterated alkyl, C₁₋₆ haloalkyl, C₁₋₆ hydroxyalkyl, C₁₋₆ alkoxy, C₂₋₆ alkenyl, C₂₋₆ alkynyl, C₃₋₁₂ cycloalkyl, 3 to 12 membered heterocyclyl, C₆₋₁₂ aryl and 5 to 12 membered heteroaryl can be each optionally further substituted by one or more substituents selected from the group consisting of hydroxy, nitro, thiol, oxo, cyano, halogen, C₁₋₆ deuterated alkyl, C₁₋₆ haloalkyl, C₁₋₆ hydroxyalkyl, amino, C₁₋₆ alkyl, C₁₋₆ alkoxy, C₂₋₆ alkenyl, C₂₋₆ alkynyl, C₃₋₈ cycloalkyl, C₆₋₁₀ aryl, 5 to 10 membered heteroaryl and 3 to 12 membered heterocyclyl.

In some embodiments of the present invention, each R^{c} is independently selected from the group consisting of hydrogen, deuterium, halogen, nitro, hydroxy, thiol, oxo, cyano, amino, C₁₋₆ alkyl, C₁₋₆ deuterated alkyl, C₁₋₆ haloalkyl, C₁₋₆ hydroxyalkyl, C₁₋₆ alkoxy, C₂₋₆ alkenyl, C₂₋₆ alkynyl, C₃₋₁₂ cycloalkyl, 3 to 12 membered heterocyclyl, C₆₋₁₂ aryl, 5 to 12 membered heteroaryl, -(CH₂)ₙR_{c1}, -(CH₂)ₙOR_{c1}, -(CH₂)ₙC(O)R_{c1}, -(CH₂)ₙC(O)OR_{c1}, -(CH₂)ₙS(O)ₘR_{c1}, -(CH₂)ₙNR_{c2}R_{c3}, -(CH₂)ₙNR_{c2}C(O)OR_{c3}, -(CH₂)ₙNR_{c2}C(O)(CH₂)ₙ₁R_{c3}, -(CH₂)ₙNRC₂C(O)NR_{c2}R_{c3}, -(CH₂)ₙC(O)NR_{c2}(CH₂)ₙ₁R_{c3}, -OC(R_{c1}R_{c2})ₙ(CH₂)ₙ₁R_{c3} and -(CH₂)ₙNR_{c2}S(O)ₘR_{c3}, the amino, C₁₋₆ alkyl, C₁₋₆ deuterated alkyl, C₁₋₆ haloalkyl, C₁₋₆ hydroxyalkyl, C₁₋₆ alkoxy, C₂₋₆ alkenyl, C₂₋₆ alkynyl, C₃₋₁₂ cycloalkyl, 3 to 12 membered heterocyclyl, C₆₋₁₂ aryl and 5 to 12 membered heteroaryl can be each optionally further substituted by one or more substituents selected from the group consisting of hydroxy, halogen, amino, C₁₋₆ alkyl and 3 to 12 membered heterocyclyl.

In some embodiments of the present invention, each R^{c} is independently selected from the group consisting of hydrogen, deuterium, halogen, nitro, hydroxy, thiol, oxo, cyano, amino, C₁₋₃ alkyl, C₁₋₃ deuterated alkyl, C₁₋₃ haloalkyl, C₁₋₃ hydroxyalkyl, C₁₋₃ alkoxy, C₂₋₃ alkenyl, C₂₋₃ alkynyl, C₃₋₆ cycloalkyl, 3 to 6 membered heterocyclyl, C₆₋₁₀ aryl, 5 to 10 membered heteroaryl, -(CH₂)ₙR_{c1}, -(CH₂)ₙOR_{c1}, -(CH₂)ₙC(O)R_{c1}, -(CH₂)ₙC(O)OR_{c1}, -(CH₂)ₙS(O)ₘR_{c1}, -(CH₂)ₙNR_{c2}R_{c3}, -(CH₂)ₙNR_{c2}C(O)OR_{c3}, -(CH₂)ₙNR_{c2}C(O)(CH₂)ₙ₁R_{c3}, -(CH₂)ₙNR_{c2}C(O)NR_{c2}R_{c3}, -(CH₂)ₙC(O)NR_{c2}(CH₂)ₙ₁R_{c3}, -OC(R_{c1}R_{c2})ₙ(CH₂)ₙ₁R_{c3} and -(CH₂)ₙNR_{c2}S(O)ₘR_{c3}, the amino, C1_3 alkyl, C₁₋₃ deuterated alkyl, C₁₋₃ haloalkyl, C₁₋₃ hydroxyalkyl, C₁₋₃ alkoxy, C₂₋₃ alkenyl, C₂₋₃ alkynyl, C₃₋₆ cycloalkyl, 3 to 6 membered heterocyclyl, C₆₋₁₀ aryl and 5 to 10 membered heteroaryl can be each optionally further substituted by one or more substituents selected from the group consisting of hydroxy, halogen, amino, C₁₋₃ alkyl and 3 to 6 membered heterocyclyl.

In some embodiments of the present invention, R_{c1}, R_{c2} and R_{c3} are each independently selected from the group consisting of hydrogen, deuterium, halogen, nitro, hydroxy, thiol, cyano, amino, C₁₋₆ alkyl, C₁₋₆ deuterated alkyl, C₁₋₆ haloalkyl, C₁₋₆ hydroxyalkyl, C₁₋₆ alkoxy, C₂₋₆ alkenyl, C₂₋₆ alkynyl, C₃₋₁₂ cycloalkyl, 3 to 12 membered heterocyclyl, C₆₋₁₂ aryl and 5 to 12 membered heteroaryl, the amino, C₁₋₆ alkyl, C₁₋₆ deuterated alkyl, C₁₋₆ haloalkyl, C₁₋₆ hydroxyalkyl, C₁₋₆ alkoxy, C₂₋₆ alkenyl, C₂₋₆ alkynyl, C₃₋₁₂ cycloalkyl, 3 to 12 membered heterocyclyl, C₆₋₁₂ aryl and 5 to 12 membered heteroaryl can be each optionally further substituted by one or more substituents selected from the group consisting of hydroxy, halogen, amino, C₁₋₆ alkyl and 3 to 12 membered heterocyclyl.

In some embodiments of the present invention, each R^{c} is independently selected from the group consisting of hydrogen, deuterium, halogen, nitro, hydroxy, thiol, oxo, cyano, amino optionally substituted by C₁₋₃ alkyl, C₁₋₃ alkyl, C₁₋₃ alkoxy, C₁₋₃ haloalkyl and C₃₋₆ cycloalkyl.

In some embodiments of the present invention, each R^{c} is independently selected from the group consisting of hydrogen, deuterium, halogen, nitro, hydroxy, thiol, oxo, cyano, amino, C₁₋₃ alkyl, C₁₋₃ haloalkyl and C₃₋₆ cycloalkyl.

In some embodiments of the present invention, each R^{c} is independently selected from the group consisting of hydrogen, deuterium, F, chlorine, hydroxy, methyl, methoxy, oxo and cyano.

In some embodiments of the present invention, each R^{c} is independently selected from the group consisting of hydrogen, deuterium, F, hydroxy, methyl, methoxy, oxo and cyano.

In some embodiments of the present invention, each R^{c} is independently selected from the group consisting of hydrogen, F, hydroxy, methyl, methoxy, oxo and cyano.

In some embodiments of the present invention, each R^{c} is independently selected from the group consisting of hydrogen, F, methyl, oxo and cyano.

In some embodiments of the present invention, each R^{c} is independently selected from the group consisting of hydrogen, F, methyl and oxo.

In some embodiments of the present invention, z is 1 or 2.

In some embodiments of the present invention, each R^{d} is independently selected from the group consisting of hydrogen, deuterium, halogen, nitro, hydroxy, thiol, oxo, cyano, amino, C₁₋₆ alkyl, C₁₋₆ deuterated alkyl, C₁₋₆ haloalkyl, C₁₋₆ hydroxyalkyl, C₁₋₆ alkoxy, C₂₋₆ alkenyl, C₂₋₆ alkynyl, C₃₋₁₂ cycloalkyl, 3 to 12 membered heterocyclyl, C₆₋₁₂ aryl, 5 to 12 membered heteroaryl, -(CH₂)ₙR_{d1}, -(CH₂)ₙOR_{d1}, -(CH₂)ₙC(O)R_{d1}, -(CH₂)ₙC(O)OR_{d1}, -(CH₂)ₙS(O)ₘR_{c1}, -(CH₂)ₙNR_{d2}R_{d3}, -(CH₂)ₙNR_{d2}C(O)OR_{d3}, -(CH₂)ₙNR_{d2}C(O)(CH₂)ₙ₁R_{d3}, -(CH₂)ₙNR_{d2}C(O)NR_{d2}R_{d3}, -(CH₂)ₙC(O)NR_{d2}(CH₂)ₙ₁R_{d3}, -OC(R_{d1}R_{d2})ₙ(CH₂)ₙ₁R_{d3} and -(CH₂)ₙNR_{d2}S(O)ₘR_{d3}, the amino, C₁₋₆ alkyl, C₁₋₆ deuterated alkyl, C₁₋₆ haloalkyl, C₁₋₆ hydroxyalkyl, C₁₋₆ alkoxy, C₂₋₆ alkenyl, C₂₋₆ alkynyl, C₃₋₁₂ cycloalkyl, 3 to 12 membered heterocyclyl, C₆₋₁₂ aryl and 5 to 12 membered heteroaryl can be each optionally further substituted by one or more substituents selected from the group consisting of hydroxy, nitro, thiol, oxo, cyano, halogen, C₁₋₆ deuterated alkyl, C₁₋₆ haloalkyl, C₁₋₆ hydroxyalkyl, amino, C₁₋₆ alkyl, C₁₋₆ alkoxy, C₂₋₆ alkenyl, C₂₋₆ alkynyl, C₃₋₈ cycloalkyl, C₆₋₁₀ aryl, 5 to 10 membered heteroaryl and 3 to 12 membered heterocyclyl.

In some embodiments of the present invention, each R^{d} is independently selected from the group consisting of hydrogen, deuterium, halogen, nitro, hydroxy, thiol, oxo, cyano, amino, C₁₋₆ alkyl, C₁₋₆ deuterated alkyl, C₁₋₆ haloalkyl, C₁₋₆ hydroxyalkyl, C₁₋₆ alkoxy, C₂₋₆ alkenyl, C₂₋₆ alkynyl, C₃₋₁₂ cycloalkyl, 3 to 12 membered heterocyclyl, C₆₋₁₂ aryl, 5 to 12 membered heteroaryl, -(CH₂)ₙR_{d1}, -(CH₂)ₙOR_{d1}, -(CH₂)ₙC(O)R_{d1}, -(CH₂)ₙC(O)OR_{d1}, -(CH₂)ₙS(O)ₘR_{c1}, -(CH₂)ₙNR_{d2}R_{d3}, -(CH₂)ₙNR_{d2}C(O)OR_{d3}, -(CH₂)ₙNR_{d2}C(O)(CH₂)ₙ₁R_{d3}, -(CH₂)ₙNR_{d2}C(O)NR_{d2}R_{d3}, -(CH₂)ₙC(O)NR_{d2}(CH₂)ₙ₁R_{d3}, -OC(R_{d1}R_{d2})ₙ(CH₂)ₙ₁R_{d3} and -(CH₂)ₙNR_{d2}S(O)ₘR_{d3}, the amino, C₁₋₆ alkyl, C₁₋₆ deuterated alkyl, C₁₋₆ haloalkyl, C₁₋₆ hydroxyalkyl, C₁₋₆ alkoxy, C₂₋₆ alkenyl, C₂₋₆ alkynyl, C₃₋₁₂ cycloalkyl, 3 to 12 membered heterocyclyl, C₆₋₁₂ aryl and 5 to 12 membered heteroaryl can be each optionally further substituted by one or more substituents selected from the group consisting of hydroxy, halogen, amino, C₁₋₆ alkyl and 3 to 12 membered heterocyclyl.

In some embodiments of the present invention, each R^{d} is independently selected from the group consisting of hydrogen, deuterium, halogen, nitro, hydroxy, thiol, oxo, cyano, amino, C₁₋₃ alkyl, C₁₋₃ deuterated alkyl, C₁₋₃ haloalkyl, C₁₋₃ hydroxyalkyl, C₁₋₃ alkoxy, C₂₋₃ alkenyl, C₂₋₃ alkynyl, C₃₋₆ cycloalkyl, 3 to 6 membered heterocyclyl, C₆₋₁₀ aryl, 5 to 10 membered heteroaryl, -(CH₂)ₙR_{d1}, -(CH₂)ₙOR_{d1}, -(CH₂)ₙC(O)R_{d1}, -(CH₂)ₙC(O)OR_{d1}, -(CH₂)ₙS(O)ₘR_{c1}, -(CH₂)ₙNR_{d2}R_{d3}, -(CH₂)ₙNR_{d2}C(O)OR_{d3}, -(CH₂)ₙNR_{d2}C(O)(CH₂)ₙ₁R_{d3}, -(CH₂)ₙNR_{d2}C(O)NR_{d2}R_{d3}, -(CH₂)ₙC(O)NR_{d2}(CH₂)ₙ₁R_{d3}, -OC(R_{d1}R_{d2})ₙ(CH₂)ₙ₁R_{d3} and -(CH₂)ₙNR_{d2}S(O)ₘR_{d3}, the amino, C₁₋₃ alkyl, C₁₋₃ deuterated alkyl, C₁₋₃ haloalkyl, C₁₋₃ hydroxyalkyl, C₁₋₃ alkoxy, C₂₋₃ alkenyl, C₂₋₃ alkynyl, C₃₋₆ cycloalkyl, 3 to 6 membered heterocyclyl, C₆₋₁₀ aryl and 5 to 10 membered heteroaryl can be each optionally further substituted by one or more substituents selected from the group consisting of hydroxy, halogen, amino, C₁₋₃ alkyl and 3 to 6 membered heterocyclyl.

In some embodiments of the present invention, R_{d1}, R_{d2} and R_{d3} are each independently selected from the group consisting of hydrogen, deuterium, halogen, nitro, hydroxy, thiol, cyano, amino, C₁₋₆ alkyl, C₁₋₆ deuterated alkyl, C₁₋₆ haloalkyl, C₁₋₆ hydroxyalkyl, C₁₋₆ alkoxy, C₂₋₆ alkenyl, C₂₋₆ alkynyl, C₃₋₁₂ cycloalkyl, 3 to 12 membered heterocyclyl, C₆₋₁₂ aryl and 5 to 12 membered heteroaryl, the amino, C₁₋₆ alkyl, C₁₋₆ deuterated alkyl, C₁₋₆ haloalkyl, C₁₋₆ hydroxyalkyl, C₁₋₆ alkoxy, C₂₋₆ alkenyl, C₂₋₆ alkynyl, C₃₋₁₂ cycloalkyl, 3 to 12 membered heterocyclyl, C₆₋₁₂ aryl and 5 to 12 membered heteroaryl can be each optionally further substituted by one or more substituents selected from the group consisting of hydroxy, halogen, amino, C₁₋₆ alkyl and 3 to 12 membered heterocyclyl.

In some embodiments of the present invention, each R^{d} is independently selected from the group consisting of hydrogen, deuterium, halogen, nitro, hydroxy, thiol, oxo, cyano, amino optionally substituted by C₁₋₃ alkyl, C₁₋₃ alkyl, C₁₋₃ alkoxy, C₁₋₃ haloalkyl and C₃₋₆ cycloalkyl.

In some embodiments of the present invention, each R^{d} is independently selected from the group consisting of hydrogen, deuterium, cyclopropyl, isopropyl, cyano, F, Cl, methyl, -CD₃, -NHCH₃, -NHCD₃, methoxy and oxo.

In some embodiments of the present invention, each R^{d} is independently selected from the group consisting of hydrogen, cyclopropyl, cyano, F, Cl, methyl, -NHCH₃, methoxy and oxo.

In some embodiments of the present invention, each R^{d} is independently selected from the group consisting of hydrogen, cyclopropyl, cyano, F, methyl, -NHCH₃, methoxy and oxo.

In some embodiments of the present invention, each R^{d} is independently selected from the group consisting of hydrogen, deuterium, halogen, nitro, hydroxy, thiol, oxo, cyano, amino, C₁₋₃ alkyl, C₁₋₃ alkoxy, C₁₋₃ haloalkyl and C₃₋₆ cycloalkyl.

In some embodiments of the present invention, each R^{d} is independently selected from the group consisting of hydrogen, cyclopropyl, cyano, F, methyl, methoxy and oxo.

In some embodiments of the present invention, w is 1 or 2.

In some embodiments of the present invention, w is 1.

In some embodiments of the present invention, the compound of formula (I) is a compound of formula (VII): 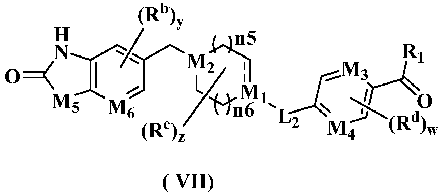 wherein,
L₂ is a bond or O;
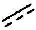 is a single bond or double bond;
M₁ is C or CR₂;
M₂ is N or CR₃;
M₃ is N or CR₄;
M₄ is N or CR₅;
M₅ is -CR₆R₇-, -CR₆R₇-CR₈R₉-, -CR₆=CR₈-, -CR₆R₇-NR₈-, -NR₈-C(=O)-, -CR₆R₇-O- or -CR₆=N-;
M₆ is N or CR₁₀;
R₁ is selected from the group consisting of C₁₋₆ alkyl, C₁₋₆ deuterated alkyl, C₁₋₆ haloalkyl, C₁₋₆ hydroxyalkyl, C₁₋₆ alkoxy, -(CH₂)ₙR₁₁, -(CH₂)ₙOR₁₁, -(CH₂)ₙC(O)R₁₁, -(CH₂)ₙC(O)OR₁₁, -(CH₂)ₙNR₂₂R₃₃ and -(CH₂)ₙNR₂₂C(O)OR₃₃;
R₁₁, R₂₂ and R₃₃ are each independently selected from the group consisting of hydrogen, deuterium, halogen, amino, hydroxy, cyano, nitro, C₁₋₆ alkyl, C₂₋₆ alkenyl, C₂₋₆ alkynyl, C₁₋₆ deuterated alkyl, C₁₋₆ haloalkyl, C₁₋₆ alkoxy, C₁₋₆ haloalkoxy, C₁₋₆ hydroxyalkyl, C₃₋₁₂ cycloalkyl, 3 to 12 membered heterocyclyl, C₆₋₁₂ aryl and 5 to 12 membered heteroaryl;
R₂, R₃, R₄ and R₅ are identical or different, and are each independently selected from the group consisting of hydrogen, deuterium, halogen, nitro, hydroxy, thiol, cyano, amino optionally substituted by C₁₋₃ alkyl, C₁₋₆ alkyl, C₁₋₆ deuterated alkyl, C₁₋₆ haloalkyl, C₁₋₆ hydroxyalkyl, C₁₋₆ alkoxy, C₂₋₆ alkenyl, C₂₋₆ alkynyl, C₃₋₁₂ cycloalkyl, 3 to 12 membered heterocyclyl, C₆₋₁₂ aryl and 5 to 12 membered heteroaryl; preferably, R₂, R₃, R₄ and R₅ are each independently hydrogen, deuterium, halogen, C₁₋₃ alkyl or C₃₋₆ cycloalkyl;
R^{b} is selected from the group consisting of hydrogen, deuterium, halogen, nitro, hydroxy, thiol, oxo, cyano, amino optionally substituted by C₁₋₃ alkyl, C₁₋₆ alkyl, C₁₋₆ haloalkyl, C₁₋₆ alkoxy and C₃₋₆ cycloalkyl;
R^{c} is selected from the group consisting of hydrogen, deuterium, halogen, nitro, hydroxy, thiol, oxo, cyano, amino optionally substituted by C₁₋₃ alkyl, C₁₋₆ alkyl, C₁₋₆ haloalkyl, C₁₋₆ alkoxy and C₃₋₆ cycloalkyl;
R^{d} is selected from the group consisting of hydrogen, deuterium, halogen, nitro, hydroxy, thiol, oxo, cyano, amino optionally substituted by C₁₋₃ alkyl, C₁₋₆ alkyl, C₁₋₆ haloalkyl, C₁₋₆ alkoxy and C₃₋₆ cycloalkyl;
R₆, R₇, R₈, R₉ and R₁₀ are identical or different, and are each independently selected from the group consisting of hydrogen, deuterium, halogen, nitro, hydroxy, thiol, cyano, amino optionally substituted by C₁₋₃ alkyl, C₁₋₆ alkyl, C₁₋₆ deuterated alkyl, C₁₋₆ haloalkyl, C₁₋₆ hydroxyalkyl, C₁₋₆ alkoxy, C₂₋₆ alkenyl, C₂₋₆ alkynyl, C₃₋₁₂ cycloalkyl, 3 to 12 membered heterocyclyl, C₆₋₁₂ aryl and 5 to 12 membered heteroaryl; preferably, R₆, R₇, R₈ and R₉ are each independently hydrogen, deuterium, halogen, cyano, C₁₋₃ alkyl or C₃₋₆ cycloalkyl;
or, R₆, R₇ together with the adjacent carbon atom are bonded to form a C₃₋₆ cycloalkyl, the C₃₋₆ cycloalkyl is optionally substituted by one or more substituents selected from the group consisting of hydrogen, deuterium, halogen, nitro, hydroxy, thiol, cyano and amino;
or, R₆, R₈ together with the adjacent carbon atom are bonded to form a C₃₋₆ cycloalkyl, the C₃₋₆ cycloalkyl is optionally substituted by one or more substituents selected from the group consisting of hydrogen, deuterium, halogen, nitro, hydroxy, thiol, cyano and amino;
n5 and n6 are each independently 0, 1 or 2;
w is 1 or 2;
y is 1 or 2;
z is 1 or 2; and
n is 0, 1, 2 or 3.

In some embodiments of the present invention, M₆ is N or C-CN.

In some embodiments of the present invention, the compound of formula (I) is a compound of formula (VI): 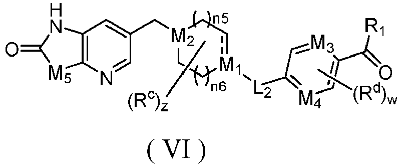 wherein,
L₂ is a bond or O;
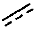 is a single bond or double bond;
M₁ is C or CR₂;
M₂ is N or CR₃;
M₃ is N or CR₄;
M₄ is N or CR₅;
M₅ is -CR₆R₇-, -CR₆R₇-CR₈R₉-, -CR₆=CR₈-, -CR₆R₇-NR₈-, -NR₈-C(=O)-, -CR₆R₇-O- or -CR₆=N-;
R₁ is selected from the group consisting of C₁₋₆ alkyl, C₁₋₆ deuterated alkyl, C₁₋₆ haloalkyl, C₁₋₆ hydroxyalkyl, C₁₋₆ alkoxy, -(CH₂)ₙR₁₁, -(CH₂)ₙOR₁₁, -(CH₂)ₙC(O)R₁₁, -(CH₂)ₙC(O)OR₁₁, -(CH₂)ₙNR₂₂R₃₃ and -(CH₂)ₙNR₂₂C(O)OR₃₃;
R₁₁, R₂₂ and R₃₃ are each independently selected from the group consisting of hydrogen, deuterium, halogen, amino, hydroxy, cyano, nitro, C₁₋₆ alkyl, C₂₋₆ alkenyl, C₂₋₆ alkynyl, C₁₋₆ deuterated alkyl, C₁₋₆ haloalkyl, C₁₋₆ alkoxy, C₁₋₆ haloalkoxy, C₁₋₆ hydroxyalkyl, C₃₋₁₂ cycloalkyl, 3 to 12 membered heterocyclyl, C₆₋₁₂ aryl and 5 to 12 membered heteroaryl;
R₂, R₃, R₄ and R₅ are identical or different, and are each independently selected from the group consisting of hydrogen, deuterium, halogen, nitro, hydroxy, thiol, cyano, amino optionally substituted by C₁₋₃ alkyl, C₁₋₆ alkyl, C₁₋₆ deuterated alkyl, C₁₋₆ haloalkyl, C₁₋₆ hydroxyalkyl, C₁₋₆ alkoxy, C₂₋₆ alkenyl, C₂₋₆ alkynyl, C₃₋₁₂ cycloalkyl, 3 to 12 membered heterocyclyl, C₆₋₁₂ aryl and 5 to 12 membered heteroaryl; preferably, R₂, R₃, R₄ and R₅ are each independently hydrogen, deuterium, halogen, C₁₋₃ alkyl or C₃₋₆ cycloalkyl;
R^{c} is selected from the group consisting of hydrogen, deuterium, halogen, nitro, hydroxy, thiol, oxo, cyano, amino optionally substituted by C₁₋₃ alkyl, C₁₋₆ alkyl, C₁₋₆ haloalkyl, C₁₋₆ alkoxy and C₃₋₆ cycloalkyl;
R^{d} is selected from the group consisting of hydrogen, deuterium, halogen, nitro, hydroxy, thiol, oxo, cyano, amino optionally substituted by C₁₋₃ alkyl, C₁₋₆ alkyl, C₁₋₆ haloalkyl, C₁₋₆ alkoxy and C₃₋₆ cycloalkyl;
R₆, R₇, R₈ and R₉ are identical or different, and are each independently selected from the group consisting of hydrogen, deuterium, halogen, nitro, hydroxy, thiol, cyano, amino optionally substituted by C₁₋₃ alkyl, C₁₋₆ alkyl, C₁₋₆ deuterated alkyl, C₁₋₆ haloalkyl, C₁₋₆ hydroxyalkyl, C₁₋₆ alkoxy, C₂₋₆ alkenyl, C₂₋₆ alkynyl, C₃₋₁₂ cycloalkyl, 3 to 12 membered heterocyclyl, C₆₋₁₂ aryl and 5 to 12 membered heteroaryl; preferably, R₆, R₇, R₈ and R₉ are each independently hydrogen, deuterium, halogen, C₁₋₃ alkyl or C₃₋₆ cycloalkyl;
or, R₆, R₇ together with the adjacent carbon atom are bonded to form a C₃₋₆ cycloalkyl, the C₃₋₆ cycloalkyl is optionally substituted by one or more substituents selected from the group consisting of hydrogen, deuterium, halogen, nitro, hydroxy, thiol, cyano and amino;
or, R₆, R₈ together with the adjacent carbon atom are bonded to form a C₃₋₆ cycloalkyl, the C₃₋₆ cycloalkyl is optionally substituted by one or more substituents selected from the group consisting of hydrogen, deuterium, halogen, nitro, hydroxy, thiol, cyano and amino;
n5 and n6 are each independently 0, 1 or 2;
w is 1 or 2;
z is 1 or 2; and
n is 0, 1, 2 or 3.

In some embodiments of the present invention, M₃ is N, CH or CF.

In some embodiments of the present invention, M₁ is CH or C.

In some embodiments of the present invention, M₁ is CH or CD.

In some embodiments of the present invention, R₆, R₇, R₈ and R₉ are each independently hydrogen, methyl, ethyl, methoxy, ethynyl, propynyl or cyclopropyl; or, R₆, R₇ together with the adjacent carbon atom are bonded to form a cyclopropyl; or, R₆, R₈ together with the adjacent carbon atom are bonded to form a cyclopropyl.

In some embodiments of the present invention, R₆, R₇, R₈ and R₉ are each independently hydrogen, methyl, ethyl, methoxy, ethynyl or cyclopropyl; or, R₆, R₇ together with the adjacent carbon atom are bonded to form a cyclopropyl; or, R₆, R₈ together with the adjacent carbon atom are bonded to form a cyclopropyl.

In some embodiments of the present invention, R₁ is -NHCH₃ or -NH-cyclopropyl.

In some embodiments of the present invention, R^{d} is hydrogen, fluorine, chlorine or cyclopropyl.

In some embodiments of the present invention, R^{d} is hydrogen or cyclopropyl.

In some embodiments of the present invention, the compound of formula (I) is a compound of formula (V): 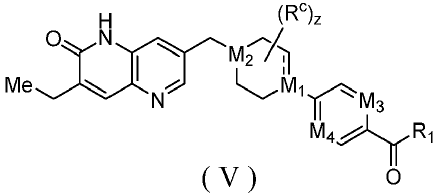 wherein, 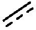 is a single bond or double bond;
M₁ is C or CR₂;
M₂ is N or CR₃;
M₃ is N or CR₄;
M₄ is N or CR₅;
R₁ is selected from the group consisting of C₁₋₆ alkyl, C₁₋₆ deuterated alkyl, C₁₋₆ haloalkyl, C₁₋₆ hydroxyalkyl, C₁₋₆ alkoxy, -(CH₂)ₙR₁₁, -(CH₂)ₙOR₁₁, -(CH₂)ₙC(O)R₁₁, -(CH₂)ₙC(O)OR₁₁, -(CH₂)ₙNR₂₂R₃₃ and -(CH₂)ₙNR₂₂C(O)OR₃₃;
or, R₁ is selected from the group consisting of hydrogen, deuterium, halogen, nitro, hydroxy, thiol, cyano, amino, C₁₋₆ alkyl, C₁₋₆ deuterated alkyl, C₁₋₆ haloalkyl, C₁₋₆ hydroxyalkyl, C₁₋₆ alkoxy, C₂₋₆ alkenyl, C₂₋₆ alkynyl, C₃₋₁₂ cycloalkyl, 3 to 12 membered heterocyclyl, C₆₋₁₂ aryl and 5 to 12 membered heteroaryl; preferably, R₁ is selected from the group consisting of hydrogen, deuterium, halogen, nitro, hydroxy, thiol, cyano, amino, C₁₋₃ alkyl, C₁₋₃ deuterated alkyl, C₁₋₃ haloalkyl, C₁₋₃ hydroxyalkyl, C₁₋₃ alkoxy, C₂₋₆ alkenyl, C₂₋₆ alkynyl, C₃₋₁₂ cycloalkyl, 3 to 10 membered heterocyclyl; more preferably, R₁ is selected from the group consisting of hydrogen, deuterium, cyano, methyl, ethyl, propyl, isopropyl, hydroxymethyl, hydroxyethyl, hydroxypropyl, methoxy, ethoxy, propoxy, cyclopropyl, cyclobutyl, cyclopentyl, cyclohexyl and 3 to 8 membered heterocyclyl containing 1 to 3 oxygen, nitrogen or sulfur atoms, the cyclopropyl, cyclobutyl, cyclopentyl, cyclohexyl and 3 to 8 membered heterocyclyl containing 1 to 3 oxygen, nitrogen or sulfur atoms can be each optionally further substituted;
R₁₁, R₂₂ and R₃₃ are each independently selected from the group consisting of hydrogen, deuterium, halogen, amino, hydroxy, cyano, nitro, C₁₋₆ alkyl, C₂₋₆ alkenyl, C₂₋₆ alkynyl, C₁₋₆ deuterated alkyl, C₁₋₆ haloalkyl, C₁₋₆ alkoxy, C₁₋₆ haloalkoxy, C₁₋₆ hydroxyalkyl, C₃₋₁₂ cycloalkyl, 3 to 12 membered heterocyclyl, C₆₋₁₂ aryl and 5 to 12 membered heteroaryl;
R₂, R₃, R₄ and R₅ are identical or different, and are each independently selected from the group consisting of hydrogen, deuterium, halogen, nitro, hydroxy, thiol, cyano, amino, C₁₋₆ alkyl, C₁₋₆ deuterated alkyl, C₁₋₆ haloalkyl, C₁₋₆ hydroxyalkyl, C₁₋₆ alkoxy, C₂₋₆ alkenyl, C₂₋₆ alkynyl, C₃₋₁₂ cycloalkyl, 3 to 12 membered heterocyclyl, C₆₋₁₂ aryl and 5 to 12 membered heteroaryl; preferably, R₂, R₃, R₄ and R₅ are each independently hydrogen, deuterium, halogen, C₁₋₃ alkyl or C₃₋₆ cycloalkyl;
R^{c} is selected from the group consisting of hydrogen, deuterium, halogen, nitro, hydroxy, thiol, oxo, cyano, amino, C₁₋₆ alkyl, C₁₋₆ haloalkyl and C₃₋₆ cycloalkyl;
z is 1 or 2; and
n is 0, 1, 2 or 3.

In some embodiments of the present invention, M₁ is C or CH.

In some embodiments of the present invention, M₂ is CH.

In some embodiments of the present invention, M₄ is CH.

In some embodiments of the present invention, M₃ is N or CF.

In some embodiments of the present invention, R₁ is selected from the group consisting of C₁₋₃ alkyl, C₁₋₃ deuterated alkyl, C₁₋₃ haloalkyl, C₁₋₃ hydroxyalkyl, C₁₋₃ alkoxy, -(CH₂)ₙR₁₁, -(CH₂)ₙOR₁₁, -(CH₂)ₙC(O)R₁₁, (CH₂)ₙC(O)OR₁₁, -(CH₂)ₙNR₂₂R₃₃ and -(CH₂)ₙNR₂₂C(O)OR₃₃.

In some embodiments of the present invention, R₁₁, R₂₂ and R₃₃ are each independently selected from the group consisting of hydrogen, deuterium, halogen, amino, hydroxy, cyano, nitro, C₁₋₃ alkyl, C₂₋₃ alkenyl, C₂₋₃ alkynyl, C₁₋₃ deuterated alkyl, C₁₋₃ haloalkyl, C₁₋₃ alkoxy, C₁₋₃ haloalkoxy, C₁₋₃ hydroxyalkyl, C₃₋₆ cycloalkyl, 3 to 6 membered heterocyclyl, C₆₋₁₀ aryl and 5 to 10 membered heteroaryl.

In some embodiments of the present invention, R₁ is -NHCH₃,

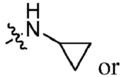

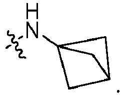

In some embodiments of the present invention, provided is the compound of formula (V), the stereoisomer thereof or the pharmaceutically acceptable salt thereof, wherein:
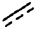 is a double bond;
M₁ is C;
M₂ is N or CH;
M₃ is N or CH;
M₄ is N or CH;
R₁ is selected from the group consisting of C₁₋₃ alkyl, C₁₋₃ deuterated alkyl, C₁₋₃ haloalkyl, C₁₋₃ hydroxyalkyl, C₁₋₃ alkoxy, -R₁₁, -OR₁₁, -C(O)R₁₁, -C(O)OR₁₁, -NR₂₂R₃₃ and -(CH₂)ₙNR₂₂C(O)OR₃₃;
R₁₁, R₂₂ and R₃₃ are each independently selected from the group consisting of hydrogen, deuterium, halogen, amino, hydroxy, cyano, nitro, C₁₋₃ alkyl, C₂₋₄ alkenyl, C₂₋₄ alkynyl, C₁₋₃ deuterated alkyl, C₁₋₃ haloalkyl, C₁₋₃ alkoxy, C₁₋₃ haloalkoxy, C₁₋₃ hydroxyalkyl, C₃₋₆ cycloalkyl, 4 to 6 membered heterocyclyl, phenyl and 5 to 6 membered heteroaryl;
R^{c} is selected from the group consisting of hydrogen, deuterium, halogen, nitro, hydroxy, thiol, oxo, cyano, amino, C₁₋₃ alkyl, C₁₋₃ haloalkyl and C₃₋₃ cycloalkyl;
z is 1 or 2.

In some embodiments of the present invention, the compound is further as shown in formula (IX): 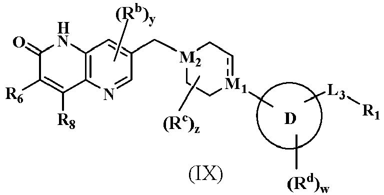 wherein, ring D is a 9 to 10 membered heterocyclyl, C₆₋₁₀ aryl or 9 to 10 membered heteroaryl; preferably a 6 membered heteroaromatic ring fused with 6 membered heteroaromatic ring, 6 membered heteroaromatic ring fused with 6 membered heterocyclic ring, or 6 membered heteroaromatic ring fused with 5 membered heterocyclic ring; more preferably 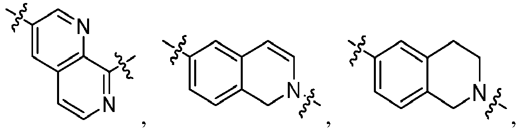 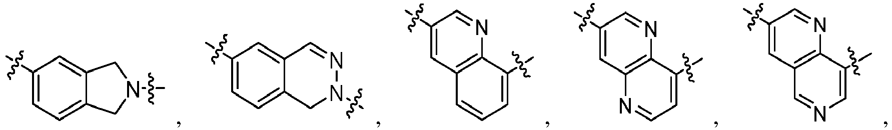 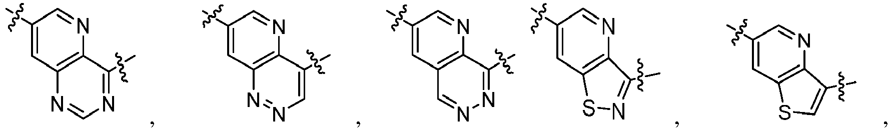 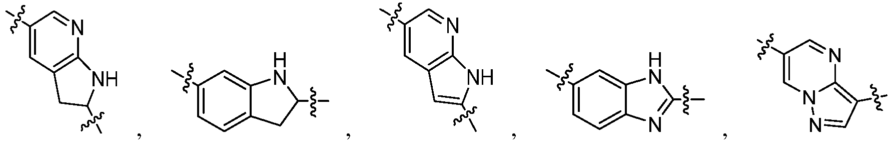 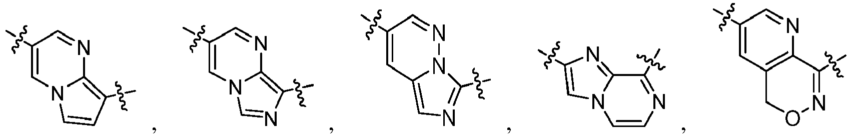 or

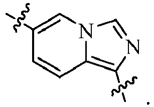

In some embodiments of the present invention, the compound of formula (I) is a compound of formula (VIII): 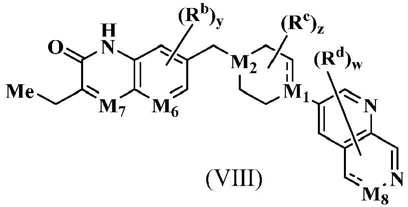 wherein,
= represents a single bond or double bond;
M₁ is N, C or CR₂;
M₂ is N or CR₃;
M₆ is N or CR₁₀;
M₇ is CR₁₂ or N;
M₈ is CR₁₃ or O; preferably, -̅ -̅M₈ is -̅ -̅CR₁₃ or -O.
R₂, R₃, R₁₀, R₁₂ and R₁₃ are identical or different, and are each independently selected from the group consisting of hydrogen, deuterium, halogen, nitro, hydroxy, thiol, cyano, amino optionally substituted by one or more C₁₋₃ alkyl, C₁₋₆ alkyl, C₁₋₆ deuterated alkyl, C₁₋₆ haloalkyl, C₁₋₆ hydroxyalkyl, C₁₋₆ alkoxy, C₂₋₆ alkenyl, C₂₋₆ alkynyl, C₃₋₁₂ cycloalkyl, 3 to 12 membered heterocyclyl, C₆₋₁₂ aryl and 5 to 12 membered heteroaryl; preferably, R₂, R₃, R₁₀, R₁₂ and R₁₃ are identical or different, and are each independently selected from the group consisting of hydrogen, deuterium, halogen, nitro, hydroxy, thiol, cyano, amino optionally substituted by one or more C₁₋₃ alkyl, C₁₋₃ alkyl, C₁₋₃ deuterated alkyl, C₁₋₃ haloalkyl, C₁₋₃ hydroxyalkyl and C₁₋₃ alkoxy;
R^{b} is selected from the group consisting of hydrogen, deuterium, halogen, nitro, hydroxy, thiol, oxo, cyano, amino optionally substituted by one or more C₁₋₃ alkyl, C₁₋₆ alkyl, C₁₋₆ haloalkyl, C₁₋₆ alkoxy and C₃₋₆ cycloalkyl; preferably hydrogen, deuterium, halogen, nitro, hydroxy, thiol, oxo, cyano, amino optionally substituted by one or more C₁₋₃ alkyl, C₁₋₃ alkyl, C₁₋₃ haloalkyl and C₁₋₃ alkoxy;
R^{c} is selected from the group consisting of hydrogen, deuterium, halogen, nitro, hydroxy, thiol, oxo, cyano, amino optionally substituted by one or more C₁₋₃ alkyl, C₁₋₆ alkyl, C₁₋₆ haloalkyl, C₁₋₆ alkoxy and C₃₋₆ cycloalkyl; preferably hydrogen, deuterium, halogen, nitro, hydroxy, thiol, oxo, cyano, amino optionally substituted by one or more C₁₋₃ alkyl, C₁₋₃ alkyl, C₁₋₃ haloalkyl and C₁₋₃ alkoxy;
R^{d} is selected from the group consisting of hydrogen, deuterium, halogen, nitro, hydroxy, thiol, oxo, cyano, amino optionally substituted by one or more C₁₋₃ alkyl, C₁₋₆ alkyl, C₁₋₆ haloalkyl, C₁₋₆ alkoxy and C₃₋₆ cycloalkyl; preferably hydrogen, deuterium, halogen, nitro, hydroxy, thiol, oxo, cyano, amino optionally substituted by one or more C₁₋₃ alkyl, C₁₋₃ alkyl, C₁₋₃ haloalkyl and C₁₋₃ alkoxy;
w, y and z are each independently 1, 2, 3 or 4.

In formula (VIII), -̅ -̅M₁ is preferably =C or -N.

In formula (VIII), M₂ is preferably CH.

In formula (VIII), M₆ is preferably N or CH.

In formula (VIII), M₇ is preferably CH or N.

In formula (VIII), -̅ -̅M₈ is preferably -̅ -̅CH or -O.

In formula (VIII), R^{b} is preferably hydrogen, deuterium or F.

In formula (VIII), R^{c} is preferably hydrogen.

In formula (VIII), R^{d} is preferably hydrogen, deuterium, F, Cl, methyl, -CD₃, -NHCH₃.

In formula (VIII), w is preferably 1 or 2.

In formula (VIII), y is preferably 1.

The present invention also provides a method for preparing the compound, the stereoisomer thereof or the pharmaceutically acceptable salt thereof, wherein: the compound is as shown in formula (III), a compound of formula (III-1) and a compound of formula (III-2) are subjected to the following reaction, 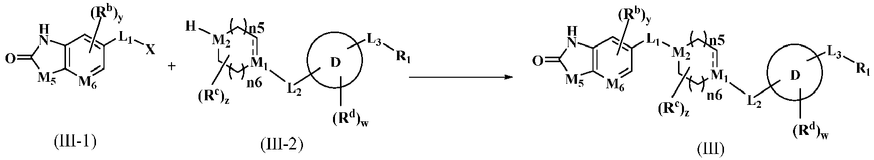
wherein, X is halogen, preferably fluorine, chlorine, bromine or iodine;
preferably, the reaction is carried out in the presence of a base and a catalyst; the base is preferably DIPEA; the catalyst is preferably potassium iodide;
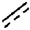 , M_{1,} M₂, M₅, M₆, R^{b}, R^{c}, R^{d}, L₁, L₂, L₃, R₁, ring D, n5, n6, y, z and w are as defined above.

The present invention also provides a compound of formula (III-2), 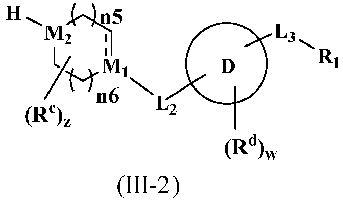 wherein, 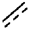 , M₁, M₂, R^{c}, R^{d}, L₂, L₃, R₁, ring D, n5, n6, z and w are as defined above.

The present invention further relates to a pharmaceutical composition comprising a therapeutically effective dose of any one of the compound of formula (I), the stereoisomer thereof or the pharmaceutically acceptable salt thereof, and one or more pharmaceutically acceptable carriers, diluents or excipients.

The present invention further relates to a use of any one of the compound of formula (I), the stereoisomer thereof or the pharmaceutically acceptable salt thereof, or the pharmaceutical composition comprising the same in the preparation of a PARP inhibitor medicament, wherein the PARP is preferably PARP1.

The present invention further relates to a use of the compound of formula (I), the stereoisomer thereof or the pharmaceutically acceptable salt thereof, or the pharmaceutical composition comprising the same in the preparation of a medicament for treating cancer, ischemic disease, or neurodegenerative disease, wherein the cancer is preferably selected from the group consisting of breast cancer, ovarian cancer, pancreatic cancer, prostate cancer, blood cancer, gastric cancer, colorectal cancer, gastrointestinal cancer and lung cancer.

The present invention further relates to a method for treating cancer, ischemic disease, or neurodegenerative disease by the compound of formula (I), the stereoisomer thereof or the pharmaceutically acceptable salt thereof, or the pharmaceutical composition comprising the same.

The present invention also relates to a method for preventing and/or treating ischemic disease, neurodegenerative disease, breast cancer, ovarian cancer, pancreatic cancer, prostate cancer, blood cancer, gastrointestinal cancer or lung cancer, comprising a step of administering a therapeutically effective dose of the compound of formula (I), the stereoisomer thereof or the pharmaceutically acceptable salt thereof, or the pharmaceutical composition comprising the same to a patient. Preferably, the gastrointestinal cancer is selected from the group consisting of gastric cancer and colorectal cancer.

The present invention also provides a method for treating disease condition by using the compound or the pharmaceutical composition of the present invention, wherein the disease condition includes, but is not limited to, condition associated with PARP kinase dysfunction. The PARP is preferably PARP1 or PARP2.

The present invention also relates to a method for treating cancer in a mammal, comprising a step of administering a therapeutically effective amount of the compound or the pharmaceutically acceptable salt, ester, prodrug, solvate, hydrate or derivative thereof of the present invention to the mammal.

In some embodiments of the present invention, the method relates to the treatment of conditions such as cancer, ischemic disease or neurodegenerative disease.

In some embodiments of the present invention, the method relates to the treatment of breast cancer, ovarian cancer, pancreatic cancer, prostate cancer, blood cancer, gastrointestinal cancer or lung cancer; preferably, the gastrointestinal cancer is selected from the group consisting of gastric cancer and colorectal cancer.

In some embodiments of the present invention, the method relates to the treatment of ovarian cancer or breast cancer.

In some embodiments of the present invention, the cancer is breast cancer, ovarian cancer, pancreatic cancer, prostate cancer.

### DEFINITIONS

Unless otherwise stated, the terms used in the specification and claims have the meanings described below.

The term "alkyl" refers to a saturated aliphatic hydrocarbon group, which is a straight or branched chain group comprising 1 to 20 carbon atoms, preferably an alkyl having 1 to 8 carbon atoms, more preferably an alkyl having 1 to 6 carbon atoms, and most preferably an alkyl having 1 to 3 carbon atoms. Non-limiting examples include methyl, ethyl, *n*-propyl, isopropyl, *n*-butyl, isobutyl, *tert-*butyl*, sec*-butyl, *n*-pentyl, 1,1-dimethylpropyl, 1,2-dimethylpropyl, 2,2-dimethylpropyl, 1-ethylpropyl, 2-methylbutyl, 3-methylbutyl, *n*-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, n-heptyl, 2-methylhexyl, 3-methylhexyl, 4-methylhexyl, 5-methylhexyl, 2,3-dimethylpentyl, 2,4-dimethylpentyl, 2,2-dimethylpentyl, 3,3-dimethylpentyl, 2-ethylpentyl, 3-ethylpentyl, *n*-octyl, 2,3-dimethylhexyl, 2,4-dimethylhexyl, 2,5-dimethylhexyl, 2,2-dimethylhexyl, 3,3-dimethylhexyl, 4,4-dimethylhexyl, 2-ethylhexyl, 3-ethylhexyl, 4-ethylhexyl, 2-methyl-2-ethylpentyl, 2-methyl-3-ethylpentyl, *n*-nonyl, 2-methyl-2-ethylhexyl, 2-methyl-3-ethylhexyl, 2,2-diethylpentyl, *n*-decyl, 3,3-diethylhexyl, 2,2-diethylhexyl, and various branched isomers thereof. More preferably, the alkyl is a lower alkyl having 1 to 6 carbon atoms, and non-limiting examples include methyl, ethyl, *n*-propyl, isopropyl, *n*-butyl, isobutyl, *tert-*butyl*, sec*-butyl, *n*-pentyl, 1,1-dimethylpropyl, 1,2-dimethylpropyl, 2,2-dimethylpropyl, 1-ethylpropyl, 2-methylbutyl, 3-methylbutyl, *n*-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl and the like. The alkyl can be substituted or unsubstituted. When substituted, the substituent(s) can be substituted at any available connection point. The substituent(s) is preferably one or more group(s) independently selected from the group consisting of alkyl, alkenyl, alkynyl, alkoxy, alkylthio, alkylamino, halogen, thiol, hydroxy, nitro, cyano, cycloalkyl, heterocyclyl, aryl, heteroaryl, cycloalkoxy, heterocycloalkoxy, cycloalkylthio, heterocyclylthio, oxo, carboxy and alkoxycarbonyl. The alkyl of the present invention is preferably selected from the group consisting of methyl, ethyl, isopropyl, *tert*-butyl, haloalkyl, deuterated alkyl, alkoxy-substituted alkyl and hydroxy-substituted alkyl.

The term "alkylene" refers to an alkyl of which a hydrogen atom is further substituted, for example, "methylene" refers to -CH₂-, "ethylene" refers to -(CH₂)₂-, "propylene" refers to -(CH₂)₃-, "butylene" refers to -(CH₂)₄- and the like. The term "alkenyl" refers to an alkyl as defined above that consists of at least two carbon atoms and at least one carbon-carbon double bond, for example, ethenyl, 1-propenyl, 2-propenyl, 1-, 2- or 3-butenyl and the like. The alkenyl can be substituted or unsubstituted. When substituted, the substituent(s) is preferably one or more groups independently selected from the group consisting of alkyl, alkenyl, alkynyl, alkoxy, alkylthio, alkylamino, halogen, thiol, hydroxy, nitro, cyano, cycloalkyl, heterocyclyl, aryl, heteroaryl, cycloalkoxy, heterocycloalkoxy, cycloalkylthio and heterocyclylthio.

The term "cycloalkyl" refers to a saturated or partially unsaturated monocyclic or polycyclic hydrocarbon substituent group having 3 to 20 carbon atoms, preferably 3 to 12 carbon atoms, and more preferably 3 to 6 carbon atoms. Non-limiting examples of monocyclic cycloalkyl include cyclopropyl, cyclobutyl, cyclopentyl, cyclopentenyl, cyclohexyl, cyclohexenyl, cyclohexadienyl, cycloheptyl, cycloheptatrienyl, cyclooctyl and the like. Polycyclic cycloalkyl includes a cycloalkyl having a spiro ring, fused ring or bridged ring. The cycloalkyl is preferably cyclopropyl, cyclobutyl, cyclohexyl, cyclopentyl and cycloheptyl.

The term "spiro cycloalkyl" refers to a 5 to 20 membered polycyclic group with individual rings connected through one shared carbon atom (called a spiro atom), wherein the rings can contain one or more double bonds, but none of the rings has a completely conjugated π-electron system. The spiro cycloalkyl is preferably a 6 to 14 membered spiro cycloalkyl, and more preferably a 7 to 10 membered spiro cycloalkyl. According to the number of the spiro atoms shared between the rings, the spiro cycloalkyl can be divided into a mono-spiro cycloalkyl, a di-spiro cycloalkyl, or a poly-spiro cycloalkyl, and the spiro cycloalkyl is preferably a mono-spiro cycloalkyl or di-spiro cycloalkyl, and more preferably a 4-membered/4-membered, 4-membered/5-membered, 4-membered/6-membered, 5-membered/5-membered, or 5-membered/6-membered mono-spiro cycloalkyl. Non-limiting examples of spiro cycloalkyl include: 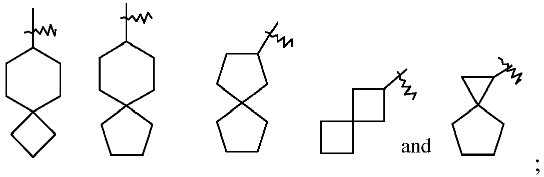 and also include spiro cycloalkyl in which a cycloalkyl and a heterocyclyl are connected through one spiro atom, non-limiting examples thereof include:

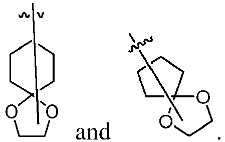

The term "fused cycloalkyl" refers to a 5 to 20 membered all-carbon polycyclic group, wherein each ring in the system shares an adjacent pair of carbon atoms with another ring, one or more rings can contain one or more double bonds, but none of the rings has a completely conjugated π-electron system. The fused cycloalkyl is preferably a 6 to 14 membered fused cycloalkyl, and more preferably a 7 to 10 membered fused cycloalkyl. According to the number of membered rings, the fused cycloalkyl can be divided into a bicyclic, tricyclic, tetracyclic or polycyclic fused cycloalkyl, and the fused cycloalkyl is preferably a bicyclic or tricyclic fused cycloalkyl, and more preferably a 5-membered/5-membered or 5-membered/6-membered bicyclic fused cycloalkyl. Non-limiting examples of fused cycloalkyl include:

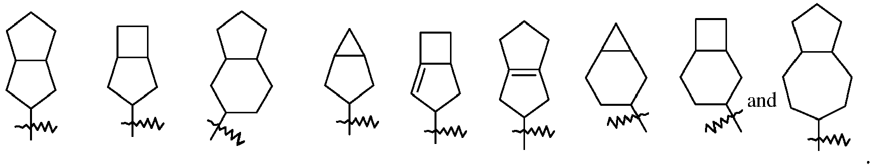

The term "bridged cycloalkyl" refers to a 5 to 20 membered all-carbon polycyclic group, wherein every two rings in the system share two disconnected carbon atoms, the rings can have one or more double bonds, but none of the rings has a completely conjugated π-electron system. The bridged cycloalkyl is preferably a 6 to 14 membered bridged cycloalkyl, and more preferably a 7 to 10 membered bridged cycloalkyl. According to the number of membered rings, the bridged cycloalkyl can be divided into a bicyclic, tricyclic, tetracyclic or polycyclic bridged cycloalkyl, and the bridged cycloalkyl is preferably a bicyclic, tricyclic or tetracyclic bridged cycloalkyl, and more preferably a bicyclic or tricyclic bridged cycloalkyl. Non-limiting examples of bridged cycloalkyl include:

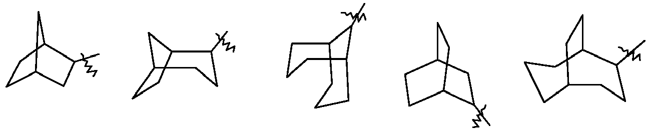

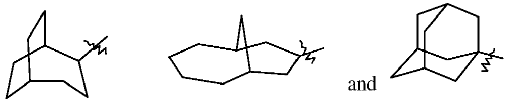

The cycloalkyl ring can be fused to the ring of aryl, heteroaryl or heterocyclyl, wherein the ring bound to the parent structure is cycloalkyl. Non-limiting examples include indanyl, tetrahydronaphthyl, benzocycloheptyl and the like. The cycloalkyl can be optionally substituted or unsubstituted. When substituted, the substituent(s) is preferably one or more group(s) independently selected from the group consisting of alkyl, alkenyl, alkynyl, alkoxy, alkylthio, alkylamino, halogen, thiol, hydroxy, nitro, cyano, cycloalkyl, heterocyclyl, aryl, heteroaryl, cycloalkoxy, heterocycloalkoxy, cycloalkylthio, heterocyclylthio, oxo, carboxy and alkoxycarbonyl.

The term "heterocyclyl" refers to a 3 to 20 membered saturated or partially unsaturated monocyclic or polycyclic hydrocarbon group, wherein one or more ring atoms are heteroatoms selected from the group consisting of nitrogen, oxygen and S(O)ₘ (wherein m is an integer of 0 to 2), but excluding -O-O-, -O-S- or -S-S- in the ring, with the remaining ring atoms being carbon atoms; wherein the ring atom can be further boron or P(O)ₚ (wherein p is an integer of 0 to 2). Preferably, the heterocyclyl has 3 to 12 ring atoms wherein 1 to 4 atoms are heteroatoms; more preferably, 3 to 8 ring atoms; and most preferably 3 to 8 ring atoms. Non-limiting examples of monocyclic heterocyclyl include pyrrolidinyl, imidazolidinyl, tetrahydrofuranyl, tetrahydrothienyl, dihydroimidazolyl, dihydrofuranyl, dihydropyrazolyl, dihydropyrrolyl, piperidinyl, piperazinyl, morpholinyl, thiomorpholinyl, homopiperazinyl, pyranyl and the like, preferably tetrahydrofuryl, pyrazolidinyl, morpholinyl, piperazinyl and pyranyl. Polycyclic heterocyclyl includes a heterocyclyl having a spiro ring, fused ring or bridged ring. The heterocyclyl having a spiro ring, fused ring or bridged ring is optionally bonded to other group via a single bond, or further bonded to other cycloalkyl, heterocyclyl, aryl and heteroaryl via any two or more atoms on the ring.

Non-limiting examples of heterocyclyl include

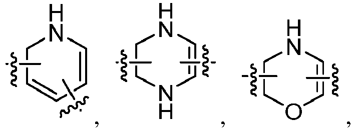

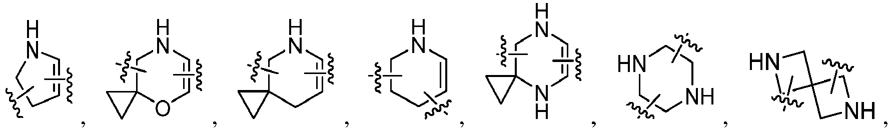

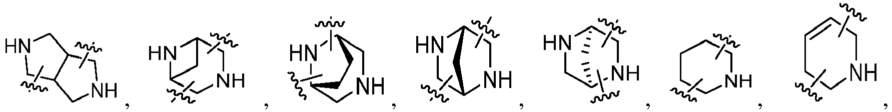

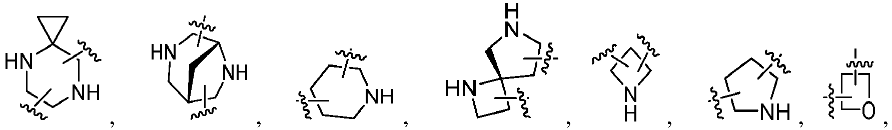

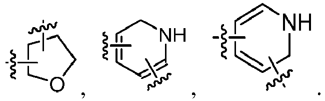

The term "spiro heterocyclyl" refers to a 5 to 20 membered polycyclic heterocyclyl group with individual rings connected through one shared atom (called a spiro atom), wherein one or more ring atoms are heteroatoms selected from the group consisting of nitrogen, oxygen and S(O)ₘ (wherein m is an integer of 0 to 2), with the remaining ring atoms being carbon atoms, and the rings can contain one or more double bonds, but none of the rings has a completely conjugated π-electron system. The spiro heterocyclyl is preferably a 6 to 14 membered spiro heterocyclyl, and more preferably a 7 to 10 membered spiro heterocyclyl. According to the number of the spiro atoms shared between the rings, the spiro heterocyclyl can be divided into a mono-spiro heterocyclyl, di-spiro heterocyclyl, or poly-spiro heterocyclyl, and the spiro heterocyclyl is preferably a mono-spiro heterocyclyl or di-spiro heterocyclyl, and more preferably a 4-membered/4-membered, 4-membered/5-membered, 4-membered/6-membered, 5-membered/5-membered, or 5-membered/6-membered mono-spiro heterocyclyl. Non-limiting examples of spiro heterocyclyl include:

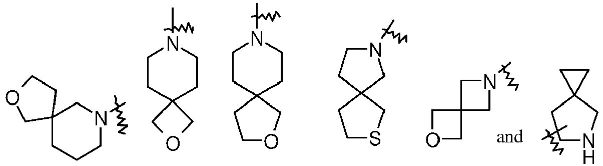

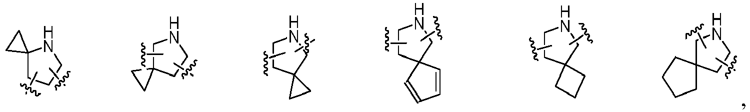

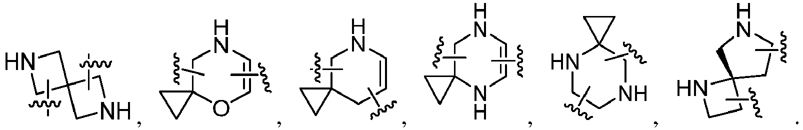

The term "fused heterocyclyl" refers to a 5 to 20 membered polycyclic heterocyclyl group, wherein each ring in the system shares an adjacent pair of atoms with another ring, one or more rings can contain one or more double bonds, but none of the rings has a completely conjugated π-electron system, and one or more ring atoms are heteroatoms selected from the group consisting of nitrogen, oxygen and S(O)ₘ (wherein m is an integer of 0 to 2), with the remaining ring atoms being carbon atoms. The fused heterocyclyl is preferably a 6 to 14 membered fused heterocyclyl, and more preferably a 7 to 10 membered fused heterocyclyl. According to the number of membered rings, the fused heterocyclyl can be divided into a bicyclic, tricyclic, tetracyclic or polycyclic fused heterocyclyl, and preferably a bicyclic or tricyclic fused heterocyclyl, and more preferably a 5-membered/5-membered or 5-membered/6-membered bicyclic fused heterocyclyl. Non-limiting examples of fused heterocyclyl include:

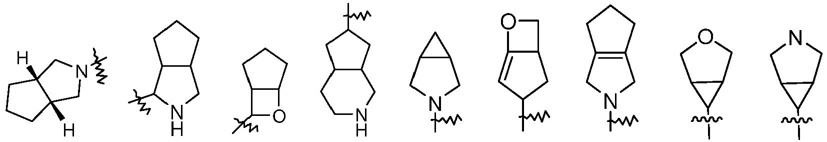

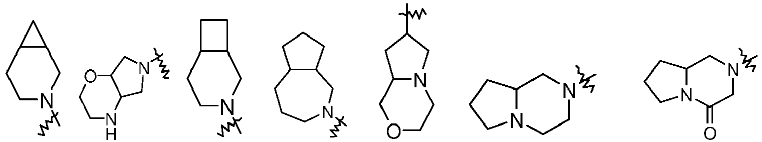

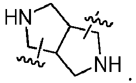

The term "bridged heterocyclyl" refers to a 5 to 14 membered polycyclic heterocyclyl group, wherein every two rings in the system share two disconnected atoms, wherein the rings can have one or more double bond(s), but none of the rings has a completely conjugated π-electron system, and one or more ring atoms are heteroatoms selected from the group consisting of nitrogen, oxygen and S(O)ₘ (wherein m is an integer of 0 to 2), with the remaining ring atoms being carbon atoms. The bridged heterocyclyl is preferably a 6 to 14 membered bridged heterocyclyl, and more preferably a 7 to 10 membered bridged heterocyclyl. According to the number of membered rings, the bridged heterocyclyl can be divided into a bicyclic, tricyclic, tetracyclic or polycyclic bridged heterocyclyl, and the bridged heterocyclyl is preferably a bicyclic, tricyclic or tetracyclic bridged heterocyclyl, and more preferably a bicyclic or tricyclic bridged heterocyclyl. Non-limiting examples of bridged heterocyclyl include:

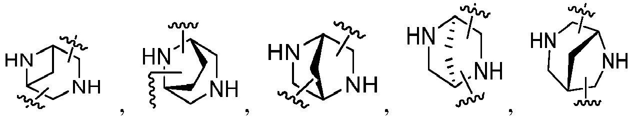

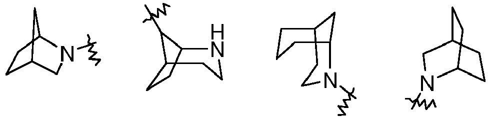

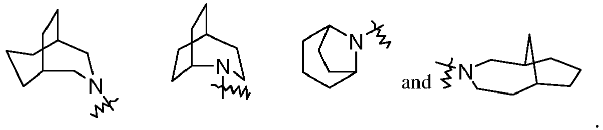

The heterocyclyl ring can be fused to the ring of aryl, heteroaryl or cycloalkyl, wherein the ring bound to the parent structure is heterocyclyl. Non-limiting examples include: 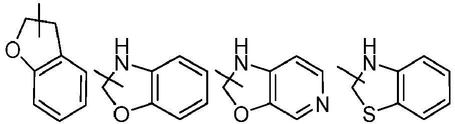 and the like.

The heterocyclyl can be optionally substituted or unsubstituted. When substituted, the substituent(s) is preferably one or more group(s) independently selected from the group consisting of alkyl, alkenyl, alkynyl, alkoxy, alkylthio, alkylamino, halogen, thiol, hydroxy, nitro, cyano, cycloalkyl, heterocyclyl, aryl, heteroaryl, cycloalkoxy, heterocycloalkoxy, cycloalkylthio, heterocyclylthio, oxo, carboxy and alkoxycarbonyl.

The term "aryl" refers to a 6 to 14 membered all-carbon monocyclic ring or polycyclic fused ring (*i.e.* each ring in the system shares an adjacent pair of carbon atoms with another ring in the system) having a conjugated π-electron system, preferably a 6 to 10 membered aryl, for example, phenyl and naphthyl. The aryl is more preferably phenyl. The aryl ring can be fused to the ring of heteroaryl, heterocyclyl or cycloalkyl, wherein the ring bound to the parent structure is aryl ring. Non-limiting examples include:

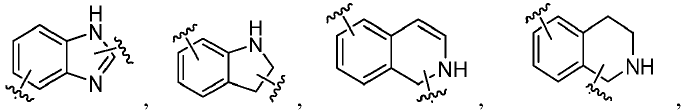

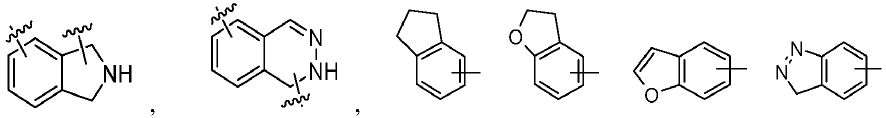

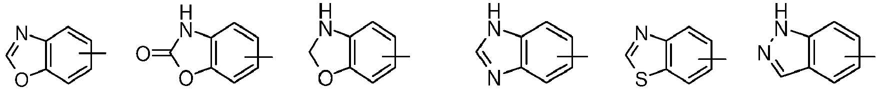

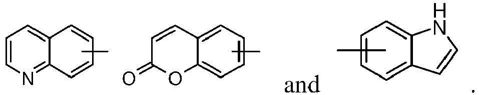

The aryl can be substituted or unsubstituted. When substituted, the substituent(s) is preferably one or more group(s) independently selected from the group consisting of alkyl, alkenyl, alkynyl, alkoxy, alkylthio, alkylamino, halogen, thiol, hydroxy, nitro, cyano, cycloalkyl, heterocyclyl, aryl, heteroaryl, cycloalkoxy, heterocycloalkoxy, cycloalkylthio, heterocyclylthio, carboxy and alkoxycarbonyl.

The term "heteroaryl" refers to a 5 to 14 membered heteroaromatic system having 1 to 4 heteroatoms selected from the group consisting of oxygen, sulfur and nitrogen. The heteroaryl is preferably a 5 to 10 membered heteroaryl, and more preferably a 5 or

6 membered heteroaryl, for example imidazolyl, furanyl, thienyl, thiazolyl, pyrazolyl, oxazolyl, pyrrolyl, triazolyl, tetrazolyl, pyridyl, pyrimidinyl, thiadiazolyl, pyrazinyl and the like, preferably triazolyl, thienyl, imidazolyl, pyrazolyl or pyrimidinyl, thiazolyl, and more preferably triazolyl, pyrrolyl, thienyl, thiazolyl and pyrimidinyl. The heteroaryl ring can be fused to the ring of aryl, heterocyclyl or cycloalkyl, wherein the ring bound to the parent structure is heteroaryl ring. Non-limiting examples thereof include:

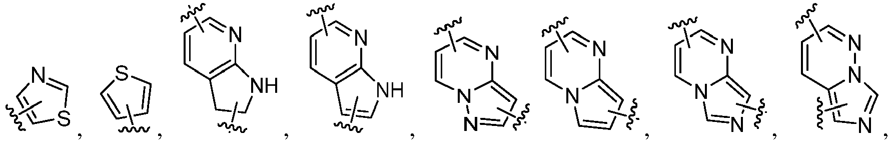

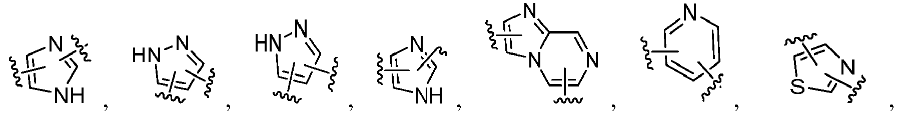

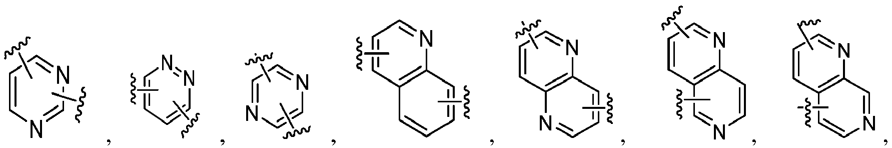

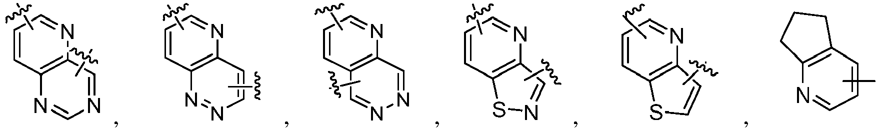

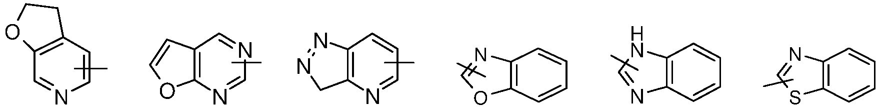

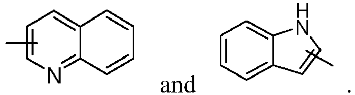

The heteroaryl can be optionally substituted or unsubstituted. When substituted, the substituent(s) is preferably one or more group(s) independently selected from the group consisting of alkyl, alkenyl, alkynyl, alkoxy, alkylthio, alkylamino, halogen, thiol, hydroxy, nitro, cyano, cycloalkyl, heterocyclyl, aryl, heteroaryl, cycloalkoxy, heterocycloalkoxy, cycloalkylthio, heterocyclylthio, carboxy and alkoxycarbonyl.

The term "alkoxy" refers to an -O-(alkyl) or an -O-(unsubstituted cycloalkyl) group, wherein the alkyl is as defined above. Non-limiting examples of alkoxy include methoxy, ethoxy, propoxy, butoxy, cyclopropyloxy, cyclobutyloxy, cyclopentyloxy, cyclohexyloxy. The alkoxy can be optionally substituted or unsubstituted. When substituted, the substituent(s) is preferably one or more group(s) independently selected from the group consisting of alkyl, alkenyl, alkynyl, alkoxy, alkylthio, alkylamino, halogen, thiol, hydroxy, nitro, cyano, cycloalkyl, heterocyclyl, aryl, heteroaryl, cycloalkoxy, heterocycloalkoxy, cycloalkylthio, heterocyclylthio, carboxy and alkoxycarbonyl.

"Haloalkyl" refers to an alkyl substituted by one or more halogen(s), wherein the alkyl is as defined above.

"Haloalkoxy" refers to an alkoxy substituted by one or more halogen(s), wherein the alkoxy is as defined above.

"Hydroxyalkyl" refers to an alkyl substituted by hydroxy(s), wherein the alkyl is as defined above.

"Alkenyl" refers to a chain olefin, also known as alkene group. The alkenyl can be further substituted by other related group, for example alkyl, alkenyl, alkynyl, alkoxy, alkylthio, alkylamino, halogen, thiol, hydroxy, nitro, cyano, cycloalkyl, heterocyclyl, aryl, heteroaryl, cycloalkoxy, heterocycloalkoxy, cycloalkylthio, heterocyclylthio, carboxy or alkoxycarbonyl.

"Alkynyl" refers to (CH=C-). The alkynyl can be further substituted by other related group, for example alkyl, alkenyl, alkynyl, alkoxy, alkylthio, alkylamino, halogen, thiol, hydroxy, nitro, cyano, cycloalkyl, heterocyclyl, aryl, heteroaryl, cycloalkoxy, heterocycloalkoxy, cycloalkylthio, heterocyclylthio, carboxy or alkoxycarbonyl.

"Hydroxy" refers to an -OH group.

"Halogen" refers to fluorine, chlorine, bromine or iodine.

"Amino" refers to a -NH₂ group.

"Cyano" refers to a -CN group.

"Nitro" refers to a -NO₂ group.

"Carboxy" refers to a -C(O)OH group.

"THF" refers to tetrahydrofuran.

"EtOAc" refers to ethyl acetate.

"MeOH" refers to methanol.

"DMF" refers to N,N-dimethylformamide.

"DIPEA" refers to diisopropylethylamine.

"TFA" refers to trifluoroacetic acid.

"MeCN" refers to acetonitrile.

"DMA" refers to N,N-dimethylacetamide.

"Et₂O" refers to diethyl ether.

"DCE" refers to 1,2-dichloroethane.

"DIPEA" refers to N,N-diisopropylethylamine.

"NBS" refers to N-bromosuccinimide.

"NIS" refers to N-iodosuccinimide.

"Cbz-Cl" refers to benzyl chloroformate.

"Pd₂(dba)₃" refers to tris(dibenzylideneacetone)dipalladium.

"Dppf" refers to 1,1'-bisdiphenylphosphinoferrocene.

"HATU" refers to 2-(7-azabenzotriazol-1-yl)-N,N,N',N'-tetramethyluronium hexafluorophosphate.

"KHMDS" refers to potassium hexamethyldisilazide.

"LiHMDS" refers to lithium bis(trimethylsilyl)amide.

"MeLi" refers to methyl lithium.

"n-BuLi" refers to n-butyl lithium.

"NaBH(OAc)₃" refers to sodium triacetoxyborohydride.

Different expressions such as "X is selected from the group consisting of A, B or C", "X is selected from the group consisting of A, B and C", "X is A, B or C", "X is A, B and C" are the same meaning, that is, X can be any one or more of A, B and C.

The hydrogen atom of the present invention can be replaced by its isotope deuterium. Any of the hydrogen atoms in the compounds of the examples of the present invention can also be substituted by deuterium atom.

"Optional" or "optionally" means that the event or circumstance described subsequently can, but need not, occur, and such a description includes the situation in which the event or circumstance does or does not occur. For example, "the heterocyclyl optionally substituted by an alkyl" means that an alkyl group can be, but need not be, present, and such a description includes the situation of the heterocyclyl being substituted by an alkyl and the heterocyclyl being not substituted by an alkyl.

"Substituted" refers to one or more hydrogen atoms in a group, preferably up to 5, and more preferably 1 to 3 hydrogen atoms, independently substituted by a corresponding number of substituents. It goes without saying that the substituents only exist in their possible chemical position. The person skilled in the art is able to determine whether the substitution is possible or impossible by experiments or theory without excessive efforts. For example, the combination of amino or hydroxy having free hydrogen and carbon atoms having unsaturated bonds (such as olefinic) may be unstable.

A "pharmaceutical composition" refers to a mixture of one or more of the compounds according to the present invention or physiologically/pharmaceutically acceptable salts or prodrugs thereof with other chemical components such as physiologically/pharmaceutically acceptable carriers and excipients. The purpose of the pharmaceutical composition is to facilitate administration of a compound to an organism, which is conducive to the absorption of the active ingredient so as to exert biological activity.

A "pharmaceutically acceptable salt" refers to a salt of the compound of the present invention, which is safe and effective in mammals and has the desired biological activity.

### DESCRIPTION OF DRAWINGS

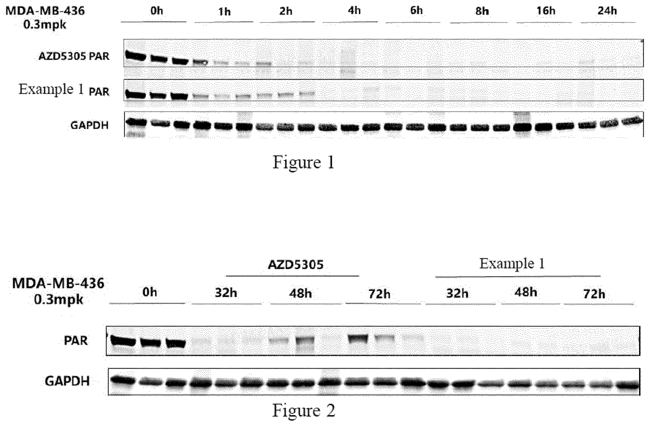

Figure 1 Inhibition on PAR within 24 hours after a single administration of Example 1 in the MDA-MB-436 model;
Figure 2 Inhibition on PAR within 24 to 72 hours after a single administration of Example 1 in the MDA-MB-436 model.

### DETAILED DESCRIPTION OF THE INVENTION

The present invention will be further described with reference to the following examples, but the examples should not be considered as limiting the scope of the present invention.

### EXAMPLES

The structures of the compounds of the present invention were identified by nuclear magnetic resonance (NMR) and/or liquid chromatography-mass spectrometry (LC-MS). NMR shifts (δ) are given in parts per million (ppm). NMR is determined by a Bruker AVANCE-400 instrument. The solvents for determination are deuterated-dimethyl sulfoxide (DMSO-*d₆*), deuterated-methanol (CD₃OD) and deuterated-chloroform (CDCl₃), and the internal standard is tetramethylsilane (TMS).

Liquid chromatography-mass spectrometry (LC-MS) was determined on an Agilent 1200 Infinity Series mass spectrometer. High performance liquid chromatography (HPLC) was determined on an Agilent 1200DAD high pressure liquid chromatograph (Sunfire C18 150×4.6 mm column), and a Waters 2695-2996 high pressure liquid chromatograph (Gimini C18 150×4.6 mm column).

Yantai Huanghai HSGF254 or Qingdao GF254 silica gel plate was used as the thin-layer silica gel chromatography (TLC) plate. The dimension of the silica gel plate used in TLC is 0.15 mm to 0.2 mm, and the dimension of the silica gel plate used in product purification is 0.4 mm to 0.5 mm. Yantai Huanghai 200 to 300 mesh silica gel was generally used as a carrier for column chromatography.

The raw materials used in the examples of the present invention are known and commercially available, or can be synthesized by or according to known methods in the art.

Unless otherwise stated, all reactions of the present invention are carried out under continuous magnetic stirring under a dry nitrogen or argon atmosphere. The solvent is dry, and the reaction temperature is in degrees celsius.

### Example 1

### 1'-((7-Ethyl-6-oxo-5,6-dihydro-1,5-naphthyridin-3-yl)methyl)-N-methyl-1',2',3',6'-tetrahydro-[3,4'-bipyridine]-6-carboxamide

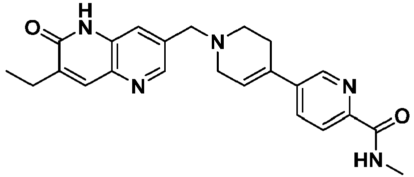

### Step 1: Preparation of ethyl 6-formyl-5-nitronicotinate

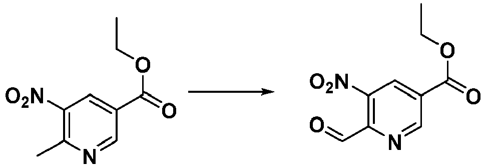

Selenium dioxide (3.96 g, 35.68 mmol) was added to a solution of ethyl 6-methyl-5-nitronicotinate (5.0 g, 23.8 mmol) in 1,4-dioxane (25 mL). The reaction solution was heated to 110°C and stirred for 20 hours. The reaction solution was cooled to room temperature, and filtered through diatomaceous earth under reduced pressure. The filtrate was concentrated under reduced pressure to remove the organic solvent, and the resulting residue was purified by column chromatography to obtain the brown oily compound ethyl 6-formyl-5-nitronicotinate (4.8 g, 90%).

MS m/z (ES⁺): 224.1 [M]⁺.

### Step 2: Preparation of ethyl (E)-6-(2-(ethoxycarbonyl)but-1-en-1-yl)-5-nitronicotinate

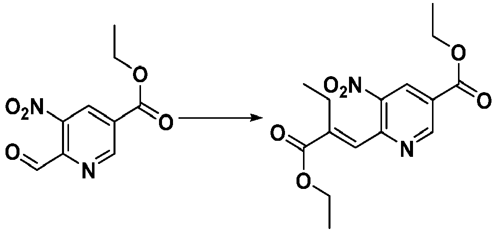

In an ice bath, triethyl 2-phosphonobutyrate (12.8 g, 50.6 mmol) was slowly added dropwise to a solution of NaH (60 wt%, 2.0 g, 50.6 mmol) in tetrahydrofuran (30 mL). The reaction solution was stirred in the ice bath for 0.5 hours, then warmed up to room temperature and stirred for 0.5 hours, then heated to 40°C and stirred for 10 minutes. The reaction solution was cooled to -78°C, and a solution of ethyl 6-formyl-5-nitronicotinate (4.8 g, 21 mmol) in tetrahydrofuran (20 mL) was slowly added dropwise, and stirred at -78°C for 1 hour. The reaction solution was quenched with saturated aqueous ammonium chloride, and extracted three times with ethyl acetate. The organic phases were combined, and washed with saturated brine. The organic phase was separated, dried over anhydrous sodium sulfate and filtered. The filtrate was concentrated under reduced pressure to remove the organic solvent, and the resulting residue was purified by column chromatography to obtain the title compound ethyl (E)-6-(2-(ethoxycarbonyl)but-1-en-1-yl)-5-nitronicotinate (5.1 g, 75%).

MS m/z (ES⁺): 322.2 [M]⁺.

### Step 3: Preparation of ethyl 7-ethyl-6-oxo-5,6,7,8-tetrahydro-1,5-naphthyridine-3-carboxylate

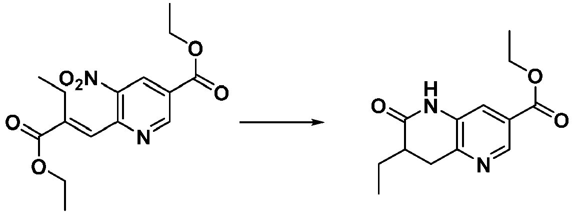

Palladium on carbon (1.86 g, 1.76 mmol) was added to a solution of ethyl (E)-6-(2-(ethoxycarbonyl)but-1-en-1-yl)-5-nitronicotinate (3.76 g, 11.6 mmol) in ethanol (50 mL). The reaction solution was purged with nitrogen for five minutes, placed under a hydrogen atmosphere, and stirred overnight at room temperature. The reaction solution was filtered through diatomaceous earth under reduced pressure. The filtrate was concentrated under reduced pressure to obtain the crude product ethyl 7-ethyl-6-oxo-5,6,7,8-tetrahydro-1,5-naphthyridine-3-carboxylate (2.8 g), which was used directly in the next step.

MS m/z (ESI): 249.2 [M+H]⁺.

### Step 4: Preparation of ethyl 7-ethyl-6-oxo-5,6-dihydro-1,5-naphthyridine-3-carboxylate

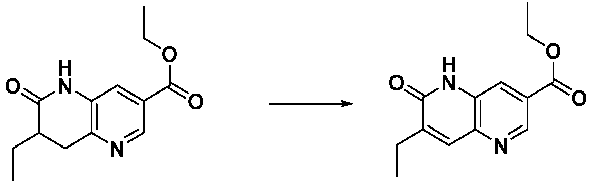

DDQ (2.85 g, 12.5 mmol) was added to a solution of ethyl 7-ethyl-6-oxo-5,6,7,8-tetrahydro-1,5-naphthyridine-3-carboxylate (2.8 g, 11.3 mmol) in 1,4-dioxane (50 mL), heated to 110°C and stirred for 4 hours. The reaction solution was cooled to room temperature, and filtered through diatomaceous earth under reduced pressure. The filtrate was concentrated under reduced pressure, and the resulting residue was purified by column chromatography to obtain the compound ethyl 7-ethyl-6-oxo-5,6-dihydro-1,5-naphthyridine-3-carboxylate (2.1 g, 76%).

MS m/z (ESI): 247.2 [M+H]⁺.

### Step 5: Preparation of 3-ethyl-7-(hydroxymethyl)-1,5-naphthyridin-2(1H)-one

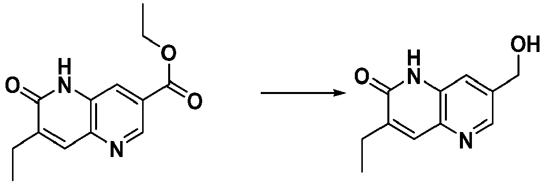

In an ice bath, a solution of lithium aluminum hydride in THF (2 M, 8.5 mL, 17.0 mmol) was slowly added to a solution of 7-ethyl-6-oxo-5,6-dihydro-1,5-naphthyridine-3-carboxylate (2.1 g, 8.5 mmol) in THF (30 mL), and stirred under the ice bath for 2 hours. The reaction solution was quenched with sodium sulfate decahydrate, and filtered through diatomaceous earth under reduced pressure. The filtrate was concentrated under reduced pressure, and the resulting residue was purified by column chromatography to obtain the compound 3-ethyl-7-(hydroxymethyl)-1,5-naphthyridin-2(1H)-one (1.5 g, 86%).

MS m/z (ESI): 205.2 [M+H]⁺.

### Step 6: Preparation of 7-(chloromethyl)-3-ethyl-1,5-naphthyridin-2(1H)-one

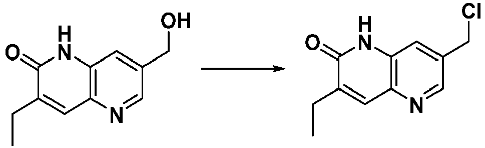

In an ice bath, thionyl chloride (3.2 mL, 44.1 mmol) was added to a solution of 3-ethyl-7-(hydroxymethyl)-1,5-naphthyridin-2(1H)-one (1.5 g, 7.4 mmol) in dichloromethane (30 mL). DMF (0.06 mL, 0.77 mmol) was added, and the reaction solution was stirred at room temperature for 6 hours. The reaction solution was concentrated under reduced pressure to remove the organic solvent, so as to obtain the crude product 7-(chloromethyl)-3-ethyl-1,5-naphthyridin-2(1H)-one (1.61 g), which was used directly in the next step.

MS m/z (ESI): 223.1 [M+H]⁺.

### Step 7: Preparation of 1'-(tert-butyl) 6-methyl 3',6'-dihydro-[3,4'-bipyridine]-1',6(2'H)-dicarboxylate

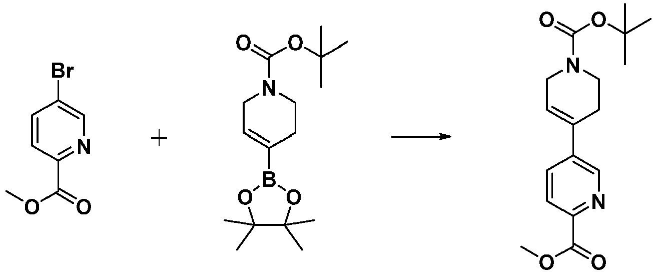

At room temperature, methyl 5-bromopicolinate (1.0 g, 4.6 mmol), tert-butyl 4-(4,4,5,5-tetramethyl-1,3,2-dioxaborolan-2-yl)-3,6-dihydropyridine-1(2H)-carboxylate (1.6 g, 5.1 mmol), 1,1'-bis(diphenylphosphino)ferrocene palladium(II)dichloride (146 mg, 0.2 mmol) and potassium carbonate (1.6 g, 11.6 mmol) were dissolved in N,N-dimethylformamide (10 mL). The reaction solution was purged with nitrogen for 1 minute, heated to 140°C and reacted under microwave for 30 minutes. Water was added to the reaction system, and extracted with ethyl acetate. The organic phase was separated, washed with saturated brine, dried over anhydrous sodium sulfate and filtered. The filtrate was concentrated under reduced pressure to remove the organic solvent, and the resulting residue was purified by column chromatography to obtain the brown oily compound 1'-(*tert*-butyl) 6-methyl 3',6'-dihydro-[3,4'-bipyridine]-1',6(2'H)-dicarboxylate (620 mg, 42%).

MS m/z (ES+): 319.1 [M+H]⁺.

### Step 8: Preparation of tert-butyl 6-(methylcarbamoyl)-3',6'-dihydro-[3,4'-bipyridine]-1'(2'H)-carboxylate

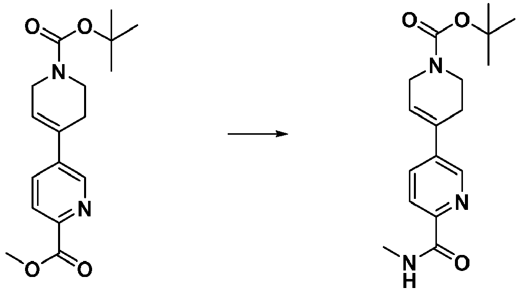

At room temperature, a solution of methylamine in alcohol (30 wt%, 2.0 g, 19.5 mmol) was added to a solution of 1'-(*tert*-butyl) 6-methyl 3',6'-dihydro-[3,4'-bipyridine]-1',6(2'H)-dicarboxylate (620 mg, 1.9 mmol) in methanol (8 mL), and stirred at room temperature for 4 hours. The reaction solution was concentrated under reduced pressure, saturated aqueous ammonium chloride solution was added and extracted three times with DCM. The organic phases were combined, dried over anhydrous sodium sulfate and filtered. The filtrate was concentrated under reduced pressure to remove the organic solvent, so as to obtain the crude product *tert-*butyl 6-(methylcarbamoyl)-3',6'-dihydro-[3,4'-bipyridine]-1'(2'H)-carboxylate (600 mg), which was used directly in the next step without further purification.

MS m/z (ESI): 318.2 [M+H]⁺.

### Step 9: Preparation of N-methyl-1',2',3',6'-tetrahydro-[3,4'-bipyridine]-6-carboxamide

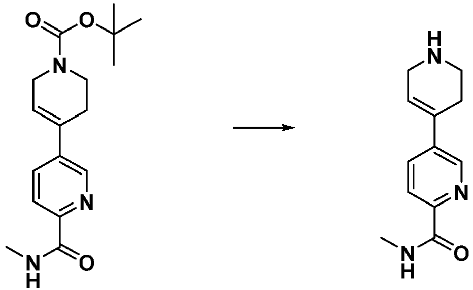

In an ice bath, trifluoroacetic acid (1 mL) was added to a solution of tert-butyl 6-(methylcarbamoyl)-3',6'-dihydro-[3,4'-bipyridine]-1'(2'H)-carboxylate (200 mg, 0.6 mmol) in dichloromethane (5 mL), and stirred at room temperature for 4 hours. The reaction solution was concentrated under reduced pressure to remove the organic solvent, so as to obtain the crude product N-methyl-1',2',3',6'-tetrahydro-[3,4'-bipyridine]-6-carboxamide (155 mg), which was used directly in the next step without further purification.

MS m/z (ESI): 218.2 [M+H]⁺.

### Step 10: Preparation of 1'-((7-ethyl-6-oxo-5,6-dihydro-1,5-naphthyridin-3-yl)methyl)-N-methyl-1',2',3',6'-tetrahydro-[3,4'-bipyridine]-6-carboxamide

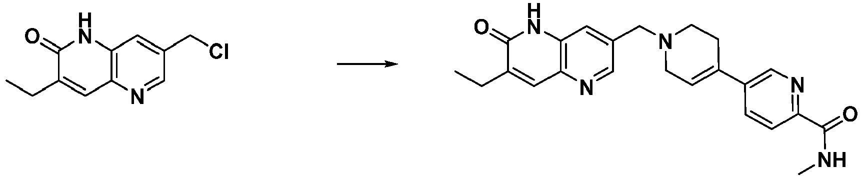

DIPEA (58 mg, 0.45 mmol) and potassium iodide (3 mg, 0.02 mmol) were added to a solution of 7-(chloromethyl)-3-ethyl-1,5-naphthyridin-2(1H)-one (20 mg, 0.09 mmol) and N-methyl-1',2',3',6'-tetrahydro-[3,4'-bipyridine]-6-carboxamide (52 mg, 0.24 mmol) in acetonitrile (3 mL). The reaction solution was heated to 80°C and stirred for 2 hours. The reaction solution was cooled to room temperature, and filtered under reduced pressure. The filtrate was concentrated under reduced pressure, and the resulting residue was purified by column chromatography to obtain the compound 1'-((7-ethyl-6-oxo-5,6-dihydro-1,5-naphthyridin-3-yl)methyl)-N-methyl-1',2',3',6'-tetr ahydro-[3,4'-bipyridine]-6-carboxamide (6.2 mg, 17%).
¹H NMR (400 MHz, DMSO-*d₆*) *δ* 11.76-11.91 (m, 1H), 8.68-8.73 (m, 2H), 8.40-8.44 (m, 1H), 8.02-7.95 (m, 2H), 7.76 (s, 1H), 7.65 (s, 1H), 6.41-6.44 (m, 1H), 3.69-3.76 (m, 2H), 3.19-3.13 (m, 2H), 2.77-2.85 (m, 3H), 2.66-2.74 (m, 2H), 2.51-2.59 (m, 4H), 1.18 (t, *J* = 7.4 Hz, 3H);
MS m/z (ESI): 404.2 [M+H]⁺.

### Example 2

### 5-(1-((7-Ethyl-6-oxo-5,6-dihydro-1,5-naphthyridin-3-yl)methyl)piperidin-4-yl)-N-methylpicolinamide

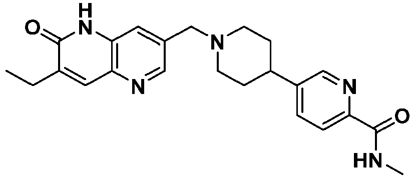

### Step 1: Preparation of tert-butyl 4-(6-(methylcarbamoyl)pyridin-3-yl)piperidine-1-carboxylate

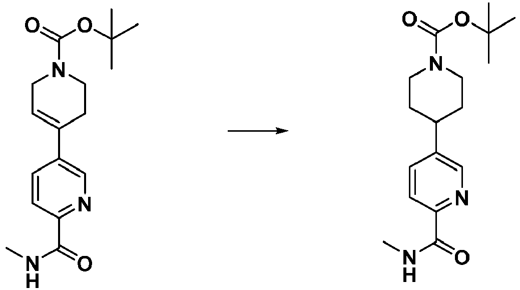

Under a nitrogen atmosphere, Pd/C (50 mg) was added to a solution of *tert*-butyl 6-(methylcarbamoyl)-3',6'-dihydro-[3,4'-bipyridine]-1'(2'H)-carboxylate (400 mg, 1.3 mmol) in methanol (8 mL). The reaction solution was purged with hydrogen three times, and stirred at room temperature for 16 hours. The reaction solution was filtered, concentrated under reduced pressure to remove the organic solvent, so as to obtain the crude product *tert*-butyl 4-(6-(methylcarbamoyl)pyridin-3-yl)piperidine-1-carboxylate (280 mg), which was used directly in the next step without further purification.

MS m/z (ESI): 320.2 [M+H]⁺.

### Step 2: Preparation of N-methyl-5-(piperidin-4-yl)picolinamide

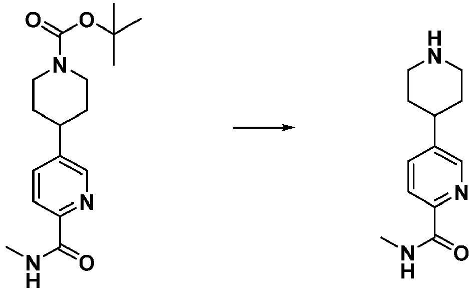

In an ice bath, hydrochloride acid in dioxane (2 mL) was added to a solution of *tert*-butyl 4-(6-(methylcarbamoyl)pyridin-3-yl)piperidine-1-carboxylate (280 mg, 0.88 mmol) in dichloromethane (5 mL), and stirred at room temperature for 4 hours. The reaction solution was concentrated under reduced pressure to remove the organic solvent, so as to obtain the crude product N-methyl-5-(piperidin-4-yl)picolinamide (230 mg), which was used directly in the next step without further purification.

MS m/z (ESI): 220.2 [M+H]⁺.

### Step 3: Preparation of 5-(1-((7-ethyl-6-oxo-5,6-dihydro-1,5-naphthyridin-3-yl)methyl)piperidin-4-yl)-N-methylpicolinamide

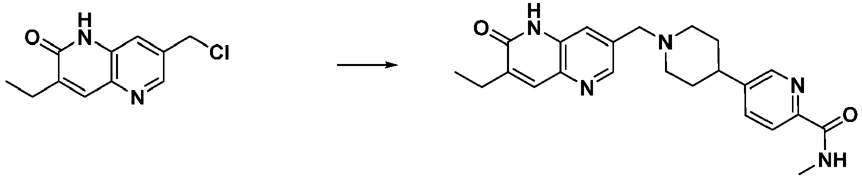

DIPEA (58 mg, 0.45 mmol) and potassium iodide (3 mg, 0.02 mmol) were added to a solution of 7-(chloromethyl)-3-ethyl-1,5-naphthyridin-2(1H)-one (20 mg, 0.09 mmol) and N-methyl-5-(piperidin-4-yl)picolinamide (52 mg, 0.24 mmol) in acetonitrile (3 mL). The reaction solution was heated to 80°C and stirred for 2 hours. The reaction solution was cooled to room temperature, and filtered under reduced pressure. The filtrate was concentrated under reduced pressure, and the resulting residue was purified by column chromatography to obtain the compound 5-(1-((7-ethyl-6-oxo-5,6-dihydro-1,5-naphthyridin-3-yl)methyl)piperidin-4-yl)-N-methylpicolinamide (6.5 mg, 18%).
¹H NMR (400 MHz, DMSO-*d₆*) *δ* 11.79-11.87 (m, 1H), 8.66-8.72 (m, 1H), 8.53 (s, 1H), 8.38-8.42 (m, 1H), 7.92-7.97 (m, 1H), 7.84-7.88 (m, 1H), 7.75 (s, 1H), 7.61 (s, 1H), 3.58-3.65 (m, 2H), 2.91-2.97 (m, 2H), 2.77-2.84 (m, 3H), 2.63-2.72 (m, 1H), 2.51-2.58 (m, 2H), 2.08-2.17 (m, 2H), 1.67-1.84 (m, 4H), 1.18 (t, *J=* 7.4 Hz, 3H);
MS m/z (ESI): 406.2 [M+H]⁺.

### Example 3

### 3-Ethyl-7-((4-(8-(methylamino)-1,7-naphthyridin-3-yl)piperazin-1-yl)methyl)-1,5-naphthyridin-2(1H)-one

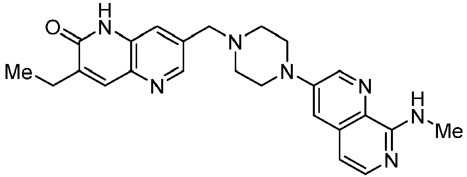

3-ethyl-7-((4-(8-(methylamino)-1,7-naphthyridin-3-yl)piperazin-1-yl)methyl)-1,5-naphthyridin-2(1H)-one was obtained by the preparation method referred to in Example 1.

It can also be synthesized according to the following steps:

### Step 1: Preparation of tert-butyl 4-(8-chloro-1,7-naphthyridin-3-yl)piperazine-1-carboxylate

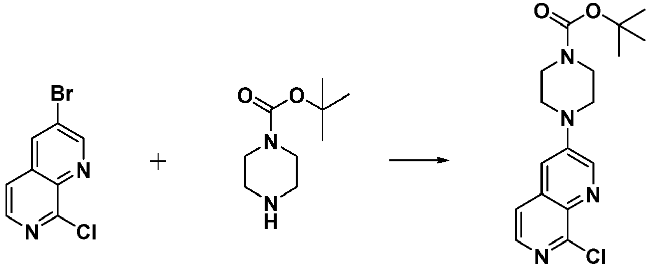

3-Bromo-8-chloro-1,7-naphthyridine (1 g, 4.10 mmol), *tert-*butyl piperazine-1-carboxylate (918 mg, 4.93 mmol), 1,1'-bis(diphenylphosphino)ferrocene palladium(II)dichloride (146 mg, 0.20 mmol) and potassium carbonate (1.61 g, 11.65 mmol) were mixed in DMF (10 mL), warmed up to 100°C and reacted for 12 hours. The reaction solution was cooled to room temperature, diluted with water (100 mL), and extracted with ethyl acetate (100 mL×3). The organic phases were combined, washed with saturated sodium chloride solution (100 mL), dried over anhydrous sodium sulfate, and filtered. The filtrate was concentrated under reduced pressure, and the resulting residue was purified by silica gel column chromatography to obtain the target compound *tert*-butyl 4-(8-chloro-1,7-naphthyridin-3-yl)piperazine-1-carboxylate (510 mg, 35.7%).

MS m/z (ESI): 349.1 [M+H]⁺.

### Step 2: Preparation of tert-butyl 4-(8-(methylamino)-1,7-naphthyridin-3-yl)piperazine-1-carboxylate

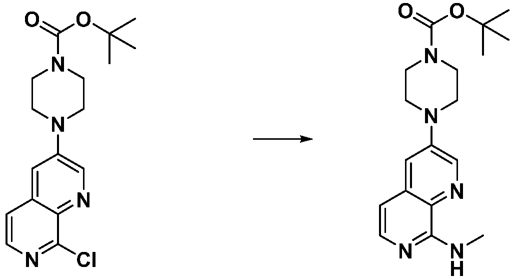

A solution of methylamine in alcohol (30 wt%, 2.0 g, 19.5 mmol) was added to a solution of *tert*-butyl 4-(8-chloro-1,7-naphthyridin-3-yl)piperazine-1-carboxylate (510 mg, 1.46 mmol) in methanol (8 mL), warmed up to 80°C and stirred for 12 hours. The reaction solution was concentrated under reduced pressure, and partitioned between DCM and water. The organic phase was dried over anhydrous sodium sulfate and filtered. The filtrate was concentrated under reduced pressure to remove the organic solvent, so as to obtain the crude target compound *tert-*butyl 4-(8-(methylamino)-1,7-naphthyridin-3-yl)piperazine-1-carboxylate (500 mg), which was used directly in the next step.

MS m/z (ESI): 344.2 [M+H]⁺.

### Step 3: Preparation of N-methyl-3-(piperazin-1-yl)-1,7-naphthyridin-8-amine

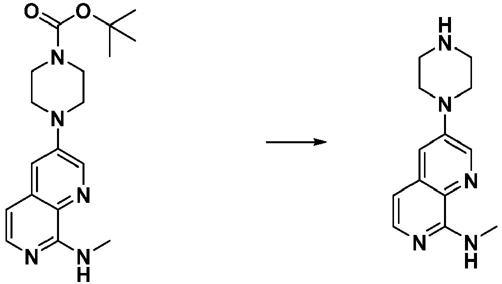

In an ice bath, trifluoroacetic acid (1 mL) was added to a solution of *tert*-butyl 4-(8-(methylamino)-1,7-naphthyridin-3-yl)piperazine-1-carboxylate (200 mg, 0.58 mmol) in dichloromethane (5 mL), and stirred at room temperature for 4 hours. The reaction solution was concentrated under reduced pressure to obtain the crude target compound N-methyl-3-(piperazin-1-yl)-1,7-naphthyridin-8-amine (140 mg), which was used directly in the next step.

MS m/z (ESI): 244.2 [M+H]⁺.

### Step 4: Preparation of 3-ethyl-7-((4-(8-(methylamino)-1,7-naphthyridin-3-yl)piperazin-1-yl)methyl)-1,5-naphthyridin-2(1H)-one

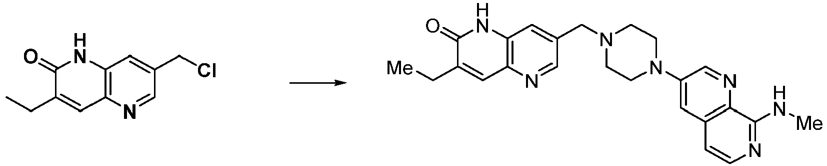

DIPEA (52 mg, 0.41 mmol) and potassium iodide (3 mg, 0.02 mmol) were added to a solution of 7-(chloromethyl)-3-ethyl-1,5-naphthyridin-2(1H)-one (30 mg, 0.13 mmol) and N-methyl-3-(piperazin-1-yl)-1,7-naphthyridin-8-amine (49 mg, 0.20 mmol) in acetonitrile (3 mL). The reaction solution was heated to 80°C and reacted for 3 hours. The reaction solution was cooled to room temperature and filtered. The filtrate was concentrated under reduced pressure, and the resulting residue was purified by column chromatography to obtain the target compound 3-ethyl-7-((4-(8-(methylamino)-1,7-naphthyridin-3-yl)piperazin-1-yl)methyl)-1,5-naphthyridin-2(1H)-one (12 mg, 21.5%).
¹H NMR (400 MHz, DMSO-*d₆*) *δ* 11.76 (s, 1H), 8.33 (s, 1H), 7.65-7.70 (s, 2H), 7.55 (s, 1H), 7.52-7.76 (m, 1H), 6.64-6.73 (m, 2H), 3.57 (s, 2H), 3.32-3.35 (m, 4H), 2.68 (d, *J* = 4.4 Hz, 3H), 2.47-2.51 (m, 6H), 1.11 (t, *J* = 7.4 Hz, 3H);
MS m/z (ESI): 430.2 [M+H]⁺.

### Example 4

### 3-Ethyl-7-((4-(2-methyl-1-oxo-1,2-dihydroisoquinolin-6-yl)piperazin-1-yl)methyl)-1,5-naphthyridin-2(1H)-one

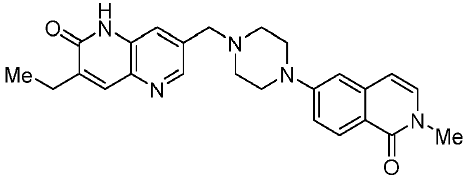

3-ethyl-7-((4-(2-methyl-1-oxo-1,2-dihydroisoquinolin-6-yl)piperazin-1-yl)methyl)-1,5-naphthyridin-2(1H)-one was obtained by the preparation method referred to in Example 3.

MS m/z (ESI): 430.2 [M+H]⁺.

### Example 5

### 3-Ethyl-7-((4-(2-methyl- 1-oxo-1,2,3,4-tetrahydroisoquinolin-6-yl)piperazin-1-yl) methyl)-1,5-naphthyridin-2(1H)-one

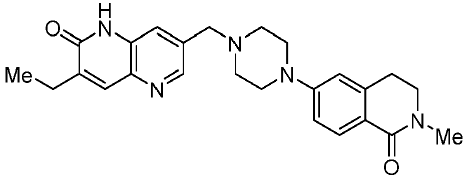

3-ethyl-7-((4-(2-methyl-1-oxo-1,2,3,4-tetrahydroisoquinolin-6-yl)piperazin-1-yl) methyl)-1,5-naphthyridin-2(1H)-one was obtained by the preparation method referred to in Example 3.

MS m/z (ESI): 432.2 [M+H]⁺.

### Example 6

### 3-Ethyl-7-((4-(2-methyl-1-oxoisoindolin-5-yl)piperazin-1-yl)methyl)-1,5-naphthyridin-2(1H)-one

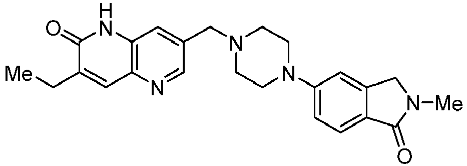

3-ethyl-7-((4-(2-methyl-1-oxoisoindolin-5-yl)piperazin-1-yl)methyl)-1,5-naphthyridin-2 (1H)-one was obtained by the preparation method referred to in Example 3.

MS m/z (ESI): 418.2 [M+H]⁺.

### Example 7

### 3-Ethyl-7-((4-(2-methyl-1-oxo-1,2-dihydrophthalazin-6-yl)piperazin-1-yl)methyl)-1,5-naphthyridin-2(1H)-one

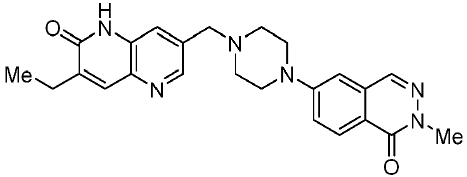

3-ethyl-7-((4-(2-methyl-1-oxo-1,2-dihydrophthalazin-6-yl)piperazin-1-yl)methyl)-1,5-naphthyridin-2(1H)-one was obtained by the preparation method referred to in Example 3.

MS m/z (ESI): 431.2 [M+H]⁺.

### Example 8

### 3-Ethyl-7-((4-(8-(methylamino)quinolin-3-yl)piperazin-1-yl)methyl)-1,5-naphthyridin-2 (1H)-one

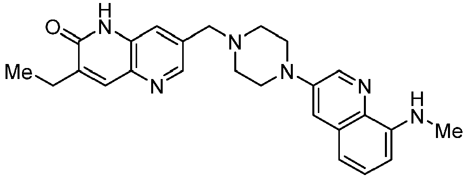

3-ethyl-7-((4-(8-(methylamino)quinolin-3-yl)piperazin-1-yl)methyl)-1,5-naphthyridin-2 (1H)-one was obtained by the preparation method referred to in Example 3.
¹H NMR (400 MHz, DMSO-*d₆*) *δ* 11.86 (s, 1H), 8.64 (d, *J=* 2.7 Hz, 1H), 8.43 (d, *J* = 1.5 Hz, 1H), 7.76 (s, 1H), 7.65 (s, 1H), 7.40 (d, *J* = 2.7 Hz, 1H), 7.23-7.27 (m, 1H), 6.88 (d, *J=* 7.8 Hz, 1H), 6.35-6.40 (m, 2H), 3.64-3.68 (m, 2H), 3.28-3.31 (m, 4H), 2.87 (d, *J=* 5.1 Hz, 3H), 2.58-2.62 (m, 4H), 2.53-2.59 (m, 2H), 1.19 (t, *J* = 7.4 Hz, 3H);
MS m/z (ESI): 429.2 [M+H]⁺.

### Example 9

### 3-Ethyl-7-((4-(8-(methylamino)-1,5-naphthyridin-3-yl)piperazin-1-yl)methyl)-1,5-naphthyridin-2(1H)-one

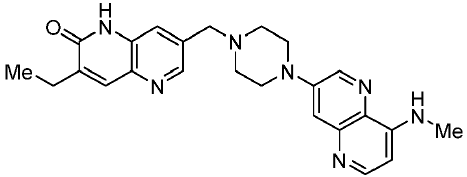

3-ethyl-7-((4-(8-(methylamino)-1,5-naphthyridin-3-yl)piperazin-1-yl)methyl)-1,5-naphthyridin-2(1H)-one was obtained by the preparation method referred to in Example 3.

MS m/z (ESI): 430.2 [M+H]⁺.

### Example 10

### 3-Ethyl-7-((4-(8-(methylamino)-1,6-naphthyridin-3-yl)piperazin-1-yl)methyl)-1,5-naphthyridin-2(1H)-one

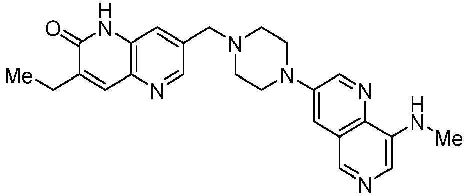

### 3-ethyl-7-((4-(8-(methylamino)-1,6-naphthyridin-3-yl)piperazin-1-yl)methyl)-1,5-naphthyridin-2(1H)-one was obtained by the preparation method referred to in Example 3.

MS m/z (ESI): 430.2 [M+H]⁺.

### Example 11

### 3-Ethyl-7-((4-(4-(methylamino)pyrido[3,2-d]pyrimidin-7-yl)piperazin-1-yl)methyl)-1,5-naphthyridin-2(1H)-one

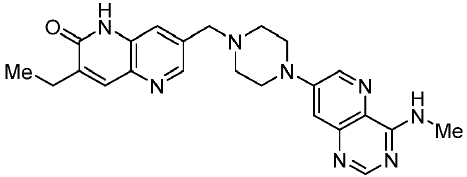

3-ethyl-7-((4-(4-(methylamino)pyrido[3,2-d]pyrimidin-7-yl)piperazin-1-yl)methyl)-1,5-naphthyridin-2(1H)-one was obtained by the preparation method referred to in Example 3.

MS m/z (ESI): 431.2 [M+H]⁺.

### Example 12

### 3-Ethyl-7-((4-(4-(methylamino)pyrido[3,2-c]pyridazin-7-yl)piperazin-1-yl)methyl)-1,5-naphthyridin-2(1H)-one

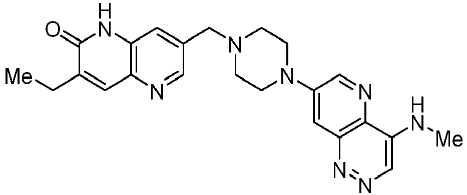

3-ethyl-7-((4-(4-(methylamino)pyrido[3,2-c]pyridazin-7-yl)piperazin-1-yl)methyl)-1,5-naphthyridin-2(1H)-one was obtained by the preparation method referred to in Example 3.

MS m/z (ESI): 431.2 [M+H]⁺.

### Example 13

### 3-Ethyl-7-((4-(8-(methylamino)pyrido[2,3-d]pyridazin-3-yl)piperazin-1-yl)methyl)-1,5-naphthyridin-2(1H)-one

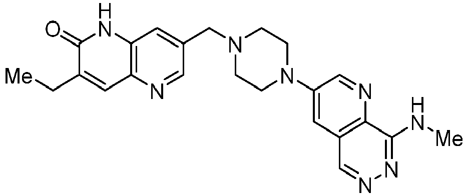

3-ethyl-7-((4-(8-(methylamino)pyrido[2,3-d]pyridazin-3-yl)piperazin-1-yl)methyl)-1,5-naphthyridin-2(1H)-one was obtained by the preparation method referred to in Example 3.

MS m/z (ESI): 431.2 [M+H]⁺.

### Example 14

### 3-Ethyl-7-((4-(3-(methylamino)isothiazolo[4,5-b]pyridin-6-yl)piperazin-1-yl)methyl)-1,5-naphthyridin-2(1H)-one

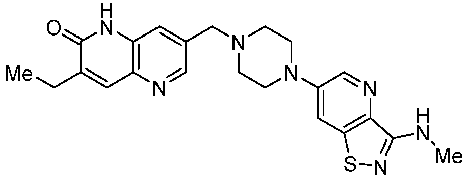

3-ethyl-7-((4-(3-(methylamino)isothiazolo[4,5-b]pyridin-6-yl)piperazin-1-yl)methyl)-1,5-naphthyridin-2(1H)-one was obtained by the preparation method referred to in Example 3.

MS m/z (ESI): 436.2 [M+H]⁺.

### Example 15

### 3-Ethyl-7-((4-(2-fluoro-3-(methylamino)thieno[3,2-b]pyridin-6-yl)piperazin-1-yl) methyl)-1,5-naphthyridin-2(1H)-one

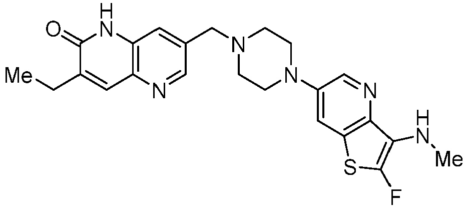

3-ethyl-7-((4-(2-fluoro-3-(methylamino)thieno[3,2-b]pyridin-6-yl)piperazin-1-yl) methyl)-1,5-naphthyridin-2(1H)-one was obtained by the preparation method referred to in Example 3.

MS m/z (ESI): 453.2 [M+H]⁺.

### Example 16

### 1'-((3-Ethyl-2-oxo-1,2,3,4-tetrahydropyrido[3,2-d]pyrimidin-7-yl)methyl)-N-methyl-1',2',3',6'-tetrahydro-[3,4'-bipyridine]-6-carboxamide

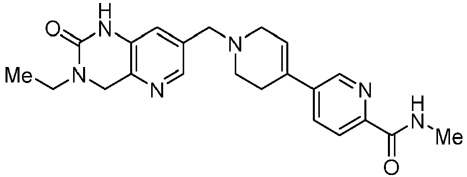

1'-((3-ethyl-2-oxo-1,2,3,4-tetrahydropyrido[3,2-d]pyrimidin-7-yl)methyl)-N-methyl-1', 2',3',6'-tetrahydro-[3,4'-bipyridine]-6-carboxamide was obtained by the preparation method referred to in Example 1.

MS m/z (ESI): 407.2 [M+H]⁺.

### Example 17

### 3-Ethyl-7-((4-(1-oxoisoindolin-5-yl)piperazin-1-yl)methyl)-1,5-naphthyridin-2(1H)-one

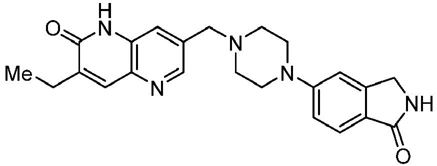

3-ethyl-7-((4-(1-oxoisoindolin-5-yl)piperazin-1-yl)methyl)-1,5-naphthyridin-2(1H)-one was obtained by the preparation method referred to in Example 3.

MS m/z (ESI): 404.2 [M+H]⁺.

### Example 18

### 3-Ethyl-7-((4-(1-oxo-1,2-dihydroisoquinolin-6-yl)piperazin-1-yl)methyl)-1,5-naphthyridin-2(1H)-one

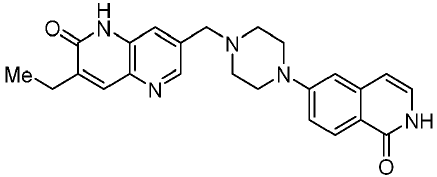

3-ethyl-7-((4-(1-oxo-1,2-dihydroisoquinolin-6-yl)piperazin-1-yl)methyl)-1,5-naphthyridin-2(1H)-one was obtained by the preparation method referred to in Example 3.

MS m/z (ESI): 416.2 [M+H]⁺.

### Example 19

### 3-Ethyl-7-((4-(1-oxo-1,2,3,4-tetrahydroisoquinolin-6-yl)piperazin-1-yl)methyl)-1,5-naphthyridin-2(1H)-one

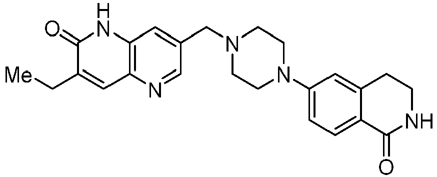

3-ethyl-7-((4-(1-oxo-1,2,3,4-tetrahydroisoquinolin-6-yl)piperazin-1-yl)methyl)-1,5-naphthyridin-2(1H)-one was obtained by the preparation method referred to in Example 3.

MS m/z (ESI): 418.2 [M+H]⁺.

### Example 20

### 3-Ethyl-7-((4-(1-oxo-1,2-dihydrophthalazin-6-yl)piperazin-1-yl)methyl)-1,5-naphth-yridin-2(1H)-one

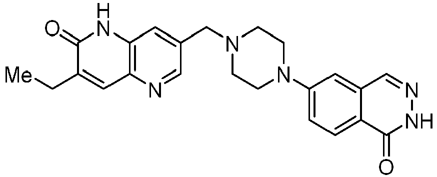

3-ethyl-7-((4-(1-oxo-1,2-dihydrophthalazin-6-yl)piperazin-1-yl)methyl)-1,5-naphth-yridin-2(1H)-one was obtained by the preparation method referred to in Example 3. MS m/z (ESI): 417.2 [M+H]⁺.

### Example 21

### 3-Ethyl-7-((4-(2-oxo-2,3-dihydro-1H-pyrrolo[2,3-b]pyridin-5-yl)piperazin-1-yl)methyl) -1,5-naphthyridin-2(1H)-one

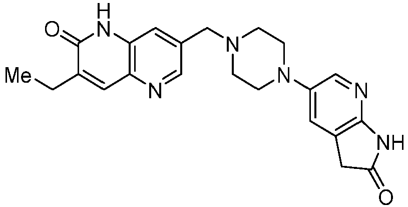

3-ethyl-7-((4-(2-oxo-2,3-dihydro-1H-pyrrolo[2,3-b]pyridin-5-yl)piperazin-1-yl)methyl)-1,5-naphthyridin-2(1H)-one was obtained by the preparation method referred to in Example 3.

MS m/z (ESI): 405.2 [M+H]⁺.

### Example 22

### 3-Ethyl-7-((4-(2-oxoindolin-6-yl)piperazin-1-yl)methyl)-1,5-naphthyridin-2(1H)-one

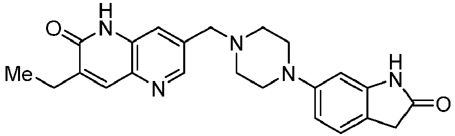

3-ethyl-7-((4-(2-oxoindolin-6-yl)piperazin-1-yl)methyl)-1,5-naphthyridin-2(1H)-one was obtained by the preparation method referred to in Example 3.

MS m/z (ESI): 404.2 [M+H]⁺.

### Example 23

### 7-((4-(1H-Pyrrolo[2,3-b]pyridin-5-yl)piperazin-1-yl)methyl)-3-ethyl-1,5-naphthyridin-2 (1H)-one

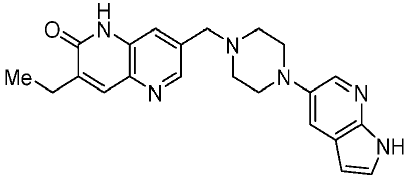

7-((4-(1H-pyrrolo[2,3-b]pyridin-5-yl)piperazin-1-yl)methyl)-3-ethyl-1,5-naphthyridin-2 (1H)-one was obtained by the preparation method referred to in Example 3.

MS m/z (ESI): 389.2 [M+H]⁺.

### Example 24

### 3-Ethyl-7-((4-(2-methyl-1H-benzo[d]imidazol-6-yl)piperazin-1-yl)methyl)-1,5-naphth-yridin-2(1H)-one

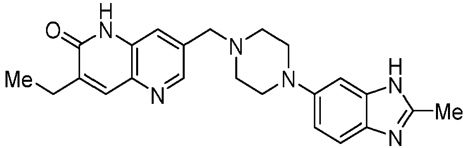

3-ethyl-7-((4-(2-methyl-1H-benzo[d]imidazol-6-yl)piperazin-1-yl)methyl)-1,5-naphth-yridin-2(1H)-one was obtained by the preparation method referred to in Example 3.
¹H NMR (400 MHz, DMSO-*d₆*) *δ* 11.83-11.90 (br s, 2H), 8.41 (s, 1H), 7.76 (s, 1H), 7.64 (s, 1H), 7.28 (d, *J* = 8.0 Hz, 1H), 6.89 (s, 1H), 6.83 (d, *J* = 8.0 Hz, 1H), 3.65 (s, 2H), 3.06-3.10 (m, 4H), 2.50-2.59 (m, 6H), 2.42 (s, 3H), 1.18 (t, *J=* 8.0 Hz, 3H);
MS m/z (ESI): 403.2 [M+H]⁺.

### Example 25

### 3-Ethyl-7-((4-(3-(methylamino)pyrazolo[1,5-a]pyrimidin-6-yl)piperazin-1-yl)methyl)-1,5-naphthyridin-2(1H)-one

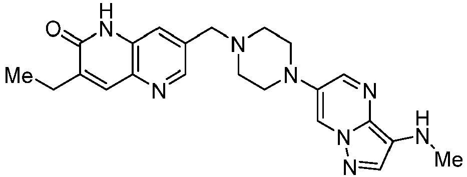

3-ethyl-7-((4-(3-(methylamino)pyrazolo[1,5-a]pyrimidin-6-yl)piperazin-1-yl)methyl)-1,5-naphthyridin-2(1H)-one was obtained by the preparation method referred to in Example 3.

MS m/z (ESI): 419.2 [M+H]⁺.

### Example 26

### 3-Ethyl-7-((4-(8-(methylamino)pyrrolo[1,2-a]pyrimidin-3-yl)piperazin-1-yl)methyl)-1,5-naphthyridin-2(1H)-one

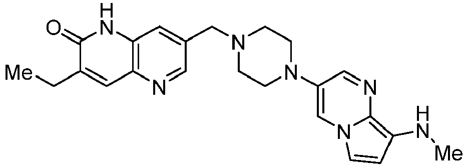

3-ethyl-7-((4-(8-(methylamino)pyrrolo[1,2-a]pyrimidin-3-yl)piperazin-1-yl)methyl)-1,5-naphthyridin-2(1H)-one was obtained by the preparation method referred to in Example 3.

MS m/z (ESI): 418.2 [M+H]⁺.

### Example 27

### 3-Ethyl-7-((4-(8-(methylamino)imidazo[1,5-a]pyrimidin-3-yl)piperazin-1-yl)methyl)-1,5-naphthyridin-2(1H)-one

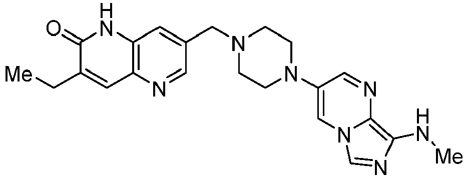

3-ethyl-7-((4-(8-(methylamino)imidazo[1,5-a]pyrimidin-3-yl)piperazin-1-yl)methyl)-1,5-naphthyridin-2(1H)-one was obtained by the preparation method referred to in Example 3.

MS m/z (ESI): 419.2 [M+H]⁺.

### Example 28

### 3-Ethyl-7-((4-(7-(methylamino)imidazo[1,5-b]pyridazin-3-yl)piperazin-1-yl)methyl)-1,5-naphthyridin-2(1H)-one

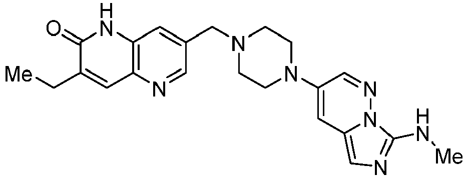

3-ethyl-7-((4-(7-(methylamino)imidazo[1,5-b]pyridazin-3-yl)piperazin-1-yl)methyl)-1,5-naphthyridin-2(1H)-one was obtained by the preparation method referred to in Example 3.

MS m/z (ESI): 419.2 [M+H]⁺.

### Example 29

### N-Cyclopropyl-1'-((7-ethyl-6-oxo-5,6-dihydro-1,5-naphthyridin-3-yl)methyl)-1',2',3',6'-tetrahydro-[3,4'-bipyridine]-6-carboxamide

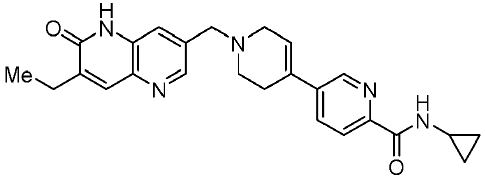

N-cyclopropyl-1'-((7-ethyl-6-oxo-5,6-dihydro-1,5-naphthyridin-3-yl)methyl)-1',2',3',6'-tetrahydro-[3,4'-bipyridine]-6-carboxamide was obtained by the preparation method referred to in Example 1.
¹H NMR (400 MHz, DMSO-*d₆*) *δ* 11.77 (s, 1H), 8.60 (d, *J=* 4.2 Hz, 2H), 8.30-8.43 (m, 1H), 7.85-8.01 (m, 2H), 7.69 (s, 1H), 7.58 (s, 1H), 6.34 (s, 1H), 3.65 (s, 2H), 3.09 (d, *J* = 3.5 Hz, 2H), 2.83 (q, *J =* 6.4, 5.2 Hz, 1H), 2.63 (t, *J =* 5.5 Hz, 2H), 2.48 (t, *J =* 7.2 Hz, 4H), 1.13 (q, *J =* 9.3, 7.4 Hz, 3H), 0.54-0.72 (m, 4H);
MS m/z (ESI): 430.2 [M+H]⁺.

### Example 30

### 1'-((7-Ethyl-6-oxo-5,6-dihydro-1,5-naphthyridin-3-yl)methyl)-N-methoxy-1',2',3',6'-tetrahydro-[3,4'-bipyridine]-6-carboxamide

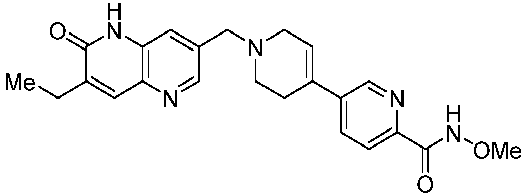

1'-((7-ethyl-6-oxo-5,6-dihydro-1,5-naphthyridin-3-yl)methyl)-N-methoxy-1',2',3',6'-tetrahydro-[3,4'-bipyridine]-6-carboxamide was obtained by the preparation method referred to in Example 1.
¹H NMR (400 MHz, DMSO-*d₆*) *δ* 11.92 (s, 1H), 11.78 (s, 1H), 8.61 (d, *J =* 2.2 Hz, 1H), 8.35 (d, *J=* 1.9 Hz, 1H), 7.94 (dd, *J* = 8.4, 2.3 Hz, 1H), 7.87 (d, *J=* 8.3 Hz, 1H), 7.69 (s, 1H), 7.58 (s, 1H), 6.36 (s, 1H), 3.64 (d, *J=* 13.4 Hz, 5H), 3.09 (d, *J=* 3.4 Hz, 2H), 2.64 (t, *J =* 5.6 Hz, 2H), 2.48 (d, *J =* 7.5 Hz, 4H), 1.12 (t, *J =* 7.4 Hz, 3H);
MS m/z (ESI): 420.2 [M+H]⁺.

### Example 31

### (S)-1'-((7-Ethyl-6-oxo-5,6-dihydro-1,5-naphthyridin-3-yl)methyl)-N-(tetrahydrofuran-3-yl)-1',2',3',6'-tetrahydro-[3,4'-bipyridine]-6-carboxamide

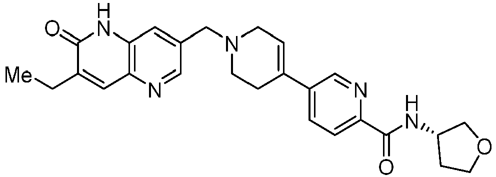

(S)-1'-((7-ethyl-6-oxo-5,6-dihydro-1,5-naphthyridin-3-yl)methyl)-N-(tetrahydrofuran-3-yl)-1',2',3',6'-tetrahydro-[3,4'-bipyridine]-6-carboxamide was obtained by the preparation method referred to in Example 1.

MS m/z (ESI): 460.2 [M+H]⁺.

### Example 32

### (R)-1'-((7-Ethyl-6-oxo-5,6-dihydro-1,5-naphthyridin-3-yl)methyl)-N-(tetrahydrofuran-3-yl)-1',2',3',6'-tetrahydro-[3,4'-bipyridine]-6-carboxamide

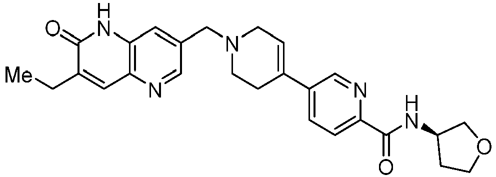

(R)-1'-((7-ethyl-6-oxo-5,6-dihydro-1,5-naphthyridin-3-yl)methyl)-N-(tetrahydrofuran-3-yl)-1',2',3',6'-tetrahydro-[3,4'-bipyridine]-6-carboxamide was obtained by the preparation method referred to in Example 1.

MS m/z (ESI): 460.2 [M+H]⁺.

### Example 33

### 4-(1-((7-Ethyl-6-oxo-5,6-dihydro-1,5-naphthyridin-3-yl)methyl)-1,2,3,6-tetrahydro-pyridin-4-yl)-2-fluoro-N-methylbenzamide

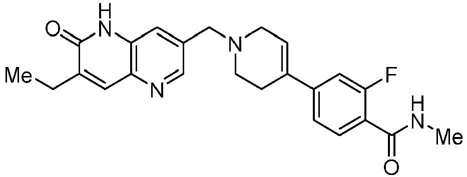

4-(1-((7-ethyl-6-oxo-5,6-dihydro-1,5-naphthyridin-3-yl)methyl)-1,2,3,6-tetrahydro-pyridin-4-yl)-2-fluoro-N-methylbenzamide was obtained by the preparation method referred to in Example 1.
¹H NMR (400 MHz, DMSO-*d₆*) *δ* 11.80-11.89 (m, 1H), 8.38-8.45 (m, 1H), 8.13-8.21 (m, 1H), 7.75 (s, 1H), 7.57-7.67 (m, 2H), 7.31-7.38 (m, 2H), 6.33-6.39 (m, 1H), 3.71 (s, 2H), 3.10-3.17 (m, 2H), 2.74-2.81 (m, 3H), 2.65-2.71 (m, 2H), 2.50-2.60 (m, 4H), 1.18 (t, *J* = 7.4 Hz, 3H);
MS m/z (ESI): 421.2 [M+H]⁺.

### Example 34

### 1'-((7-Ethyl-6-oxo-5,6-dihydro-1,5-naphthyridin-3-yl)methyl)-2-methoxy-N-methyl-1',2',3',6'-tetrahydro-[3,4'-bipyridine]-6-carboxamide

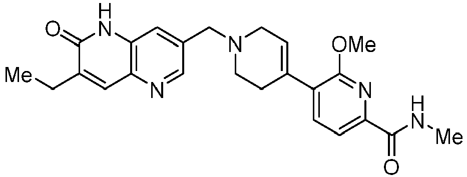

1'-((7-ethyl-6-oxo-5,6-dihydro-1,5-naphthyridin-3-yl)methyl)-2-methoxy-N-methyl-1',2',3',6'-tetrahydro-[3,4'-bipyridine]-6-carboxamide was obtained by the preparation method referred to in Example 1.

MS m/z (ESI): 434.2 [M+H]⁺.

### Example 35

### 2-Cyano-1'-((7-ethyl-6-oxo-5,6-dihydro-1,5-naphthyridin-3-yl)methyl)-N-methyl-1',2',3',6'-tetrahydro-[3,4'-bipyridine]-6-carboxamide

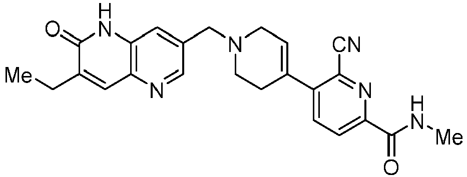

2-cyano-1'-((7-ethyl-6-oxo-5,6-dihydro-1,5-naphthyridin-3-yl)methyl)-N-methyl-1',2',3',6'-tetrahydro-[3,4'-bipyridine]-6-carboxamide was obtained by the preparation method referred to in Example 1.
¹H NMR (400 MHz, DMSO-*d₆*) *δ* 11.78 (s, 1H), 8.74 (q, *J* = 4.8 Hz, 1H), 8.36 (d, *J =* 1.8 Hz, 1H), 8.04-8.21 (m, 2H), 7.69 (s, 1H), 7.59 (d, *J* = 1.9 Hz, 1H), 6.17 (d, *J* = 4.1 Hz, 1H), 3.68 (s, 2H), 3.12 (d, *J* = 3.3 Hz, 2H), 2.76 (d, *J* = 4.8 Hz, 3H), 2.66 (t, *J* = 5.5 Hz, 2H), 2.48 (d, *J* = 7.0 Hz, 4H), 1.12 (t, *J* = 7.4 Hz, 3H);
MS m/z (ESI): 429.2 [M+H]⁺.

### Example 36

### 1'-((7-Ethyl-6-oxo-5,6-dihydro-1,5-naphthyridin-3-yl)methyl)-2-fluoro-N-methyl-1',2',3',6'-tetrahydro-[3,4'-bipyridine]-6-carboxamide

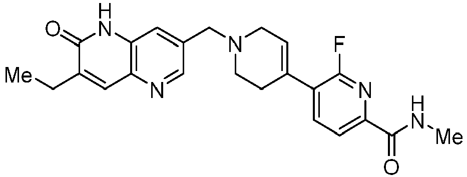

1'-((7-ethyl-6-oxo-5,6-dihydro-1,5-naphthyridin-3-yl)methyl)-2-fluoro-N-methyl-1',2',3',6'-tetrahydro-[3,4'-bipyridine]-6-carboxamide was obtained by the preparation method referred to in Example 1.
¹H NMR (400 MHz, DMSO-*d₆*) *δ* 11.78 (s, 1H), 8.57 (d, *J =* 5.0 Hz, 1H), 8.35 (d, *J* = 1.8 Hz, 1H), 8.02 (dd, *J* = 9.9, 7.7 Hz, 1H), 7.81-7.90 (m, 1H), 7.69 (s, 1H), 7.58 (s, 1H), 6.19 (s, 1H), 3.65 (s, 2H), 3.10 (s, 2H), 2.72 (d, *J =* 4.8 Hz, 3H), 2.62 (t, *J =* 5.6 Hz, 2H), 2.48 (dd, *J =* 10.3, 4.7 Hz, 4H), 1.12 (t, *J =* 7.4 Hz, 3H);
MS m/z (ESI): 422.2 [M+H]⁺.

### Example 37

### N-Methyl-1'-((2'-oxo-1',4'-dihydro-2'H-spiro[cyclopropane-1,3'-quinolin]-7'-yl) methyl)-1',2',3',6'-tetrahydro-[3,4'-bipyridine]-6-carboxamide

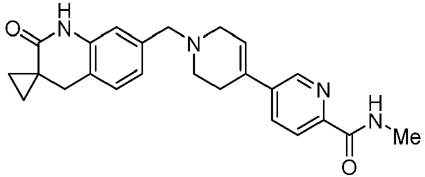

N-methyl-1'-((2'-oxo-1',4'-dihydro-2'H-spiro[cyclopropane-1,3'-quinolin]-7'-yl) methyl)-1',2',3',6'-tetrahydro-[3,4'-bipyridine]-6-carboxamide was obtained by the preparation method referred to in Example 1.

MS m/z (ESI): 403.2 [M+H]⁺.

### Example 38

### N-Methyl-1'-((2'-oxo-1',4'-dihydro-2'H-spiro[cyclopropane-1,3'-[1,5]naphthyridin]-7'-yl)methyl)-1',2',3',6'-tetrahydro-[3,4'-bipyridine]-6-carboxamide

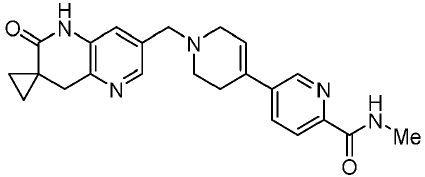

N-methyl-1'-((2'-oxo-1',4'-dihydro-2'H-spiro[cyclopropane-1,3'-[1,5]naphthyridin]-7'-yl)methyl)-1',2',3',6'-tetrahydro-[3,4'-bipyridine]-6-carboxamide was obtained by the preparation method referred to in Example 1.

MS m/z (ESI): 404.2 [M+H]⁺.

### Example 39

### (R)-N-Methyl-1'-((3-methyl-2-oxo-2,3-dihydro-1H-pyrrolo[3,2-b]pyridin-6-yl)methyl)-1',2',3',6'-tetrahydro-[3,4'-bipyridine]-6-carboxamide

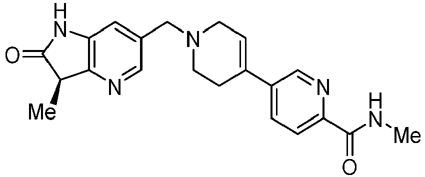

(R)-N-methyl-1'-((3-methyl-2-oxo-2,3-dihydro-1H-pyrrolo[3,2-b]pyridin-6-yl)methyl)-1',2',3',6'-tetrahydro-[3,4'-bipyridine]-6-carboxamide was obtained by the preparation method referred to in Example 1.

MS m/z (ESI): 378.2 [M+H]⁺.

### Example 40

### (S)-N-Methyl-1'-((3-methyl-2-oxo-2,3-dihydro-1H-pyrrolo[3,2-b]pyridin-6-yl)methyl)-1',2',3',6'-tetrahydro-[3,4'-bipyridine]-6-carboxamide

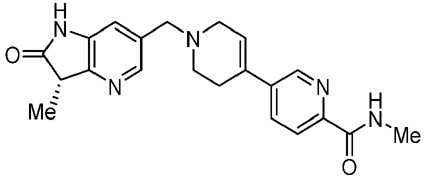

(S)-N-methyl-1'-((3-methyl-2-oxo-2,3-dihydro-1H-pyrrolo[3,2-b]pyridin-6-yl)methyl)-1',2',3',6'-tetrahydro-[3,4'-bipyridine]-6-carboxamide was obtained by the preparation method referred to in Example 1.

MS m/z (ESI): 378.2 [M+H]⁺.

### Example 41

### 2-Cyclopropyl-1'-((7-ethyl-6-oxo-5,6-dihydro-1,5-naphthyridin-3-yl)methyl)-N-methyl-1',2',3',6'-tetrahydro-[3,4'-bipyridine]-6-carboxamide

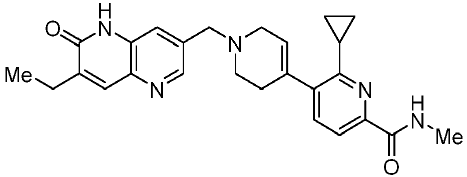

2-cyclopropyl-1'-((7-ethyl-6-oxo-5,6-dihydro-1,5-naphthyridin-3-yl)methyl)-N-methyl-1',2',3',6'-tetrahydro-[3,4'-bipyridine]-6-carboxamide was obtained by the preparation method referred to in Example 1.

MS m/z (ESI): 444.2 [M+H]⁺.

### Example 42

### 1'-((5-Cyano-3-ethyl-2-oxo-1,2-dihydroquinolin-7-yl)methyl)-N-methyl-1',2',3',6' -tetrahydro-[3,4'-bipyridine]-6-carboxamide

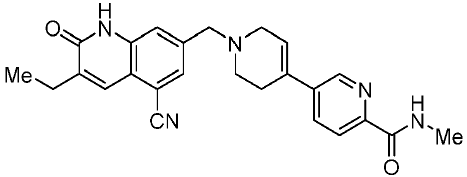

1'-((5-cyano-3-ethyl-2-oxo-1,2-dihydroquinolin-7-yl)methyl)-N-methyl-1',2',3',6'-tetrahydro-[3,4'-bipyridine]-6-carboxamide was obtained by the preparation method referred to in Example 1.
¹H NMR (400 MHz, DMSO-*d₆*) *δ* 12.10 (s, 1H), 8.66-8.70 (m, 2H), 7.96-8.04 (m, 2H), 7.76 (s, 1H), 7.68 (s, 1H), 7.62 (m, 1H), 6.43 (s, 1H), 3.72 (s, 2H), 3.16 (s, 2H), 2.78-2.82 (m, 3H), 2.66-2.74 (m, 2H), 2.55-2.62 (m, 4H), 1.19 (t, *J* = 7.6 Hz, 3H);
MS m/z (ESI): 428.2 [M+H]⁺.

### Example 43

### 1'-((3-Ethyl-5-fluoro-2-oxo-1,2-dihydroquinolin-7-yl)methyl)-N-methyl-1',2',3',6'-tetrahydro-[3,4'-bipyridine]-6-carboxamide

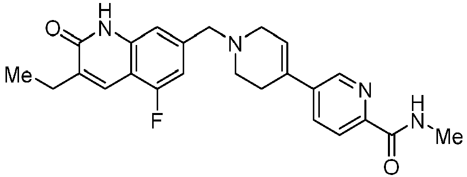

1'-((3-ethyl-5-fluoro-2-oxo-1,2-dihydroquinolin-7-yl)methyl)-N-methyl-1',2',3',6'-tetrahydro-[3,4'-bipyridine]-6-carboxamide was obtained by the preparation method referred to in Example 1.

MS m/z (ESI): 421.2 [M+H]⁺.

### Example 44

### 1'-((3-Ethyl-2-oxo-5-(trifluoromethyl)-1,2-dihydroquinolin-7-yl)methyl)-N-methyl-1',2',3',6'-tetrahydro-[3,4'-bipyridine]-6-carboxamide

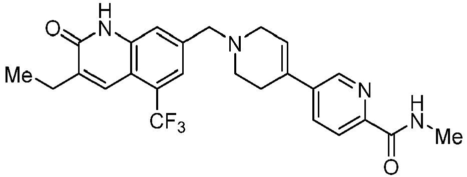

1'-((3-ethyl-2-oxo-5-(trifluoromethyl)-1,2-dihydroquinolin-7-yl)methyl)-N-methyl-1',2',3',6'-tetrahydro-[3,4'-bipyridine]-6-carboxamide was obtained by the preparation method referred to in Example 1.

MS m/z (ESI): 471.2 [M+H]⁺.

### Example 45

### 1'-((7-Cyclopropyl-6-oxo-5,6-dihydro-1,5-naphthyridin-3-yl)methyl)-N-methyl-1',2',3',6'-tetrahydro-[3,4'-bipyridine]-6-carboxamide

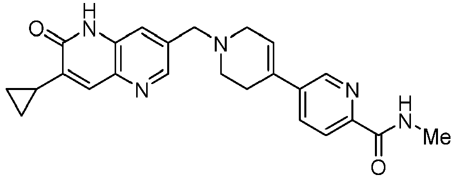

1'-((7-cyclopropyl-6-oxo-5,6-dihydro-1,5-naphthyridin-3-yl)methyl)-N-methyl-1',2',3',6'-tetrahydro-[3,4'-bipyridine]-6-carboxamide was obtained by the preparation method referred to in Example 1.
¹H NMR (400 MHz, DMSO-*d₆*) *δ* 11.83-11.91 (m, 1H), 8.67-8.73 (m, 2H), 8.38-8.41 (m, 1H), 7.95-8.02 (m, 2H), 7.60-7.64 (m, 1H), 7.42 (s, 1H), 6.39-6.45 (m, 1H), 3.71 (s, 2H), 3.12-3.19 (m, 2H), 2.78-2.86 (m, 3H), 2.68-2.74 (m, 2H), 2.53-2.59 (m, 2H), 2.10-2.19 (m, 1H), 0.94-1.00 (m, 2H), 0.80-0.85 (m, 2H);
MS m/z (ESI): 416.2 [M+H]⁺.

### Example 46

### 5-(1-((7-Ethyl-6-oxo-5,6-dihydro-1,5-naphthyridin-3-yl)methyl)-1,2,3,6-tetrahydro-pyridin-4-yl)-N-methylthiazole-2-carboxamide

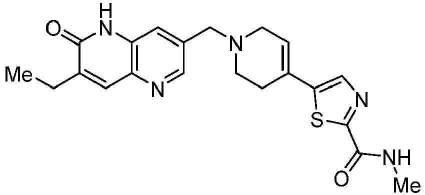

5-(1-((7-ethyl-6-oxo-5,6-dihydro-1,5-naphthyridin-3-yl)methyl)-1,2,3,6-tetrahydro-pyridin-4-yl)-N-methylthiazole-2-carboxamide was obtained by the preparation method referred to in Example 1.

MS m/z (ESI): 410.2 [M+H]⁺.

### Example 47

### 1'-((3-Ethyl-2,4-dioxo-1,2,3,4-tetrahydropyrido[3,2-d]pyrimidin-7-yl)methyl)-N-methyl-1',2',3',6'-tetrahydro-[3,4'-bipyridine]-6-carboxamide

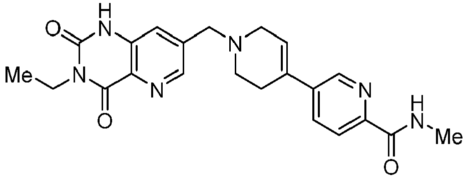

1'-((3-ethyl-2,4-dioxo-1,2,3,4-tetrahydropyrido[3,2-d]pyrimidin-7-yl)methyl)-N-methyl -1',2',3',6'-tetrahydro-[3,4'-bipyridine]-6-carboxamide was obtained by the preparation method referred to in Example 1.

MS m/z (ESI): 421.2 [M+H]⁺.

### Example 48

### 1'-((3-Ethyl-2,4-dioxo-1,2,3,4-tetrahydroquinazolin-7-yl)methyl)-N-methyl-1',2',3',6'-tetrahydro-[3,4'-bipyridine]-6-carboxamide

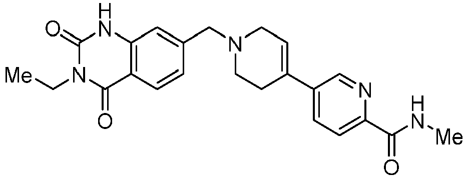

1'-((3-ethyl-2,4-dioxo-1,2,3,4-tetrahydroquinazolin-7-yl)methyl)-N-methyl-1',2',3',6'-tetrahydro-[3,4'-bipyridine]-6-carboxamide was obtained by the preparation method referred to in Example 1.

MS m/z (ESI): 420.2 [M+H]⁺.

### Example 49

### 1'-((7-Ethynyl-6-oxo-5,6-dihydro-1,5-naphthyridin-3-yl)methyl)-N-methyl-1',2',3',6'-tetrahydro-[3,4'-bipyridine]-6-carboxamide

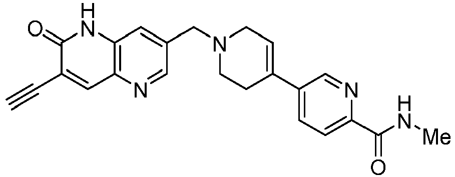

1'-((7-ethynyl-6-oxo-5,6-dihydro-1,5-naphthyridin-3-yl)methyl)-N-methyl-1',2',3',6'-tetrahydro-[3,4'-bipyridine]-6-carboxamide was obtained by the preparation method referred to in Example 1.

MS m/z (ESI): 400.2 [M+H]⁺.

### Example 50

### 3-(1-((7-Ethyl-6-oxo-5,6-dihydro-1,5-naphthyridin-3-yl)methyl)-1,2,3,6-tetrahydro-pyridin-4-yl)-4-methoxy-N,1-dimethyl-1H-pyrazole-5-carboxamide

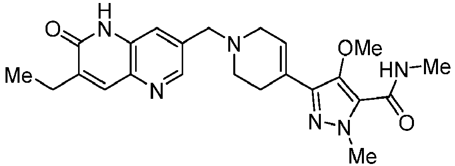

3-(1-((7-ethyl-6-oxo-5,6-dihydro-1,5-naphthyridin-3-yl)methyl)-1,2,3,6-tetrahydro-pyridin-4-yl)-4-methoxy-N,1-dimethyl-1H-pyrazole-5-carboxamide was obtained by the preparation method referred to in Example 1.

MS m/z (ESI): 437.2 [M+H]⁺.

### Example 51

### 4-(1-((7-Ethyl-6-oxo-5,6-dihydro-1,5-naphthyridin-3-yl)methyl)-1,2,3,6-tetrahydro-£pyridin-4-yl)-N,1-dimethyl-1H-imidazole-2-carboxamide

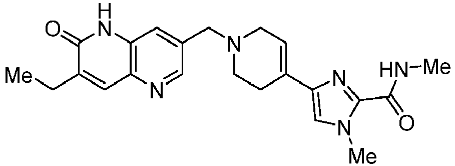

4-(1-((7-ethyl-6-oxo-5,6-dihydro-1,5-naphthyridin-3-yl)methyl)-1,2,3,6-tetrahydro-pyridin-4-yl)-N,1-dimethyl-1H-imidazole-2-carboxamide was obtained by the preparation method referred to in Example 1.

MS m/z (ESI): 407.2 [M+H]⁺.

### Example 52

### 3-Ethyl-7-((4-(8-(methylamino)imidazo[1,2-a]pyrazin-2-yl)-3,6-dihydropyridin-1(2H)-yl)methyl)-1,5-naphthyridin-2(1H)-one

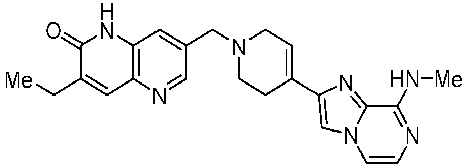

3-ethyl-7-((4-(8-(methylamino)imidazo[1,2-a]pyrazin-2-yl)-3,6-dihydropyridin-1(2H)-yl)methyl)-1,5-naphthyridin-2(1H)-one was obtained by the preparation method referred to in Example 1.

MS m/z (ESI): 416.2 [M+H]⁺.

### Example 53

### 7-((4-(1,5-Dimethyl-1H-imidazol-2-yl)-3,6-dihydropyridin-1(2H)-yl)methyl)-3-ethyl-1,5-naphthyridin-2(1H)-one

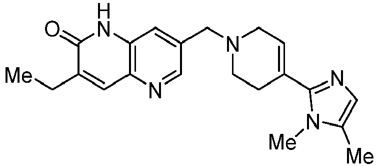

7-((4-(1,5-dimethyl-1H-imidazol-2-yl)-3,6-dihydropyridin-1(2H)-yl)methyl)-3-ethyl-1,5-naphthyridin-2(1H)-one was obtained by the preparation method referred to in Example 1.
¹H NMR (400 MHz, DMSO-*d₆*) *δ* 11.84 (s, 1H), 8.41 (s, 1H), 7.75 (s, 1H), 7.65 (s, 1H), 6.62 (t, *J* = 0.9 Hz, 1H), 5.93 (s, 1H), 3.70 (d, *J* = 2.7 Hz, 2H), 3.50 (s, 3H), 3.13 (t, *J =* 3.3 Hz, 2H), 2.62 (t, *J* = 5.5 Hz, 2H), 2.53-2.58 (m, 2H), 2.51-2.52 (m, 2H), 2.15 (s, 3H), 1.18 (t, *J* = 7.4 Hz, 3H);
MS m/z (ESI): 364.2 [M+H]⁺.

### Example 54

### 3-Ethyl-7-((4-(4-methyl-4H-1,2,4-triazol-3-yl)-3,6-dihydropyridin-1(2H)-yl)methyl)-1,5-naphthyridin-2(1H)-one

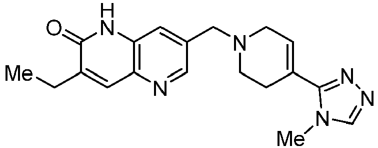

3-ethyl-7-((4-(4-methyl-4H-1,2,4-triazol-3-yl)-3,6-dihydropyridin-1(2H)-yl)methyl)-1,5-naphthyridin-2(1H)-one was obtained by the preparation method referred to in Example 1.
¹H NMR (400 MHz, DMSO-*d₆*) *δ* 11.75 (s, 1H), 8.48 (s, 2H), 7.75 (s, 1H), 7.60 (s, 1H), 6.25 (s, 1H), 3.75 (s, 2H), 3.57 (s, 3H), 3.10-3.25 (m, 2H), 2.61-2.74 (m, 2H), 2.51-2.60 (m, 4H), 1.20 (t, *J* = 7.4 Hz, 3H);
MS m/z (ESI): 351.2 [M+H]⁺.

### Example 55

### 1'-((7-Ethyl-6-oxo-5,6-dihydro-1,5-naphthyridin-3-yl)methyl)-N,2-dimethyl-1',2',3',6'-tetrahydro-[3,4'-bipyridine]-6-carboxamide

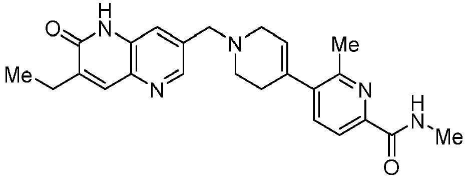

1'-((7-ethyl-6-oxo-5,6-dihydro-1,5-naphthyridin-3-yl)methyl)-N,2-dimethyl-1',2',3',6'-tetrahydro-[3,4'-bipyridine]-6-carboxamide was obtained by the preparation method referred to in Example 1.

MS m/z (ESI): 418.2 [M+H]⁺.

### Example 56

### 2-Chloro-1'-((7-ethyl-6-oxo-5,6-dihydro-1,5-naphthyridin-3-yl)methyl)-N-methyl-1',2',3',6'-tetrahydro-[3,4'-bipyridine]-6-carboxamide

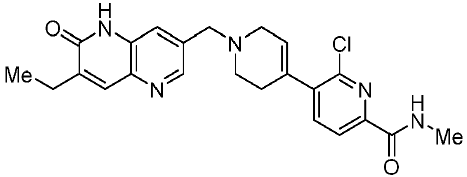

2-chloro-1'-((7-ethyl-6-oxo-5,6-dihydro-1,5-naphthyridin-3-yl)methyl)-N-methyl-1',2',3',6'-tetrahydro-[3,4'-bipyridine]-6-carboxamide was obtained by the preparation method referred to in Example 1.
¹H NMR (400 MHz, DMSO-*d₆*) *δ* 11.80 (s, 1H), 8.82 (d, *J =* 2.8 Hz, 1H), 8.70 (s,1H), 7.98 (d, *J =* 1.8 Hz, 1H), 7.90 (d, *J =* 1.8 Hz, 1H), 7.75 (s, 1H), 7.60 (s, 1H), 6.80 (s, 1H), 3.76 (s, 2H), 3.114-3.24 (m, 2H), 2.85 (d, *J* = 4.8 Hz, 3H), 2.65-2.70 (m, 2H), 2.43-2.61 (m, 4H), 1.20 (t, *J =* 7.2 Hz, 3H);
MS m/z (ESI): 438.2 [M+H]⁺.

### Example 57

### N-Cyano-1'-((7-ethyl-6-oxo-5,6-dihydro-1,5-naphthyridin-3-yl)methyl)-1',2',3',6'-tetrahydro-[3,4'-bipyridine]-6-carboxamide

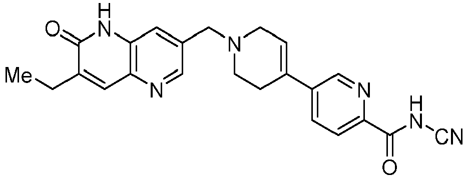

N-cyano-1' -((7-ethyl-6-oxo-5,6-dihydro-1,5-naphthyridin-3-yl)methyl)-1 ' ,2' ,3 ' ,6' - tetrahydro-[3,4'-bipyridine]-6-carboxamide was obtained by the preparation method referred to in Example 1.

MS m/z (ESI): 415.2 [M+H]⁺.

### Example 58

### N-(Cyanomethyl)-1'-((7-ethyl-6-oxo-5,6-dihydro-1,5-naphthyridin-3-yl)methyl)-1',2',3',6'-tetrahydro-[3,4'-bipyridine]-6-carboxamide

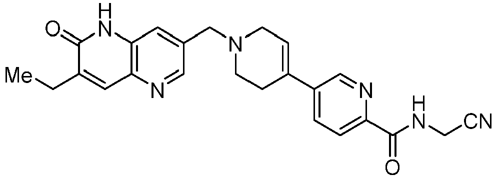

N-(cyanomethyl)-1'-((7-ethyl-6-oxo-5,6-dihydro-1,5-naphthyridin-3-yl)methyl)-1',2',3',6'-tetrahydro-[3,4'-bipyridine]-6-carboxamide was obtained by the preparation method referred to in Example 1.
¹H NMR (400 MHz, DMSO-*d₆*) *δ* 11.85 (s, 1H), 9.41-9.48 (m, 1H), 8.75 (s, 1H), 8.40 (s, 1H), 8.01-8.10 (m, 2H), 7.74 (s, 1H), 7.67 (s, 1H), 5.97 (s, 1H), 4.28-4.35 (m, 2H), 3.74 (s, 2H), 3.12-3.21 (m, 2H), 2.70-2.78 (m, 2H), 2.49-2.60 (m, 4H), 1.21 (t, *J =* 7.4 Hz, 3H);
MS m/z (ESI): 429.2 [M+H]⁺.

### Example 59

### N-(1-Cyanocyclopropyl)-1'-((7-ethyl-6-oxo-5,6-dihydro-1,5-naphthyridin-3-yl)methyl)-1',2',3',6'-tetrahydro-[3,4'-bipyridine]-6-carboxamide

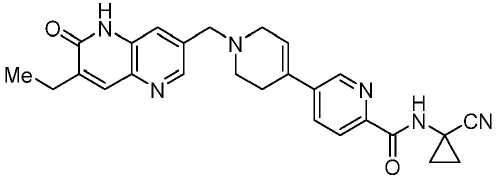

N-(1-cyanocyclopropyl)-1'-((7-ethyl-6-oxo-5,6-dihydro-1,5-naphthyridin-3-yl)methyl)-1',2',3',6'-tetrahydro-[3,4'-bipyridine]-6-carboxamide was obtained by the preparation method referred to in Example 1.
¹H NMR (400 MHz, DMSO-*d₆*) *δ* 11.84 (s, 1H), 9.67 (s, 1H), 8.71 (s, 1H), 8.41 (s, 1H), 8.00-8.04 (m, 2H), 7.76 (s, 1H), 7.65 (d, *J=* 5.6 Hz, 1H), 6.46 (s, 1H), 3.73 (s, 2H), 3.16 (d, *J=* 7.2 Hz, 2H), 2.70-2.73 (m, 2H), 2.53-2.57 (m, 4H), 1.51-1.54 (m, 2H), 1.38-1.40 (m, 2H), 1.22 (t, *J* = 7.2 Hz, 3H);
MS m/z (ESI): 455.2 [M+H]⁺.

### Example 60

### 1'-(1-(7-Ethyl-6-oxo-5,6-dihydro-1,5-naphthyridin-3-yl)cyclopropyl)-N-methyl-1',2',3',6'-tetrahydro-[3,4'-bipyridine]-6-carboxamide

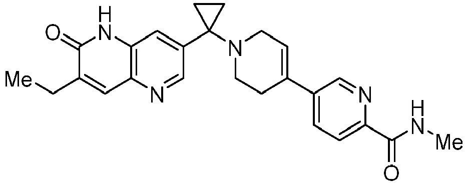

1'-(1-(7-ethyl-6-oxo-5,6-dihydro-1,5-naphthyridin-3-yl)cyclopropyl)-N-methyl-1',2',3',6'-tetrahydro-[3,4'-bipyridine]-6-carboxamide was obtained by the preparation method referred to in Example 1.

MS m/z (ESI): 430.2 [M+H]⁺.

### Example 61

### 1'-(2-(7-Ethyl-6-oxo-5,6-dihydro-1,5-naphthyridin-3-yl)propan-2-yl)-N-methyl-1',2',3',6'-tetrahydro-[3,4'-bipyridine]-6-carboxamide

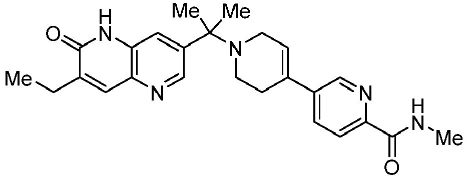

1'-(2-(7-ethyl-6-oxo-5,6-dihydro-1,5-naphthyridin-3-yl)propan-2-yl)-N-methyl-1',2',3',6'-tetrahydro-[3,4'-bipyridine]-6-carboxamide was obtained by the preparation method referred to in Example 1.

MS m/z (ESI): 432.2 [M+H]⁺.

### Example 62

### 1'-((6-Ethyl-7-oxo-7,8-dihydro-1,8-naphthyridin-2-yl)methyl)-N-methyl-1',2',3',6'-tetrahydro-[3,4'-bipyridine]-6-carboxamide

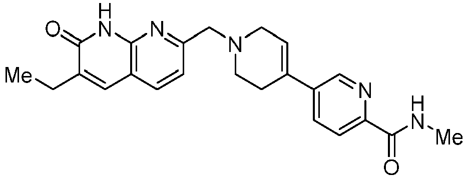

1'-((6-ethyl-7-oxo-7,8-dihydro-1,8-naphthyridin-2-yl)methyl)-N-methyl-1',2',3',6'-tetrahydro-[3,4'-bipyridine]-6-carboxamide was obtained by the preparation method referred to in Example 1.

MS m/z (ESI): 404.2 [M+H]⁺.

### Example 63

### N-Cyclopropyl-1'-((7-cyclopropyl-6-oxo-5,6-dihydro-1,5-naphthyridin-3-yl)methyl)-1',2',3',6'-tetrahydro-[3,4'-bipyridine]-6-carboxamide

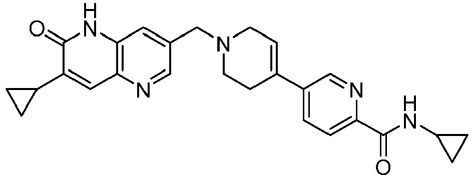

N-cyclopropyl-1'-((7-cyclopropyl-6-oxo-5,6-dihydro-1,5-naphthyridin-3-yl)methyl)-1',2',3',6'-tetrahydro-[3,4'-bipyridine]-6-carboxamide was obtained by the preparation method referred to in Example 1.

MS m/z (ESI): 442.2 [M+H]⁺.

### Example 64

### N-Methyl-1'-((6-oxo-7-(prop-1-yn-1-yl)-5,6-dihydro-1,5-naphthyridin-3-yl)methyl)-1',2',3',6'-tetrahydro-[3,4'-bipyridine]-6-carboxamide

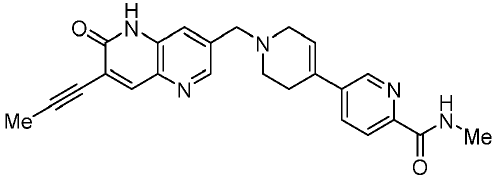

N-methyl-1'-((6-oxo-7-(prop-1-yn-1-yl)-5,6-dihydro-1,5-naphthyridin-3-yl)methyl)-1', 2',3',6'-tetrahydro-[3,4'-bipyridine]-6-carboxamide was obtained by the preparation method referred to in Example 1.

MS m/z (ESI): 414.2 [M+H]⁺.

### Example 65

### 3-Chloro-4-(1-((7-ethyl-6-oxo-5,6-dihydro-1,5-naphthyridin-3-yl)methyl)-1,2,3,6-tetrahydropyridin-4-yl)-2-fluoro-N-methylbenzamide

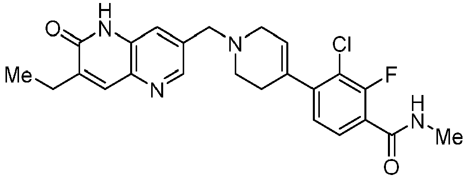

3-chloro-4-(1-((7-ethyl-6-oxo-5,6-dihydro-1,5-naphthyridin-3-yl)methyl)-1,2,3,6-tetrahydropyridin-4-yl)-2-fluoro-N-methylbenzamide was obtained by the preparation method referred to in Example 1.

MS m/z (ESI): 455.2 [M+H]⁺.

### Example 66

### 4-(1-((7-Ethyl-6-oxo-5,6-dihydro-1,5-naphthyridin-3-yl)methyl)-1,2,3,6-tetrahydro-pyridin-4-yl)-2-fluoro-N,3-dimethylbenzamide

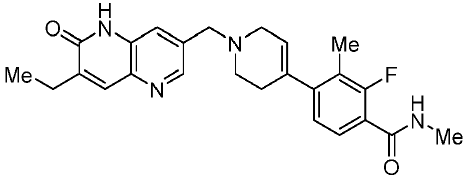

4-(1-((7-ethyl-6-oxo-5,6-dihydro-1,5-naphthyridin-3-yl)methyl)-1,2,3,6-tetrahydro-pyridin-4-yl)-2-fluoro-N,3-dimethylbenzamide was obtained by the preparation method referred to in Example 1.

MS m/z (ESI): 435.2 [M+H]⁺.

### Example 67

### 4-(1-((7-Ethyl-6-oxo-5,6-dihydro-1,5-naphthyridin-3-yl)methyl)-1,2,3,6-tetrahydro-pyridin-4-yl)-2,3-difluoro-N-methylbenzamide

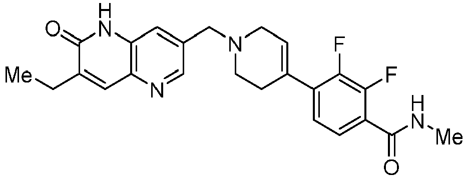

4-(1-((7-ethyl-6-oxo-5,6-dihydro-1,5-naphthyridin-3-yl)methyl)-1,2,3,6-tetrahydro-pyridin-4-yl)-2,3-difluoro-N-methylbenzamide was obtained by the preparation method referred to in Example 1.
¹H NMR (400 MHz, DMSO-*d₆*) *δ* 11.84 (s, 1H), 8.41(s, 1H), 8.34 (s, 1H), 7.76 (s, 1H), 7.65 (s, 1H), 7.35-7.39 (m, 1H), 7.22-7.26 (m, 1H), 6.13 (s, 1H), 3.72 (s, 2H), 3.14 (s, 2H), 2.74-2.81 (m, 3H), 2.64-2.71 (m, 2H), 2.50-2.62 (m, 3H), 2.33 (s, 1H), 1.18 (t, *J* = 7.4 Hz, 3H);
MS m/z (ESI): 439.2 [M+H]⁺.

### Example 68

### 3-Ethyl-7-((4-(2-methyl-8-(methylamino)-1,7-naphthyridin-3-yl)piperazin-1-yl)methyl) -1,5-naphthyridin-2(1H)-one

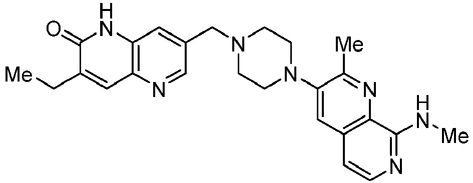

3-ethyl-7-((4-(2-methyl-8-(methylamino)-1,7-naphthyridin-3-yl)piperazin-1-yl)methyl)-1,5-naphthyridin-2(1H)-one was obtained by the preparation method referred to in Example 3.

MS m/z (ESI): 444.2 [M+H]⁺.

### Example 69

### 3-Ethyl-7-((4-(2-(methyl-d3)-8-(methylamino)- 1,7-naphthyridin-3-yl)piperazin-1-yl) methyl)-1,5-naphthyridin-2(1H)-one

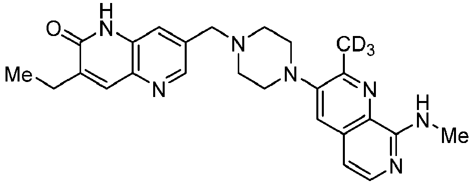

3-ethyl-7-((4-(2-(methyl-d3)-8-(methylamino)-1,7-naphthyridin-3-yl)piperazin-1-yl) methyl)-1,5-naphthyridin-2(1H)-one was obtained by the preparation method referred to in Example 3.

MS m/z (ESI): 447.2 [M+H]⁺.

### Example 70

### 3-Ethyl-7-((4-(2-fluoro-8-(methylamino)-1,7-naphthyridin-3-yl)piperazin-1-yl)methyl)-1,5-naphthyridin-2(1H)-one

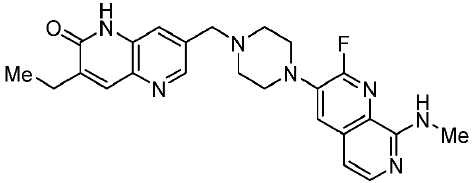

3-ethyl-7-((4-(2-fluoro-8-(methylamino)-1,7-naphthyridin-3-yl)piperazin-1-yl)methyl)-1,5-naphthyridin-2(1H)-one was obtained by the preparation method referred to in Example 3.

MS m/z (ESI): 448.2 [M+H]⁺.

### Example 71

### 7-((4-(2-Chloro-8-(methylamino)-1,7-naphthyridin-3-yl)piperazin-1-yl)methyl)-3-ethyl-1,5-naphthyridin-2(1H)-one

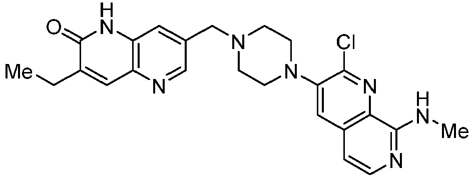

7-((4-(2-chloro-8-(methylamino)-1,7-naphthyridin-3-yl)piperazin-1-yl)methyl)-3-ethyl-1,5-naphthyridin-2(1H)-one was obtained by the preparation method referred to in Example 3.

MS m/z (ESI): 464.2 [M+H]⁺.

### Example 72

### 3-Ethyl-7-((4-(6-methyl-8-(methylamino)- 1,7-naphthyridin-3-yl)piperazin- 1-yl)methyl) -1,5-naphthyridin-2(1H)-one

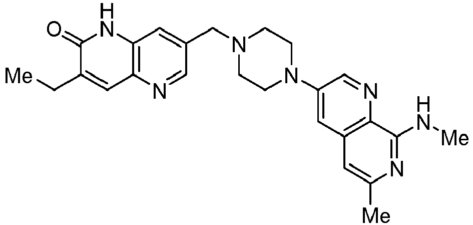

3-ethyl-7-((4-(6-methyl-8-(methylamino)-1,7-naphthyridin-3-yl)piperazin-1-yl)methyl)-1,5-naphthyridin-2(1H)-one was obtained by the preparation method referred to in Example 3.

MS m/z (ESI): 444.2 [M+H]⁺.

### Example 73

### 3-Ethyl-7-((4-(6-(methyl-d3)-8-(methylamino)- 1,7-naphthyridin-3-yl)piperazin-1-yl) methyl)-1,5-naphthyridin-2(1H)-one

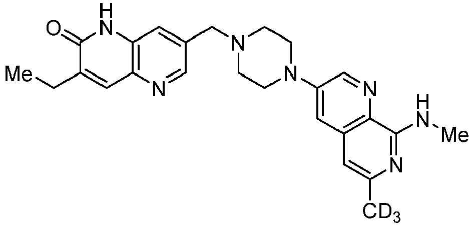

3-ethyl-7-((4-(6-(methyl-d3)-8-(methylamino)-1,7-naphthyridin-3-yl)piperazin-1-yl) methyl)-1,5-naphthyridin-2(1H)-one was obtained by the preparation method referred to in Example 3.

MS m/z (ESI): 447.2 [M+H]⁺.

### Example 74

### 3-Ethyl-7-((4-(6-fluoro-8-(methylamino)-1,7-naphthyridin-3-yl)piperazin-1-yl)methyl)-1,5-naphthyridin-2(1H)-one

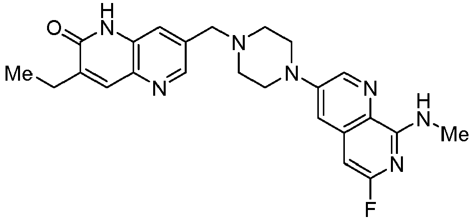

3-ethyl-7-((4-(6-fluoro-8-(methylamino)-1,7-naphthyridin-3-yl)piperazin-1-yl)methyl)-1,5-naphthyridin-2(1H)-one was obtained by the preparation method referred to in Example 3.

MS m/z (ESI): 448.2 [M+H]⁺.

### Example 75

### 7-((4-(6-Chloro-8-(methylamino)-1,7-naphthyridin-3-yl)piperazin-1-yl)methyl)-3-ethyl-1,5-naphthyridin-2(1H)-one

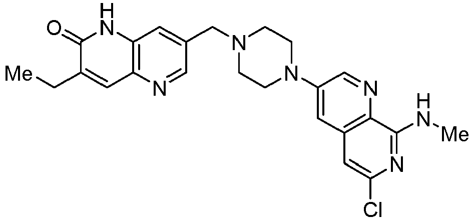

7-((4-(6-chloro-8-(methylamino)-1,7-naphthyridin-3-yl)piperazin-1-yl)methyl)-3-ethyl-1,5-naphthyridin-2(1H)-one was obtained by the preparation method referred to in Example 3.

MS m/z (ESI): 464.2 [M+H]⁺.

### Example 76

### 3-Ethyl-7-((4-(8-(methylamino)-1,7-naphthyridin-3-yl-6-d)piperazin-1-yl)methyl)-1,5-naphthyridin-2(1H)-one

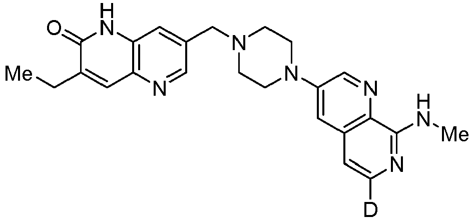

3-ethyl-7-((4-(8-(methylamino)-1,7-naphthyridin-3-yl-6-d)piperazin-1-yl)methyl)-1,5-naphthyridin-2(1H)-one was obtained by the preparation method referred to in Example 3.

MS m/z (ESI): 431.2 [M+H]⁺.

### Example 77

### 3-Ethyl-7-((4-(8-(methylamino)-1,7-naphthyridin-3-yl-4-d)piperazin-1-yl)methyl)-1,5-naphthyridin-2(1H)-one

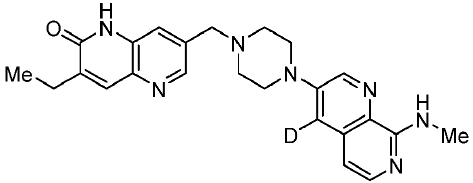

3-ethyl-7-((4-(8-(methylamino)-1,7-naphthyridin-3-yl-4-d)piperazin-1-yl)methyl)-1,5 -naphthyridin-2(1H)-one was obtained by the preparation method referred to in Example 3.

MS m/z (ESI): 431.2 [M+H]⁺.

### Example 78

### 3-Ethyl-7-((4-(8-(methylamino)-5H-pyrido[2,3-d][1,2]oxazin-3-yl)piperazin-1-yl) methyl)-1,5-naphthyridin-2(1H)-one

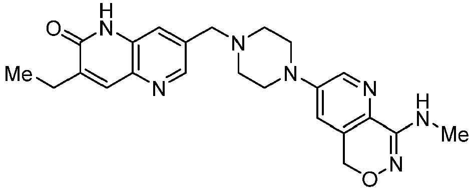

3-ethyl-7-((4-(8-(methylamino)-SH-pyrido[2,3-d] [1,2]oxazin-3-yl)piperazin-1-yl) methyl)-1,5-naphthyridin-2(1H)-one was obtained by the preparation method referred to in Example 3.

MS m/z (ESI): 434.2 [M+H]⁺.

### Example 79

### 5-(4-((7-Ethyl-6-oxo-5,6-dihydro-1,5-naphthyridin-3-yl)methyl)piperazin-1-yl)-2-methylisoindoline-1,3-dione

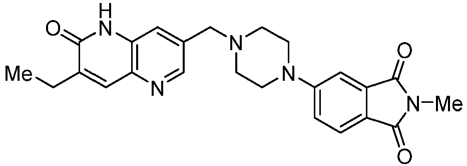

5-(4-((7-ethyl-6-oxo-5,6-dihydro-1,5-naphthyridin-3-yl)methyl)piperazin-1-yl)-2-methylisoindoline-1,3-dione was obtained by the preparation method referred to in Example 3.

MS m/z (ESI): 432.2 [M+H]⁺.

### Example 80

### 6-(4-((7-Ethyl-6-oxo-5,6-dihydro-1,5-naphthyridin-3-yl)methyl)piperazin-1-yl)-N-methylimidazo[1,5-a]pyridine-1-carboxamide

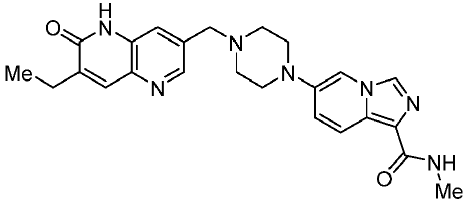

6-(4-((7-ethyl-6-oxo-5,6-dihydro-1,5-naphthyridin-3-yl)methyl)piperazin-1-yl)-N-methylimidazo[1,5-a]pyridine-1-carboxamide was obtained by the preparation method referred to in Example 3.

MS m/z (ESI): 446.2 [M+H]⁺.

### Example 81

### 1'-((7-Ethyl-6-oxo-5,6-dihydro-1,5-naphthyridin-3-yl)methyl)-5'-fluoro-N-methyl-1',2',3',6'-tetrahydro-[3,4'-bipyridine]-6-carboxamide

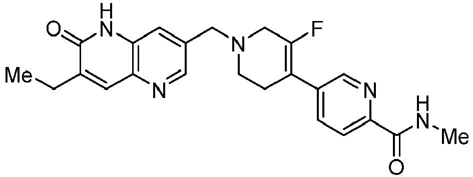

1'-((7-ethyl-6-oxo-5,6-dihydro-1,5-naphthyridin-3-yl)methyl)-5'-fluoro-N-methyl-1',2',3',6'-tetrahydro-[3,4'-bipyridine]-6-carboxamide was obtained by the preparation method referred to in Example 1.

MS m/z (ESI): 422.2 [M+H]⁺.

### Example 82

### 5-(1-((7-Ethyl-6-oxo-5,6-dihydro-1,5-naphthyridin-3-yl)methyl)-1,2,3,6-tetrahydro-pyridin-4-yl)-N-methylthiazole-2-carboxamide

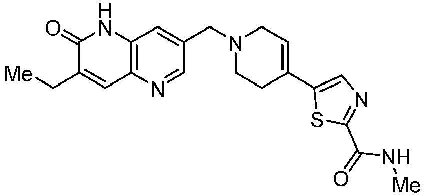

5-(1-((7-ethyl-6-oxo-5,6-dihydro-1,5-naphthyridin-3-yl)methyl)-1,2,3,6-tetrahydro-pyridin-4-yl)-N-methylthiazole-2-carboxamide was obtained by the preparation method referred to in Example 1.

MS m/z (ESI): 410.2 [M+H]⁺.

### Example 83

### 1'-((7-Ethyl-6-oxo-5,6-dihydro-1,5-naphthyridin-3-yl)methyl)-N-(2,2,2-trifluoroethyl)-1',2',3',6'-tetrahydro-[3,4'-bipyridine]-6-carboxamide

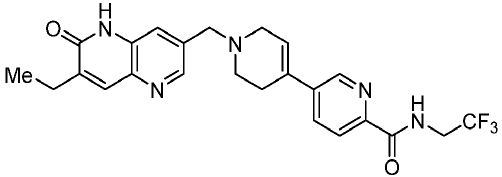

1'-((7-ethyl-6-oxo-5,6-dihydro-1,5-naphthyridin-3-yl)methyl)-N-(2,2,2-trifluoroethyl)-1',2',3',6'-tetrahydro-[3,4'-bipyridine]-6-carboxamide was obtained by the preparation method referred to in Example 1.

MS m/z (ESI): 472.2 [M+H]⁺.

### Example 84

### N-Methyl-1'-((6-oxo-7-(trifluoromethyl)-5,6-dihydro-1,5-naphthyridin-3-yl)methyl)-1',2',3',6'-tetrahydro-[3,4'-bipyridine]-6-carboxamide

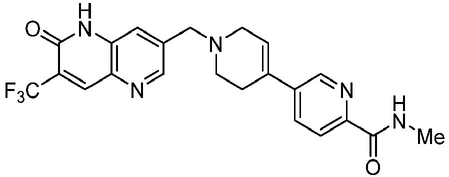

N-methyl-1'-((6-oxo-7-(trifluoromethyl)-5,6-dihydro-1,5-naphthyridin-3-yl)methyl)-1',2',3',6'-tetrahydro-[3,4'-bipyridine]-6-carboxamide was obtained by the preparation method referred to in Example 1.

MS m/z (ESI): 444.2 [M+H]⁺.

### Example 85

### 2-Chloro-1'-((3-ethyl-2-oxo-2,3-dihydro-1H-pyrido[2,3-b][1,4]oxazin-7-yl)methyl)-N-methyl-1',2',3',6'-tetrahydro-[3,4'-bipyridine]-6-carboxamide

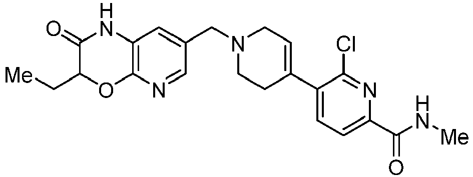

2-chloro-1'-((3-ethyl-2-oxo-2,3-dihydro-1H-pyrido[2,3-b][1,4]oxazin-7-yl)methyl)-N-methyl-1',2',3',6'-tetrahydro-[3,4'-bipyridine]-6-carboxamide was obtained by the preparation method referred to in Example 1.

MS m/z (ESI): 442.2 [M+H]⁺

### Example 86

### 1'-((3-Ethyl-2-oxo-2,3-dihydro-1H-pyrido[2,3-b] [1,4]oxazin-7-yl)methyl)-N,2-dimethyl- 1' ,2' ,3 ' ,6' -tetrahydro- [3,4' -bipyridine] -6-carboxamide

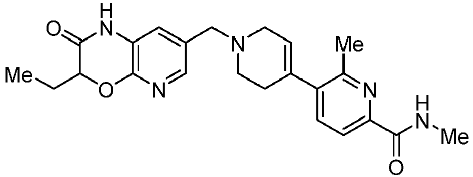

1'-((3-ethyl-2-oxo-2,3-dihydro-1H-pyrido[2,3-b] [1,4]oxazin-7-yl)methyl)-N,2-dimethyl-1',2',3',6'-tetrahydro-[3,4'-bipyridine]-6-carboxamide was obtained by the preparation method referred to in Example 1.

MS m/z (ESI): 422.2 [M+H]⁺.

### Example 87

### 2-Chloro-1'-((2-ethyl-3-oxo-3,4-dihydro-2H-benzo[b][1,4]oxazin-6-yl)methyl)-N-methyl-1',2',3',6'-tetrahydro-[3,4'-bipyridine]-6-carboxamide

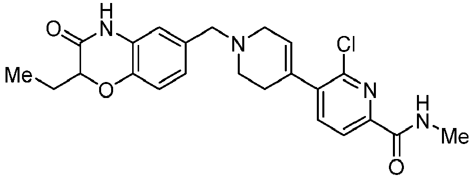

2-chloro-1'-((2-ethyl-3-oxo-3,4-dihydro-2H-benzo[b][1,4]oxazin-6-yl)methyl)-N-methyl-1',2',3',6'-tetrahydro-[3,4'-bipyridine]-6-carboxamide was obtained by the preparation method referred to in Example 1.
¹H NMR (400 MHz, DMSO-*d*₆) *δ* 10.62 (s, 1H), 8.61 (d, *J* = 2.8 Hz, 1H), 7.96 (d, *J* = 2.8 Hz, 1H), 7.89 (d, *J* = 2.8 Hz, 1H), 6.86-6.93 (m, 3H), 5.84 (s, 1H), 3.48 (m, 1H), 3.48-3.51 (m, 2H), 3.03-3.06 (m, 2H), 2.81 (d, *J* = 4.9, 3H), 2.63 (t, *J* = 8.0 Hz, 2H), 2.37-2.42 (m, 2H), 1.74-1.82 (m, 2H), 0.99 (t, *J* = 7.4 Hz, 3H);
MS m/z (ESI): 441.2 [M+H]⁺.

### Example 88

### 1'-((2-Ethyl-3-oxo-3,4-dihydro-2H-benzo[b] [1,4]oxazin-6-yl)methyl)-N,2-dimethyl-1',2',3',6'-tetrahydro-[3,4'-bipyridine]-6-carboxamide

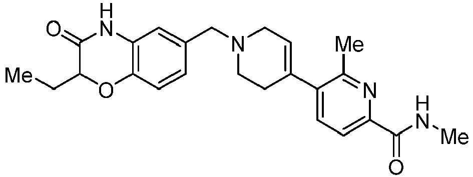

1'-((2-ethyl-3-oxo-3,4-dihydro-2H-benzo[b] [1,4]oxazin-6-yl)methyl)-N,2-dimethyl-1',2',3',6'-tetrahydro-[3,4'-bipyridine]-6-carboxamide was obtained by the preparation method referred to in Example 1.

MS m/z (ESI): 421.2 [M+H]⁺.

### Example 89

### 2-Chloro- I'-((3-cyclopropyl-2,4-dioxo- 1,2,3,4-tetrahydroquinazolin-7-yl)methyl)-N-methyl-1',2',3',6'-tetrahydro-[3,4'-bipyridine]-6-carboxamide

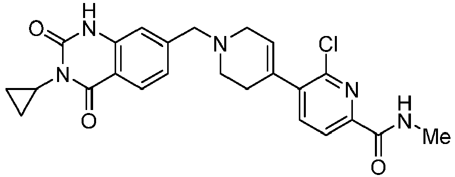

2-chloro-1'-((3-cyclopropyl-2,4-dioxo-1,2,3,4-tetrahydroquinazolin-7-yl)methyl)-N-methyl-1',2',3',6'-tetrahydro-[3,4'-bipyridine]-6-carboxamide was obtained by the preparation method referred to in Example 1.
¹H NMR (400 MHz, DMSO-*d*₆) *δ* 11.20 (s, 1H), 8.6 (d, *J* = 3.0 Hz, 1H), 7.90 (d, *J* = 2.8 Hz, 1H), 7.75-7.80 (m, 2H), 7.20 (s, 1H), 5.82 (s,1H), 3.64 (s, 2H), 3.02-3.15 (m, 2H), 2.75 (d, *J* = 4.8 Hz, 3H), 2.52-2.69 (m, 3H), 2.32-2.41 (m, 2H), 0.82-0.98 (m, 2H), 0.58-0.70 (m, 2H);
MS m/z (ESI): 466.2 [M+H]⁺.

### Example 90

### 1'-((3-Ethyl-2-oxo-2,3-dihydro-1H-pyrido[2,3-b] [1,4]oxazin-7-yl)methyl)-2-fluoro-N-methyl-1',2',3',6'-tetrahydro-[3,4'-bipyridine]-6-carboxamide

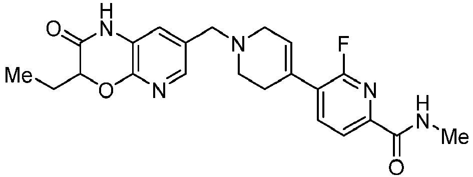

1'-((3-ethyl-2-oxo-2,3-dihydro-1H-pyrido[2,3-b] [1,4]oxazin-7-yl)methyl)-2-fluoro-N-methyl-1',2',3',6'-tetrahydro-[3,4'-bipyridine]-6-carboxamide was obtained by the preparation method referred to in Example 1.

MS m/z (ESI): 426.2 [M+H]⁺.

### Example 91

### 1'-((3-Ethyl-2,4-dioxo-1,2,3,4-tetrahydroquinazolin-7-yl)methyl)-2-fluoro-N-methyl-1',2',3',6'-tetrahydro-[3,4'-bipyridine]-6-carboxamide

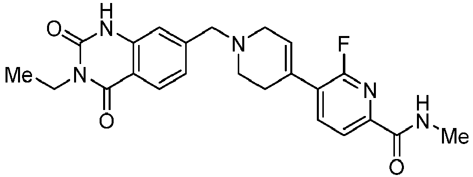

1'-((3-ethyl-2,4-dioxo-1,2,3,4-tetrahydroquinazolin-7-yl)methyl)-2-fluoro-N-methyl-1',2',3',6'-tetrahydro-[3,4'-bipyridine]-6-carboxamide was obtained by the preparation method referred to in Example 1.
¹H NMR (400 MHz, DMSO-*d*₆) *δ* 11.42 (s, 1H), 8.71 (s, 1H), 8.03-8.15 (m, 1H), 7.82-7.98 (m, 2H), 7.20-7.30 (m, 2H), 6.28 (s,1H), 3.84-3.95 (m, 2H), 3.65 (s, 2H), 3.13-3.22 (m, 2H), 2.75 (d, *J* = 4.8 Hz, 3H), 2.50-2.72 (m, 4H), 1.18 (t, *J* = 7.4 Hz, 3H);
MS m/z (ESI): 438.2 [M+H]⁺.

### Example 92

### 1'-((3-Ethyl-2,4-dioxo-1,2,3,4-tetrahydroquinazolin-7-yl)methyl)-N,2-dimethyl-1',2',3',6'-tetrahydro-[3,4'-bipyridine]-6-carboxamide

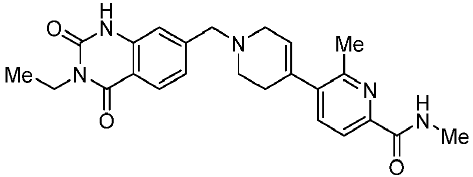

1'-((3-ethyl-2,4-dioxo-1,2,3,4-tetrahydroquinazolin-7-yl)methyl)-N,2-dimethyl-1',2',3',6'-tetrahydro-[3,4'-bipyridine]-6-carboxamide was obtained by the preparation method referred to in Example 1.
¹H NMR (400 MHz, DMSO-*d₆*) *δ* 11.38 (s, 1H), 8.50 (d, *J* = 2.8 Hz, 1H), 7.83 (d, *J =* 1.8 Hz, 1H), 7.75 (d, *J* = 2.0 Hz, 1H), 7.58 (d, *J* = 2.4 Hz, 1H), 7.10-7.21 (m, 2H), 5.60 (s, 1H), 3.79-3.92 (m, 2H), 3.60 (s, 2H), 3.11-3.15 (m, 2H), 2.75 (d, *J* = 5.0 Hz, 3H), 2.62-2.71 (m, 2H), 2.48 (s, 3H), 2.25-2.40 (m, 2H), 1.12 (t, *J* = 7.2 Hz, 3H);
MS m/z (ESI): 434.2 [M+H]⁺.

### Example 93

### 1'-(1-(7-Ethyl-6-oxo-5,6-dihydro-1,5-naphthyridin-3-yl)ethyl)-N-methyl-1',2',3',6'-tetrahydro-[3,4'-bipyridine]-6-carboxamide

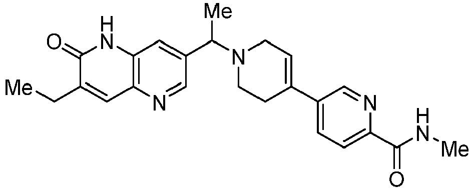

1'-(1-(7-ethyl-6-oxo-5,6-dihydro-1,5-naphthyridin-3-yl)ethyl)-N-methyl-1',2',3',6'-tetrahydro-[3,4'-bipyridine]-6-carboxamide was obtained by the preparation method referred to in Example 1.
¹H NMR (400 MHz, DMSO-*d₆*) *δ* 11.82 (s, 1H), 8.67-8.73 (m, 2H), 8.46 (s, 1H), 7.96 (d, *J* = 8.5 Hz, 2H), 7.76 (s, 1H), 7.64 (s, 1H), 6.43 (s, 1H), 3.72 (q, *J* = 6.8 Hz, 1H), 3.09-3.11 (m, 2H), 2.81 (d, *J* = 4.7 Hz, 3H), 2.64 (t, *J* = 10.0 Hz, 3H), 2.52-2.58 (m, 3H), 1.40 (d, *J* = 6.5 Hz, 3H), 1.18 (t, *J* = 7.4 Hz, 3H);
MS m/z (ESI): 418.2 [M+H]⁺.

### Example 94

### 3-Ethyl-7-((4-(5-fluoro-8-(methylamino)-1,7-naphthyridin-3-yl)piperazin-1-yl)methyl)-1,5-naphthyridin-2(1H)-one

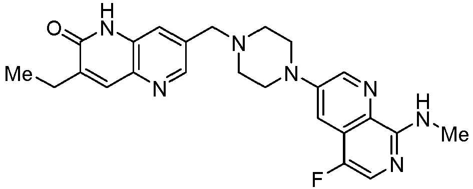

3-ethyl-7-((4-(5-fluoro-8-(methylamino)-1,7-naphthyridin-3-yl)piperazin-1-yl)methyl)-1,5-naphthyridin-2(1H)-one was obtained by the preparation method referred to in Example 3.
¹H NMR (400 MHz, DMSO-*d₆*) *δ* 11.85 (s, 1H), 8.71 (d, *J* = 2.7 Hz, 1H), 8.40 (d, *J* = 1.6 Hz, 1H), 7.78 (d, *J* = 2.2 Hz, 1H), 7.74 (s, 1H), 7.62 (s, 1H), 7.22-7.25 (m, 2H), 3.62-3.65 (m, 2H), 3.39-3.43 (m, 4H), 2.93 (d, *J* = 4.8 Hz, 3H), 2.54-2.58 (m, 4H), 2.49-2.52 (m, 2H), 1.17 (t, *J* = 7.4 Hz, 3H);
MS m/z (ESI): 448.2 [M+H]⁺.

### Example 95

### 1'-((7-Ethyl-6-oxo-5,6-dihydro-1,5-naphthyridin-3-yl)methyl)-N,5'-dimethyl-1',2',3',6'-tetrahydro-[3,4'-bipyridine]-6-carboxamide

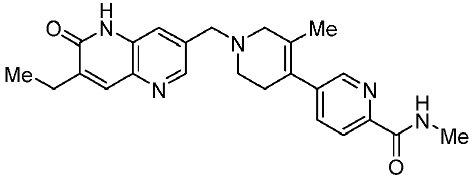

1'-((7-ethyl-6-oxo-5,6-dihydro-1,5-naphthyridin-3-yl)methyl)-N,5'-dimethyl-1',2',3',6'-tetrahydro-[3,4'-bipyridine]-6-carboxamide was obtained by the preparation method referred to in Example 1.

MS m/z (ESI): 418.2 [M+H]⁺.

### Example 96

### 5'-Chloro-1'-((7-ethyl-6-oxo-5,6-dihydro-1,5-naphthyridin-3-yl)methyl)-N-methyl-1',2',3',6'-tetrahydro-[3,4'-bipyridine]-6-carboxamide

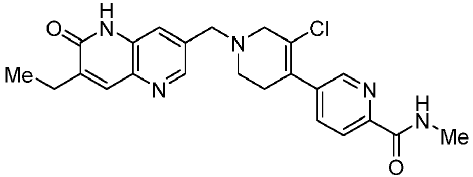

5'-chloro-1'-((7-ethyl-6-oxo-5,6-dihydro-1,5-naphthyridin-3-yl)methyl)-N-methyl-1',2',3',6'-tetrahydro-[3,4'-bipyridine]-6-carboxamide was obtained by the preparation method referred to in Example 1.

MS m/z (ESI): 438.2 [M+H]⁺.

### Example 97

### 3-Ethyl-7-((4-(8-(methylamino)-1,7-naphthyridin-3-yl-5-d)piperazin-1-yl)methyl)-1,5-naphthyridin-2(1H)-one

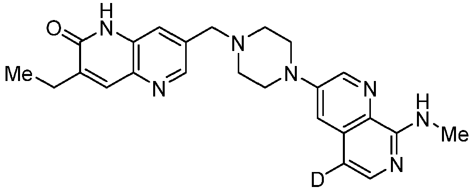

3-ethyl-7-((4-(8-(methylamino)-1,7-naphthyridin-3-yl-5-d)piperazin-1-yl)methyl)-1,5-naphthyridin-2(1H)-one was obtained by the preparation method referred to in Example 3.
¹H NMR (400 MHz, DMSO-*d₆*) *δ* 11.86 (s, 1H), 8.64 (d, *J* = 2.8 Hz, 1H), 8.42 (d, *J* = 1.7 Hz, 1H), 7.77 (d, *J* = 4.9 Hz, 2H), 7.64 (s, 1H), 7.29-7.32 (m, 2H), 3.64-3.67 (m, 2H), 2.95 (d, *J* = 4.9 Hz, 3H), 2.58-2.61 (m, 4H), 2.55 (d, *J* = 7.4 Hz, 2H), 2.51-2.53 (m, 4H), 1.19 (t, *J* = 7.4 Hz, 3H);
MS m/z (ESI): 431.2 [M+H]⁺.

### Example 98

### 3-Ethyl-7-((4-(8-((methyl-d3)amino)-1,7-naphthyridin-3-yl)piperazin-1-yl)methyl)-1,5-naphthyridin-2(1H)-one

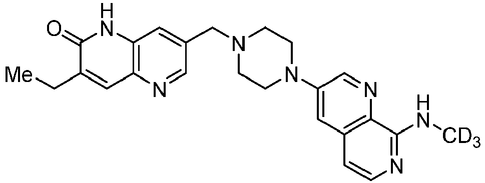

3-ethyl-7-((4-(8-((methyl-d3)amino)-1,7-naphthyridin-3-yl)piperazin-1-yl)methyl)-1,5-naphthyridin-2(1H)-one was obtained by the preparation method referred to in Example 3.
¹H NMR (400 MHz, DMSO-*d₆*) *δ* 11.79 (s, 1H), 8.57 (d, *J* = 2.7 Hz, 1H), 8.35 (s, 1H), 7.70 (d, *J* = 5.7 Hz, 2H), 7.57 (s, 1H), 7.23 (d, *J* = 2.6 Hz, 2H), 6.62 (d, *J* = 5.9 Hz, 1H), 3.55-3.60 (m, 2H), 3.25-3.31 (m, 4H), 2.47-2.53 (m, 4H), 2.48 (d, *J* = 7.4 Hz, 2H), 1.12 (t, *J* = 7.4 Hz, 3H);
MS m/z (ESI): 433.3 [M+H]⁺.

### Example 99

### 3-Ethyl-7-((4-(4-isopropyl-8-(methylamino)-1,7-naphthyridin-3-yl)piperazin-1-yl) methyl)-1,5-naphthyridin-2(1H)-one

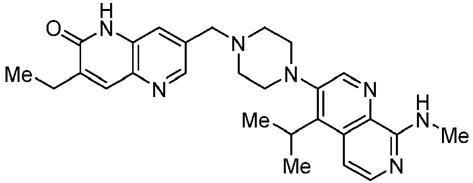

3-ethyl-7-((4-(4-isopropyl-8-(methylamino)-1,7-naphthyridin-3-yl)piperazin-1-yl) methyl)-1,5-naphthyridin-2(1H)-one was obtained by the preparation method referred to in Example 3.
¹H NMR (400 MHz, DMSO-*d*₆) *δ* 11.86 (s, 1H), 8.62 (s, 1H), 8.41 (s, 1H), 7.82 (d, *J* = 6.1 Hz, 1H), 7.74 (s, 1H), 7.61 (s, 1H), 7.48-7.54 (m, 1H), 7.05 (d, *J* = 6.1 Hz, 1H), 3.78-4.03 (m, 2H), 3.62-3.66 (m, 2H), 2.89-3.07 (m, 8H), 2.55-2.62 (m, 4H), 1.40 (d, *J* = 7.0 Hz, 6H), 1.16 (t, *J* = 7.3 Hz, 3H);
MS m/z (ESI): 472.2 [M+H]⁺.

### Example 100

### 3-Ethyl-7-((4-(2-isopropyl-8-(methylamino)-1,7-naphthyridin-3-yl)piperazin-1-yl) methyl)-1,5-naphthyridin-2(1H)-one

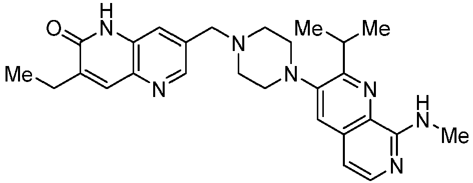

3-ethyl-7-((4-(2-isopropyl-8-(methylamino)-1,7-naphthyridin-3-yl)piperazin-1-yl) methyl)-1,5-naphthyridin-2(1H)-one was obtained by the preparation method referred to in Example 3.
¹H NMR (400 MHz, DMSO-*d*₆) *δ* 11.87 (s, 1H), 8.42 (s, 1H), 7.80 (d, *J* = 5.7 Hz, 1H), 7.75 (s, 1H), 7.63 (s, 1H), 7.60 (s, 1H), 7.21-7.24 (m, 1H), 6.74 (d, *J* = 5.8 Hz, 1H), 3.64-3.69 (m, 2H), 3.46-3.66 (m, 2H), 2.87-3.04 (m, 8H), 2.52-2.69 (m, 4H), 1.27 (d, *J* = 6.6 Hz, 6H), 1.18 (t, *J* = 7.4 Hz, 3H);
MS m/z (ESI): 472.2 [M+H]⁺.

### Example 101

### 3-Ethyl-7-((4-(5-methyl-8-(methylamino)-1,7-naphthyridin-3-yl)piperazin-1-yl)methyl) -1,5-naphthyridin-2(1H)-one

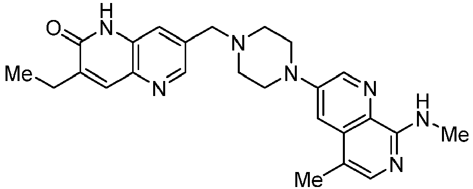

3-ethyl-7-((4-(5-methyl-8-(methylamino)-1,7-naphthyridin-3-yl)piperazin-1-yl)methyl)-1,5-naphthyridin-2(1H)-one was obtained by the preparation method referred to in Example 3.
¹H NMR (400 MHz, DMSO-*d*₆) *δ* 11.86 (s, 1H), 8.65 (d, *J* = 2.6 Hz, 1H), 8.42 (d, *J* = 1.6 Hz, 1H), 7.76 (s, 1H), 7.63-7.64 (m, 2H), 7.21 (d, *J* = 2.6 Hz, 1H), 7.15 (s, 1H), 3.64-3.68 (m, 2H), 3.39-3.42 (m, 4H), 2.93 (d, *J* = 4.8 Hz, 3H), 2.57-2.61 (m, 4H), 2.52-2.58 (m, 2H), 2.27 (s, 3H), 1.19 (t, *J* = 7.4 Hz, 3H);
MS m/z (ESI): 444.2 [M+H]⁺.

### Example 102

### 3-Ethyl-7-((4-(8-(methylamino)-1,7-naphthyridin-3-yl)-3,6-dihydropyridin-1(2H)-yl)-methyl)-1,5-naphthyridin-2(1H)-one

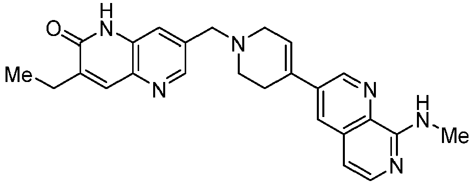

3-ethyl-7-((4-(8-(methylamino)-1,7-naphthyridin-3-yl)-3,6-dihydropyridin-1(2H)-yl) methyl)-1,5-naphthyridin-2(1H)-one was obtained by the preparation method referred to in Example 1.
¹H NMR (400 MHz, DMSO-*d*₆) *δ* 11.84 (s, 1H), 8.92 (d, *J* = 2.2 Hz, 1H), 8.43 (d, *J* = 1.7 Hz, 1H), 8.06 (d, *J* = 2.2 Hz, 1H), 7.90 (d, *J* = 5.8 Hz, 1H), 7.76 (s, 1H), 7.66 (s, 1H), 7.57 (q, *J* = 4.7 Hz, 1H), 6.84 (d, *J* = 5.8 Hz, 1H), 6.53 (s, 1H), 3.70-3.74 (m, 2H), 3.19 (d, *J* = 2.6 Hz, 2H), 2.99 (d, *J* = 4.9 Hz, 3H), 2.72-2.75 (m, 2H), 2.58-2.62 (m, 2H), 2.51-2.57 (m, 2H), 1.19 (t, *J* = 7.4 Hz, 3H);
MS m/z (ESI): 427.2 [M+H]⁺.

### Example 103

### 1'-(7-Ethyl-6-oxo-5,6-dihydro-1,5-naphthyridine-3-yl)-N-methyl-1',2',3',6'-tetrahydro-[3,4'-bipyridine]-6-carboxamide

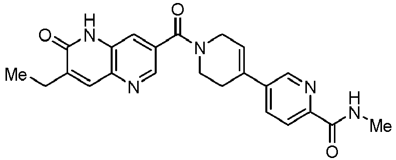

1'-(7-ethyl-6-oxo-5,6-dihydro-1,5-naphthyridine-3-yl)-N-methyl-1',2',3',6'-tetrahydro-[3,4'-bipyridine]-6-carboxamide was obtained by the preparation method referred to in Example 1.

MS m/z (ESI): 418.2 [M+H]⁺.

### Biological Assay and Evaluation

The present invention is further illustrated below in combination with the following test examples, which are not intended to limit the scope of the present invention.

### Test Example 1. Determination of the inhibitory activity of the compounds of the present invention on PARP1 enzyme

1. Experimental objective: The experimental objective of this Test Example is to determine the inhibitory activity of the compounds on PARP1 enzyme.
2. Experimental instruments:
   Centrifuge (Eppendorf 5810R)
   Microplate reader (BioTek Synergy H1 or PerkinElmer Envision) Pipette (Eppendorf or Rainin)
3. Experimental reagents:
   PARP1 Chemiluminescent Assay Kit, purchased from BPS bioscience, article number: 80569
   20×PBST, purchased from Thermo, article number: 28352
   PBS, purchased from Gibco, article number: 10010023
4. Experimental method:
   The inhibitory activity of the compounds on PARP1 enzyme was determined by the chemiluminescence method in the experiment. The experiment was carried out in a 384-well plate. Coating histones in a 384-well plate: 5×histone solution was diluted 5 folds with PBS, added to a 384-well ELISA plate (25 µL per well), and incubated at 4°C overnight. The coated ELISA plate was rinsed with 1×PBST buffer, blocked with the blocking buffer (Blocking buffer 3 in the kit, 100 µL per well) for 30 to 120 minutes, and rinsed with 1×PBST buffer 3 to 6 times. A mixture of PARP reaction biotin-labeled substrate, activated DNA, 10×PARP buffer and water was added (12.5 µL per well). Compound solutions with different concentrations were formulated using the experimental buffer (10% DMSO aqueous solution containing 1.25 mM DTT). The final detection concentration started from 100 nM, with 3-fold dilution and 8 concentrations. The compound solutions were added to the reaction wells of the 384-well plate (2.5 µL per well). 2.5 µL of 10% DMSO aqueous solution containing 1.25 mM DTT was added to the positive control well and blank well (2.5 µL per well). 10 µL of PARP1 enzyme solution formulated with 1× PARP buffer was added to start the reaction. The plate was centrifuged at 1000 rpm for 1 minute, and reacted at room temperature for 60 minutes. After completion of the reaction, the reaction solution was poured off, and the plate was rinsed with 1×PBST buffer. Streptavidin-HRP solution diluted 50 folds with Blocking buffer 3 was added (25 µL per well), and the plate was incubated at room temperature for 30 minutes. The reaction solution was poured off, and the plate was rinsed with 1×PBST buffer 3 to 6 times. The luminescence reaction solution obtained by mixing ECL substrate A and ELISA ECL substrate B (1:1) was added (50 µL per well) for reaction. The chemiluminescence values were measured immediately with a BioTek Synergy H1 or Envision instrument.
5. Processing method of the experimental data:
   The chemiluminescence values were measured with a BioTek Synergy H1 or Envision instrument. The inhibition rate was calculated, and the concentration and inhibition rate were fitted to a nonlinear regression curve using Graphpad Prism software to obtain the IC₅₀ value.

The inhibitory activity of some compounds of the examples of the present invention on PARP1 enzyme is shown in the following table:

| Example | PARP1 IC₅₀ (nM) |
|---|---|
| Example 1 | 0.93 |
| Example 2 | 0.76 |
| Example 3 | 0.40 |
| Example 6 | 2.90 |
| Example 17 | 4.50 |
| Example 23 | 3.30 |
| Example 24 | 1.70 |
| Example 29 | 0.98 |
| Example 30 | 1.40 |
| Example 31 | 1.00 |
| Example 32 | 1.00 |
| Example 33 | 0.91 |
| Example 34 | 1.60 |
| Example 35 | 1.00 |
| Example 36 | 0.80 |
| Example 42 | 1.30 |
| Example 45 | 0.80 |
| Example 46 | 0.60 |
| Example 53 | 0.96 |
| Example 56 | 1.30 |
| Example 57 | 2.80 |
| Example 58 | 0.90 |
| Example 59 | 0.80 |
| Example 65 | 2.20 |
| Example 66 | 1.70 |
| Example 67 | 2.10 |
| Example 79 | 3.30 |
| Example 80 | 5.00 |
| Example 81 | 0.90 |
| Example 82 | 0.60 |
| Example 83 | 1.40 |
| Example 84 | 2.70 |
| Example 85 | 3.50 |
| Example 86 | 5.10 |
| Example 87 | 4.90 |
| Example 88 | 5.00 |
| Example 90 | 2.70 |
| Example 91 | 3.00 |
| Example 93 | 2.10 |

### 6. Experimental conclusion:

The compounds of the present invention show excellent biological activity in the PARP1 enzyme inhibition experiment.

### Test Example 2. Determination of the inhibitory activity of the compounds of the present invention on PARP2 enzyme

1. Experimental objective: The experimental objective of this Test Example is to determine the inhibitory activity of the compounds on PARP2 enzyme.
2. Experimental instruments:
   Centrifuge (Eppendorf 5810R)
   Microplate reader (BioTek Synergy H1 or PerkinElmer Envision) Pipette (Eppendorf or Rainin)
3. Experimental reagents:
   PARP2 Chemiluminescent Assay Kit, purchased from BPS bioscience, article number: 80552
   20×PBST, purchased from Thermo, article number: 28352
   PBS, purchased from Gibco, article number: 10010023
4. Experimental method:
   The inhibitory activity of the compounds on PARP2 enzyme was determined by the chemiluminescence method in the experiment. The experiment was carried out in a 96-well plate. Coating histones in a 96-well plate: 5×histone solution was diluted 5 folds with PBS, added to a 96-well ELISA plate (50 µL per well), and incubated at 4°C overnight. The coated ELISA plate was rinsed with 1×PBST buffer, blocked with the blocking buffer (Blocking buffer 3 in the kit, 200 µL per well) for 30 to 120 minutes, and rinsed with 1×PBST buffer 3 to 6 times. A mixture of PARP reaction biotin-labeled substrate, activated DNA, 10×PARP buffer and water was added (25 µL per well). Compound solutions with different concentrations were formulated using the experimental buffer (10% DMSO aqueous solution containing 1.25 mM DTT). The final detection concentration started from 10 µM, with 3-fold dilution and 8 concentrations. The compound solutions were added to the reaction wells of the 96-well plate (5 µL per well). 5 µL of 10% DMSO aqueous solution containing 1.25 mM DTT was added to the positive control well and blank well (5 µL per well). 20 µL of PARP2 enzyme solution formulated with 1× PARP buffer was added to start the reaction. The plate was centrifuged at 1000 rpm for 1 minute, and reacted at room temperature for 60 minutes. After completion of the reaction, the reaction solution was poured off, and the plate was rinsed with 1×PBST buffer. Streptavidin-HRP solution diluted 50 folds with Blocking buffer 3 was added (50 µL per well), and the plate was incubated at room temperature for 30 minutes. The reaction solution was poured off, and the plate was rinsed with 1×PBST buffer 3 to 6 times. The luminescence reaction solution obtained by mixing ECL substrate A and ELISA ECL substrate B (1:1) was added (100 µL per well) for reaction. The chemiluminescence values were measured immediately with a BioTek Synergy H1 or Envision instrument.
5. Processing method of the experimental data:
   The chemiluminescence values were measured with a BioTek Synergy H1 or Envision instrument. The inhibition rate was calculated, and the concentration and inhibition rate were fitted to a nonlinear regression curve using Graphpad Prism software to obtain the IC₅₀ value.
6. Experimental conclusion:
   The compounds of the present invention show high selectivity for PARP2 in the PARP2 enzyme inhibitory activity experiment.

### Test Example 3. Determination of the inhibitory effect of the compounds of the present invention on the proliferation activity of BRCA2 Knockout DLD-1 cells

1. Experimental objective: The experimental objective of this Test Example is to determine the inhibitory effect of the compounds on the proliferation activity of BRCA2 Knockout DLD-1 cells.
2. Experimental instruments:
   Centrifuge (Eppendorf 5810R)
   Microplate reader (BioTek Synergy H1 or PerkinElmer Envision) Pipette (Eppendorf or Rainin)
3. Experimental reagents:
   BRCA2 Knockout DLD-1 cells, purchased from Creative Biogene
   Cell Titer-Glo, purchased from Promega, article number: G7573
   RPMI 1640, purchased from Gibco, article number: 22400089
   FBS, purchased from Gibco, article number: 10091148
   PBS, purchased from Gibco, article number: 10010023
   Trypsin, purchased from Gibco, article number: 25200056
   Cell culture plate, purchased from Corning, article number: 3610
4. Experimental method:
   BRCA2 Knockout DLD-1 cells were cultured using RPMI1640 culture medium containing 10% FBS to an appropriate cell density. The cells were collected, and adjusted to an appropriate cell concentration using the complete culture medium. The cell suspension was spread on a 96-well plate (90 µL per well), and placed in a 37°C, 5% CO₂ incubator overnight. Compound solutions of different concentrations were formulated using DMSO and culture medium, and vehicle control was set up. The compound solutions were added to the 96-well plate (10 µL per well), and the plate was incubated in a 37°C, 5% CO₂ incubator for about 144 hours. CellTiter-Glo solution was added, and the plate was shaken to mix evenly, and incubated in the dark for 10 to 30 minutes. The values were measured with a Synergy H1 or Envision microplate reader.
5. Processing method of the experimental data:
   The inhibition rate was calculated using the luminescence signal value. The concentration and inhibition rate were fitted to a nonlinear regression curve using Graphpad Prism software to obtain the IC₅₀ value.

The inhibitory effect of some compounds of the examples of the present invention on the proliferation activity of BRCA2 Knockout DLD-1 cells is shown in the following table:

| Example | IC₅₀ (nM) |
|---|---|
| Example 1 | 1.2 |
| Example 29 | 1.9 |
| Example 30 | 3.2 |
| Example 35 | 2.3 |
| Example 36 | 1.6 |
| Example 42 | 3.0 |
| Example 45 | 3.5 |
| Example 53 | 7.5 |
| Example 56 | 1.0 |
| Example 58 | 2.4 |
| Example 59 | 2.7 |
| Example 72 | 4.8 |
| Example 81 | 1.5 |
| Example 83 | 1.4 |
| Example 84 | 5.1 |
| Example 93 | 3.6 |
| Example 94 | 8.4 |
| Example 98 | 1.7 |
| Example 102 | 5.2 |

### 6. Experimental conclusion:

The compounds of the present invention show excellent biological activity in the BRCA2 Knockout DLD-1 cell proliferation activity inhibition experiment.

### Test Example 4. Bidirectional permeability test of the compounds through Caco-2 cell model

### 1. Experimental objective:

The objective of this experiment is to determine the bidirectional permeability of the compounds through Caco-2 cell model.

### 2. Experimental instruments and materials:

Liquid chromatography-mass spectrometer, centrifuge, vortexer, pipette, 24-well test plate, acetonitrile solution containing internal standard, Caco-2 cells (ATCC), Hank's balanced salt solution (HBSS), dimethyl sulfoxide (DMSO)

### 3. Experimental process:

1) Cultivation of Caco-2 monolayer cells: Caco-2 cells in good condition were spread on a plate, and cultured for 21 to 28 days (the culture medium was replaced every 2 to 3 days) to form a dense cell monolayer for permeability test.
2) Evaluation of the permeability of the test compounds:
   a. 100 µL of transport buffer (HBSS containing 10 µM test compound, 0.5% BSA, and 0.5% DMSO) was added to the administration end of A to B
   b. 300 µL of transport buffer (HBSS containing 0.5% BSA) was added to the receiving end of A to B
   c. 300 µL of transport buffer (HBSS containing 10 µM test compound, 0.5% BSA, and 0.5% DMSO) was added to the administration end of B to A
   d. 100 µL of transport buffer (HBSS containing 0.5% BSA) was added to the receiving end of B to A
   e. Incubation for 2 hours
   d. Samples were taken, processed and detected by mass spectrometry.

### 4. Chromatographic conditions:

Instrument: liquid chromatography;
Chromatographic column: Waters XSelect HSS T3 C18 (2.1*50 mm, 2.5 µm);
Mobile phase: phase A: water containing 0.1% formic acid; phase B: acetonitrile containing 0.1% formic acid.

### 5. Mass spectrometry conditions:

Instrument: API4000 liquid chromatography-mass spectrometer;
Ion source: electrospray ionization source (ESI);
Detection method: positive ion detection;
Scan mode: multiple reaction monitoring (MRM) mode.

### 6. Experimental results:

The bidirectional permeability of the compounds of the examples of the present invention through the Caco-2 cell model is shown in the table below:

| Example No. | Pₐₚₚ(10⁻⁶ cm·s⁻¹) | | Pₐₚₚ(B-A)/ Pₐₚₚ(A-B) |
|---|---|---|---|
| | A-B | B-A | |
| 1 | 12.77 | 19.23 | 1.5 |
| 2 | 9.42 | 13.47 | 1.4 |
| 3 | 7.15 | 24.12 | 3.4 |
| 29 | 12.20 | 19.00 | 1.6 |
| 35 | 5.19 | 15.69 | 3.0 |
| 36 | 14.76 | 15.62 | 1.1 |
| 45 | 9.80 | 15.35 | 1.6 |
| 72 | 5.23 | 12.71 | 2.5 |

### 7. Experimental conclusion:

It can be seen from the experimental results in the above table that the compounds of the examples of the present invention have high permeability.

### Test Example 5. Pharmacokinetic assay in Balb/C mice

### 1. Experimental objective:

Balb/C mice were used as test animals. The pharmacokinetic behavior of the compounds of Examples was studied in mouse body (plasma) by orally administration at a dose of 1 mg/kg.

### 2. Experimental protocol

### 2.1 Test compounds:

Compounds of the examples of the present invention, prepared by the applicant.

### 2.2 Test animals:

Male Balb/C mice (6 mice per example), purchased from Shanghai Jiesijie Laboratory Animal Co., LTD, with Certificate No.: SCXK (Shanghai) 2013-0006 N0.311620400001794.

### 2.3 Formulation of the compound:

5 g of hydroxyethyl cellulose (HEC, CMC-Na, viscosity: 800-1200 Cps) was weighed and dissolved in 1000 mL of purified water, followed by the addition of 10 g of Tween 80. The mixture was mixed well to obtain a clear solution.

2.05 mg of the compound was weighed and dissolved in the solution. The mixture was shaken well, crushed in a cell crusher for 1 minute, and sonicated for 15 minutes to obtain a suspension with a concentration of 0.1 mg/mL.

### 2.4 Administration:

After an overnight fast, male Balb/C mice were administered p.o. with the test compound at a dose of 1 mg/kg and a volume of 10 mL/kg.

### 2.5 Sample collection:

0.04 mL of blood was taken from the orbit of the mouse at 0, 0.5, 1, 2, 4, 6, 8 and 24 hours after the administration. The samples were stored in EDTA-K₂ tubes, and centrifuged for 6 minutes at 4°C, 6000 rpm to separate the plasma. The plasma samples were stored at -80°C. The mouse was fed 4 hours after the administration.

### 2.6 Sample process:

1) 160 µL of acetonitrile was added to 40 µL of the plasma sample for precipitation, and then the mixture was centrifuged at 3500 × g for 5 to 20 minutes.
2) After the above process, 100 µL of the supernatant was taken to analyze the concentration of the test compound by LC/MS/MS.

### 2.7 Liquid chromatography analysis

• Liquid chromatography condition: Shimadzu LC-20AD pump
• Mass spectrometry condition: AB Sciex API 4000 mass spectrometer
• Chromatographic column: phenomenex Gemiu 5 µm C18 50 × 4.6 mm
• Mobile phase: Eluent A was 0.1% formic acid in water, and Eluent B was acetonitrile
• Flow rate: 0.8 mL/min
• Elution time: 0-4.0 minutes, the eluent is as follows:

| Time/minute | Eluent A | Eluent B |
|---|---|---|
| 0.01 | 90% | 10% |
| 0.5 | 90% | 10% |
| 0.8 | 5% | 95% |
| 2.4 | 5% | 95% |
| 2.5 | 90% | 10% |
| 4.0 | Stop | |

### 3. Experimental results and analysis

The main parameters of pharmacokinetics were calculated by WinNonlin 8.2. The results of pharmacokinetic test in mice are shown in the following table:

| Example No. | Pharmacokinetic test (1 mg/kg) | | | |
|---|---|---|---|---|
| | Peak time | Area under curve | Plasma concentration | Half life |
| | tₘₐₓ(h) | AUC₀₋ₜ(ng/mL*h) | Cₘₐₓ(ng/mL) | t_{1/2}(h) |
| 1 | 1.0 | 119232 | 9933 | 6.9 |
| 3 | 0.5 | 12732 | 1573 | 3.7 |
| 29 | 0.5 | 60550 | 8267 | 3.7 |
| 35 | 0.5 | 43203 | 6100 | 2.7 |
| 36 | 0.5 | 61917 | 6547 | 4.6 |
| 45 | 1.0 | 221085 | 8977 | 23.0 |

| | | | | |
|---|---|---|---|---|
| Note: 0.5% CMC-Na (1% Tween 80) | | | | |

### 4. Experimental conclusion:

It can be seen from the results of pharmacokinetic test in mice in the table that the compounds of the examples of the present invention show good absorption and metabolism properties, both the exposure AUC and maximum plasma concentration Cₘₐₓ are good.

### Test Example 6. PK/PD study of the compounds in human breast cancer cell line MDA-MB-436 subcutaneous xenograft tumor model in nude mice

### 1. Experimental objective

To evaluate the distribution of the compound in the plasma and tumor, and the inhibitory effect on PAR in tumor tissue after a single oral administration in the human breast cancer cell line MDA-MB-436 subcutaneous xenograft tumor model in nude mice.

### 2. Experimental instruments and reagents

### 2.1 Instruments

Refrigerator (BCD-268TN, Haier)
Biological safety cabinet (BSC-1300II A2, The medical equipment factory of Shanghai Boxun Industrial Co., Ltd.)
Ultra clean workbench (CJ-2F, Suzhou Fengshi Laboratory Equipment Co., Ltd.) Electric pipetting aid (Easypet 3, Eppendorf)
Constant temperature water bath (HWS-12, Shanghai Yiheng Scientific Instruments Co., Ltd.)
CO₂ incubator (Thermo-311, Thermo)
Centrifuge (Centrifuge 5720R, Eppendorf)
Automated cell counter (Countess II, Life Technologies)
Vernier caliper (CD-6"AX, Mitutoyo, Japan)
Cell culture flask (T25/T75/T225, Corning)
Electronic balance (CPA2202S, Sartorius)
Electronic balance (BSA2202S-CW, Sartorius)
Ultrasonic cleaner (115F0032, Shanghai Kudos Ultrasonic Instrument Co., Ltd.)
Pure water meter (Pacific TII, Thermo)
Magnetic stirrer (08-2G, Chijiu)
Centrifuge (Centrifuge 5418R, Eppendorf)
Small protein vertical electrophoresis and transfer system (PowerPac Universal Power Supply, Bio-Rad)
Microplate reader (H1MFD, Biotek)
Molecular imaging system (ChemiDoc^{™} MP, Bio-Rad)
Semi-dry film transfer apparatus (690BR027087, Bio-Rad)
Tissue grinder (TISS-48, Shanghai Jingxin Experimental Equipment Co., Ltd.)
Pipette (METTLER TOLEDO/Eppendorf)
Dry thermostat (MK200-2, Hangzhou Aokangsheng Instrument Co., Ltd.)

### 2.2 Reagents

DMEM culture medium (31600-034, Gibco)
Fetal bovine serum (FBS) (10099-141C, Gibco)
Insulin-Transferrin-Selenium (ITS-G) (41400-045, Gibco)
Phosphate buffered saline (PBS) (10010-023, Gibco)
Tween 80 (30189828, Sinopharm Chemical Reagent Co., Ltd.)
Sodium carboxymethylcellulose (30036365, Sinopharm Chemical Reagent Co., Ltd.) Matrigel Matrix (356234, Corning)
Trans-Blot Turbo Transfer Pack (1704157, Bio-Rad)
4-15% Criterion^{™} TGX^{™} Gel (5671085, Bio-Rad)
GAPDH (D4C6R) Mouse (97166S, CST)
IRDye^{®} 800CW Goat anti-Mouse IgG (H + L) (P/N 926-32210, LI-COR)
IRDye^{®} 680RD Goat anti-Rabbit IgG (H + L) (P/N 926-68071, LI-COR)
Poly/Mono-ADP Ribose (E6F6A)Rabbit mAb (83732S, CST)
Pierce BCA Protein Assay Kit (23227, Thermo Fisher)
QuickBlock Western blocking buffer (P0252-500ml, Beyotime)
NuPAGE^{®} Sample Reducing Agent (10×) (NP0009, Thermo Fisher)
NuPAGE^{™} LDS Sample Buffer (4×) (NP0007, Thermo Fisher)
Pierce 20× TBS with Tween-20 (28360, Thermo Fisher)

### 3. Experimental method

### 3.1 Animals

BALB/c nude mice, 6 to 8 weeks old, ♀, purchased from the Experimental Animal Management Department of Shanghai Institute of Family Planning

### 3.2 Cell culture and preparation of cell suspension

a. A strain of MDA-MB-436 cell was taken from the cell bank, and recovered with DMEM culture medium (DMEM + 10% FBS + 1% ITS-G). The recovered cells were plated in a cell culture flask (the cell type, date, name of the experimenter and the like were labeled on the wall of the flask), and cultured in a CO₂ incubator (the temperature was 37°C and the CO₂ concentration was 5% in the incubator).
b. Cells were passaged once every three days. After passage, the cells continued to be cultured in the CO₂ incubator. This process was repeated until the number of cells was sufficient for the *in vivo* efficacy test.
c. The cultured cells were collected, and counted with an automatic cell counter. According to the counting results, the cells were resuspended with PBS, and mixed with Matrigel Matrix at a ratio of 1:1 to a final concentration of 5×10⁷/mL. The resulting suspension was placed in an ice box for later use.

### 3.3 Cell inoculation

a. The nude mice were labeled with disposable universal ear tags for rats and mice before inoculation.
b. During the inoculation, the cell suspension was mixed well. 0.1 to 1 mL of the cell suspension was drawn with a 1 mL syringe, air bubbles were removed, and then the syringe was placed on an ice pack for later use.
c. The nude mouse was bound with the left hand. The position on the right side of the back close to the right shoulder of the nude mouse (inoculation site) was disinfected with 75% alcohol. Inoculation started after 30 seconds.
d. The test nude mice were successively inoculated (each mouse was inoculated with 0.1 mL of cell suspension).

### 3.4 PK/PD test

a. Grouping: According to the growth of the tumor, when the tumor grew to a volume of 300-500 mm³, tumor-bearing mice were selected according to the experimental design and randomly grouped (3 mice per each time point) to start the PK/PD test.
b. Fasting: All tumor-bearing mice were fasted (with free access to water) overnight (fasting > 8 hours) before the administration.
c. Administration: Except for the blank control group, a single oral administration was carried out according to the time of the experimental design, and the administration volume was 10 mL/kg.
d. Sample collection: The mice were euthanized by CO₂ asphyxiation according to the time of the experimental design, and the samples were collected. One plasma sample and three tumor tissue samples were collected from each animal.

The mice were euthanized by CO₂ asphyxiation according to the time of the experimental design, and the samples were collected.

Plasma collection: After the mouse was euthanized, blood was collected from the heart. The collected blood was added to a centrifuge tube containing EDTA-K₂. The tube was manually inverted 3 to 4 times, placed on ice, and centrifuged at 8000 rpm at 4°C for 5 minutes. 100 µL of the centrifuged plasma was transferred to a new labeled centrifuge tube. One aliquot of plasma was quickly freezed on dry ice, and stored in a refrigerator at -80±10°C for PK test.

Tumor tissue collection: After the blood collection, the tumor tissue was collected. The collected tumor tissue was divided into 3 aliquots (~0.1 g each), placed into labeled 2 mL centrifuge tubes, and stored in a refrigerator at -80±10°C for PK or PD test.

The remaining tumor-bearing mice were used to collect blank plasma and blank tumor tissue.

### 3.5 PK test

### a. Sample process:

1) 4 parts by weight of 20% methanol in water was added to 1 part by weight of the tumor tissue sample, and homogenized at 40 Hz for 400 seconds. 100 µL of acetonitrile was added to 20 µL of the resulting homogenate for precipitation, and then the mixture was centrifuged at 3500 × g for 5 to 20 minutes.
2) After the above process, 100 µL of the supernatant was taken to analyze the concentration of the test compound by LC/MS/MS.

### b. Liquid chromatography analysis

• Liquid chromatography condition: Shimadzu LC-20AD pump
• Mass spectrometry condition: AB Sciex API 4000 mass spectrometer
• Chromatographic column: phenomenex Gemiu 5 µm C18 50 × 4.6 mm
• Mobile phase: Eluent A was 0.1% formic acid in water, and Eluent B was acetonitrile
• Flow rate: 0.8 mL/min
• Elution time: 0-4.0 minutes, the eluent is as follows:

| Time/minute | Eluent A | Eluent B |
|---|---|---|
| 0.01 | 90% | 10% |
| 0.5 | 90% | 10% |
| 0.8 | 5% | 95% |
| 2.4 | 5% | 95% |
| 2.5 | 90% | 10% |
| 4.0 | Stop | |

### 3.6 PD test

### a. Tumor tissue sample lysis

1 mL of tumor lysis solution was added to each tube of tumor tissue sample, followed by the addition of steel beads. The sample was placed into a tissue grinder for tissue homogenization, lysed on ice for 20 minutes, and centrifuged at 10,000 g at 4°C for 5 minutes. The protein supernatant was collected.

### b. Preparation of protein sample

Protein quantification was performed by BCA protein quantification kit. According to the concentration, the protein supernatant sample, 10× Sample Reducing Agent, 4× LDS Sample Buffer and lysate were formulated into protein loading solution with consistent concentration. The protein loading solution was placed into a preheated dry thermostat, and incubated at 100°C for 10 minutes to denature the protein.

### c. Western blot experiment of the protein sample

1) Electrophoresis: The protein sample was loaded on 4-15% Criterion^{™} TGX^{™} Gel protein gel (15 µL for each protein sample). The gel was placed into an electrophoresis tank filled with electrophoresis solution, and the protein gel electrophoresis was carried out at 150V for 60 minutes.
2) Transfer: Trans-Blot Turbo Transfer Pack Kit was used, multi-layer filter paper, PVDF membrane, protein gel and thick filter paper were placed in order and put into the transfer machine. Transfer was carried out by the program MIXED MW (2.5A-25V-7min).
3) Blocking and incubating antibodies: The PVDF membrane was taken out from the transfer machine, placed into the QuickBlock Western blocking buffer, and shaken on a shaker at room temperature for more than 1 hour to block the protein. The PVDF membrane was added to the PAR (1:500) or GAPDH (1:5000) primary antibody dilution diluted with QuickBlock Western blocking buffer, and incubated overnight at 4°C. The primary antibody dilution was removed, and the membrane was rinsed with 1×TBST six times. The goat anti-rabbit (1:3000) and goat anti-mouse (1:5000) fluorescent secondary antibody solution diluted with QuickBlock Western blocking buffer was added, and incubated at room temperature in the dark for 1 hour. The antibody dilution was removed, and the membrane was rinsed with 1×TBST six times.
4) Imaging: The cleaned PVDF membrane was placed into the Biorad ChemiDoc^{™} MP imager for imaging. PAR was subjected to fluorescence imaging, and Gapdh internal control was subjected to fluorescence imaging using IRDye 800 CW channel.

### 4. Experimental results

| **Example** | **Dosage (mg/kg)** | **Time (h)** | **Mean plasma concentration (ng/mL)** | **Mean tumor tissue concentration (ng/g)** |
|---|---|---|---|---|
| AZD5305 | 0.3 | 1 | 1463 | 41 |
| | | 2 | 1373 | 100 |
| | | 4 | 1119 | 131 |
| | | 6 | 713 | 117 |
| | | 8 | 584 | 95 |
| | | 16 | 252 | 94 |
| | | 24 | 102 | 61 |
| | | 32 | 41 | 59 |
| | | 48 | 10 | 44 |
| | | 72 | 6 | 31 |

| **Example** | **Dosage (mg/kg)** | **Time (h)** | **Mean plasma concentration (ng/mL)** | **Mean tumor tissue concentration (ng/g)** |
|---|---|---|---|---|
| Example 1 | 0.3 | 1 | 2497 | 41 |
| | | 2 | 2120 | 148 |
| | | 4 | 2327 | 198 |
| | | 6 | 1767 | 232 |
| | | 8 | 1633 | 214 |
| | | 16 | 591 | 119 |
| | | 24 | 362 | 91 |
| | | 32 | 319 | 67 |
| | | 48 | 105 | 61 |
| | | 72 | 37 | 36 |

### 5. Experimental conclusion

In the MDA-MB-436 (breast cancer, BRCA1 mutation) model, the plasma concentration of the compound of Example 1 within 24 hours after a single administration was higher than that of AZD5305, and the inhibition of intratumoral PAR was comparable. The single administration of the compound of Example 1 can inhibit intratumoral PAR for 72 hours, which is better than AZD5305.

### Test Example 7: In vivo pharmacodynamic study of the compounds in human colorectal cancer cell line DLD-1 BRCA2^{-/-} subcutaneous xenograft tumor model in nude mice

### 1. Experimental objective

To evaluate the *in vivo* efficacy of the compounds in human colorectal cancer cell line DLD-1 BRCA2^{-/-} subcutaneous xenograft tumor model in nude mice.

### 2. Experimental instruments and reagents

### 2.1 Instruments

Refrigerator (BCD-268TN, Haier)
Biological safety cabinet (BSC-1300II A2, The medical equipment factory of Shanghai Boxun Industrial Co., Ltd.)
Ultra clean workbench (CJ-2F, Suzhou Fengshi Laboratory Equipment Co., Ltd.) Electric pipetting aid (Easypet 3, Eppendorf)
Constant temperature water bath (HWS-12, Shanghai Yiheng Scientific Instruments Co., Ltd.)
CO₂ incubator (Thermo-311, Thermo)
Centrifuge (Centrifuge 5720R, Eppendorf)
Automated cell counter (Countess II, Life Technologies)
Vernier caliper (CD-6"AX, Mitutoyo, Japan)
Cell culture flask (T25/T75/T225, Corning)
Electronic balance (CPA2202S, Sartorius)
Electronic balance (BSA2202S-CW, Sartorius)
Ultrasonic cleaner (115F0032, Shanghai Kudos Ultrasonic Instrument Co., Ltd.)
Pure water meter (Pacific TII, Thermo)
Magnetic stirrer (08-2G, Chijiu)

### 2.2 Reagents

RPMI-1640 culture medium (22400-089, Gibco)
Fetal bovine serum (FBS) (10099-141C, Gibco)
Phosphate buffered saline (PBS) (10010-023, Gibco)
Tween 80 (30189828, Sinopharm Chemical Reagent Co., Ltd.)
Sodium carboxymethylcellulose (30036365, Sinopharm Chemical Reagent Co., Ltd.)

### 3. Experimental process and data process

### 3.1 Animals

BALB/c nude mice, 6 to 8 weeks old, ♀, purchased from the Experimental Animal Management Department of Shanghai Institute of Family Planning

### 3.2 Cell culture and preparation of cell suspension

a. A strain of DLD-1 BRCA2^{-/-} cell was taken from the cell bank, and recovered with RPMI-1640 culture medium (RPMI-1640 + 10% FBS). The recovered cells were plated in a cell culture flask (the cell type, date, name of the experimenter and the like were labeled on the wall of the flask), and cultured in a CO₂ incubator (the temperature was 37°C and the CO₂ concentration was 5% in the incubator).
b. Cells were passaged once every three days. After passage, the cells continued to be cultured in the CO₂ incubator. This process was repeated until the number of cells was sufficient for the *in vivo* efficacy test.
c. The cultured cells were collected, and counted with an automatic cell counter. According to the counting results, the cells were resuspended with PBS to obtain a cell suspension (density: 5×10⁷/mL), which was placed in an ice box for later use.

### 3.3 Cell inoculation

a. The nude mice were labeled with disposable universal ear tags for rats and mice before inoculation.
b. During the inoculation, the cell suspension was mixed well. 0.1 to 1 mL of the cell suspension was drawn with a 1 mL syringe, air bubbles were removed, and then the syringe was placed on an ice pack for later use.
c. The nude mouse was bound with the left hand. The position on the right side of the back close to the right shoulder of the nude mouse (inoculation site) was disinfected with 75% alcohol. Inoculation started after 30 seconds.
d. The test nude mice were successively inoculated (each mouse was inoculated with 0.1 mL of cell suspension).

### 3.4 Tumor measurement, grouping and administration of the tumor-bearing mice

a. According to the tumor growth, the tumor was measured on 10 to 18 days after the inoculation, and the tumor size was calculated. Calculation of tumor volume: tumor volume (mm³) = length (mm) × width (mm) × width (mm)/2
b. The tumor-bearing mice were grouped according to their body weight and tumor size by random grouping.
c. The test compounds were administered according to the grouping results (administration route: oral administration; administration volume: 10 mL/kg; administration frequency: once/day; administration cycle: 21 days; vehicle: 0.5% CMC-Na/1% Tween 80).
d. Tumors were measured and the mice were weighed twice a week after the administration of test compounds began.
e. Animals were euthanized at the end of the experiment.
f. Data were processed by using softwares such as Excel. Calculation of the tumor growth inhibition rate TGI (%) of the compound: when the tumor does not regress, TGI (%)=[1-(average tumor volume of the treatment group at the end of the administration-average tumor volume of the treatment group at the beginning of the administration)/(average tumor volume of the vehicle control group at the end of the treatment-average tumor volume of the vehicle control group at the beginning of the treatment)]×100%. When the tumor regress, TGI (%)=[1-(average tumor volume of the treatment group at the end of the administration-average tumor volume of the treatment group at the beginning of the administration)/average tumor volume of the treatment group at the beginning of the administration]×100%.

### 1.4 Experimental results and conclusion:

Compounds of Examples 1, 3, 29, 35 and 46 of the present invention show excellent tumor inhibitory effect in this model experiment, and their tumor growth inhibition rate TGI (%) is >90%, the tumor growth inhibition rate TGI (%) of the preferred compounds is > 150%, and there is no significant decrease in animal's body weight.

## Claims

1. A compound of formula (I), a stereoisomer thereof or a pharmaceutically acceptable salt thereof: 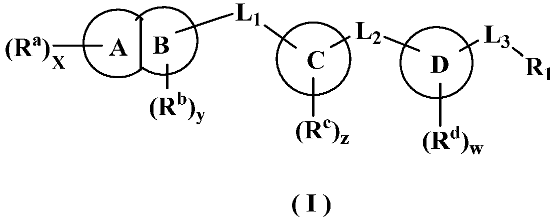
wherein, ring A, ring B, ring C and ring D are each independently selected from the group consisting of cycloalkyl, heterocyclyl, aryl and heteroaryl;
R₁ is selected from the group consisting of hydrogen, deuterium, halogen, nitro, hydroxy, thiol, cyano, amino, alkyl, deuterated alkyl, haloalkyl, hydroxyalkyl, alkoxy, alkenyl, alkynyl, cycloalkyl, heterocyclyl, aryl, heteroaryl, -(CH₂)ₙR₁₁, -(CH₂)ₙOR₁₁, -(CH₂)ₙC(O)R₁₁, -(CH₂)ₙC(O)OR₁₁, -(CH₂)ₙS(O)ₘR₁₁, -(CH₂)ₙNR₂₂R₃₃, -(CH₂)ₙNR₂₂C(O)OR₃₃, -(CH₂)ₙNR₂₂C(O)(CH₂)ₙ₁R₃₃, -(CH₂)ₙNR₂₂C(O)NR₂₂R₃₃, -(CH₂)ₙC(O)NR₂₂(CH₂)ₙ₁R₃₃, -OC(R₁₁R₂₂)ₙ(CH₂)ₙ₁R₃₃ and -(CH₂)ₙNR₂₂S(O)ₘR₃₃, the amino, alkyl, deuterated alkyl, haloalkyl, hydroxyalkyl, alkoxy, alkenyl, alkynyl, cycloalkyl, heterocyclyl, aryl and heteroaryl can be each optionally further substituted;
R₁₁, R₂₂ and R₃₃ are each independently selected from the group consisting of hydrogen, deuterium, halogen, amino, hydroxy, cyano, nitro, alkyl, alkenyl, alkynyl, deuterated alkyl, haloalkyl, alkoxy, haloalkoxy, hydroxyalkyl, cyano-substituted alkyl, cycloalkyl, heterocyclyl, aryl and heteroaryl, the amino, alkyl, alkenyl, alkynyl, deuterated alkyl, haloalkyl, alkoxy, haloalkoxy, hydroxyalkyl, cyano-substituted alkyl, cycloalkyl, heterocyclyl, aryl and heteroaryl can be each optionally further substituted;
L₁, L₂ and L₃ are each independently selected from the group consisting of a bond, substituted or unsubstituted alkenyl, substituted or unsubstituted alkynyl, -(CH₂)ₙ-, -(CH₂)ₙC(O)(CRₐₐR_{bb})ₙ₁-, -(CH₂)ₙC(O)NRₐₐ(CH₂)ₙ₁-, -(CH₂)ₙ(CRₐₐR_{bb})ₙ₂-, -(CRₐₐR_{bb})ₙO(CH₂)ₙ₁-, -(CH₂)ₙO(CRₐₐR_{bb})ₙ₁-, -(CRₐₐR_{bb})ₙ₃S(CH₂)ₙ₄-, -(CH₂)ₙS(CRₐₐR_{bb})ₙ₃-, -(CRₐₐR_{bb})ₙ₃(CH₂)ₙNR_{cc}-, -(CH₂)ₙNRₐₐ(CR_{bb}R_{cc})ₙ-, -(CH₂)ₙNRₐₐC(O)-, -(CH₂)ₙP(O)ₚRₐₐ-, -(CH₂)ₙS(O)ₘ-, -(CH₂)ₙS(O)ₘNRₐₐ- and -(CH₂)ₙNRₐₐS(O)ₘ-;
Rₐₐ, R_{bb} and R_{cc} are each independently selected from the group consisting of hydrogen, deuterium, halogen, nitro, hydroxy, thiol, cyano, amino, alkyl, deuterated alkyl, haloalkyl, hydroxyalkyl, alkoxy, alkenyl, alkynyl, cycloalkyl, heterocyclyl, aryl and heteroaryl, the amino, alkyl, deuterated alkyl, haloalkyl, hydroxyalkyl, alkoxy, alkenyl, alkynyl, cycloalkyl, heterocyclyl, aryl and heteroaryl can be each optionally further substituted;
each R^{a} is independently selected from the group consisting of hydrogen, deuterium, halogen, nitro, hydroxy, thiol, oxo, cyano, amino, alkyl, deuterated alkyl, haloalkyl, hydroxyalkyl, alkoxy, alkenyl, alkynyl, cycloalkyl, heterocyclyl, aryl, heteroaryl, -(CH₂)ₙRₐ₁, -(CH₂)ₙORₐ₁, -(CH₂)ₙC(O)Rₐ₁, -(CH₂)ₙC(O)ORₐ₁, -(CH₂)ₙS(O)ₘRₐ₁, -(CH₂)ₙNRₐ₂Rₐ₃, -(CH₂)ₙNRₐ₂C(O)ORₐ₃, -(CH₂)ₙNRₐ₂C(O)(CH₂)ₙ₁Rₐ₃, -(CH₂)ₙNRₐ₂C(O)NRₐ₂Rₐ₃, -(CH₂)ₙC(O)NRₐ₂(CH₂)ₙ₁Rₐ₃, -OC(Rₐ₁Rₐ₂)ₙ(CH₂)ₙ₁Rₐ₃ and -(CH₂)ₙNRₐ₂S(O)ₘRₐ₃, the amino, alkyl, deuterated alkyl, haloalkyl, hydroxyalkyl, alkoxy, alkenyl, alkynyl, cycloalkyl, heterocyclyl, aryl and heteroaryl can be each optionally further substituted;
Rₐ₁, Rₐ₂ and Rₐ₃ are each independently selected from the group consisting of hydrogen, deuterium, halogen, nitro, hydroxy, thiol, cyano, amino, alkyl, deuterated alkyl, haloalkyl, hydroxyalkyl, alkoxy, alkenyl, alkynyl, cycloalkyl, heterocyclyl, aryl and heteroaryl, the amino, alkyl, deuterated alkyl, haloalkyl, hydroxyalkyl, alkoxy, alkenyl, alkynyl, cycloalkyl, heterocyclyl, aryl and heteroaryl can be each optionally further substituted;
each R^{b} is independently selected from the group consisting of hydrogen, deuterium, halogen, nitro, hydroxy, thiol, oxo, cyano, amino, alkyl, deuterated alkyl, haloalkyl, hydroxyalkyl, alkoxy, alkenyl, alkynyl, cycloalkyl, heterocyclyl, aryl, heteroaryl, -(CH₂)ₙR_{b1}, -(CH₂)ₙOR_{b1}, -(CH₂)ₙC(O)R_{b1}, -(CH₂)ₙC(O)OR_{b1}, -(CH₂)ₙS(O)ₘR_{b1}, -(CH₂)ₙNR_{b2}R_{b3}, -(CH₂)ₙNR_{b2}C(O)OR_{b3}, -(CH₂)ₙNR_{b2}C(O)(CH₂)ₙ₁R_{b3}, -(CH₂)ₙNR_{b2}C(O)NR_{b2}R_{b3}, -(CH₂)ₙC(O)NR_{b2}(CH₂)ₙ₁R_{b3}, -OC(R_{b1}R_{b2})ₙ(CH₂)ₙ₁R_{b3} and -(CH₂)ₙNR_{b2}S(O)ₘR_{b3}, the amino, alkyl, deuterated alkyl, haloalkyl, hydroxyalkyl, alkoxy, alkenyl, alkynyl, cycloalkyl, heterocyclyl, aryl and heteroaryl can be each optionally further substituted;
R_{b1}, R_{b2} and R_{b3} are each independently selected from the group consisting of hydrogen, deuterium, halogen, nitro, hydroxy, thiol, cyano, amino, alkyl, deuterated alkyl, haloalkyl, hydroxyalkyl, alkoxy, alkenyl, alkynyl, cycloalkyl, heterocyclyl, aryl and heteroaryl, the amino, alkyl, deuterated alkyl, haloalkyl, hydroxyalkyl, alkoxy, alkenyl, alkynyl, cycloalkyl, heterocyclyl, aryl and heteroaryl can be each optionally further substituted;
each R^{c} is independently selected from the group consisting of hydrogen, deuterium, halogen, nitro, hydroxy, thiol, oxo, cyano, amino, alkyl, deuterated alkyl, haloalkyl, hydroxyalkyl, alkoxy, alkenyl, alkynyl, cycloalkyl, heterocyclyl, aryl, heteroaryl, -(CH₂)ₙR_{c1}, -(CH₂)ₙOR_{c1}, -(CH₂)ₙC(O)R_{c1}, -(CH₂)ₙC(O)OR_{c1}, -(CH₂)ₙS(O)ₘR_{c1}, -(CH₂)ₙNR_{c2}R_{c3}, -(CH₂)ₙNR_{c2}C(O)OR_{c3}, -(CH₂)ₙNR_{c2}C(O)(CH₂)ₙ₁R_{c3}, -(CH₂)ₙNR_{c2}C(O)NR_{c2}R_{c3}, -(CH₂)ₙC(O)NR_{c2}(CH₂)ₙ₁R_{c3}, -OC(R_{c1}R_{c2})ₙ(CH₂)ₙ₁R_{c3} and -(CH₂)ₙNR_{c2}S(O)ₘR_{c3}, the amino, alkyl, deuterated alkyl, haloalkyl, hydroxyalkyl, alkoxy, alkenyl, alkynyl, cycloalkyl, heterocyclyl, aryl and heteroaryl can be each optionally further substituted;
R_{c1}, R_{c2} and R_{c3} are each independently selected from the group consisting of hydrogen, deuterium, halogen, nitro, hydroxy, thiol, cyano, amino, alkyl, deuterated alkyl, haloalkyl, hydroxyalkyl, alkoxy, alkenyl, alkynyl, cycloalkyl, heterocyclyl, aryl and heteroaryl, the amino, alkyl, deuterated alkyl, haloalkyl, hydroxyalkyl, alkoxy, alkenyl, alkynyl, cycloalkyl, heterocyclyl, aryl and heteroaryl can be each optionally further substituted;
each R^{d} is independently selected from the group consisting of hydrogen, deuterium, halogen, nitro, hydroxy, thiol, oxo, cyano, amino, alkyl, deuterated alkyl, haloalkyl, hydroxyalkyl, alkoxy, alkenyl, alkynyl, cycloalkyl, heterocyclyl, aryl, heteroaryl, -(CH₂)ₙR_{d1}, -(CH₂)ₙOR_{d1}, -(CH₂)ₙC(O)R_{d1}, -(CH₂)ₙC(O)OR_{d1}, -(CH₂)ₙS(O)ₘR_{d1}, -(CH₂)ₙNR_{d2}R_{d3}, -(CH₂)ₙNR_{d2}C(O)OR_{d3}, -(CH₂)ₙNR_{d2}C(O)(CH₂)ₙ₁R_{d3}, -(CH₂)ₙNR_{d2}C(O)NR_{d2}R_{d3}, -(CH₂)ₙC(O)NR_{d2}(CH₂)ₙ₁R_{d3}, -OC(R_{d1}R_{d2})ₙ(CH₂)ₙ₁R_{d3} and -(CH₂)ₙNR_{d2}S(O)ₘR_{d3}, the amino, alkyl, deuterated alkyl, haloalkyl, hydroxyalkyl, alkoxy, alkenyl, alkynyl, cycloalkyl, heterocyclyl, aryl and heteroaryl can be each optionally further substituted;
R_{d1}, R_{d2} and R_{d3} are each independently selected from the group consisting of hydrogen, deuterium, halogen, nitro, hydroxy, thiol, cyano, amino, alkyl, deuterated alkyl, haloalkyl, hydroxyalkyl, alkoxy, alkenyl, alkynyl, cycloalkyl, heterocyclyl, aryl and heteroaryl, the amino, alkyl, deuterated alkyl, haloalkyl, hydroxyalkyl, alkoxy, alkenyl, alkynyl, cycloalkyl, heterocyclyl, aryl and heteroaryl can be each optionally further substituted;
w, x, y and z are each independently 1, 2, 3 or 4;
each m is independently 0, 1 or 2;
each n is independently 0, 1, 2, 3 or 4;
each p is independently 0 or 1; and
n1, n2, n3 and n4 are each independently 0, 1, 2, 3 or 4.

2. The compound, the stereoisomer thereof or the pharmaceutically acceptable salt thereof according to claim 1, wherein the compound is further as shown in formula (III): 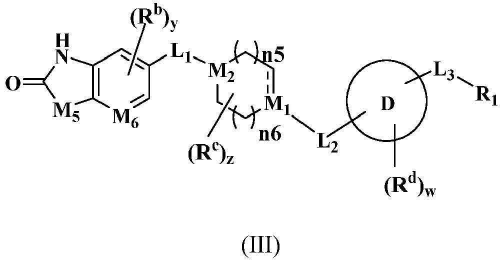 wherein,
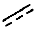 is a single bond or double bond;
L₁ is a bond, -(CH₂)ₙC(O)(CRₐₐR_{bb})ₙ₁-, -(CH₂)ₙ(CRₐₐR_{bb})ₙ₂- or -(CRₐₐR_{bb})ₙO(CH₂)ₙ₁-;
L₂ is a bond or -(CH₂)ₙ(CRₐₐR_{bb})ₙ₂-;
L₃ is selected from the group consisting of a bond, -(CH₂)ₙC(O)(CRₐₐR_{bb})ₙ₁-, -(CH₂)ₙC(O)NRₐₐ(CH₂)ₙ₁-, -(CH₂)ₙ(CRₐₐR_{bb})ₙ₂-, -(CRₐₐR_{bb})ₙO(CH₂)ₙ₁- and -(CH₂)ₙNRₐₐ(CR_{bb}R_{cc})ₙ-;
Rₐₐ, R_{bb} and R_{cc} are each independently selected from the group consisting of hydrogen, deuterium, halogen, nitro, hydroxy, thiol, cyano, amino, alkyl, deuterated alkyl, haloalkyl, hydroxyalkyl, alkoxy, alkenyl and alkynyl;
or, any two of Rₐₐ, R_{bb} and R_{cc} are bonded to form a cycloalkyl;
M₁ is N, C or CR₂;
M₂ is N or CR₃;
M₅ is -CR₆R₇-, -CR₆R₇-CR₈R₉-, -CR₆=CR₈-, -CR₆R₇-NR₈-, -NR₈-C(=O)-, -CR₆R₇-O- or -CR₆=N-;
M₆ is N or CR₁₀;
R₁ is selected from the group consisting of hydrogen, deuterium, halogen, nitro, hydroxy, thiol, cyano, amino, alkyl, deuterated alkyl, haloalkyl, hydroxyalkyl, alkoxy, alkenyl, alkynyl, cycloalkyl, heterocyclyl, aryl, heteroaryl, -(CH₂)ₙR₁₁, -(CH₂)ₙOR₁₁, -(CH₂)ₙC(O)R₁₁, -(CH₂)ₙC(O)OR₁₁, -(CH₂)ₙS(O)ₘR₁₁, -(CH₂)ₙNR₂₂R₃₃, -(CH₂)ₙNR₂₂C(O)OR₃₃, -(CH₂)ₙNR₂₂C(O)(CH₂)ₙ₁R₃₃, -(CH₂)ₙNR₂₂C(O)NR₂₂R₃₃, -(CH₂)ₙC(O)NR₂₂(CH₂)ₙ₁R₃₃, -OC(R₁₁R₂₂)ₙ(CH₂)ₙ₁R₃₃ and -(CH₂)ₙNR₂₂S(O)ₘR₃₃, the amino, alkyl, deuterated alkyl, haloalkyl, hydroxyalkyl, alkoxy, alkenyl, alkynyl, cycloalkyl, heterocyclyl, aryl and heteroaryl can be each optionally further substituted;
R₁₁, R₂₂ and R₃₃ are each independently selected from the group consisting of hydrogen, deuterium, halogen, amino, hydroxy, cyano, nitro, alkyl, alkenyl, alkynyl, deuterated alkyl, haloalkyl, alkoxy, haloalkoxy, hydroxyalkyl, cyano-substituted alkyl, cycloalkyl, heterocyclyl, aryl and heteroaryl, the amino, alkyl, alkenyl, alkynyl, deuterated alkyl, haloalkyl, alkoxy, haloalkoxy, hydroxyalkyl, cyano-substituted alkyl, cycloalkyl, heterocyclyl, aryl and heteroaryl can be each optionally further substituted;
R₂ and R₃ are identical or different, and are each independently selected from the group consisting of hydrogen, deuterium, halogen, nitro, hydroxy, thiol, cyano, amino optionally substituted by alkyl, alkyl, deuterated alkyl, haloalkyl, hydroxyalkyl, alkoxy, alkenyl, alkynyl, cycloalkyl, heterocyclyl, aryl and heteroaryl; preferably, R₂ and R₃ are each independently hydrogen, deuterium, halogen, alkyl or cycloalkyl;
R₆, R₇, R₈, R₉ and R₁₀ are identical or different, and are each independently selected from the group consisting of hydrogen, deuterium, halogen, nitro, hydroxy, thiol, cyano, amino optionally substituted by alkyl, alkyl, deuterated alkyl, haloalkyl, hydroxyalkyl, alkoxy, alkenyl, alkynyl, cycloalkyl, heterocyclyl, aryl and heteroaryl; preferably, R₆, R₇, R₈ and R₉ are each independently hydrogen, deuterium, halogen, cyano, alkyl, alkynyl or cycloalkyl;
or, R₆, R₇ together with the adjacent carbon atom are bonded to form a cycloalkyl, the cycloalkyl is optionally substituted by one or more substituents selected from the group consisting of hydrogen, deuterium, halogen, nitro, hydroxy, thiol, cyano and amino;
or, R₆, R₈ together with the adjacent carbon atom are bonded to form a cycloalkyl, the cycloalkyl is optionally substituted by one or more substituents selected from the group consisting of hydrogen, deuterium, halogen, nitro, hydroxy, thiol, cyano and amino;
each R^{b} is independently selected from the group consisting of hydrogen, deuterium, halogen, nitro, hydroxy, thiol, oxo, cyano, amino, alkyl, deuterated alkyl, haloalkyl, hydroxyalkyl, alkoxy, alkenyl, alkynyl, cycloalkyl, heterocyclyl, aryl, heteroaryl, -(CH2)nR_{b1}, -(CH₂)ₙOR_{b1}, -(CH₂)ₙC(O)R_{b1}, -(CH₂)ₙC(O)OR_{b1}, -(CH₂)ₙS(O)ₘR_{b1}, -(CH₂)ₙNR_{b2}R_{b3}, -(CH₂)ₙNR_{b2}C(O)OR_{b3}, -(CH₂)nNR_{b2}C(O)(CH₂)ₙ₁R_{b3}, -(CH₂)ₙNR_{b2}C(O)NR_{b2}R_{b3}, -(CH₂)ₙC(O)NR_{b2}(CH₂)ₙ₁R_{b3}, -OC(R_{b1}R_{b2})ₙ(CH₂)ₙ₁R_{b3} and -(CH₂)ₙNR_{b2}S(O)ₘR_{b3}, the amino, alkyl, deuterated alkyl, haloalkyl, hydroxyalkyl, alkoxy, alkenyl, alkynyl, cycloalkyl, heterocyclyl, aryl and heteroaryl can be each optionally further substituted;
R_{b1}, R_{b2} and R_{b3} are each independently selected from the group consisting of hydrogen, deuterium, halogen, nitro, hydroxy, thiol, cyano, amino, alkyl, deuterated alkyl, haloalkyl, hydroxyalkyl, alkoxy, alkenyl, alkynyl, cycloalkyl, heterocyclyl, aryl and heteroaryl, the amino, alkyl, deuterated alkyl, haloalkyl, hydroxyalkyl, alkoxy, alkenyl, alkynyl, cycloalkyl, heterocyclyl, aryl and heteroaryl can be each optionally further substituted;
each R^{c} is independently selected from the group consisting of hydrogen, deuterium, halogen, nitro, hydroxy, thiol, oxo, cyano, amino, alkyl, deuterated alkyl, haloalkyl, hydroxyalkyl, alkoxy, alkenyl, alkynyl, cycloalkyl, heterocyclyl, aryl, heteroaryl, -(CH₂)ₙR_{c1}, -(CH₂)ₙOR_{c1}, -(CH₂)ₙC(O)R_{c1}, -(CH₂)ₙC(O)OR_{c1}, -(CH₂)ₙS(O)ₘR_{c1}, -(CH₂)ₙNR_{c2}R_{c3}, -(CH₂)ₙNR_{c2}C(O)OR_{c3}, -(CH₂)ₙNR_{c2}C(O)(CH₂)ₙ₁R_{c3}, -(CH₂)ₙNR_{c2}C(O)NR_{c2}R_{c3}, -(CH₂)ₙC(O)NR_{c2}(CH₂)ₙ₁R_{c3}, -OC(R_{c1}R_{c2})ₙ(CH₂)ₙ₁R_{c3} and -(CH₂)ₙNR_{c2}S(O)ₘR_{c3}, the amino, alkyl, deuterated alkyl, haloalkyl, hydroxyalkyl, alkoxy, alkenyl, alkynyl, cycloalkyl, heterocyclyl, aryl and heteroaryl can be each optionally further substituted;
R_{c1}, R_{c2} and R_{c3} are each independently selected from the group consisting of hydrogen, deuterium, halogen, nitro, hydroxy, thiol, cyano, amino, alkyl, deuterated alkyl, haloalkyl, hydroxyalkyl, alkoxy, alkenyl, alkynyl, cycloalkyl, heterocyclyl, aryl and heteroaryl, the amino, alkyl, deuterated alkyl, haloalkyl, hydroxyalkyl, alkoxy, alkenyl, alkynyl, cycloalkyl, heterocyclyl, aryl and heteroaryl can be each optionally further substituted;
each R^{d} is independently selected from the group consisting of hydrogen, deuterium, halogen, nitro, hydroxy, thiol, oxo, cyano, amino, alkyl, deuterated alkyl, haloalkyl, hydroxyalkyl, alkoxy, alkenyl, alkynyl, cycloalkyl, heterocyclyl, aryl, heteroaryl, -(CH₂)ₙR_{d1}, -(CH₂)ₙOR_{d1}, -(CH₂)ₙC(O)R_{d1}, -(CH₂)ₙC(O)OR_{d1}, -(CH₂)ₙS(O)ₘR_{d1}, -(CH₂)ₙNR_{d2}R_{d3}, -(CH₂)ₙNR_{d2}C(O)OR_{d3}, -(CH₂)ₙNR_{d2}C(O)(CH₂)ₙ₁R_{d3}, -(CH₂)ₙNR_{d2}C(O)NR_{d2}R_{d3}, -(CH₂)ₙC(O)NR_{d2}(CH₂)ₙ₁R_{d3}, -OC(R_{d1}R_{d2})ₙ(CH₂)ₙ₁R_{d3} and -(CH₂)ₙNR_{d2}S(O)ₘR_{d3}, the amino, alkyl, deuterated alkyl, haloalkyl, hydroxyalkyl, alkoxy, alkenyl, alkynyl, cycloalkyl, heterocyclyl, aryl and heteroaryl can be each optionally further substituted;
R_{d1}, R_{d2} and R_{d3} are each independently selected from the group consisting of hydrogen, deuterium, halogen, nitro, hydroxy, thiol, cyano, amino, alkyl, deuterated alkyl, haloalkyl, hydroxyalkyl, alkoxy, alkenyl, alkynyl, cycloalkyl, heterocyclyl, aryl and heteroaryl, the amino, alkyl, deuterated alkyl, haloalkyl, hydroxyalkyl, alkoxy, alkenyl, alkynyl, cycloalkyl, heterocyclyl, aryl and heteroaryl can be each optionally further substituted;
w, y and z are each independently 1, 2, 3 or 4;
each m is independently 0, 1 or 2;
each n is independently 0, 1, 2, 3 or 4;
n1 and n2 are each independently 0, 1, 2, 3 or 4;
n5 and n6 are each independently 0, 1 or 2;
when M₁ is N and M₂ is N, then ring D is not a monocyclic ring, and when ring D is 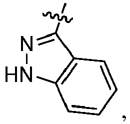 then R₁ is not hydrogen;
when M₂ is N, M₁ is CH and ring D is 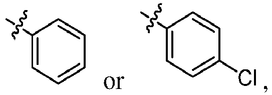 then R₁ is not hydrogen;
when M₁ is N, M₂ is CH and ring D is 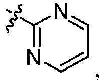 then R₁ is not hydrogen;
when 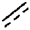 is a double bond, M₁ is C, M₂ is N and ring D is phenyl, then R₁ is not hydrogen.

3. The compound, the stereoisomer thereof or the pharmaceutically acceptable salt thereof according to claim 1, wherein the compound is further as shown in formula (II):

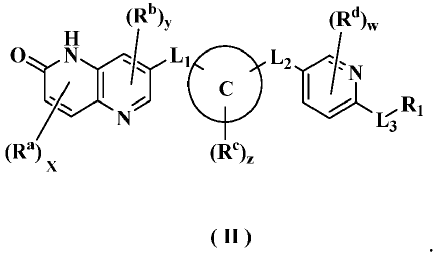

4. The compound, the stereoisomer thereof or the pharmaceutically acceptable salt thereof according to claim 1, wherein the compound is further as shown in formula (IV): 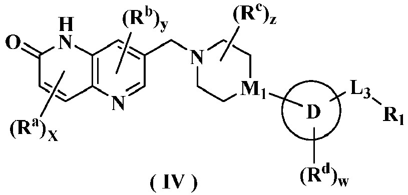 M₁ is N or CR^{a}.

5. The compound, the stereoisomer thereof or the pharmaceutically acceptable salt thereof according to claim 1 or 3, wherein:
ring C is a 3 to 10 membered heterocyclyl, and more preferably, ring C is a 4 to 8 membered heterocyclyl; the heteroatoms in the 3 to 10 membered heterocyclyl and 4 to 8 membered heterocyclyl are each independently selected from the group consisting of nitrogen, oxygen and sulfur, and the number of heteroatoms is independently 1, 2 or 3; preferably, the rings of the 3 to 10 membered heterocyclyl and 4 to 8 membered heterocyclyl are each independently monocyclic ring, bridged ring, spiro ring or fused ring;
ring C is further preferably selected from the group consisting of:

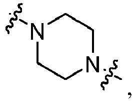

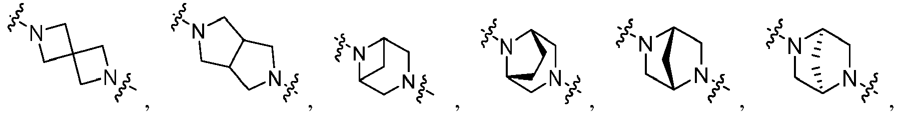

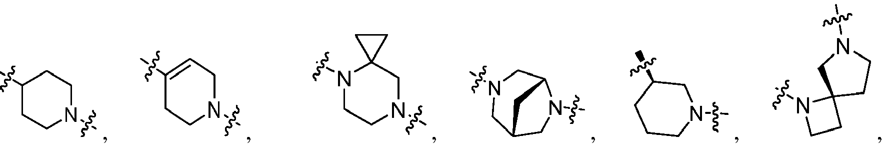

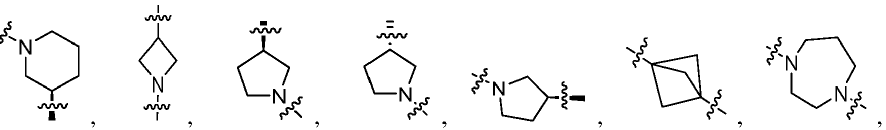

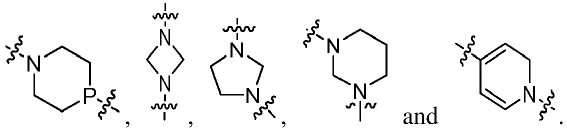

6. The compound, the stereoisomer thereof or the pharmaceutically acceptable salt thereof according to claim 1, 2 or 4, wherein:
ring D is a 6 to 10 membered heterocyclyl, C₆₋₁₀ aryl or 5 to 10 membered heteroaryl; preferably, ring D is a 5 membered heteroaromatic ring, 6 membered heteroaromatic ring, benzene ring, 5 membered heteroaromatic ring fused with 6 membered heteroaromatic ring, 6 membered heteroaromatic ring fused with 6 membered heteroaromatic ring, 6 membered heteroaromatic ring fused with 6 membered heterocyclic ring, 6 membered heteroaromatic ring fused with 5 membered heterocyclic ring, benzene ring fused with 5 membered heterocyclic ring, benzene ring fused with 6 membered heteroaromatic ring, benzene ring fused with 6 membered heterocyclic ring; wherein the heteroatoms in the 3 to 14 membered heterocyclyl, 6 to 10 membered heterocyclyl, 5 to 10 membered heteroaryl and 5 to 14 membered heteroaryl are each independently selected from the group consisting of nitrogen, oxygen and sulfur, and the number of heteroatoms is independently 1, 2 or 3;
ring D is more preferably selected from the group consisting of:

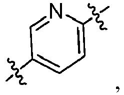

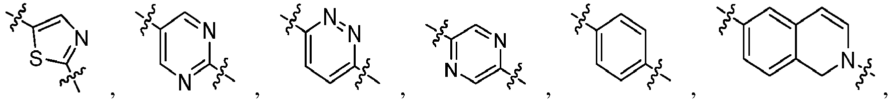

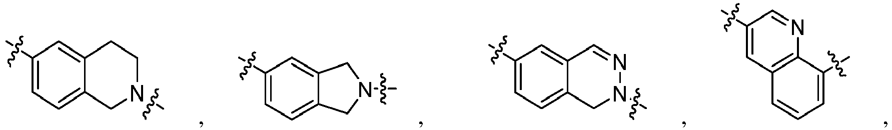

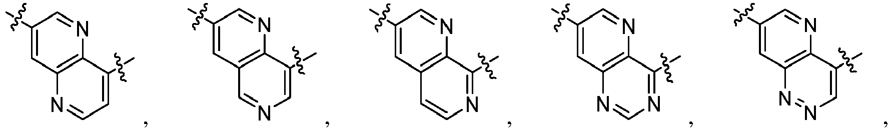

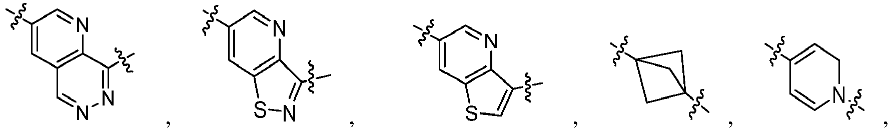

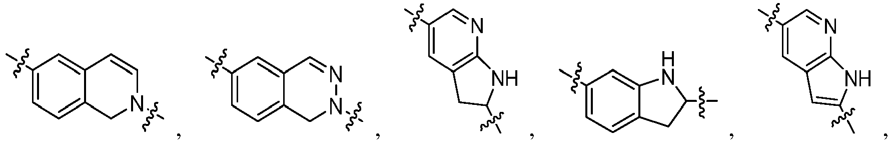

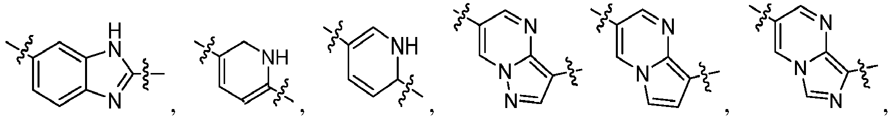

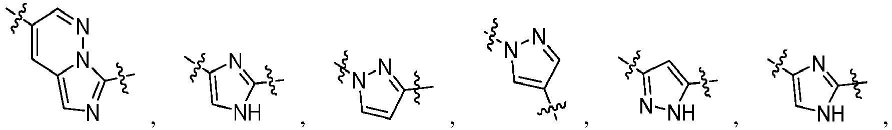

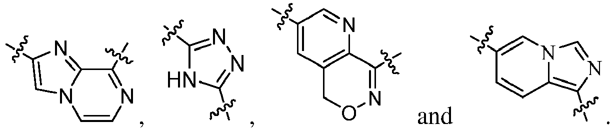

7. The compound, the stereoisomer thereof or the pharmaceutically acceptable salt thereof according to any one of claims 1 to 6, wherein:
R₁ is selected from the group consisting of hydrogen, deuterium, halogen, nitro, hydroxy, thiol, cyano, amino, C₁₋₆ alkyl, C₁₋₆ deuterated alkyl, C₁₋₆ haloalkyl, C₁₋₆ hydroxyalkyl, C₁₋₆ alkoxy, C₂₋₆ alkenyl, C₂₋₆ alkynyl, C₃₋₁₂ cycloalkyl, 3 to 12 membered heterocyclyl, C₆₋₁₂ aryl, 5 to 12 membered heteroaryl, -(CH₂)ₙR₁₁, -(CH₂)ₙOR₁₁, -(CH₂)ₙC(O)R₁₁, -(CH₂)ₙC(O)OR₁₁, -(CH₂)ₙS(O)ₘR₁₁, -(CH₂)ₙNR₂₂R₃₃, -(CH₂)ₙNR₂₂C(O)OR₃₃, -(CH₂)ₙNR₂₂C(O)(CH₂)ₙ₁R₃₃, -(CH₂)ₙNR₂₂C(O)NR₂₂R₃₃, -(CH₂)ₙC(O)NR₂₂(CH₂)ₙ₁R₃₃, -OC(R₁₁R₂₂)ₙ(CH₂)ₙ₁R₃₃ and -(CH₂)ₙNR₂₂S(O)ₘR₃₃, the amino, C₁₋₆ alkyl, C₁₋₆ deuterated alkyl, C₁₋₆ haloalkyl, C₁₋₆ hydroxyalkyl, C₁₋₆ alkoxy, C₂₋₆ alkenyl, C₂₋₆ alkynyl, C₃₋₁₂ cycloalkyl, 3 to 12 membered heterocyclyl, C₆₋₁₂ aryl and 5 to 12 membered heteroaryl can be each optionally further substituted by one or more substituents selected from the group consisting of hydroxy, halogen, nitro, hydroxy, thiol, cyano, amino, oxo, C₁₋₆ alkyl, C₁₋₆ alkoxy, C₆₋₁₂ aryl, 5 to 12 membered heteroaryl and 3 to 12 membered heterocyclyl;
preferably, R₁ is selected from the group consisting of hydrogen, deuterium, halogen, nitro, hydroxy, thiol, cyano, amino, C₁₋₆ alkyl, C₁₋₆ deuterated alkyl, C₁₋₆ haloalkyl, C₁₋₆ hydroxyalkyl, C₁₋₆ alkoxy, C₂₋₆ alkenyl, C₂₋₆ alkynyl, C₃₋₁₂ cycloalkyl, 3 to 12 membered heterocyclyl, C₆₋₁₂ aryl, 5 to 12 membered heteroaryl, -(CH₂)ₙR₁₁, -(CH₂)ₙOR₁₁, -(CH₂)ₙC(O)R₁₁, -(CH₂)ₙC(O)OR₁₁, -(CH₂)ₙS(O)ₘR₁₁, -(CH₂)ₙNR₂₂R₃₃, -(CH₂)ₙNR₂₂C(O)OR₃₃, -(CH₂)ₙNR₂₂C(O)(CH₂)ₙ₁R₃₃, -(CH₂)ₙNR₂₂C(O)NR₂₂R₃₃, -(CH₂)ₙC(O)NR₂₂(CH₂)ₙ₁R₃₃, -OC(R₁₁R₂₂)ₙ(CH₂)ₙ₁R₃₃ and -(CH₂)ₙNR₂₂S(O)ₘR₃₃, the amino, C₁₋₆ alkyl, C₁₋₆ deuterated alkyl, C₁₋₆ haloalkyl, C₁₋₆ hydroxyalkyl, C₁₋₆ alkoxy, C₂₋₆ alkenyl, C₂₋₆ alkynyl, C₃₋₁₂ cycloalkyl, 3 to 12 membered heterocyclyl, C₆₋₁₂ aryl and 5 to 12 membered heteroaryl can be each optionally further substituted by one or more substituents selected from the group consisting of hydroxy, halogen, amino, C₁₋₆ alkyl and 3 to 12 membered heterocyclyl;
R₁₁, R₂₂ and R₃₃ are each independently selected from the group consisting of hydrogen, deuterium, halogen, amino, hydroxy, cyano, nitro, C₁₋₆ alkyl, C₂₋₆ alkenyl, C₂₋₆ alkynyl, C₁₋₆ deuterated alkyl, C₁₋₆ haloalkyl, C₁₋₆ alkoxy, C₁₋₆ haloalkoxy, C₁₋₆ hydroxyalkyl, cyano-substituted C₁₋₆ alkyl, C₃₋₁₂ cycloalkyl, 3 to 12 membered heterocyclyl, C₆₋₁₂ aryl, 5 to 12 membered heteroaryl, -O(CH₂)ₙ₁R₆₆, -OC(R₄₄R₅₅)ₘ₁(CH₂)ₙ₁R₆₆, -NR₄₄(CH₂)ₙ₁R₆₆, -(CH₂)ₙ₁-, -(CH₂)ₙ₁R₆₆, -(CH₂)ₙ₁OR₆₆, -(CH₂)ₙ₁SR₆₆, -(CH₂)ₙ₁C(O)R₆₆, -(CH₂)ₙ₁C(O)OR₆₆, -(CH₂)ₙ₁S(O)ₘ₁R₆₆, -(CH₂)ₙ₁NR₄₄R₅₅, -(CH₂)ₙ₁C(O)NR₄₄R₅₅, -(CH₂)ₙ₁NR₄₄C(O)R₆₆ and -(CH₂)ₙ₁NR₄₄S(O)ₘ₁R₆₆, each optionally substituted by one or more substituents selected from the group consisting of deuterium, halogen, amino, hydroxy, cyano, nitro, C₁₋₆ alkyl and C₃₋₁₂ cycloalkyl;
R₄₄, R₅₅ and R₆₆ are each independently selected from the group consisting of hydrogen, deuterium, halogen, amino, hydroxy, cyano, nitro, C₁₋₆ alkyl, C₂₋₆ alkenyl, C₂₋₆ alkynyl, C₁₋₆ deuterated alkyl, C₁₋₆ haloalkyl, C₁₋₆ alkoxy, C₁₋₆ haloalkoxy, C₁₋₆ hydroxyalkyl, cyano-substituted C₁₋₆ alkyl, C₃₋₁₂ cycloalkyl, 3 to 12 membered heterocyclyl, C₆₋₁₂ aryl and 5 to 12 membered heteroaryl, each optionally substituted by one or more substituents selected from the group consisting of deuterium, halogen, amino, hydroxy, cyano, nitro, C₁₋₆ alkyl and C₃₋₁₂ cycloalkyl;
more preferably, R₁ is selected from the group consisting of hydrogen, deuterium, halogen, nitro, hydroxy, thiol, cyano, amino, C₁₋₆ alkyl, C₁₋₆ deuterated alkyl, C₁₋₆ haloalkyl, C₁₋₆ hydroxyalkyl, C₁₋₆ alkoxy, C₂₋₆ alkenyl, C₂₋₆ alkynyl, C₃₋₁₂ cycloalkyl, 3 to 12 membered heterocyclyl, C₆₋₁₂ aryl and 5 to 12 membered heteroaryl, each can be optionally further substituted by one or more substituents selected from the group consisting of hydroxy, halogen, amino, C₁₋₆ alkyl and 3 to 12 membered heterocyclyl;
further preferably, R₁ is selected from the group consisting of hydrogen, deuterium, cyano, C₁₋₃ deuterated alkyl, C₁₋₃ alkyl, C₁₋₃ alkoxy, C₃₋₆ cycloalkyl and 3 to 6 membered heterocyclyl, each can be optionally further substituted by one or more substituents selected from the group consisting of hydroxy, halogen, amino, C₁₋₃ alkyl and 3 to 6 membered heterocyclyl;
more further preferably, R₁ is selected from the group consisting of hydrogen, -CH₃,
- CD₃, -CH₂CN, ethyl, methoxy, cyano, cyclopropyl,

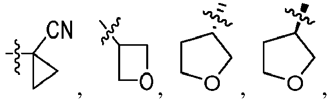

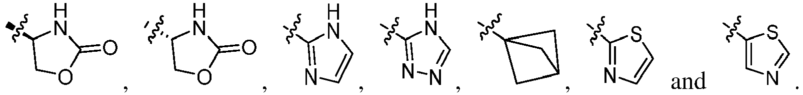

8. The compound, the stereoisomer thereof or the pharmaceutically acceptable salt thereof according to any one of claims 1, 3 to 6, wherein:
each R^{a} is independently selected from the group consisting of hydrogen, deuterium, halogen, nitro, hydroxy, thiol, oxo, cyano, amino, C₁₋₆ alkyl, C₁₋₆ deuterated alkyl, C₁₋₆ haloalkyl, C₁₋₆ hydroxyalkyl, C₁₋₆ alkoxy, C₂₋₆ alkenyl, C₂₋₆ alkynyl, C₃₋₁₂ cycloalkyl, 3 to 12 membered heterocyclyl, C₆₋₁₂ aryl, 5 to 12 membered heteroaryl, -(CH₂)ₙRₐ₁, -(CH₂)ₙORₐ₁, -(CH₂)ₙC(O)Rₐ₁, -(CH₂)ₙC(O)ORₐ₁, -(CH₂)ₙS(O)ₘRₐ₁, -(CH₂)ₙNRₐ₂Rₐ₃, -(CH₂)ₙNRₐ₂C(O)ORₐ₃, -(CH₂)ₙNRₐ₂C(O)(CH₂)ₙ₁Rₐ₃, -(CH₂)ₙNRₐ₂C(O)NRₐ₂Rₐ₃, -(CH₂)ₙC(O)NRₐ₂(CH₂)ₙ₁Rₐ₃, -OC(Rₐ₁Rₐ₂)ₙ(CH₂)ₙ₁Rₐ₃ and -(CH₂)ₙNRₐ₂S(O)ₘRₐ₃, the amino, C₁₋₆ alkyl, C₁₋₆ deuterated alkyl, C₁₋₆ haloalkyl, C₁₋₆ hydroxyalkyl, C₁₋₆ alkoxy, C₂₋₆ alkenyl, C₂₋₆ alkynyl, C₃₋₁₂ cycloalkyl, 3 to 12 membered heterocyclyl, C₆₋₁₂ aryl and 5 to 12 membered heteroaryl can be each optionally further substituted by one or more substituents selected from the group consisting of hydroxy, halogen, amino, C₁₋₆ alkyl and 3 to 12 membered heterocyclyl;
preferably, each R^{a} is independently selected from the group consisting of hydrogen, deuterium, halogen, nitro, hydroxy, thiol, oxo, cyano, amino, C₁₋₃ alkyl, C₁₋₃ deuterated alkyl, C₁₋₃ haloalkyl, C₁₋₃ hydroxyalkyl, C₁₋₃ alkoxy, C₂₋₃ alkenyl, C₂₋₃ alkynyl, C₃₋₆ cycloalkyl, 3 to 6 membered heterocyclyl, C₆₋₁₀ aryl, 5 to 10 membered heteroaryl, -(CH₂)ₙRₐ₁, -(CH₂)ₙORₐ₁, -(CH₂)ₙC(O)Rₐ₁, -(CH₂)ₙC(O)ORₐ₁, -(CH₂)ₙS(O)ₘRₐ₁, -(CH₂)ₙNRₐ₂Rₐ₃, -(CH₂)ₙNRₐ₂C(O)ORₐ₃, -(CH₂)ₙNRₐ₂C(O)(CH₂)ₙ₁Rₐ₃, -(CH₂)ₙNRₐ₂C(O)NRₐ₂Rₐ₃, -(CH₂)ₙC(O)NRₐ₂(CH₂)ₙ₁Rₐ₃, -OC(Rₐ₁Rₐ₂)ₙ(CH₂)ₙ₁Rₐ₃ and -(CH₂)ₙNRₐ₂S(O)ₘRₐ₃, the amino, C₁₋₃ alkyl, C₁₋₃ deuterated alkyl, C₁₋₃ haloalkyl, C₁₋₃ hydroxyalkyl, C₁₋₃ alkoxy, C₂₋₃ alkenyl, C₂₋₃ alkynyl, C₃₋₆ cycloalkyl, 3 to 6 membered heterocyclyl, C₆₋₁₀ aryl and 5 to 10 membered heteroaryl can be each optionally further substituted by one or more substituents selected from the group consisting of hydroxy, halogen, amino, C₁₋₃ alkyl and 3 to 6 membered heterocyclyl;
Rₐ₁, Rₐ₂ and Rₐ₃ are each independently selected from the group consisting of hydrogen, deuterium, halogen, nitro, hydroxy, thiol, cyano, amino, C₁₋₆ alkyl, C₁₋₆ deuterated alkyl, C₁₋₆ haloalkyl, C₁₋₆ hydroxyalkyl, C₁₋₆ alkoxy, C₂₋₆ alkenyl, C₂₋₆ alkynyl, C₃₋₁₂ cycloalkyl, 3 to 12 membered heterocyclyl, C₆₋₁₂ aryl and 5 to 12 membered heteroaryl, the amino, C₁₋₆ alkyl, C₁₋₆ deuterated alkyl, C₁₋₆ haloalkyl, C₁₋₆ hydroxyalkyl, C₁₋₆ alkoxy, C₂₋₆ alkenyl, C₂₋₆ alkynyl, C₃₋₁₂ cycloalkyl, 3 to 12 membered heterocyclyl, C₆₋₁₂ aryl and 5 to 12 membered heteroaryl can be each optionally further substituted by one or more substituents selected from the group consisting of hydroxy, halogen, amino, C₁₋₆ alkyl and 3 to 12 membered heterocyclyl;
more preferably, each R^{a} is independently selected from the group consisting of hydrogen, deuterium, halogen, nitro, hydroxy, thiol, oxo, cyano, amino, C₁₋₃ alkyl and C₃₋₆ cycloalkyl;
x is preferably 1, 2 or 3.

9. The compound, the stereoisomer thereof or the pharmaceutically acceptable salt thereof according to any one of claims 1 to 6, wherein:
each R^{b} is independently selected from the group consisting of hydrogen, deuterium, halogen, nitro, hydroxy, thiol, oxo, cyano, amino, C₁₋₆ alkyl, C₁₋₆ deuterated alkyl, C₁₋₆ haloalkyl, C₁₋₆ hydroxyalkyl, C₁₋₆ alkoxy, C₂₋₆ alkenyl, C₂₋₆ alkynyl, C₃₋₁₂ cycloalkyl, 3 to 12 membered heterocyclyl, C₆₋₁₂ aryl, 5 to 12 membered heteroaryl, -(CH₂)ₙR_{b1}, -(CH₂)ₙOR_{b1}, -(CH₂)ₙC(O)R_{b1}, -(CH₂)ₙC(O)OR_{b1}, -(CH₂)ₙS(O)ₘR_{b1}, -(CH₂)ₙNR_{b2}R_{b3}, -(CH₂)ₙNR_{b2}C(O)OR_{b3}, -(CH₂)ₙNR_{b2}C(O)(CH₂)ₙ₁R_{b3}, -(CH₂)ₙNR_{b2}C(O)NR_{b2}R_{b3}, -(CH₂)ₙC(O)NR_{b2}(CH₂)ₙ₁R_{b3}, -OC(R_{b1}R_{b2})ₙ(CH₂)ₙ₁R_{b3} and -(CH₂)ₙNR_{b2}S(O)ₘR_{b3}, the amino, C₁₋₆ alkyl, C₁₋₆ deuterated alkyl, C₁₋₆ haloalkyl, C₁₋₆ hydroxyalkyl, C₁₋₆ alkoxy, C₂₋₆ alkenyl, C₂₋₆ alkynyl, C₃₋₁₂ cycloalkyl, 3 to 12 membered heterocyclyl, C₆₋₁₂ aryl and 5 to 12 membered heteroaryl can be each optionally further substituted by one or more substituents selected from the group consisting of hydroxy, halogen, amino, C₁₋₆ alkyl and 3 to 12 membered heterocyclyl;
preferably, each R^{b} is independently selected from the group consisting of hydrogen, deuterium, halogen, nitro, hydroxy, thiol, oxo, cyano, amino, C₁₋₃ alkyl, C₁₋₃ deuterated alkyl, C₁₋₃ haloalkyl, C₁₋₃ hydroxyalkyl, C₁₋₃ alkoxy, C₂₋₃ alkenyl, C₂₋₃ alkynyl, C₃₋₆ cycloalkyl, 3 to 6 membered heterocyclyl, C₆₋₁₀ aryl, 5 to 10 membered heteroaryl, -(CH₂)ₙR_{b1}, -(CH₂)ₙOR_{b1}, -(CH₂)ₙC(O)R_{b1}, -(CH₂)ₙC(O)OR_{b1}, -(CH₂)ₙS(O)ₘR_{b1}, -(CH₂)ₙNR_{b2}R_{b3}, -(CH₂)ₙNR_{b2}C(O)OR_{b3}, -(CH₂)ₙNR_{b2}C(O)(CH₂)ₙ₁R_{b3}, -(CH₂)ₙNR_{b2}C(O)NR_{b2}R_{b3}, -(CH₂)ₙC(O)NR_{b2}(CH₂)ₙ₁R_{b3}, -OC(R_{b1}R_{b2})ₙ(CH₂)ₙ₁R_{b3} and -(CH₂)ₙNR_{b2}S(O)ₘR_{b3}, the amino, C₁₋₃ alkyl, C₁₋₃ deuterated alkyl, C₁₋₃ haloalkyl, C₁₋₃ hydroxyalkyl, C₁₋₃ alkoxy, C₂₋₃ alkenyl, C₂₋₃ alkynyl, C₃₋₆ cycloalkyl, 3 to 6 membered heterocyclyl, C₆₋₁₀ aryl and 5 to 10 membered heteroaryl can be each optionally further substituted by one or more substituents selected from the group consisting of hydroxy, halogen, amino, C₁₋₃ alkyl and 3 to 6 membered heterocyclyl;
R_{b1}, R_{b2} and R_{b3} are each independently selected from the group consisting of hydrogen, deuterium, halogen, nitro, hydroxy, thiol, cyano, amino, C₁₋₆ alkyl, C₁₋₆ deuterated alkyl, C₁₋₆ haloalkyl, C₁₋₆ hydroxyalkyl, C₁₋₆ alkoxy, C₂₋₆ alkenyl, C₂₋₆ alkynyl, C₃₋₁₂ cycloalkyl, 3 to 12 membered heterocyclyl, C₆₋₁₂ aryl and 5 to 12 membered heteroaryl, the amino, C₁₋₆ alkyl, C₁₋₆ deuterated alkyl, C₁₋₆ haloalkyl, C₁₋₆ hydroxyalkyl, C₁₋₆ alkoxy, C₂₋₆ alkenyl, C₂₋₆ alkynyl, C₃₋₁₂ cycloalkyl, 3 to 12 membered heterocyclyl, C₆₋₁₂ aryl and 5 to 12 membered heteroaryl can be each optionally further substituted by one or more substituents selected from the group consisting of hydroxy, halogen, amino, C₁₋₆ alkyl and 3 to 12 membered heterocyclyl;
more preferably, each R^{b} is independently selected from the group consisting of hydrogen, deuterium, halogen, nitro, hydroxy, thiol, oxo, cyano, amino, C₁₋₃ alkyl, C₁₋₃ haloalkyl and C₃₋₆ cycloalkyl;
further preferably, each R^{b} is independently selected from the group consisting of hydrogen, F, -CF₃, cyano, methyl and cyclopropyl;
y is preferably 1.

10. The compound, the stereoisomer thereof or the pharmaceutically acceptable salt thereof according to any one of claims 1 to 6, wherein:
each R^{c} is independently selected from the group consisting of hydrogen, deuterium, halogen, nitro, hydroxy, thiol, oxo, cyano, amino, C₁₋₆ alkyl, C₁₋₆ deuterated alkyl, C₁₋₆ haloalkyl, C₁₋₆ hydroxyalkyl, C₁₋₆ alkoxy, C₂₋₆ alkenyl, C₂₋₆ alkynyl, C₃₋₁₂ cycloalkyl, 3 to 12 membered heterocyclyl, C₆₋₁₂ aryl, 5 to 12 membered heteroaryl, -(CH₂)ₙR_{c1}, -(CH₂)ₙOR_{c1}, -(CH₂)ₙC(O)R_{c1}, -(CH₂)ₙC(O)OR_{c1}, -(CH₂)ₙS(O)ₘR_{c1}, -(CH₂)ₙNR_{c2}R_{c3}, -(CH₂)ₙNR_{c2}C(O)OR_{c3}, -(CH₂)ₙNR_{c2}C(O)(CH₂)ₙ₁R_{c3}, -(CH₂)ₙNR_{c2}C(O)NR_{c2}R_{c3}, -(CH₂)ₙC(O)NR_{c2}(CH₂)ₙ₁R_{c3}, -OC(R_{c1}R_{c2})ₙ(CH₂)ₙ₁R_{c3} and -(CH₂)ₙNR_{c2}S(O)ₘR_{c3}, the amino, C₁₋₆ alkyl, C₁₋₆ deuterated alkyl, C₁₋₆ haloalkyl, C₁₋₆ hydroxyalkyl, C₁₋₆ alkoxy, C₂₋₆ alkenyl, C₂₋₆ alkynyl, C₃₋₁₂ cycloalkyl, 3 to 12 membered heterocyclyl, C₆₋₁₂ aryl and 5 to 12 membered heteroaryl can be each optionally further substituted by one or more substituents selected from the group consisting of hydroxy, halogen, amino, C₁₋₆ alkyl and 3 to 12 membered heterocyclyl;
preferably, each R^{c} is independently selected from the group consisting of hydrogen, deuterium, halogen, nitro, hydroxy, thiol, oxo, cyano, amino, C₁₋₃ alkyl, C₁₋₃ deuterated alkyl, C₁₋₃ haloalkyl, C₁₋₃ hydroxyalkyl, C₁₋₃ alkoxy, C₂₋₃ alkenyl, C₂₋₃ alkynyl, C₃₋₆ cycloalkyl, 3 to 6 membered heterocyclyl, C₆₋₁₀ aryl, 5 to 10 membered heteroaryl, -(CH₂)ₙR_{c1}, -(CH₂)ₙOR_{c1}, -(CH₂)ₙC(O)R_{c1}, -(CH₂)ₙC(O)OR_{c1}, -(CH₂)ₙS(O)ₘR_{c1}, -(CH₂)ₙNR_{c2}R_{c3}, -(CH₂)NR_{c2}C(O)OR_{c3}, -(CH₂)nNR_{c2}C(O)(CH₂)ₙ₁R_{c3}, -(CH₂)ₙNR_{c2}C(O)NR_{c2}R_{c3}, -(CH₂)ₙC(O)NR_{c2}(CH₂)ₙ₁R_{c3}, -OC(R_{c1}R_{c2})ₙ(CH₂)ₙ₁R_{c3} and -(CH₂)ₙNR_{c2}S(O)ₘR_{c3}, the amino, C₁₋₃ alkyl, C₁₋₃ deuterated alkyl, C₁₋₃ haloalkyl, C₁₋₃ hydroxyalkyl, C₁₋₃ alkoxy, C₂₋₃ alkenyl, C₂₋₃ alkynyl, C₃₋₆ cycloalkyl, 3 to 6 membered heterocyclyl, C₆₋₁₀ aryl and 5 to 10 membered heteroaryl can be each optionally further substituted by one or more substituents selected from the group consisting of hydroxy, halogen, amino, C₁₋₃ alkyl and 3 to 6 membered heterocyclyl;
R_{c1}, R_{c2} and R_{c3} are each independently selected from the group consisting of hydrogen, deuterium, halogen, nitro, hydroxy, thiol, cyano, amino, C₁₋₆ alkyl, C₁₋₆ deuterated alkyl, C₁₋₆ haloalkyl, C₁₋₆ hydroxyalkyl, C₁₋₆ alkoxy, C₂₋₆ alkenyl, C₂₋₆ alkynyl, C₃₋₁₂ cycloalkyl, 3 to 12 membered heterocyclyl, C₆₋₁₂ aryl and 5 to 12 membered heteroaryl, the amino, C₁₋₆ alkyl, C₁₋₆ deuterated alkyl, C₁₋₆ haloalkyl, C₁₋₆ hydroxyalkyl, C₁₋₆ alkoxy, C₂₋₆ alkenyl, C₂₋₆ alkynyl, C₃₋₁₂ cycloalkyl, 3 to 12 membered heterocyclyl, C₆₋₁₂ aryl and 5 to 12 membered heteroaryl can be each optionally further substituted by one or more substituents selected from the group consisting of hydroxy, halogen, amino, C₁₋₆ alkyl and 3 to 12 membered heterocyclyl;
more preferably, each R^{c} is independently selected from the group consisting of hydrogen, deuterium, halogen, nitro, hydroxy, thiol, oxo, cyano, amino, C₁₋₃ alkyl, C₁₋₃ haloalkyl and C₃₋₆ cycloalkyl;
further preferably, each R^{c} is independently selected from the group consisting of hydrogen, deuterium, F, chlorine, hydroxy, methyl, methoxy, oxo and cyano;
z is preferably 1 or 2.

11. The compound, the stereoisomer thereof or the pharmaceutically acceptable salt thereof according to any one of claims 1 to 6, wherein:
each R^{d} is independently selected from the group consisting of hydrogen, deuterium, halogen, nitro, hydroxy, thiol, oxo, cyano, amino, C₁₋₆ alkyl, C₁₋₆ deuterated alkyl, C₁₋₆ haloalkyl, C₁₋₆ hydroxyalkyl, C₁₋₆ alkoxy, C₂₋₆ alkenyl, C₂₋₆ alkynyl, C₃₋₁₂ cycloalkyl, 3 to 12 membered heterocyclyl, C₆₋₁₂ aryl, 5 to 12 membered heteroaryl, -(CH₂)ₙR_{d1}, -(CH₂)ₙOR_{d1}, -(CH₂)ₙC(O)R_{d1}, -(CH₂)ₙC(O)OR_{d1}, -(CH₂)ₙS(O)ₘR_{c1}, -(CH₂)ₙNR_{d2}R_{d3}, -(CH₂)ₙNR_{d2}C(O)OR_{d3}, -(CH₂)ₙNR_{d2}C(O)(CH₂)ₙ₁R_{d3}, -(CH₂)ₙNR_{d2}C(O)NR_{d2}R_{d3}, -(CH₂)ₙC(O)NR_{d2}(CH₂)ₙ₁R_{d3}, -OC(R_{d1}R_{d2})ₙ(CH₂)ₙ₁R_{d3} and -(CH₂)ₙNR_{d2}S(O)ₘR_{d3}, the amino, C₁₋₆ alkyl, C₁₋₆ deuterated alkyl, C₁₋₆ haloalkyl, C₁₋₆ hydroxyalkyl, C₁₋₆ alkoxy, C₂₋₆ alkenyl, C₂₋₆ alkynyl, C₃₋₁₂ cycloalkyl, 3 to 12 membered heterocyclyl, C₆₋₁₂ aryl and 5 to 12 membered heteroaryl can be each optionally further substituted by one or more substituents selected from the group consisting of hydroxy, halogen, amino, C₁₋₆ alkyl and 3 to 12 membered heterocyclyl;
preferably, each R^{d} is independently selected from the group consisting of hydrogen, deuterium, halogen, nitro, hydroxy, thiol, oxo, cyano, amino, C₁₋₃ alkyl, C₁₋₃ deuterated alkyl, C₁₋₃ haloalkyl, C₁₋₃ hydroxyalkyl, C₁₋₃ alkoxy, C₂₋₃ alkenyl, C₂₋₃ alkynyl, C₃₋₆ cycloalkyl, 3 to 6 membered heterocyclyl, C₆₋₁₀ aryl, 5 to 10 membered heteroaryl, -(CH₂)ₙR_{d1}, -(CH₂)ₙOR_{d1}, -(CH₂)ₙC(O)R_{d1}, -(CH₂)ₙC(O)OR_{d1}, -(CH₂)ₙS(O)ₘR_{c1}, -(CH₂)ₙNR_{d2}R_{d3}, -(CH₂)ₙNR_{d2}C(O)OR_{d3}, -(CH₂)ₙNR_{d2}C(O)(CH₂)ₙ₁R_{d3}, -(CH₂)nNR_{d2}C(O)NR_{d2}R_{d3}, -(CH₂)ₙC(O)NR_{d2}(CH₂)ₙ₁R_{d3}, -OC(R_{d1}R_{d2})n(CH₂)ₙ₁R_{d3} and -(CH₂)ₙNR_{d2}S(O)ₘR_{d3}, the amino, C₁₋₃ alkyl, C₁₋₃ deuterated alkyl, C₁₋₃ haloalkyl, C₁₋₃ hydroxyalkyl, C₁₋₃ alkoxy, C₂₋₃ alkenyl, C₂₋₃ alkynyl, C₃₋₆ cycloalkyl, 3 to 6 membered heterocyclyl, C₆₋₁₀ aryl and 5 to 10 membered heteroaryl can be each optionally further substituted by one or more substituents selected from the group consisting of hydroxy, halogen, amino, C₁₋₃ alkyl and 3 to 6 membered heterocyclyl;
R_{d1}, R_{d2} and R_{d3} are each independently selected from the group consisting of hydrogen, deuterium, halogen, nitro, hydroxy, thiol, cyano, amino, C₁₋₆ alkyl, C₁₋₆ deuterated alkyl, C₁₋₆ haloalkyl, C₁₋₆ hydroxyalkyl, C₁₋₆ alkoxy, C₂₋₆ alkenyl, C₂₋₆ alkynyl, C₃₋₁₂ cycloalkyl, 3 to 12 membered heterocyclyl, C₆₋₁₂ aryl and 5 to 12 membered heteroaryl, the amino, C₁₋₆ alkyl, C₁₋₆ deuterated alkyl, C₁₋₆ haloalkyl, C₁₋₆ hydroxyalkyl, C₁₋₆ alkoxy, C₂₋₆ alkenyl, C₂₋₆ alkynyl, C₃₋₁₂ cycloalkyl, 3 to 12 membered heterocyclyl, C₆₋₁₂ aryl and 5 to 12 membered heteroaryl can be each optionally further substituted by one or more substituents selected from the group consisting of hydroxy, halogen, amino, C₁₋₆ alkyl and 3 to 12 membered heterocyclyl;
more preferably, each R^{d} is independently selected from the group consisting of hydrogen, deuterium, halogen, nitro, hydroxy, thiol, oxo, cyano, amino, C₁₋₃ alkyl, C₁₋₃ alkoxy, C₁₋₃ haloalkyl and C₃₋₆ cycloalkyl;
further preferably, each R^{d} is independently selected from the group consisting of hydrogen, deuterium, cyclopropyl, isopropyl, cyano, F, Cl, methyl, -CD₃, -NHCH₃, -NHCD₃, methoxy and oxo;
w is preferably 1 or 2, and more preferably 1.

12. The compound, the stereoisomer thereof or the pharmaceutically acceptable salt thereof according to claim 1 or 2, wherein the compound is further as shown in formula (VII): 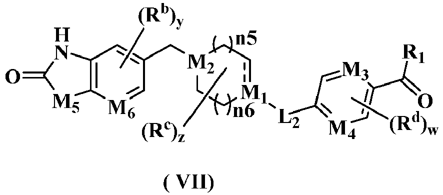 wherein,
L₂ is a bond or O;
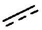 is a single bond or double bond;
M₁ is C or CR₂;
M₂ is N or CR₃;
M₃ is N or CR₄;
M₄ is N or CR₅;
M₅ is -CR₆R₇-, -CR₆R₇-CR₈R₉-, -CR₆=CR_{g}-, -CR₆R₇-NR₈-, -NR₈-C(=O)-, -CR₆R₇-O- or -CR₆=N-;
M₆ is N or CR₁₀;
R₁ is selected from the group consisting of C₁₋₆ alkyl, C₁₋₆ deuterated alkyl, C₁₋₆ haloalkyl, C₁₋₆ hydroxyalkyl, C₁₋₆ alkoxy, -(CH₂)ₙR₁₁, -(CH₂)ₙOR₁₁, -(CH₂)ₙC(O)R₁₁, -(CH₂)ₙC(O)OR₁₁, -(CH₂)ₙNR₂₂R₃₃ and -(CH₂)ₙNR₂₂C(O)OR₃₃;
R₁₁, R₂₂ and R₃₃ are each independently selected from the group consisting of hydrogen, deuterium, halogen, amino, hydroxy, cyano, nitro, C₁₋₆ alkyl, C₂₋₆ alkenyl, C₂₋₆ alkynyl, C₁₋₆ deuterated alkyl, C₁₋₆ haloalkyl, C₁₋₆ alkoxy, C₁₋₆ haloalkoxy, C₁₋₆ hydroxyalkyl, C₃₋₁₂ cycloalkyl, 3 to 12 membered heterocyclyl, C₆₋₁₂ aryl and 5 to 12 membered heteroaryl;
R₂, R₃, R₄ and R₅ are identical or different, and are each independently selected from the group consisting of hydrogen, deuterium, halogen, nitro, hydroxy, thiol, cyano, amino optionally substituted by C₁₋₃ alkyl, C₁₋₆ alkyl, C₁₋₆ deuterated alkyl, C₁₋₆ haloalkyl, C₁₋₆ hydroxyalkyl, C₁₋₆ alkoxy, C₂₋₆ alkenyl, C₂₋₆ alkynyl, C₃₋₁₂ cycloalkyl, 3 to 12 membered heterocyclyl, C₆₋₁₂ aryl and 5 to 12 membered heteroaryl; preferably, R₂, R₃, R₄ and R₅ are each independently hydrogen, deuterium, halogen, C₁₋₃ alkyl or C₃₋₆ cycloalkyl;
R^{b} is selected from the group consisting of hydrogen, deuterium, halogen, nitro, hydroxy, thiol, oxo, cyano, amino optionally substituted by C₁₋₃ alkyl, C₁₋₆ alkyl, C₁₋₆ haloalkyl, C₁₋₆ alkoxy and C₃₋₆ cycloalkyl;
R^{c} is selected from the group consisting of hydrogen, deuterium, halogen, nitro, hydroxy, thiol, oxo, cyano, amino optionally substituted by C₁₋₃ alkyl, C₁₋₆ alkyl, C₁₋₆ haloalkyl, C₁₋₆ alkoxy and C₃₋₆ cycloalkyl;
R^{d} is selected from the group consisting of hydrogen, deuterium, halogen, nitro, hydroxy, thiol, oxo, cyano, amino optionally substituted by C₁₋₃ alkyl, C₁₋₆ alkyl, C₁₋₆ haloalkyl, C₁₋₆ alkoxy and C₃₋₆ cycloalkyl;
R₆, R₇, R₈, R₉ and R₁₀ are identical or different, and are each independently selected from the group consisting of hydrogen, deuterium, halogen, nitro, hydroxy, thiol, cyano, amino optionally substituted by C₁₋₃ alkyl, C₁₋₆ alkyl, C₁₋₆ deuterated alkyl, C₁₋₆ haloalkyl, C₁₋₆ hydroxyalkyl, C₁₋₆ alkoxy, C₂₋₆ alkenyl, C₂₋₆ alkynyl, C₃₋₁₂ cycloalkyl, 3 to 12 membered heterocyclyl, C₆₋₁₂ aryl and 5 to 12 membered heteroaryl; preferably, R₆, R₇, R₈ and R₉ are each independently hydrogen, deuterium, halogen, cyano, C₁₋₃ alkyl or C₃₋₆ cycloalkyl;
or, R₆, R₇ together with the adjacent carbon atom are bonded to form a C₃₋₆ cycloalkyl, the C₃₋₆ cycloalkyl is optionally substituted by one or more substituents selected from the group consisting of hydrogen, deuterium, halogen, nitro, hydroxy, thiol, cyano and amino;
or, R₆, R₈ together with the adjacent carbon atom are bonded to form a C₃₋₆ cycloalkyl, the C₃₋₆ cycloalkyl is optionally substituted by one or more substituents selected from the group consisting of hydrogen, deuterium, halogen, nitro, hydroxy, thiol, cyano and amino;
n5 and n6 are each independently 0, 1 or 2;
w is 1 or 2;
y is 1 or 2;
z is 1 or 2; and
n is 0, 1, 2 or 3.

13. The compound, the stereoisomer thereof or the pharmaceutically acceptable salt thereof according to claim 1 or 2, wherein the compound is further as shown in formula (VI): 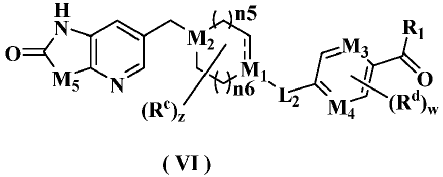 wherein,
L₂ is a bond or O;
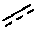 is a single bond or double bond;
M₁ is C or CR₂;
M₂ is N or CR₃;
M₃ is N or CR₄;
M₄ is N or CR₅;
M₅ is -CR₆R₇-, -CR₆R₇-CR₈R₉-, -CR₆=CR_{g}-, -CR₆R₇-NR₈-, -NR₈-C(=O)-, -CR₆R₇-O- or -CR₆=N-;
R₁ is selected from the group consisting of C₁₋₆ alkyl, C₁₋₆ deuterated alkyl, C₁₋₆ haloalkyl, C₁₋₆ hydroxyalkyl, C₁₋₆ alkoxy, -(CH₂)ₙR₁₁, -(CH₂)ₙOR₁₁, -(CH₂)ₙC(O)R₁₁, -(CH₂)ₙC(O)OR₁₁, -(CH₂)ₙNR₂₂R₃₃ and -(CH₂)ₙNR₂₂C(O)OR₃₃;
R₁₁, R₂₂ and R₃₃ are each independently selected from the group consisting of hydrogen, deuterium, halogen, amino, hydroxy, cyano, nitro, C₁₋₆ alkyl, C₂₋₆ alkenyl, C₂₋₆ alkynyl, C₁₋₆ deuterated alkyl, C₁₋₆ haloalkyl, C₁₋₆ alkoxy, C₁₋₆ haloalkoxy, C₁₋₆ hydroxyalkyl, C₃₋₁₂ cycloalkyl, 3 to 12 membered heterocyclyl, C₆₋₁₂ aryl and 5 to 12 membered heteroaryl;
R₂, R₃, R₄ and R₅ are identical or different, and are each independently selected from the group consisting of hydrogen, deuterium, halogen, nitro, hydroxy, thiol, cyano, amino optionally substituted by C₁₋₃ alkyl, C₁₋₆ alkyl, C₁₋₆ deuterated alkyl, C₁₋₆ haloalkyl, C₁₋₆ hydroxyalkyl, C₁₋₆ alkoxy, C₂₋₆ alkenyl, C₂₋₆ alkynyl, C₃₋₁₂ cycloalkyl, 3 to 12 membered heterocyclyl, C₆₋₁₂ aryl and 5 to 12 membered heteroaryl; preferably, R₂, R₃, R₄ and R₅ are each independently hydrogen, deuterium, halogen, C₁₋₃ alkyl or C₃₋₆ cycloalkyl;
R^{c} is selected from the group consisting of hydrogen, deuterium, halogen, nitro, hydroxy, thiol, oxo, cyano, amino optionally substituted by C₁₋₃ alkyl, C₁₋₆ alkyl, C₁₋₆ haloalkyl, C₁₋₆ alkoxy and C₃₋₆ cycloalkyl;
R^{d} is selected from the group consisting of hydrogen, deuterium, halogen, nitro, hydroxy, thiol, oxo, cyano, amino optionally substituted by C₁₋₃ alkyl, C₁₋₆ alkyl, C₁₋₆ haloalkyl, C₁₋₆ alkoxy and C₃₋₆ cycloalkyl;
R₆, R₇, R₈ and R₉ are identical or different, and are each independently selected from the group consisting of hydrogen, deuterium, halogen, nitro, hydroxy, thiol, cyano, amino optionally substituted by C₁₋₃ alkyl, C₁₋₆ alkyl, C₁₋₆ deuterated alkyl, C₁₋₆ haloalkyl, C₁₋₆ hydroxyalkyl, C₁₋₆ alkoxy, C₂₋₆ alkenyl, C₂₋₆ alkynyl, C₃₋₁₂ cycloalkyl, 3 to 12 membered heterocyclyl, C₆₋₁₂ aryl and 5 to 12 membered heteroaryl; preferably, R₆, R₇, R₈ and R₉ are each independently hydrogen, deuterium, halogen, C₁₋₃ alkyl or C₃₋₆ cycloalkyl;
or, R₆, R₇ together with the adjacent carbon atom are bonded to form a C₃₋₆ cycloalkyl, the C₃₋₆ cycloalkyl is optionally substituted by one or more substituents selected from the group consisting of hydrogen, deuterium, halogen, nitro, hydroxy, thiol, cyano and amino;
or, R₆, R₈ together with the adjacent carbon atom are bonded to form a C₃₋₆ cycloalkyl, the C₃₋₆ cycloalkyl is optionally substituted by one or more substituents selected from the group consisting of hydrogen, deuterium, halogen, nitro, hydroxy, thiol, cyano and amino;
n5 and n6 are each independently 0, 1 or 2;
w is 1 or 2;
z is 1 or 2; and
n is 0, 1, 2 or 3.

14. The compound, the stereoisomer thereof or the pharmaceutically acceptable salt thereof according to claim 1 or 13, wherein the compound is further as shown in formula (V): 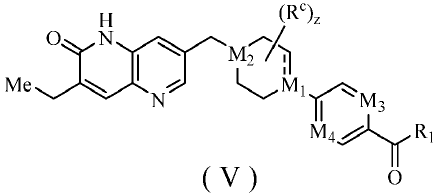
wherein, 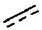 is a single bond or double bond;
M₁ is C or CR₂;
M₂ is N or CR₃;
M₃ is N or CR₄;
M₄ is N or CR₅;
R₁ is selected from the group consisting of C₁₋₆ alkyl, C₁₋₆ deuterated alkyl, C₁₋₆ haloalkyl, C₁₋₆ hydroxyalkyl, C₁₋₆ alkoxy, -(CH₂)ₙR₁₁, -(CH₂)ₙOR₁₁, -(CH₂)ₙC(O)R₁₁, -(CH₂)ₙC(O)OR₁₁, -(CH₂)ₙNR₂₂R₃₃ and -(CH₂)ₙNR₂₂C(O)OR₃₃;
R₁₁, R₂₂ and R₃₃ are each independently selected from the group consisting of hydrogen, deuterium, halogen, amino, hydroxy, cyano, nitro, C₁₋₆ alkyl, C₂₋₆ alkenyl, C₂₋₆ alkynyl, C₁₋₆ deuterated alkyl, C₁₋₆ haloalkyl, C₁₋₆ alkoxy, C₁₋₆ haloalkoxy, C₁₋₆ hydroxyalkyl, C₃₋₁₂ cycloalkyl, 3 to 12 membered heterocyclyl, C₆₋₁₂ aryl and 5 to 12 membered heteroaryl;
R₂, R₃, R₄ and R₅ are identical or different, and are each independently selected from the group consisting of hydrogen, deuterium, halogen, nitro, hydroxy, thiol, cyano, amino, C₁₋₆ alkyl, C₁₋₆ deuterated alkyl, C₁₋₆ haloalkyl, C₁₋₆ hydroxyalkyl, C₁₋₆ alkoxy, C₂₋₆ alkenyl, C₂₋₆ alkynyl, C₃₋₁₂ cycloalkyl, 3 to 12 membered heterocyclyl, C₆₋₁₂ aryl and 5 to 12 membered heteroaryl; preferably, R₂, R₃, R₄ and R₅ are each independently hydrogen, deuterium, halogen, C₁₋₃ alkyl or C₃₋₆ cycloalkyl;
R^{c} is selected from the group consisting of hydrogen, deuterium, halogen, nitro, hydroxy, thiol, oxo, cyano, amino, C₁₋₆ alkyl, C₁₋₆ haloalkyl and C₃₋₆ cycloalkyl;
z is 1 or 2; and
n is 0, 1, 2 or 3.

15. The compound, the stereoisomer thereof or the pharmaceutically acceptable salt thereof according to claim 2, wherein the compound is further as shown in formula (IX): 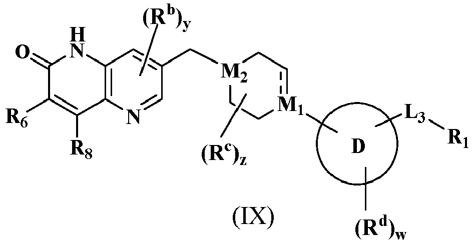 wherein, ring D is a 9 to 10 membered heterocyclyl, C₆₋₁₀ aryl or 9 to 10 membered heteroaryl; preferably a 6 membered heteroaromatic ring fused with 6 membered heteroaromatic ring, 6 membered heteroaromatic ring fused with 6 membered heterocyclic ring, or 6 membered heteroaromatic ring fused with 5 membered heterocyclic ring; and more preferably

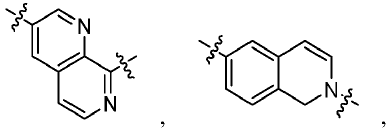

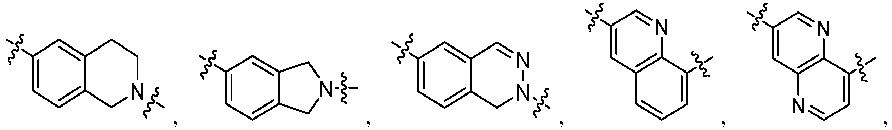

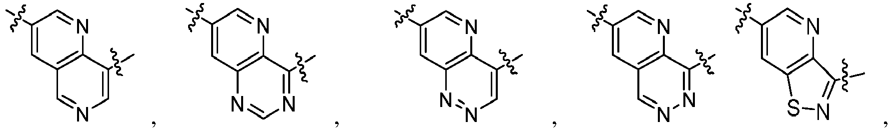

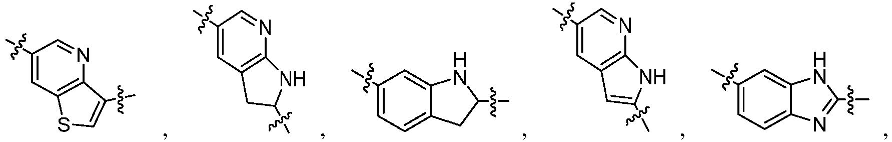

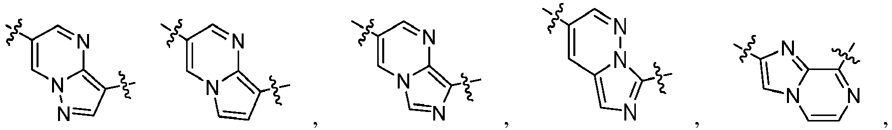

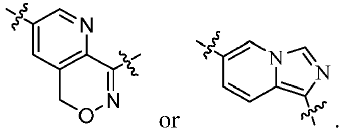

16. The compound, the stereoisomer thereof or the pharmaceutically acceptable salt thereof according to claim 1, wherein the compound is further as shown in formula (VIII): 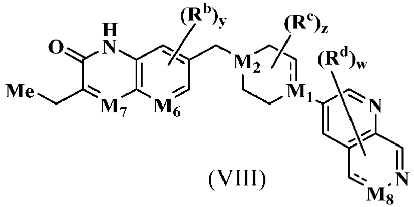 wherein,
-̅ -̅ -̅ represents a single bond or double bond;
M₁ is N, C or CR₂;
M₂ is N or CR₃;
M₆ is N or CR₁₀;
M₇ is CR₁₂ or N;
M₈ is CR₁₃ or O; preferably, -̅ -̅M₈ is -̅ -̅CR₁₃ or -O;
R₂, R₃, R₁₀, R₁₂ and R₁₃ are identical or different, and are each independently selected from the group consisting of hydrogen, deuterium, halogen, nitro, hydroxy, thiol, cyano, amino optionally substituted by one or more C₁₋₃ alkyl, C₁₋₆ alkyl, C₁₋₆ deuterated alkyl, C₁₋₆ haloalkyl, C₁₋₆ hydroxyalkyl, C₁₋₆ alkoxy, C₂₋₆ alkenyl, C₂₋₆ alkynyl, C₃₋₁₂ cycloalkyl, 3 to 12 membered heterocyclyl, C₆₋₁₂ aryl and 5 to 12 membered heteroaryl; preferably, R₂, R₃, R₁₀, R₁₂ and R₁₃ are identical or different, and are each independently selected from the group consisting of hydrogen, deuterium, halogen, nitro, hydroxy, thiol, cyano, amino optionally substituted by one or more C₁₋₃ alkyl, C₁₋₃ alkyl, C₁₋₃ deuterated alkyl, C₁₋₃ haloalkyl, C₁₋₃ hydroxyalkyl and C₁₋₃ alkoxy;
each R^{b} is independently selected from the group consisting of hydrogen, deuterium, halogen, nitro, hydroxy, thiol, oxo, cyano, amino optionally substituted by one or more C₁₋₃ alkyl, C₁₋₆ alkyl, C₁₋₆ haloalkyl, C₁₋₆ alkoxy and C₃₋₆ cycloalkyl; and preferably hydrogen, deuterium, halogen, nitro, hydroxy, thiol, oxo, cyano, amino optionally substituted by one or more C₁₋₃ alkyl, C₁₋₃ alkyl, C₁₋₃ haloalkyl and C₁₋₃ alkoxy;
each R^{c} is independently selected from the group consisting of hydrogen, deuterium, halogen, nitro, hydroxy, thiol, oxo, cyano, amino optionally substituted by one or more C₁₋₃ alkyl, C₁₋₆ alkyl, C₁₋₆ haloalkyl, C₁₋₆ alkoxy and C₃₋₆ cycloalkyl; and preferably hydrogen, deuterium, halogen, nitro, hydroxy, thiol, oxo, cyano, amino optionally substituted by one or more C₁₋₃ alkyl, C₁₋₃ alkyl, C₁₋₃ haloalkyl and C₁₋₃ alkoxy;
each R^{d} is independently selected from the group consisting of hydrogen, deuterium, halogen, nitro, hydroxy, thiol, oxo, cyano, amino optionally substituted by one or more C₁₋₃ alkyl, C₁₋₆ alkyl, C₁₋₆ haloalkyl, C₁₋₆ alkoxy and C₃₋₆ cycloalkyl; and preferably hydrogen, deuterium, halogen, nitro, hydroxy, thiol, oxo, cyano, amino optionally substituted by one or more C₁₋₃ alkyl, C₁₋₃ alkyl, C₁₋₃ haloalkyl and C₁₋₃ alkoxy;
w, y and z are each independently 1, 2, 3 or 4.

17. The compound, the stereoisomer thereof or the pharmaceutically acceptable salt thereof according to claim 1, wherein the specific structure of the compound is as follows:

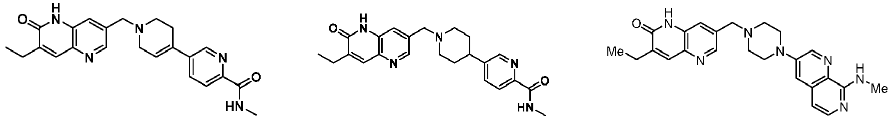

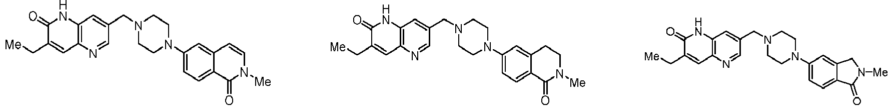

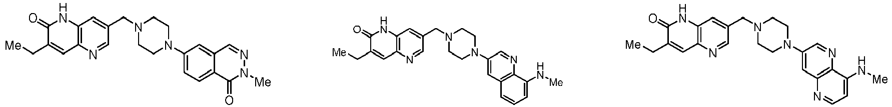

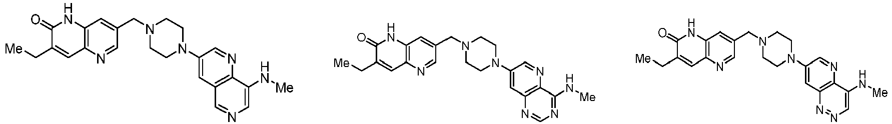

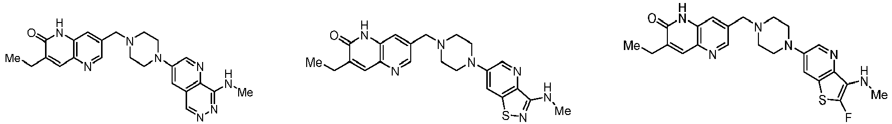

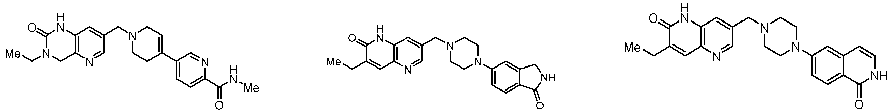

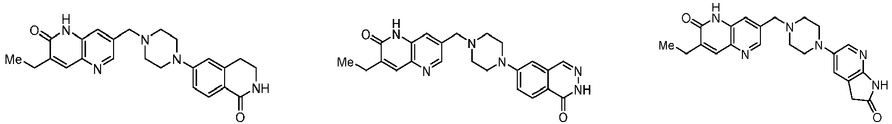

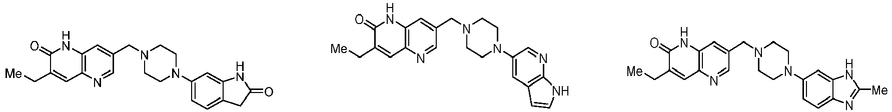

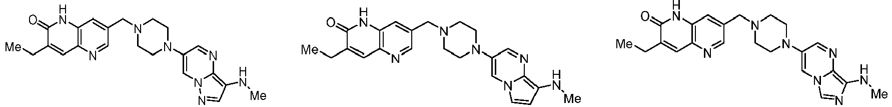

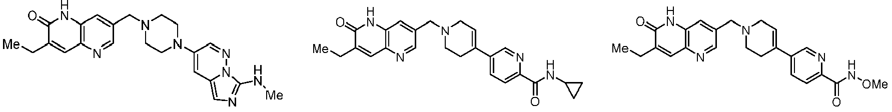

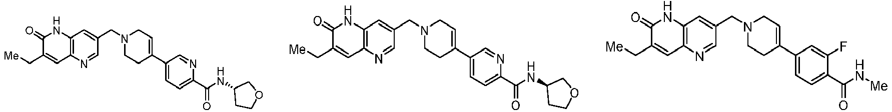

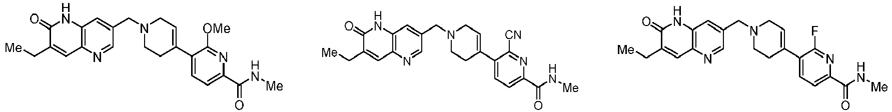

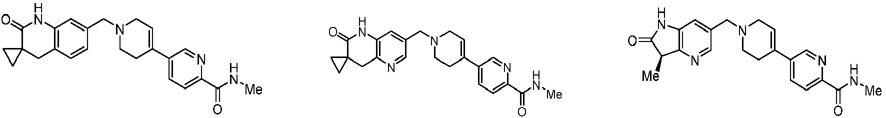

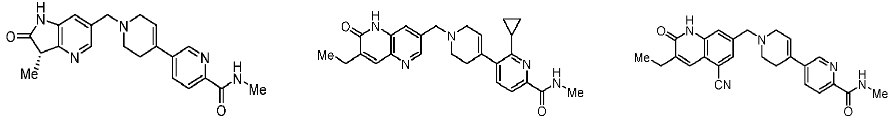

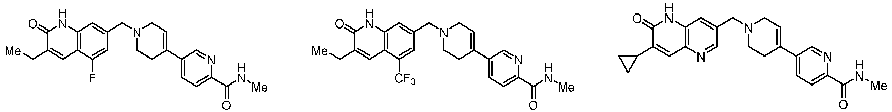

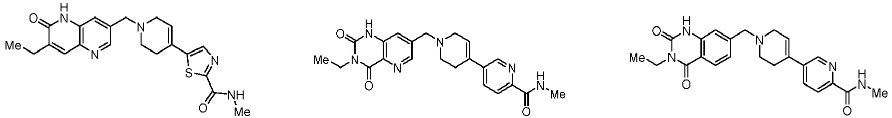

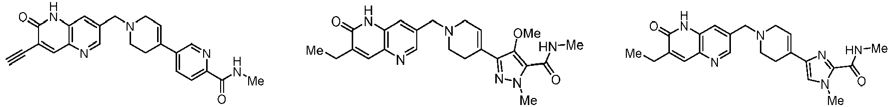

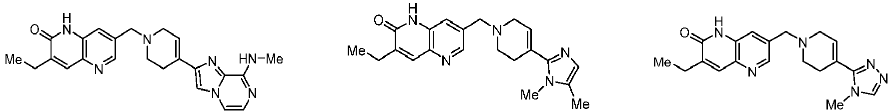

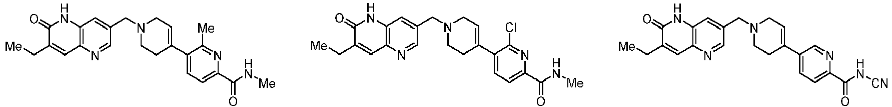

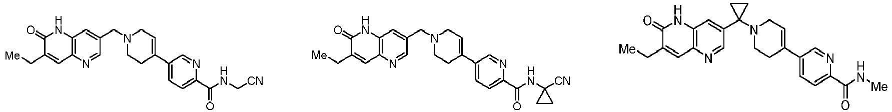

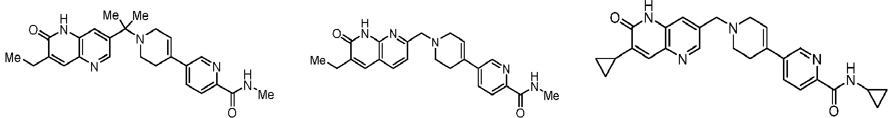

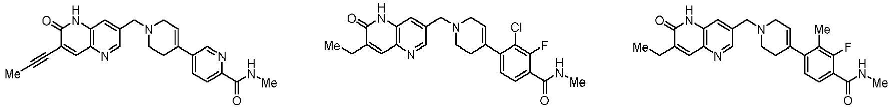

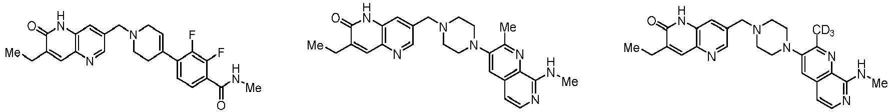

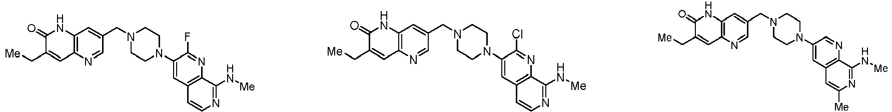

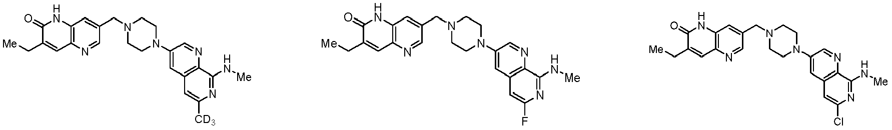

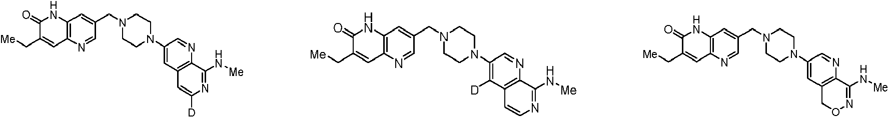

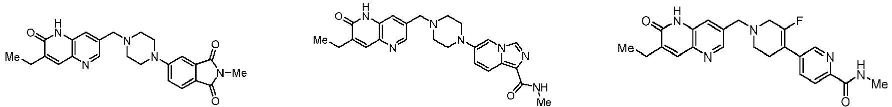

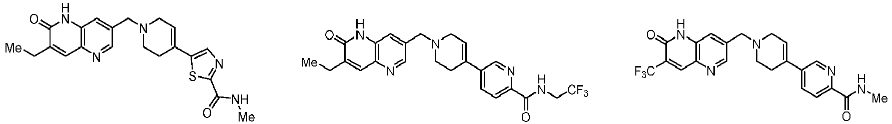

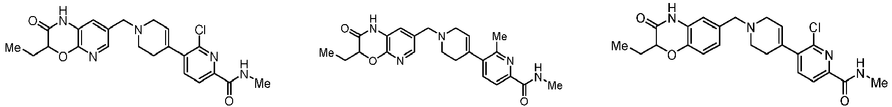

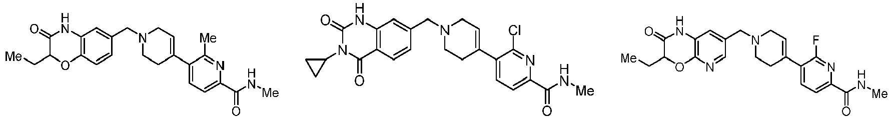

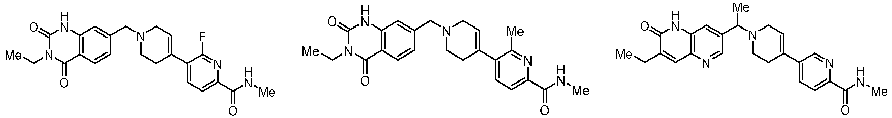

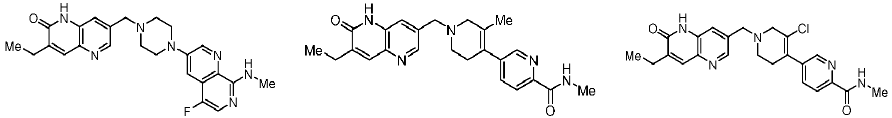

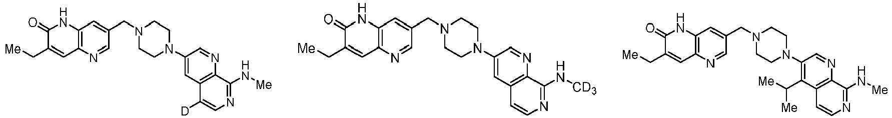

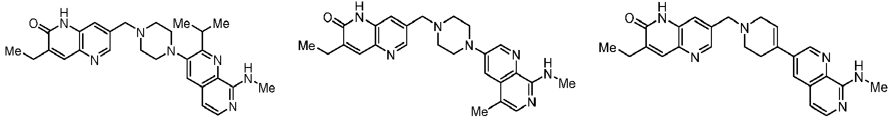

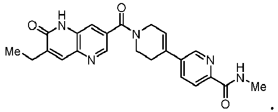

18. A method for preparing a compound of formula (III), a stereoisomer thereof or a pharmaceutically acceptable salt thereof, wherein a compound of formula (III-1) and a compound of formula (III-2) are subjected to the following reaction to obtain the compound of formula (III), 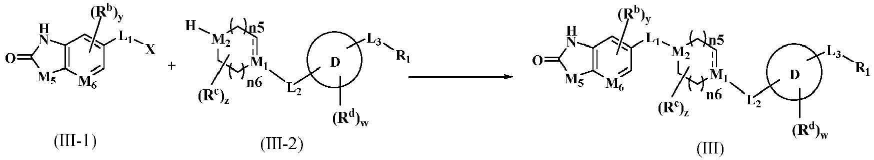
wherein, X is halogen, and preferably fluorine, chlorine, bromine or iodine;
preferably, the reaction is carried out in the presence of a base and a catalyst; the base is preferably DIPEA; the catalyst is preferably potassium iodide;
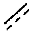 , M₁, M₂, M₄, M₆, R^{b}, R^{c}, R^{d}, L₁, L₂, L₃, R₁, ring D, n5, n6, y, z and w are as defined in claim 2.

19. A compound of formula (III-2), 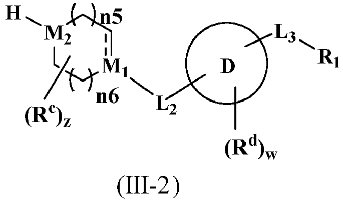 wherein, 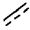 , M₁, M₂, R^{c}, R^{d}, L₂, L₃, R₁, ring D, n5, n6, z and w are as defined in claim 2.

20. A pharmaceutical composition, comprising a therapeutically effective dose of the compound, the stereoisomer thereof or the pharmaceutically acceptable salt thereof according to any one of claims 1 to 17, and one or more pharmaceutically acceptable carriers, diluents or excipients.

21. Use of the compound, the stereoisomer thereof or the pharmaceutically acceptable salt thereof according to any one of claims 1 to 17, or the pharmaceutical composition according to claim 20 in the preparation of a PARP inhibitor medicament, wherein the PARP is preferably PARP1.

22. Use of the compound, the stereoisomer thereof or the pharmaceutically acceptable salt thereof according to any one of claims 1 to 17, or the pharmaceutical composition according to claim 20 in the preparation of a medicament for treating cancer, ischemic disease, or neurodegenerative disease, wherein the cancer is preferably selected from the group consisting of breast cancer, ovarian cancer, pancreatic cancer, prostate cancer, blood cancer, gastric cancer, colorectal cancer, gastrointestinal cancer and lung cancer.
